# EUROPEAN PATENT APPLICATION

(11) **EP 4 206 206 A1**
(43) Date of publication of application: **05.07.2023**
(21) Application number: 22205060.1
(22) Date of filing: 02.11.2022
(51) Int. Cl.: C07D 491/04, C07D 495/04, C09K 11/00

(54) **ORGANIC COMPOUND, ORGANIC LIGHT EMITTING DIODE AND ORGANIC LIGHT EMITTING DEVICE INCLUDING THEREOF**

(30) Priority: 30.12.2021 KR 20210193030
(71) Applicant: LG Display Co., Ltd., SEOUL, 07336 (KR)
(72) Inventor: KIM, Sang-Beom, 10845 Paju-si (KR); YU, Young-Jun, 10845 Paju-si (KR); YOO, Seon-Keum, 10845 Paju-si (KR)
(74) Representative: Viering, Jentschura & Partner mbB Patent- und Rechtsanwälte

(57) **Abstract**

An organic compound including a first moiety having a hetero-ring fused acridine moiety and a second moiety having a quinazoline ring linked to the first moiety directly or via a bivalent linking group is disclosed. In the organic compound, at least one hydrogen atom may be substituted with deuterium or a deuterium-substituted group. An organic light emitting diode (OLED) (D, D1, D2, D3) and an organic light emitting device (100, 400) including the organic compound are also disclosed. The driving voltage of the OLED (D, D1, D2, D3) can be lowered and the luminous efficiency and the luminous lifespan of the OLED (D, D1, D2, D3) can be maximized or increased by adding the organic compound into an emissive layer (230, 530, 530') of the OLED (D, D1, D2, D3).

## Description

### BACKGROUND

### Technical Field

The present disclosure relates to an organic compound, and more specifically, to an organic compound that may have improved luminous properties, and an organic light emitting diode and an organic light emitting device including thereof.

### Discussion of the Related Art

A flat display device including an organic light emitting diode (OLED) has attracted attention as a display device that can replace a liquid crystal display device (LCD). The OLED can be formed as a thin organic film less than 2000 Å and the electrode configurations can implement unidirectional or bidirectional images. Also, the OLED can be formed even on a flexible transparent substrate, such as a plastic substrate, so that a flexible or a foldable display device can be realized with ease using the OLED. In addition, the OLED can be driven at a lower voltage and the OLED has advantageous high color purity compared to the LCD.

Since fluorescent material uses only singlet exciton energy in the luminous process, the fluorescent material in the related art shows low luminous efficiency. On the contrary, phosphorescent material can show high luminous efficiency since it uses triplet exciton energy as well as singlet exciton energy in the luminous process. However, examples of phosphorescent material include metal complexes, which have a short luminous lifespan for commercial use. Therefore, there remains a need to develop a luminous compound or an organic light emitting diode that may enhance luminous efficiency and luminous lifespan

### SUMMARY

Accordingly, embodiments of the present disclosure are directed to an organic compound, an organic light emitting diode and an organic light emitting device that substantially obviate one or more of the problems due to the limitations and disadvantages of the related art.

An object of the present disclosure is to provide an organic compound having beneficial luminous efficiency and luminous lifespan and an organic light emitting diode and an organic light emitting device including thereof.

Additional features and objects will be set forth in the description that follows, and in part will be apparent from the description, or can be learned by practice of the inventive concepts provided herein. Other features and aspects of the inventive concept can be realized and attained by the structure particularly pointed out in the written description, or derivable therefrom, and the claims hereof as well as the appended drawings.

To achieve these and other advantages and in accordance with objects of the disclosure, as embodied and broadly described, an organic compound according to claim 1 is provided. Further embodiments are described in the dependent claims. In an aspect of the present disclosure, an organic compound has a structure represented by Formula 1: 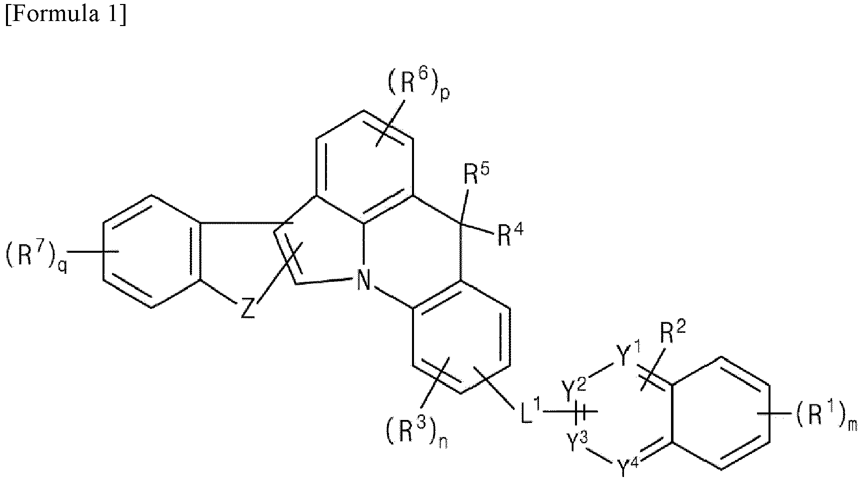
wherein in the Formula 1,
two of Y¹ to Y⁴ are nitrogen atoms, one of Y¹ to Y⁴ is a carbon atom linked to L¹, and the other of Y¹ to Y⁴ is CR²;
each of R¹ to R⁷ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
at least one of R¹ to R⁷ is a deuterium, a deuterium-substituted C₁-C₂₀ alkyl, a deuterium-substituted C₁-C₂₀ heteroalkyl, a deuterium-substituted C₂-C₂₀ alkenyl, a deuterium-substituted C₂-C₂₀ heteroalkenyl, a deuterium-substituted C₁-C₂₀ alkoxy, a deuterium-substituted C₁-C₂₀ alkyl amino, a deuterium-substituted C₃-C₂₀ alicyclic group, a deuterium-substituted C₃-C₂₀ heteroalicyclic group, a deuterium-substituted C₆-C₃₀ aromatic group, or a deuterium-substituted C₃-C₃₀ heteroaromatic group,
each R¹ is identical to or different from each other when m is 2, 3 or 4, each R³ is identical to or different from each other when n is 2 or 3, each R⁶ is identical to or different from each other when p is 2 or 3, and each R⁷ is identical to or different from each other when q is 2, 3 or 4,
each of m and q is 4 and each of n and p is 3 when each of R¹, R³, R⁶, and R⁷ is a protium,
each of m and q is independently 1, 2, 3 or 4 and each of n and p is independently 1, 2 or 3 when each of R¹, R³, R⁶, and R⁷ is not a protium;
Z is O or S; and
L¹ is a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring.

In an example embodiment, the organic compound may have a structure represented by Formula 2: 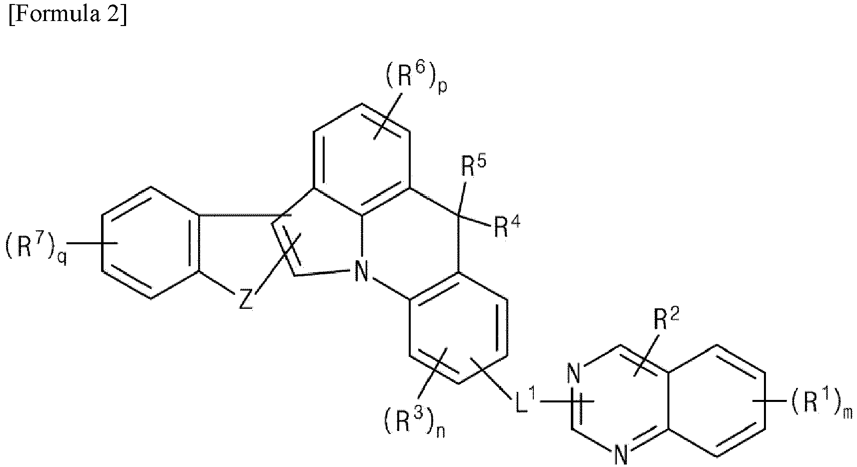
wherein in the Formula 2,
each of R¹ to R⁷, Z, L¹, m, n, p, and q is as defined in the Formula 1.

In an example embodiment, the organic compound may have a structure represented by Formula 3 or Formula 4: 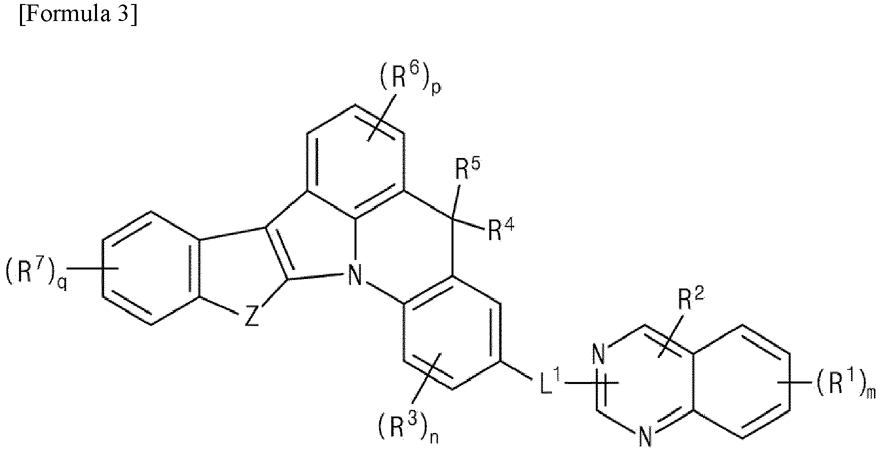 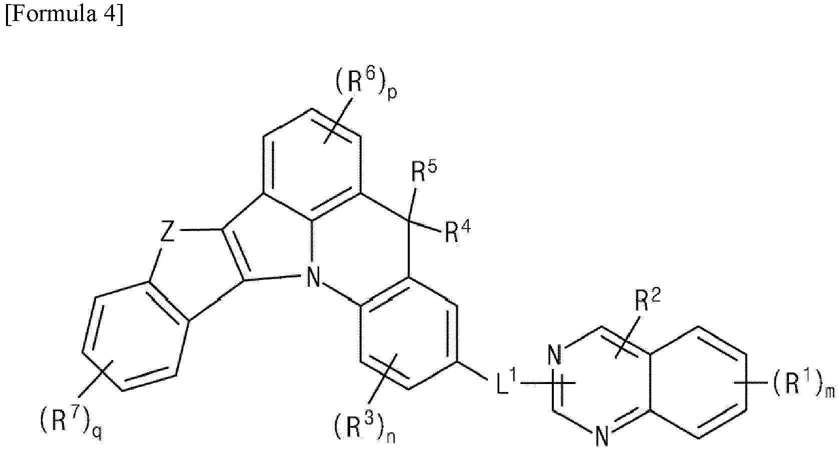
wherein, in Formulae 3 and 4,
each of R¹ to R⁷, Z, L¹, m, n, p, and q is as defined in the Formula 1.

In an example embodiment, at least one of R¹ to R⁷ may be a deuterium, a deuterium-substituted C₁-C₂₀ alkyl, a deuterium-substituted C₃-C₂₀ cycloalkyl, a deuterium-substituted C₃-C₂₀ heterocycloalkyl, a deuterium-substituted C₆-C₃₀ aryl, or a deuterium-substituted C₃-C₃₀ heteroaryl.

In another aspect of the present disclosure, an organic light emitting diode includes a first electrode; a second electrode facing the first electrode; and an emissive layer disposed between the first and second electrodes; wherein the emissive layer includes an organic compound having any one of the structures of Formulae 1 to 4.

In an example embodiment, the emissive layer may include at least one emitting material layer which includes the organic compound.

In one example embodiment, the at least one emitting material layer may further include a spiro-bifluorene-based organic compound having a structure represented by Formula 6: 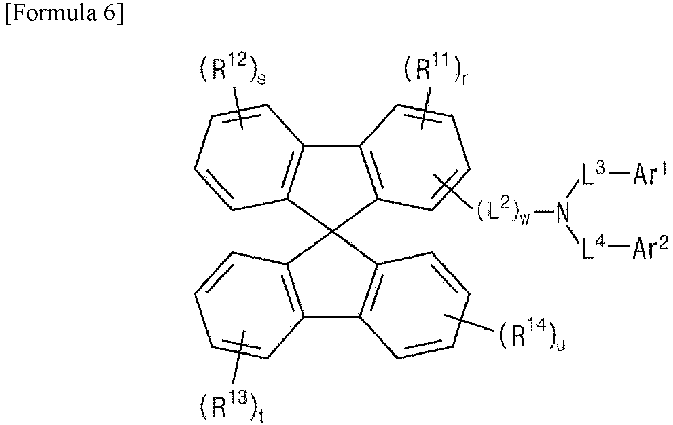
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4,
r is 3 and each of s, t, and u is 4 when each of R¹¹ to R¹⁴ is a protium,
r is 1, 2 or 3, and each of s, t, and u is independently 1, 2, 3 or 4 when each of R¹¹ to R¹⁴ is not a protium,
w is 0, 1, 2 or 3;
each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring.

In an example embodiment, the spiro-bifluorene-based organic compound may have a structure represented by Formula 7 or Formula 8: 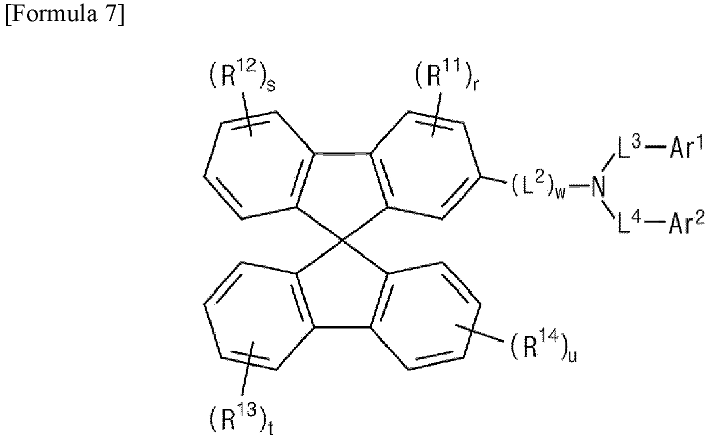 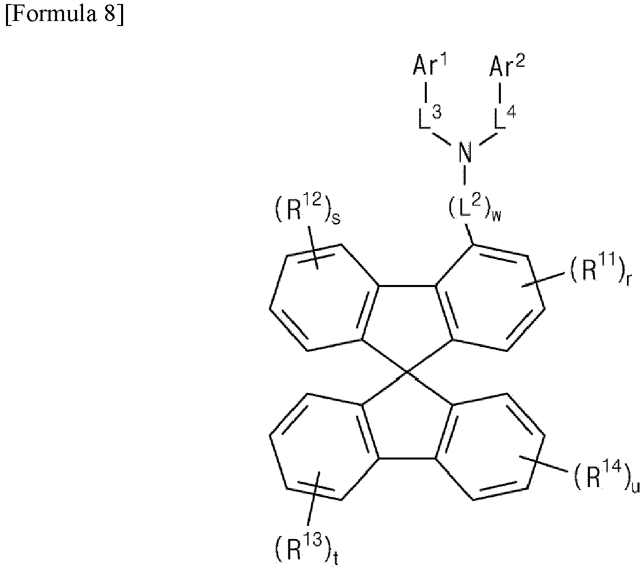
wherein in the Formulae 7 and 8,
each of R¹¹ to R¹⁴, Ar¹, Ar², L² to L⁴, r, s, t, u, and w is as defined in the Formula 6.

In an example embodiment, the at least one emitting material layer may further include a dopant.

In an example embodiment, the dopant may include a red dopant.

In an example embodiment, the emissive layer may further include at least one hole transport layer disposed between the first electrode and the at least one emitting material layer.

In an example embodiment, the at least one hole transport layer may include the spiro-bifluorene-based organic compound having any one of the structures of Formulae 6 to 8.

In an example embodiment, the emissive layer may include a first emitting part disposed between the first and second electrodes and may include a first emitting material layer; a second emitting part disposed between the first emitting part and the second electrode and may include a second emitting material layer; and a first charge generation layer disposed between the first emitting part and the second emitting part, wherein at least one of the first emitting material layer and the second emitting material layer may include the organic compound.

In an example embodiment, the second emitting material layer may include a first layer disposed between the first charge generation layer and the second electrode, and a second layer disposed between the first layer and the second electrode, and at least one of the first layer and the second layer may include the organic compound.

In an example embodiment, the second emitting material layer may further include a third layer disposed between the first layer and the second layer.

In another example embodiment, the emissive layer may further include a third emitting part disposed between the second emitting part and the second electrode and may include a third emitting material layer, and a second charge generation layer disposed between the second emitting part and the third emitting part.

In yet another aspect of the present disclosure, an organic light emitting diode includes a first electrode; a second electrode facing the first electrode; and an emissive layer disposed between the first and second electrodes, wherein the emissive layer includes a first emitting part disposed between the first and second electrodes and including a blue emitting material layer; a second emitting part disposed between the first emitting part and the second electrode and including at least one emitting material layer; and a first charge generation layer disposed between the first emitting part and the second emitting part, wherein the at least one emitting material layer may include the organic compound having any one of the structures of Formulae 1 to 4.

In an example embodiment, the at least one emitting material layer may include a first layer disposed between the first charge generation layer and the second electrode, and a second layer disposed between the first layer and the second electrode, and wherein at least one of the first layer and the second layer may include the organic compound.

In an example embodiment, the first layer may include the organic compound and a red dopant, and the second layer may be a green emitting material layer.

In an example embodiment, the first layer further includes a spiro-bifluorene-based organic compound having any one of the structures of Formulae 6 to 8.

In an example embodiment, the at least one emitting material layer may further include a third layer disposed between the first layer and the second layer, and wherein the third layer may be a yellow green emitting material layer.

In an example embodiment, the second emitting part may further include a hole transport layer disposed between the first charge generation layer and the at least one emitting material layer, and wherein the hole transport layer may include the spiro-bifluorene-based organic compound having any one of the structures of Formulae 6 to 8.

In an example embodiment, the emissive layer may further include a third emitting part disposed between the second emitting part and the second electrode, and a second charge generation layer disposed between the second emitting part and the third emitting part.

In yet another aspect of the present disclosure, an organic light emitting device, for example, an organic light emitting display device or an organic light emitting illumination device, includes a substrate and the organic light emitting diode disposed on the substrate.

The organic compound includes a hetero ring fused acridine moiety and a quinazoline moiety linked to the acridine moiety directly or via an aromatic or hetero aromatic linking group. The organic compound includes at least deuterium and/or other group substituted with at least one deuterium among the whole molecular structure.

The organic compound including the acridine moiety and the quinazoline moiety each of which includes multiple rings may have high triplet energy level and wide energy bandgap between HOMO energy level and LUMO energy level. As the acridine moiety is fused through 5-membered ring to form multiple ring systems, the compound may have beneficial thermal stability.

The organic compound may be applied into an emissive layer disposed between two electrodes. For example, the organic compound may be applied into an emitting material layer as an n-type host to transfer carriers to a dopant.

Optionally, an amine-based organic compound, a carbazole-based organic compound and/or a spiro-bifluorene-based organic compound having beneficial hole transporting properties may be used with the organic compound as the p-type host in the emitting material layer or used as hole transporting material in a hole transport layer disposed adjacent to the emitting material layer. When the organic compound is applied into the emitting material layer and the amine-based compound, the carbazole-based organic compound and/or the spiro-bifluorene-based organic compound is utilized as p-type host and/or the hole transporting material, holes and electrons can be injected into the emitting material layer in balance and can be transferred to the ultimately emitting dopant rapidly, and excitons can be recombined in the whole are of the emitting material layer. The driving voltage of the OLED and the organic light emitting device can be reduced and the luminous efficiency as well as the luminous lifespan of the OLED and the device can be improved by applying the organic compound into the emissive layer.

It is to be understood that both the foregoing general description and the following detailed description are merely by way of example and explanatory, and are intended to provide further explanation of the inventive concepts as claimed.

### BRIEF DESCRIPTION OF THE DRAWINGS

The accompanying drawings, which are included to provide a further understanding of the disclosure, are incorporated in and constitute a part of this application, illustrate embodiments of the disclosure and together with the description serve to explain principles of the disclosure.
FIG. 1 illustrates a schematic circuit diagram of an organic light emitting display device in accordance with the present disclosure.
FIG. 2 illustrates a cross-sectional view of an organic light emitting display device as an example of an organic light emitting device in accordance with an example embodiment of the present disclosure.
FIG. 3 illustrates a cross-sectional view of an organic light emitting diode having a single emitting part in accordance with an example embodiment of the present disclosure.
FIG. 4 illustrates a cross-sectional view of an organic light emitting display device in accordance with another example embodiment of the present disclosure.
FIG. 5 illustrates a cross-sectional view of an organic light emitting diode having a double-stack structure in accordance with yet another example embodiment of the present disclosure.
FIG. 6 illustrates a cross-sectional view of an organic light emitting diode having a triple-stack structure in accordance with yet another example embodiment of the present disclosure.

### DETAILED DESCRIPTION

Reference will now be made in detail to aspects of the disclosure, examples of which are illustrated in the accompanying drawings. Wherever possible, the same reference numbers will be used throughout the drawings to refer to the same or like parts.

Advantages and features of the present disclosure, and implementation methods thereof will be clarified through following example embodiments described with reference to the accompanying drawings. The present disclosure may, however, be embodied in different forms and should not be construed as limited to the example embodiments set forth herein. Rather, these example embodiments are provided so that this disclosure may be sufficiently thorough and complete to assist those skilled in the art to fully understand the scope of the present disclosure. Further, the protected scope of the present disclosure is defined by claims and their equivalents.

The shapes, sizes, ratios, angles, numbers, and the like, which are illustrated in the drawings to describe various example embodiments of the present disclosure, are merely given by way of example. Therefore, the present disclosure is not limited to the illustrations in the drawings. The same or similar elements are designated by the same reference numerals throughout the specification unless otherwise specified.

In the following description, where the detailed description of the relevant known function or configuration may unnecessarily obscure an important point of the present disclosure, a detailed description of such known function of configuration may be omitted.

In the present specification, where the terms "comprise," "have," "include," and the like are used, one or more other elements may be added unless the term, such as "only," is used. An element described in the singular form is intended to include a plurality of elements, and vice versa, unless the context clearly indicates otherwise.

In construing an element, the element is to be construed as including an error or tolerance range even where no explicit description of such an error or tolerance range is provided.

In the description of the various embodiments of the present disclosure, where positional relationships are described, for example, where the positional relationship between two parts is described using "on," "over," "under," "above," "below," "beside," "next," or the like, one or more other parts may be located between the two parts unless a more limiting term, such as "immediate(ly)," "direct(ly)," or "close(ly)" is used. For example, where an element or layer is disposed "on" another element or layer, a third layer or element may be interposed therebetween.

In describing a temporal relationship, when the temporal order is described as, for example, "after," "subsequent," "next," or "before," a case which is not continuous may be included unless a more limiting term, such as "just," "immediate(ly)," or "direct(ly)," is used.

Although the terms "first," "second," and the like may be used herein to describe various elements, the elements should not be limited by these terms. These terms are used only to identify one element from another. For example, a first element could be termed a second element, and similarly, a second element could be termed a first element, without departing from the scope of the present disclosure.

Although the terms "first," "second," A, B, (a), (b), and the like may be used herein to describe various elements, the elements should not be interpreted to be limited by these terms as they are not used to define a particular order, precedence, or number of the corresponding elements. These terms are used only to identify one element from another.

The expression that an element or layer is "connected" to another element or layer means that the element or layer can not only be directly connected to another element or layer, but also be indirectly connected or adhered to another element or layer with one or more intervening elements or layers "disposed," or "interposed" between the elements or layers, unless otherwise specified.

The term "at least one" should be understood as including any and all combinations of one or more of the associated listed items. For example, the meaning of "at least one of a first element, a second element, and a third element" encompasses the combination of all three listed elements, combinations of any two of the three elements, as well as each individual element, the first element, the second element, and the third element.

Features of various embodiments of the present disclosure may be partially or overall coupled to or combined with each other, and may be variously inter-operated with each other and driven technically as those skilled in the art can sufficiently understand. Embodiments of the present disclosure may be carried out independently from each other, or may be carried out together in a co-dependent relationship.

Hereinafter, example embodiments of the present disclosure will be described in detail with reference to the accompanying drawings. In adding reference numerals to elements of each of the drawings, although the same elements are illustrated in other drawings, like reference numerals may refer to like elements. Also, for convenience of description, a scale in which each of elements is illustrated in the accompanying drawings may differ from an actual scale. Thus, the illustrated elements are not limited to the specific scale in which they are illustrated in the drawings.

### [Organic Compound]

An organic compound present in an organic light emitting diode (OLED) may have beneficial luminous property and good affinity to carriers, and maintain stability in driving the device. The luminous materials in the OLED may be the most important factor determining the luminous efficiency of the device. The luminous materials may have high quantum efficiency as well as great hole and electron mobility and may be formed with stability and uniformity.

An organic compound in accordance with the present disclosure includes a first moiety having a hetero-ring fused acridine ring and a second moiety including a quinazoline ring having multiple nitrogen atoms. The organic compound of the present disclosure may have a structure represented by Formula 1: 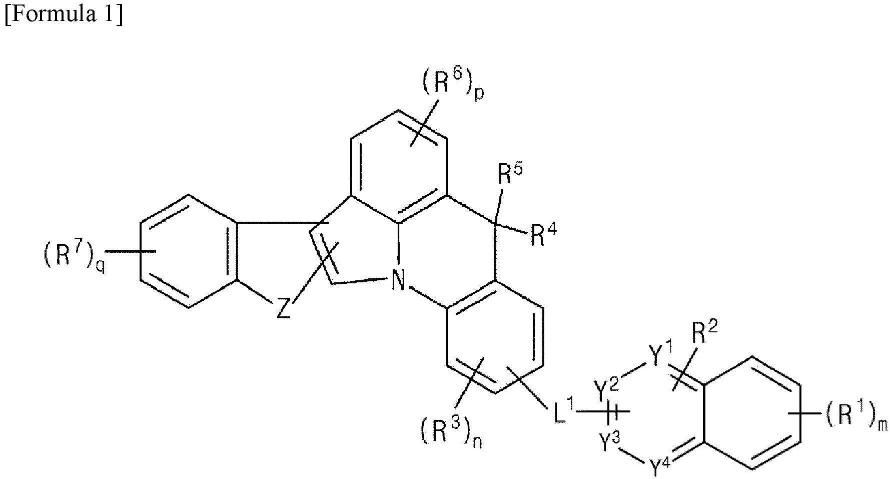
wherein in the Formula 1,
two of Y¹ to Y⁴ are nitrogen atoms, one of Y¹ to Y⁴ is a carbon atom linked to L¹, and the other of Y¹ to Y⁴ is CR²;
each of R¹ to R⁷ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring, wherein at least one of R¹ to R⁷ is a deuterium, a deuterium-substituted C₁-C₂₀ alkyl, a deuterium-substituted C₁-C₂₀ heteroalkyl, a deuterium-substituted C₂-C₂₀ alkenyl, a deuterium-substituted C₂-C₂₀ heteroalkenyl, a deuterium-substituted C₁-C₂₀ alkoxy, a deuterium-substituted C₁-C₂₀ alkyl amino, a deuterium-substituted C₃-C₂₀ alicyclic group, a deuterium-substituted C₃-C₂₀ heteroalicyclic group, a deuterium-substituted C₆-C₃₀ aromatic group, or a deuterium-substituted C₃-C₃₀ heteroaromatic group, wherein each R¹ is identical to or different from each other when m is 2, 3 or 4, each R³ is identical to or different from each other when n is 2 or 3, each R⁶ is identical to or different from each other when p is 2 or 3, and each R⁷ is identical to or different from each other when q is 2, 3 or 4;
Z is O or S;
L¹ is a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring; and
each of m and q is 4 and each of n and p is 3 when each of R¹, R³, R⁶, and R⁷ is a protium, and each of m and q is independently 0, 1, 2, 3 or 4 and each of n and p is independently 0, 1, 2 or 3 when each of R¹, R³, R⁶, and R⁷ is not a protium.

As used herein, the term "unsubstituted" means that a hydrogen atom is directly linked to a carbon atom or a hetero atom. "Hydrogen", as used herein, may refer to protium.

As used herein, "substituted" means that the hydrogen is replaced with a substituent. The substituent comprises, but is not limited to, deuterium, tritium, unsubstituted or deuterium- or halogen-substituted C₁-C₂₀ alkyl, unsubstituted or deuterium- or halogen-substituted C₁-C₂₀ alkoxy, halogen, cyano, a hydroxyl group, a carboxylic group, a carbonyl group, a sulfonate group, an amino group, an unsubstituted or deuterium- or halogen-substituted C₁-C₂₀ alkyl amino group, an unsubstituted or deuterium- or halogen-substituted C₆-C₃₀ aryl amino group, an unsubstituted or deuterium- or halogen-substituted C₃-C₃₀ heteroaryl amino group, an unsubstituted or deuterium-substituted C₃-C₃₀ alicyclic group, an unsubstituted or deuterium-substituted C₃-C₃₀ heteroalicyclic group, an unsubstituted or deuterium-substituted C₁-C₂₀ alkyl silyl group, an unsubstituted or deuterium-substituted C₁-C₂₀ alkoxy silyl group, an unsubstituted or deuterium-substituted C₃-C₃₀ alicyclic silyl group, an unsubstituted or deuterium-substituted C₃-C₃₀ heteroalicyclic group, an unsubstituted or deuterium-substituted C₆-C₃₀ aryl silyl group, an unsubstituted or deuterium-substituted C₃-C₃₀ heteroalkyl silyl group, an unsubstituted or deuterium-substituted C₆-C₃₀ aryl group, and/or an unsubstituted or deuterium-substituted C₃-C₃₀ heteroaryl group.

As used herein, the term "alkyl" refers to a branched or unbranched saturated hydrocarbon group of 1 to 20 carbon atoms, such as methyl, ethyl, or 1 to 24 carbon atoms, such as methyl, ethyl, n-propyl, isopropyl, n-butyl, isobutyl, s-butyl, t-butyl, n-pentyl, isopentyl, s-pentyl, neopentyl, hexyl, heptyl, octyl, nonyl, decyl, dodecyl, tetradecyl, hexadecyl, and the like.

As used herein, the term "alkenyl" is a hydrocarbon group of 2 to 20 carbon atoms containing at least one carbon-carbon double bond. The alkenyl group may be substituted with one or more substituents.

As used herein, the term "alicyclic" or "cycloalkyl" refers to a non-aromatic carbon-based ring composed of at least three carbon atoms. Examples of alicyclic groups include, but are not limited to, cyclopropyl, cyclobutyl, cyclopentyl, cyclohexyl, norbornyl, and the like. The alicyclic group may be substituted with one or more substituents.

As used herein, the term "alkoxy" refers to an branched or unbranched alkyl bonded through an ether linkage represented by the formula -O(-alkyl) where "alkyl" is as defined herein. Examples of alkoxy include methoxy, ethoxy, n-propoxy, isopropoxy, butoxy, and tert-butoxy, and the like.

As used herein, the term "alkyl amino" refers to a group represented by the formula -NH(-alkyl) or -N(-alkyl)₂ where "alkyl" is as defined herein. Examples of alkyl amino represented by the formula -NH(-alkyl) include, but not limited to, methylamino group, ethylamino group, propylamino group, isopropylamino group, butylamino group, isobutylamino group, (sec-butyl)amino group, (tert-butyl)amino group, pentylamino group, isopentylamino group, (tert-pentyl)amino group, hexylamino group, and the like. Examples of alkyl amino represented by the formula -N(-alkyl)₂ include, but not limited to, dimethylamino group, diethylamino group, dipropylamino group, diisopropylamino group, dibutylamino group, diisobutylamino group, di(sec-butyl)amino group, di(tert-butyl)amino group, dipentylamino group, diisopentylamino group, di(tert-pentyl)amino group, dihexylamino group, N-ethyl-N-methylamino group, N-methyl-N-propylamino group, N ethyl-N-propylamino group and the like.

As used herein, the term "aromatic" or "aryl" is well known in the art. The term includes monocyclic rings, monocyclic rings linked covalently to each other via a bond, or fused-ring polycyclic groups. An aromatic group may be unsubstituted or substituted. Examples of aromatic or aryl include phenyl, biphenyl *(e.g.,* 4-biphenyl), terphenyl, naphthyl *(e.g.,* 1-naphthyl, 2-naphthyl), anthracenyl, pentalenyl, indenyl, indeno-indenyl, heptalenyl, biphenylenyl, indacenyl, phenalenyl, phenanthrenyl, benzo-phenanthrenyl, dibenzo-phenanthrenyl, azulenyl, pyrenyl, fluoranthenyl, triphenylenyl, chrysenyl, tetraphenylenyl, tetracenyl, pleiadenyl, picenyl, pentaphenylenyl, pentacenyl, fluorenyl, indeno-fluorenyl, and spiro-fluorenyl. Substituents for the aromatic group or the aryl group are as defined herein.

As used herein, the term "alkyl silyl group" refers to any linear or branched, saturated or unsaturated acyclic alkyl, and the alkyl has 1 to 20 carbon atoms. Examples of the alkyl silyl group include a trimethylsilyl group, a triethylsilyl group, a *t*-butyldimethylsilyl group, a vinyldimethylsilyl group, a propyldimethylsilyl group, a triphenylsilyl group, a diphenylsilyl group, and a phenylsilyl group.

As used herein, the term "halogen" refers to fluorine, chlorine, bromine or iodine atom.

As used herein, the term "hetero" in terms such as "heteroalkyl", "heteroalkenyl", "a heteroalicyclic group", "a heteroaromatic group", "a heterocycloalkylene group", "a heteroarylene group", "a heteroaryl alkylene group", " a heteroaryl oxylene group", "a heterocycloalkyl group", "a heteroaryl group", "a heteroaryl alkyl group", "a heteroaryloxyl group", "a heteroaryl amino group" means that at least one carbon atom, for example, 1 to 5 carbons atoms, constituting an aliphatic chain, an alicyclic group or ring, or an aromatic group or ring is substituted with at least one hetero atom selected from the group consisting of N, O, S, and P.

As used herein, the term "heteroaromatic" or "heteroaryl" refers to a heterocycle including hetero atoms selected from N, O, and S in a ring where the ring system is an aromatic ring. The term includes monocyclic rings linked covalently or fused-ring polycyclic groups. A heteroaromatic group can be unsubstituted or substituted. The heteroaromatic group may include, but is not limited to, C₃-C₃₀ heteroaryl, C₄-C₃₀ heteroaryl alkyl, C₆-C₃₀ heteroaryl oxy and C₆-C₃₀ heteroaryl amino. Examples of heteroaromatic or heteroaryl may include, but is not limited to, an unfused or fused heteroaryl group such as pyrrolyl, pyridinyl, pyrimidinyl, pyrazinyl, pyridazinyl, triazinyl, tetrazinyl, imidazolyl, pyrazolyl, indolyl, iso-indolyl, indazolyl, indolizinyl, pyrrolizinyl, carbazolyl, benzo-carbazolyl, dibenzo-carbazolyl, indolo-carbazolyl, indeno-carbazolyl, benzofuro-carbazolyl, benzo-thieno-carbazolyl, carbolinyl, quinolinyl, iso-quinolinyl, phthlazinyl, quinoxalinyl, cinnolinyl, quinazolinyl, quinolizinyl, purinyl, benzo-quinolinyl, benzo-iso-quinolinyl, benzo-quinazolinyl, benzo-quinoxalinyl, acridinyl, phenazinyl, phenoxazinyl, phenothiazinyl, phenanthrolinyl, perimidinyl, phenanthridinyl, pteridinyl, naphthyridinyl, furanyl, pyranyl, oxazinyl, oxazolyl, oxadiazolyl, triazolyl, dioxinyl, benzo-furanyl, dibenzo-furanyl, thiopyranyl, xanthenyl, chromenyl, iso-chromenyl, thioazinyl, thiophenyl, benzo-thiophenyl, dibenzo-thiophenyl, difuro-pyrazinyl, benzofuro-dibenzo-furanyl, benzothieno-benzo-thiophenyl, benzothieno-dibenzo-thiophenyl, benzothieno-benzo-furanyl, benzothieno-dibenzo-furanyl, xanthene-linked spiro acridinyl, dihydroacridinyl substituted with at least one C₁-C₁₀ alkyl, and N-substituted spiro fluorenyl. Substituents for the heteroaromatic group or the heteroaryl group are as defined herein.

As an example, each of the aromatic group or the heteroaromatic group of R¹ to R⁷ may consist of or include one to three aromatic or heteroaromatic rings. When the number of the aromatic or heteroaromatic rings of R¹ to R⁷ becomes more than four, the conjugated structure within the whole molecule becomes too long, thus, the organic metal compound may have too narrow energy bandgap. For example, each of the aryl group or the heteroaryl group of R¹ to R⁷ may independently include, but is not limited to, phenyl, biphenyl, naphthyl, anthracenyl, pyrrolyl, triazinyl, imidazolyl, pyrazolyl, pyridinyl, pyrazinyl, pyrimidinyl, pyridazinyl, furanyl, benzo-furanyl, dibenzo-furanyl, thiophenyl, benzo-thiophenyl, dibenzo-thiophenyl, carbazolyl, acridinyl, carbolinyl, phenazinyl, phenoxazinyl and/or phenothiazinyl, each of which may be unsubstituted or substituted with at least one of deuterium, alkyl, aryl, and heteroaryl.

The C₆-C₃₀ arylene and the C₃-C₃₀ heteroarylene of L¹ may be a bivalent linking (bridging) group corresponding to the above aryl and the heteroaryl. As an example, the arylene and the heteroarylene may include, but is not limited to, phenylene, naphthylene and pyridylene, each of which is unsubstituted or substituted with at least one aryl *(e.g.,* phenyl, naphthyl, anthracenyl, phenanthrenyl) and/or deuterium.

In one example embodiment, at least one of R¹ to R⁷ in Formula 1 may be a deuterium, a deuterium-substituted C₁-C₂₀ alkyl, a deuterium-substituted C₃-C₂₀ cycloalkyl, a deuterium-substituted C₃-C₂₀ heterocycloalkyl, a deuterium-substituted C₆-C₃₀ aryl, or a deuterium-substituted C₃-C₃₀ heteroaryl. In another example embodiment, each of R¹ to R⁷ in Formula 1 may be, but is not limited to, a protium, a deuterium, or an unsubstituted or deuterium-substituted C₁-C₂₀ alkyl.

In yet another example embodiment, R² in Formula 1 may be, but is not limited to, a protium, a deuterium, a C₆-C₃₀ aryl unsubstituted or substituted with at least one of a deuterium, a C₁-C₁₀ alkyl, a C₆-C₂₀ aryl and a C₃-C₂₀ heteroaryl, or a C₃-C₃₀ heteroaryl unsubstituted or substituted with at least one of a deuterium, a C₁-C₁₀ alkyl, a C₆-C₂₀ aryl and a C₃-C₂₀ heteroaryl (in this case, each of the substituents, the C₁-C₁₀ alkyl, the C₆-C₂₀ aryl and the C₃-C₂₀ heteroaryl, may be independently unsubstituted or further substituted with a deuterium). As an example, R² in Formula 1 may be, but is not limited to, phenyl, biphenyl, naphthyl (*e.g*., 1-naphthyl or 2-napthyl), carbazolyl, dibenzofuranyl, dibenzothiophenyl, and fluorenyl, each of which may be independently unsubstituted or substituted with at least one of a deuterium, a C₁-C₁₀ alkyl, a C₆-C₂₀ aryl *(e.g.,* phenyl or naphthyl) and a C₃-C₃₀ heteroaryl (*e.g.,* carbazolyl) (in this case, each of the substituents, C₁-C₁₀ alkyl, C₆-C₂₀ aryl and C₃-C₂₀ heteroaryl, may be independently unsubstituted or further substituted with a deuterium).

In yet another example embodiment, the quinazoline ring constituting the second moiety may include a 1,2-quinazoline ring, a 1-3-quinazoline ring or a 1,4-quinazoline ring. In another example embodiment, the second moiety having the quinazoline ring may be linked to position-1, position-2, position-3 or position-4 of the acridine ring constituting the first moiety directly or via the linking group.

The organic compound having the structure of Formula 1 include multiple rings, and thus, it may have relatively high triplet energy level and relatively wide energy bandgap between HOMO (Highest Occupied Molecular Orbital) energy level and LUMO (Lowest Unoccupied Molecular Orbital) energy level compared to dopants in an emitting material layer. In addition, the acridine ring of the first moiety is fused through a 5-membered heteroring to form multiple ring systems, it has beneficial thermal stability.

The organic compound may be used in an emissive layer disposed between two electrodes. As an example, the organic compound having the structure of Formula 1 may be added to an emitting material layer as an n-type host so that it can transfer carriers to the ultimately emitting dopant rapidly. The driving voltage of the OLED and organic light emitting device may be reduced and the luminous efficiency and luminous lifespan of the OLED and the device may be improved by including the organic compound in the emissive layer.

In one example embodiment, the quinazoline ring of the second moiety may be a 1,3-quinazoline ring. The organic compound having the 1,3-quinazoline ring may have a structure represented by Formula 2: 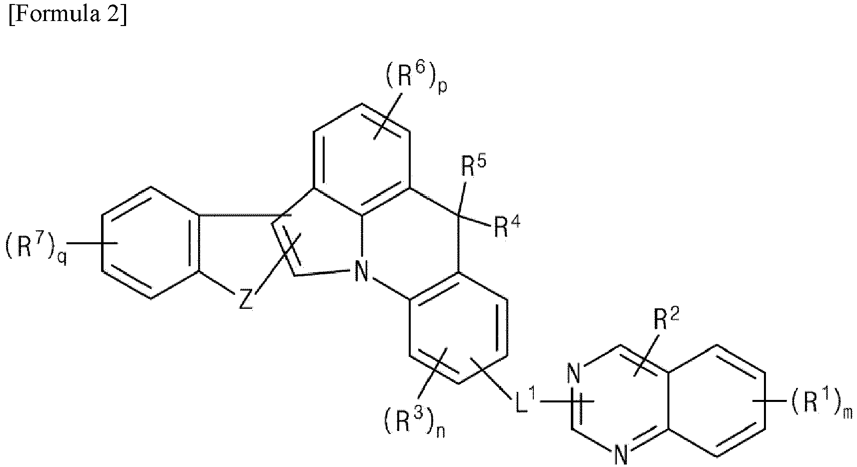
wherein in the Formula 2,
each of R¹ to R⁷, Z, L¹, m, n, p, and q is as defined in the Formula 1.

In yet another example embodiment, the second moiety having the quinazoline ring may be linked to position-3 of the acridine ring of the first moiety. The organic compound having this arrangement may have a structure represented by Formula 3 or Formula 4: 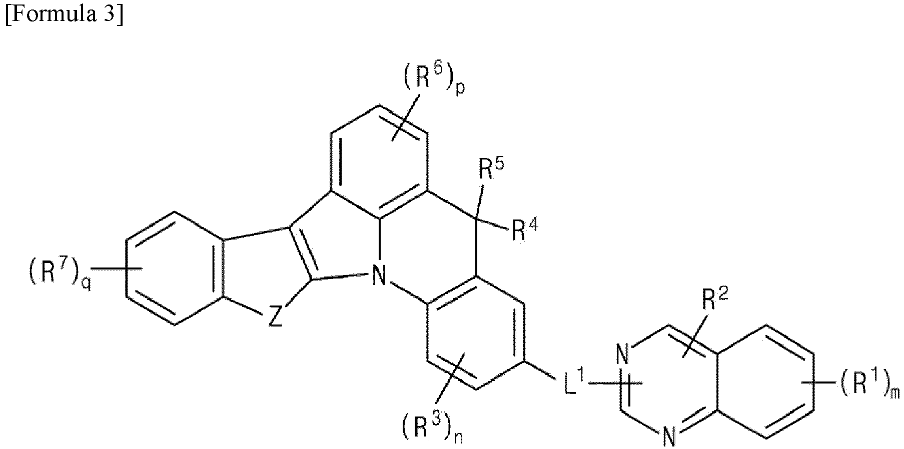 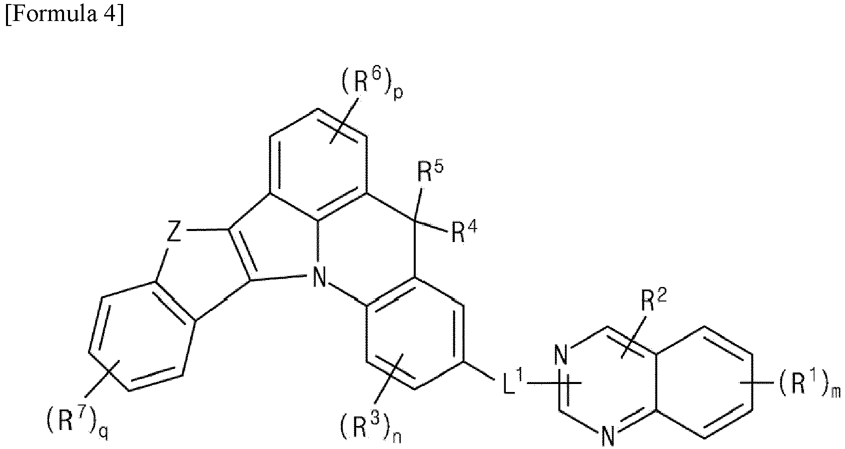
wherein in the Formulae 3 and 4,
each of R¹ to R⁷, Z, L¹, m, n, p, and q is as defined in the Formula 1.

In an example embodiment, the organic compound having any one of the structures of Formulae 1 to 4 may include at least one of, or may be selected from, but is not limited to, the following organic compounds of Formula 5:

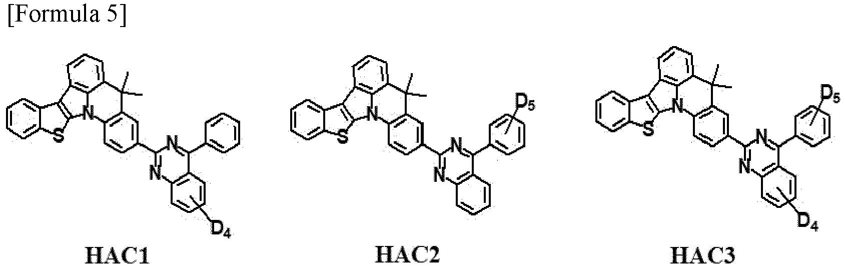

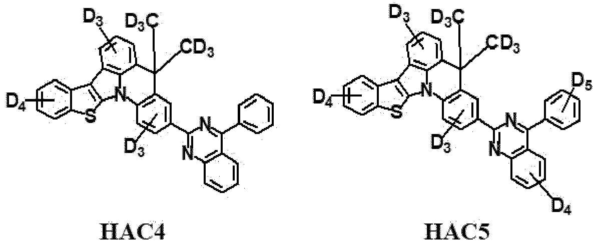

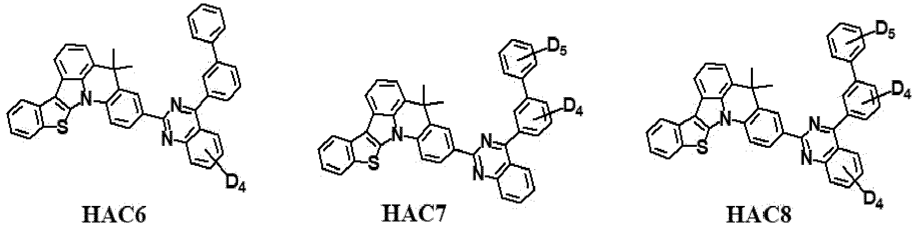

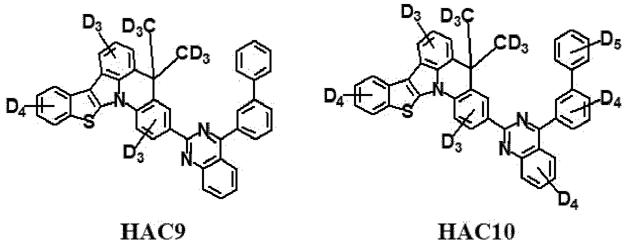

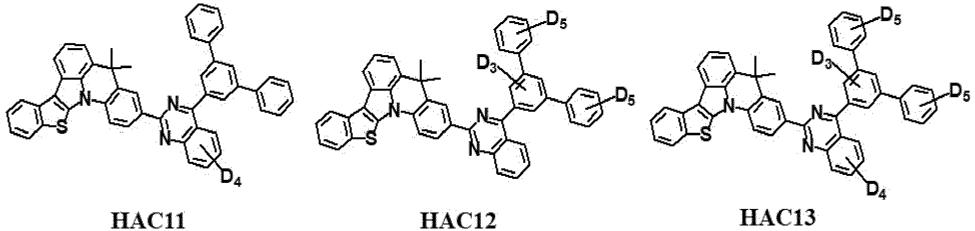

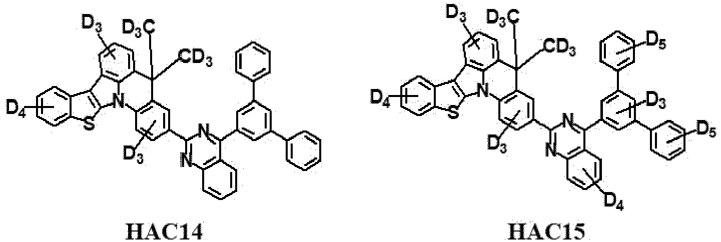

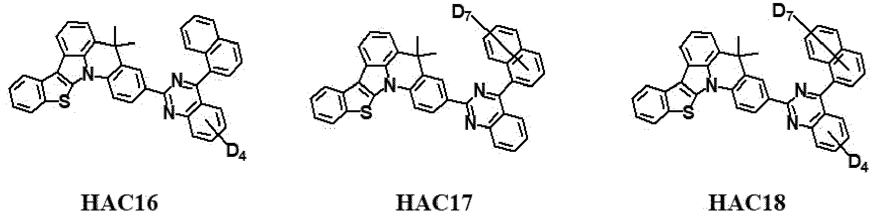

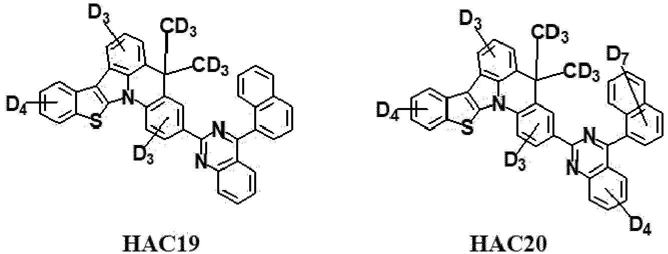

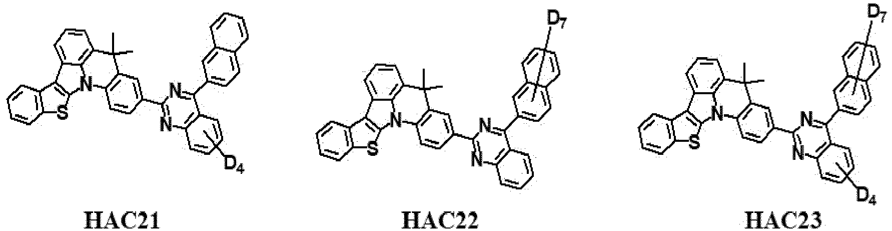

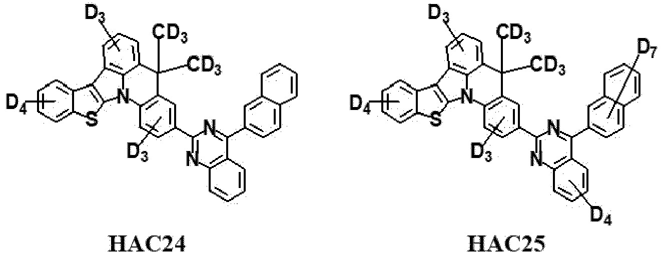

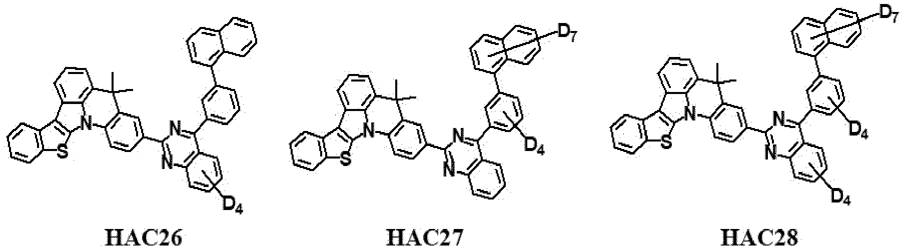

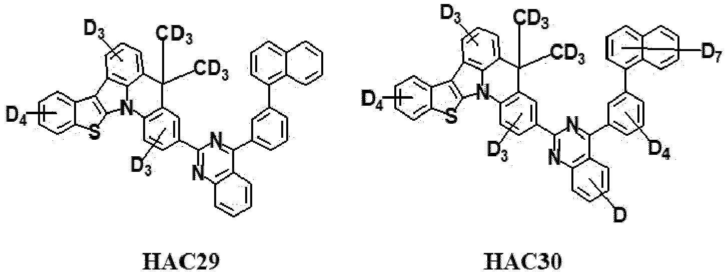

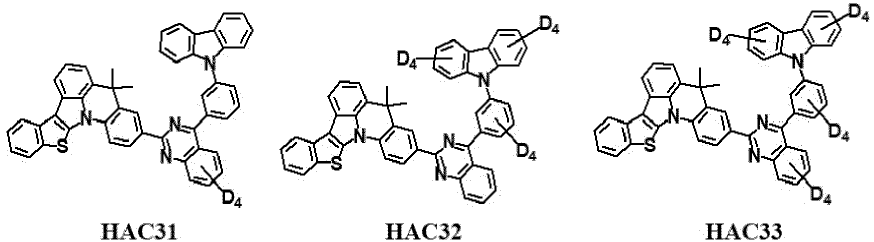

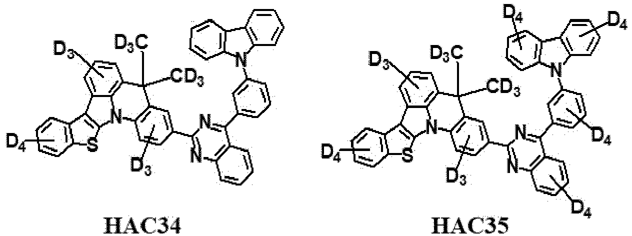

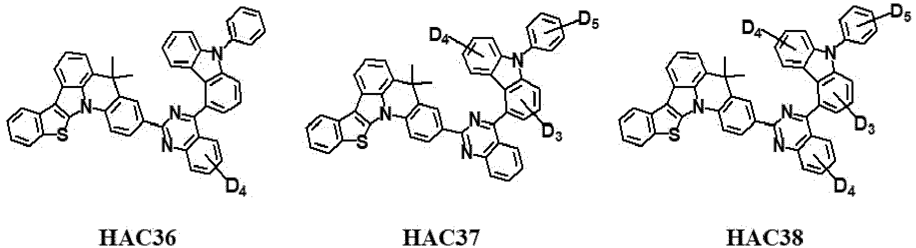

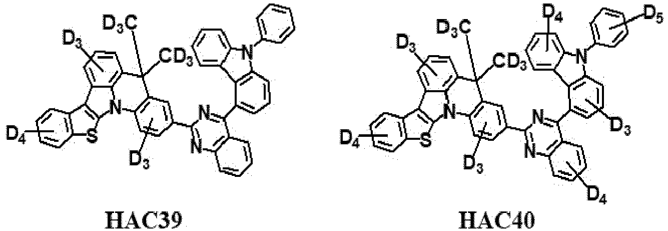

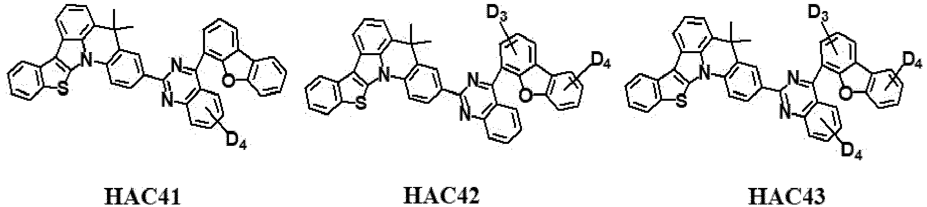

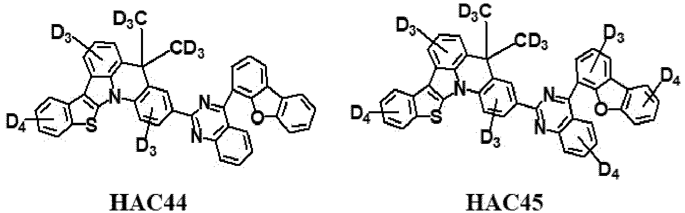

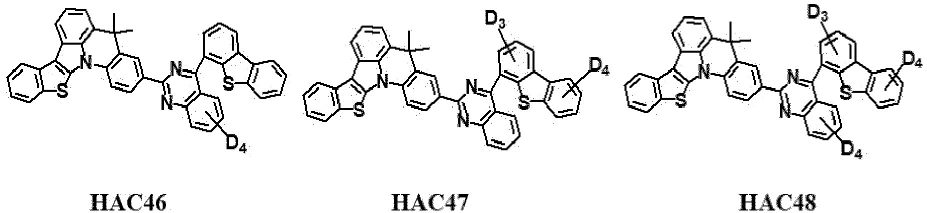

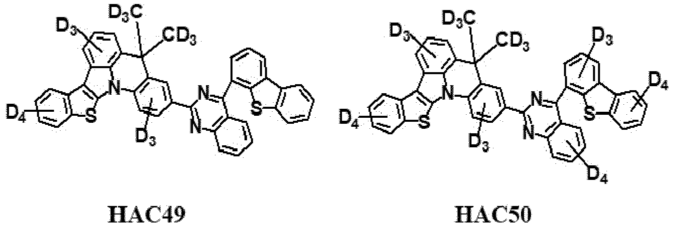

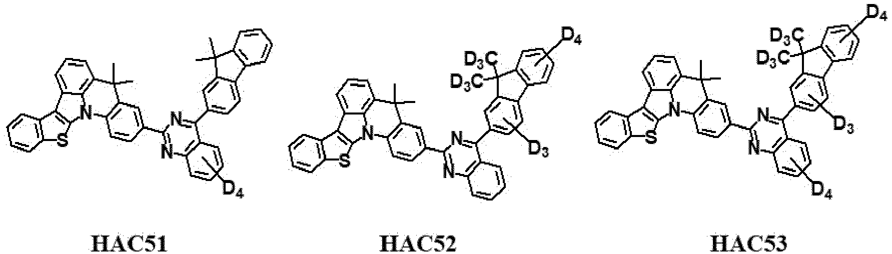

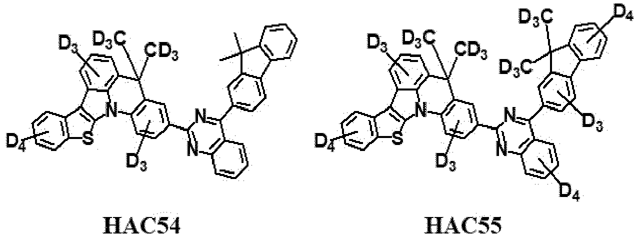

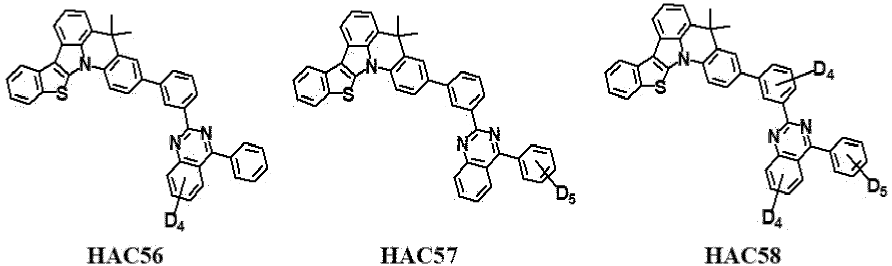

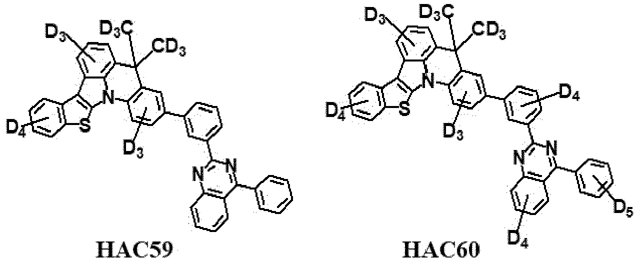

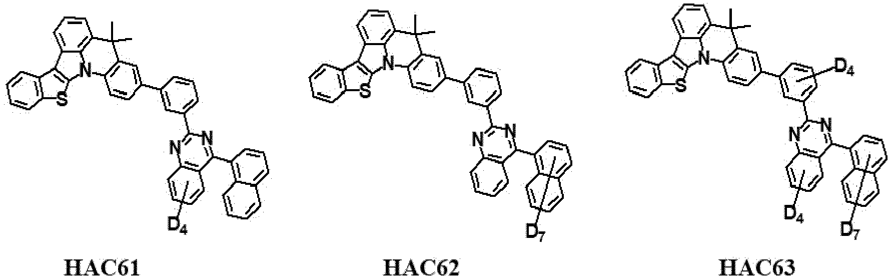

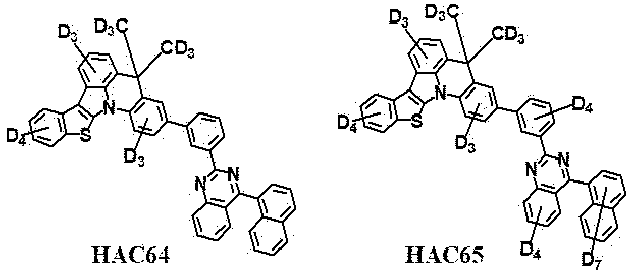

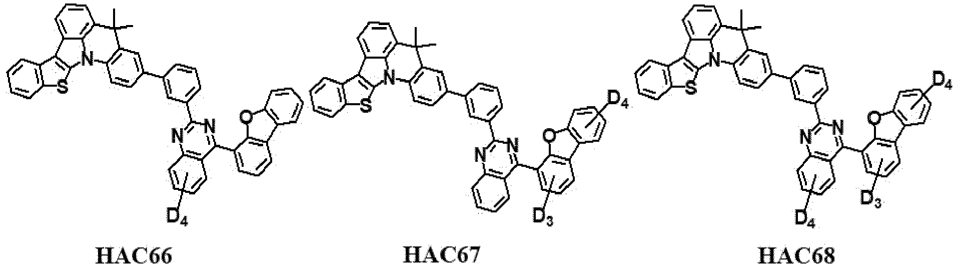

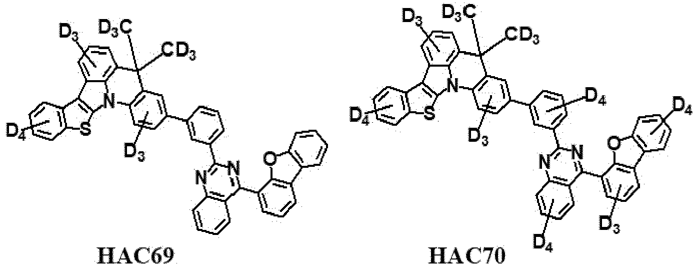

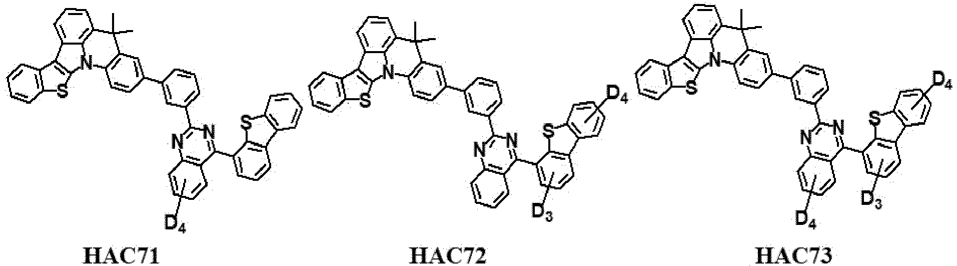

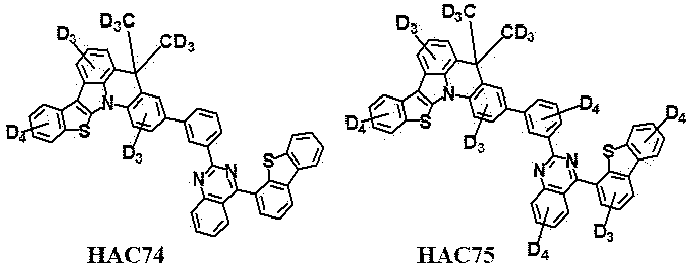

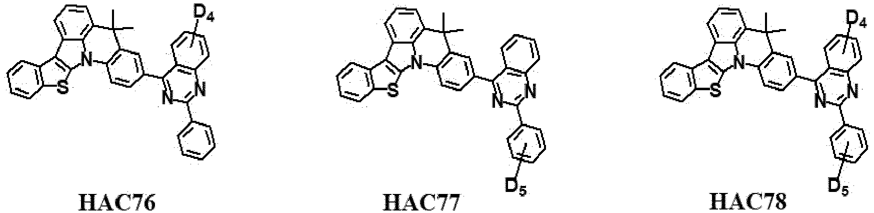

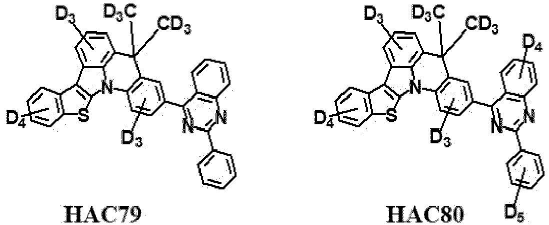

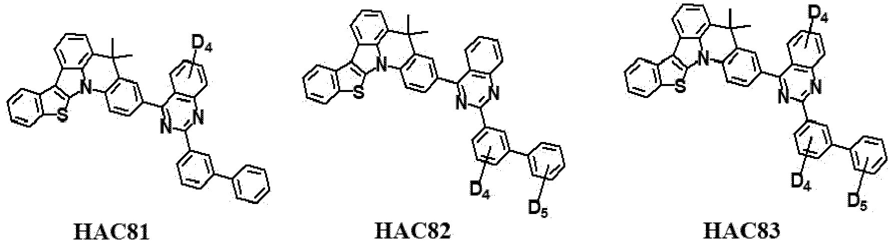

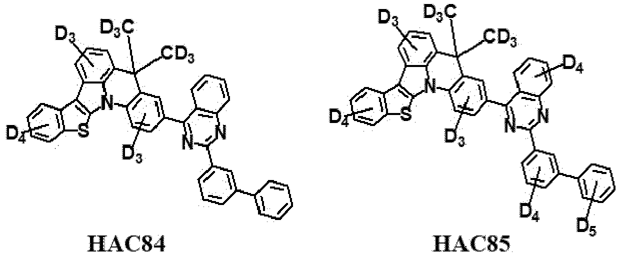

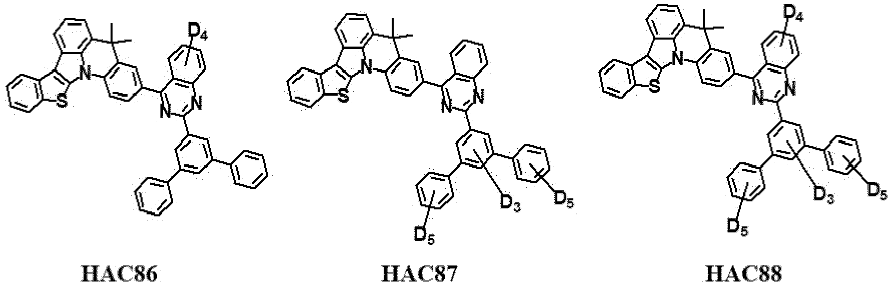

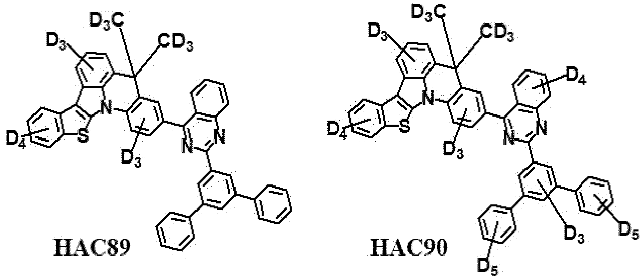

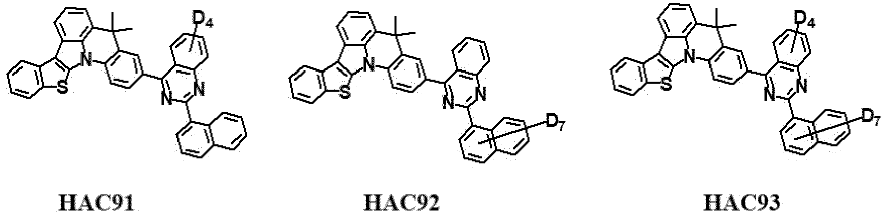

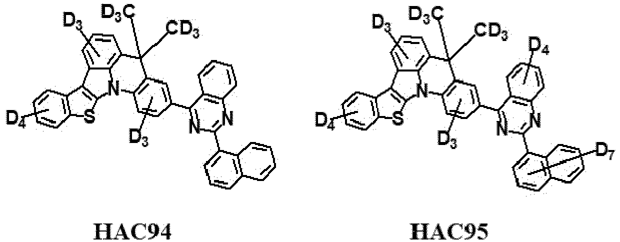

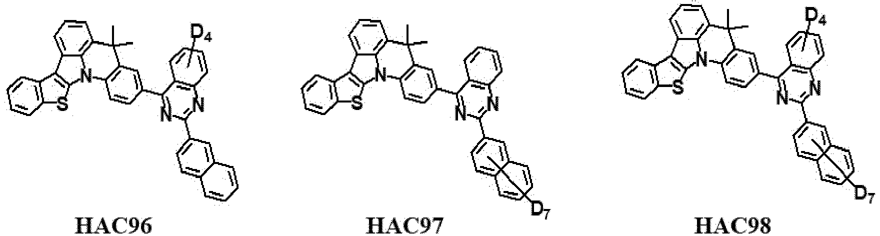

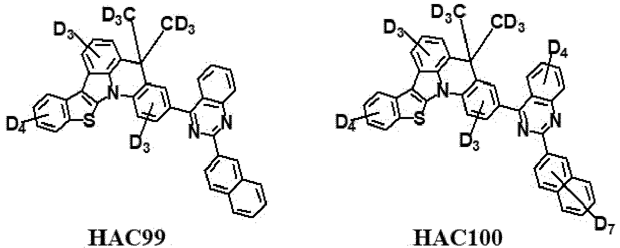

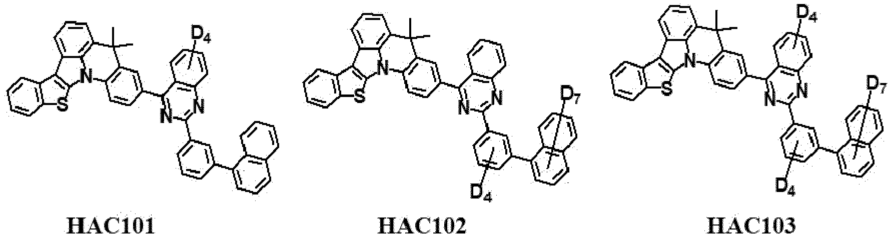

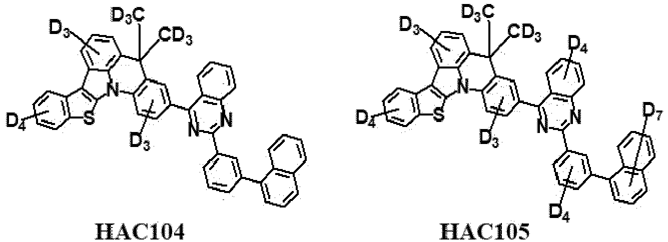

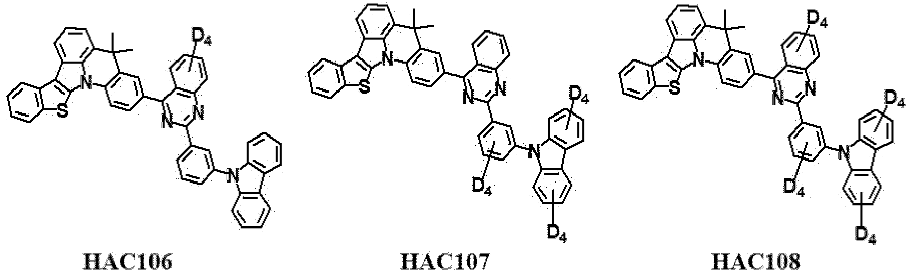

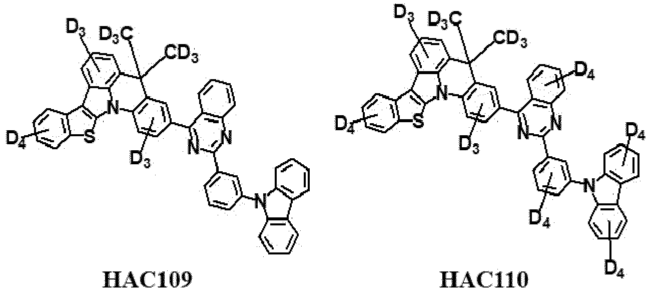

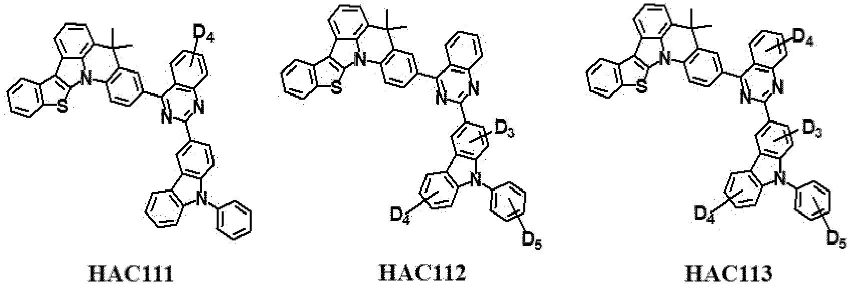

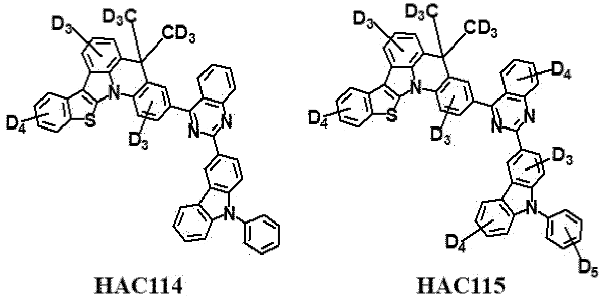

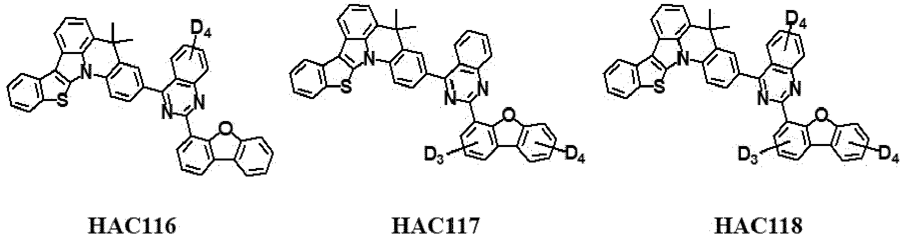

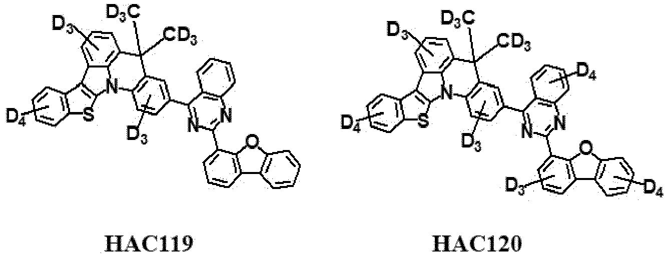

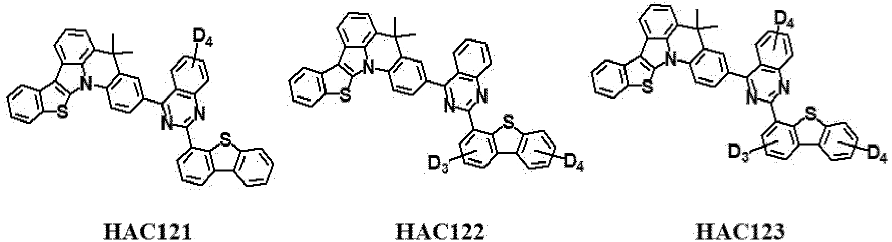

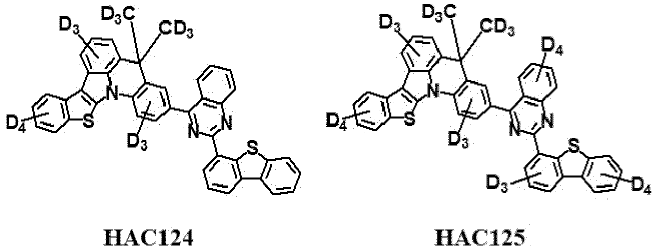

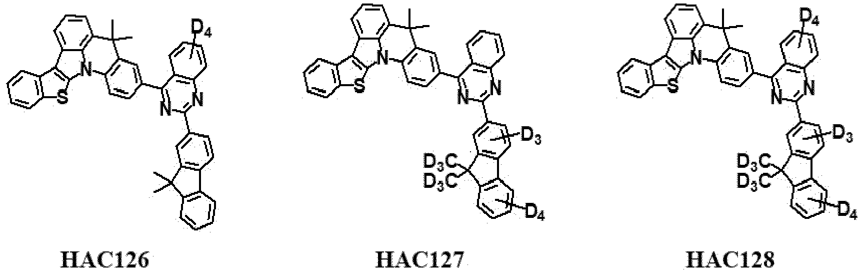

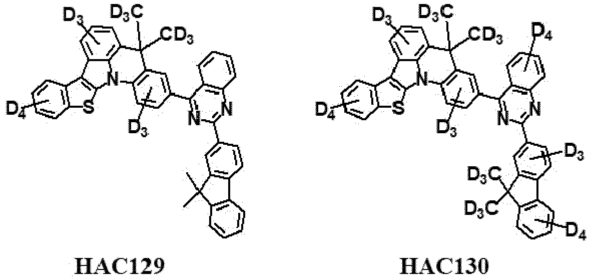

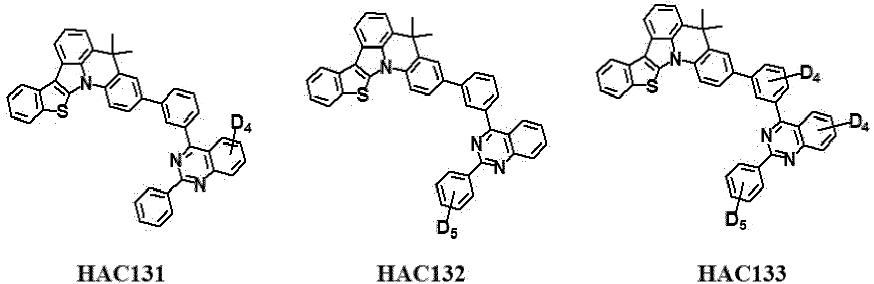

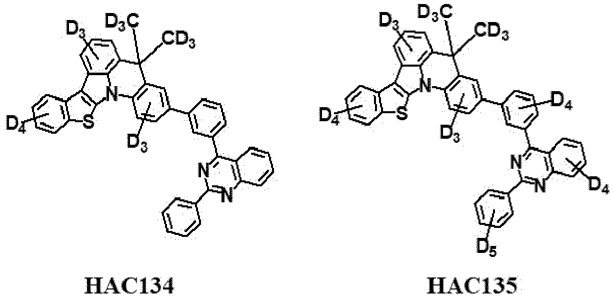

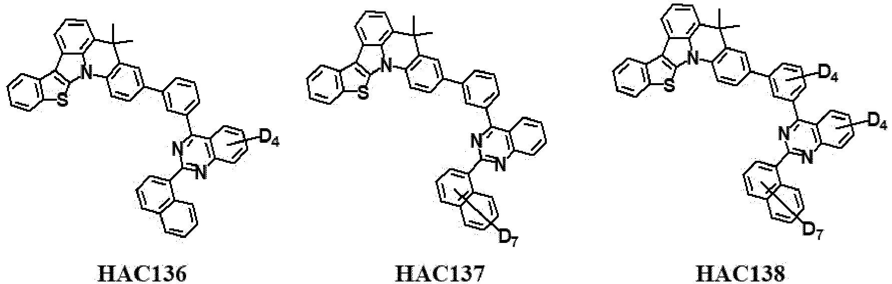

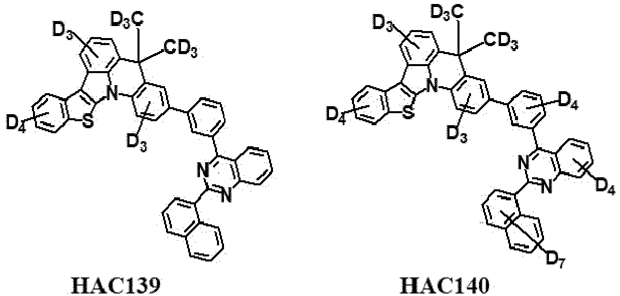

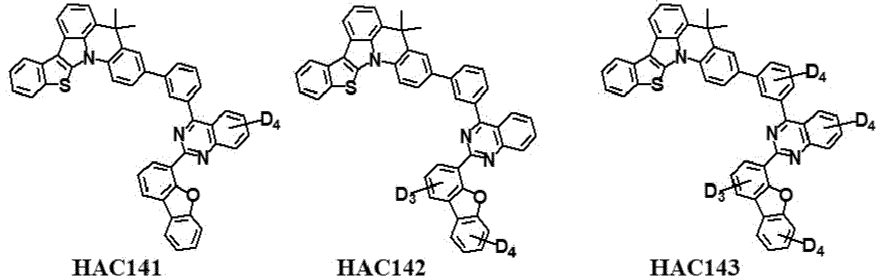

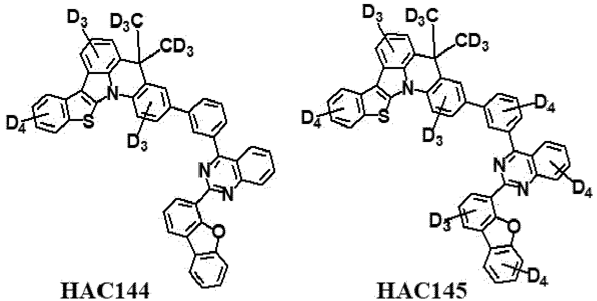

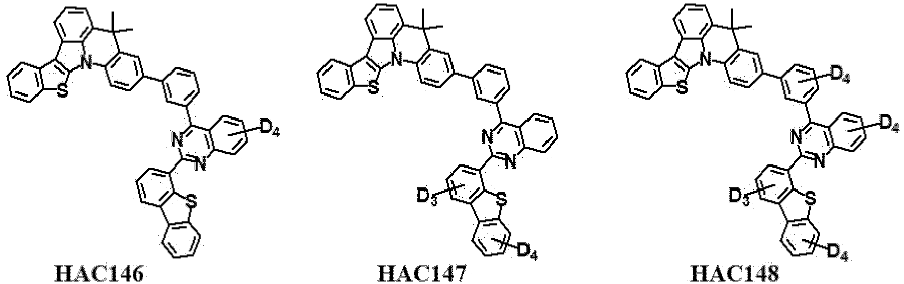

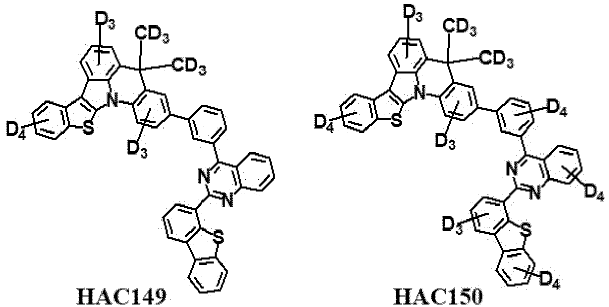

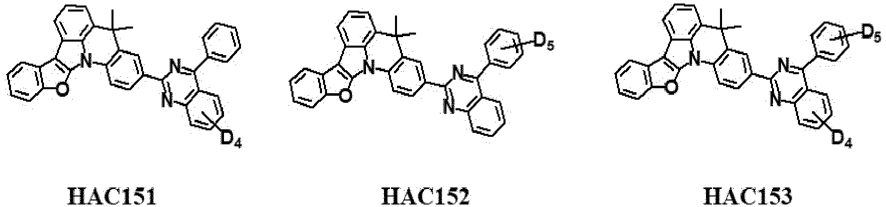

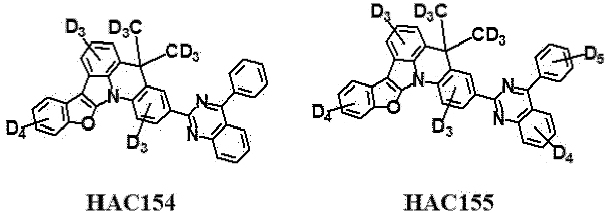

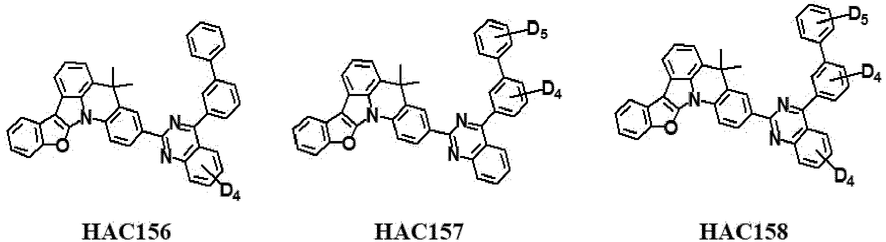

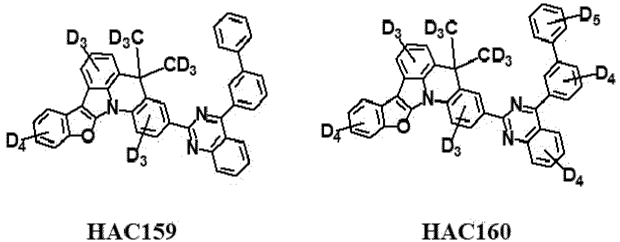

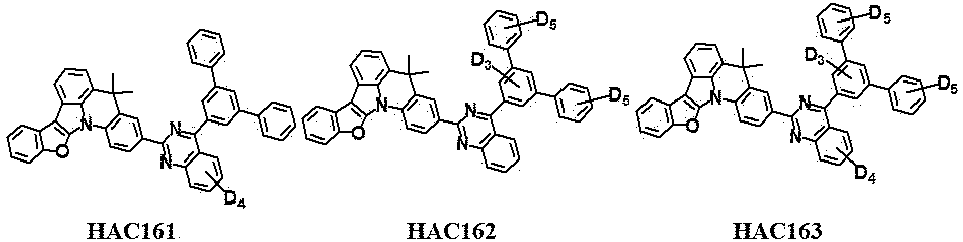

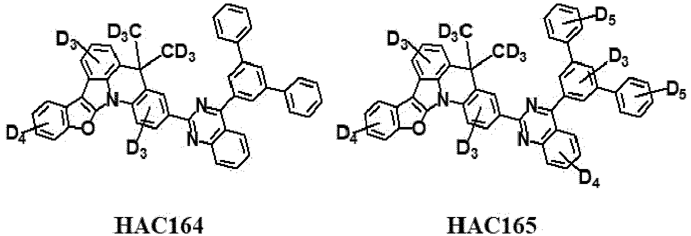

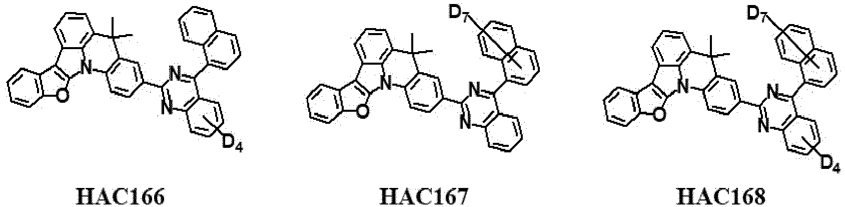

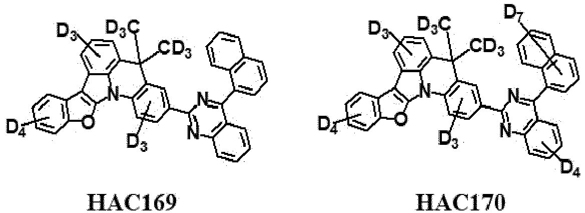

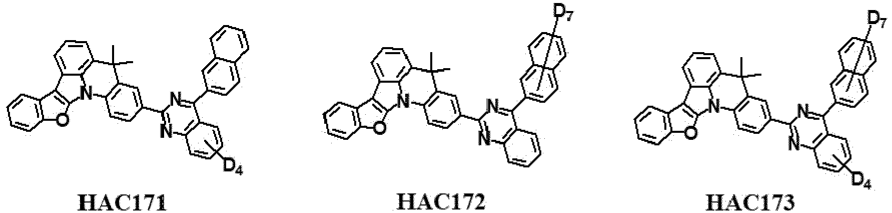

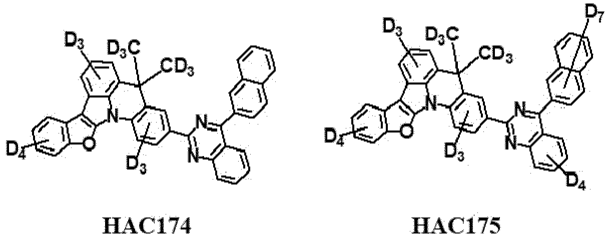

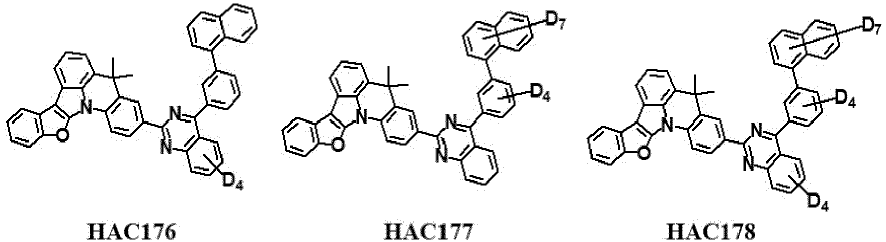

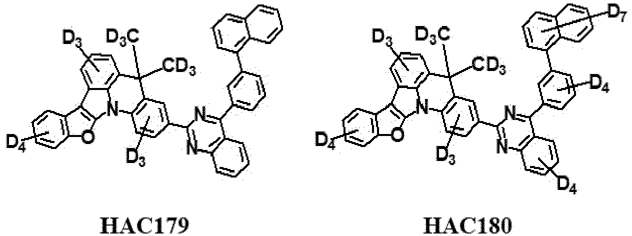

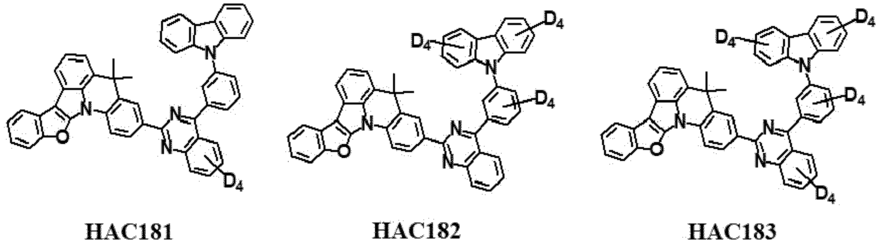

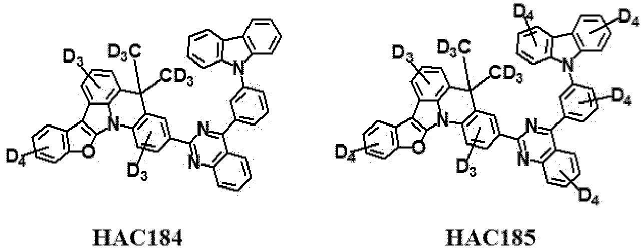

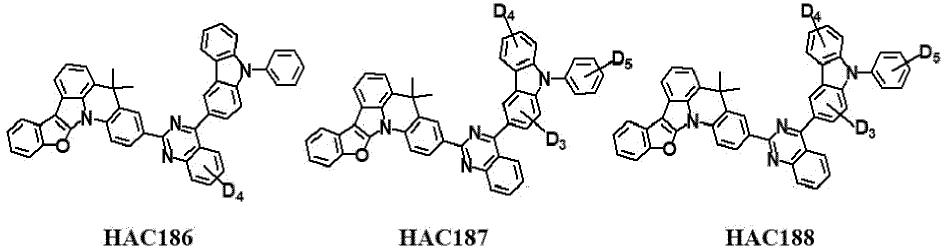

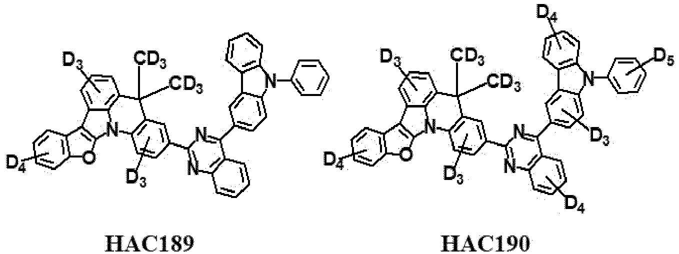

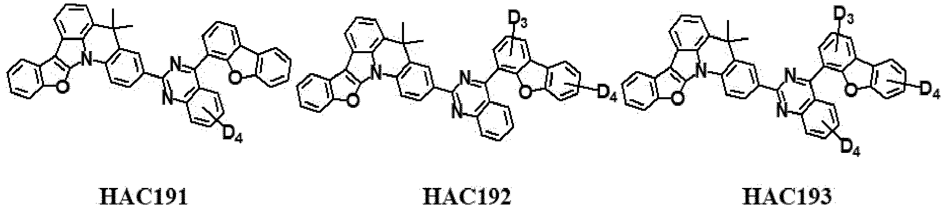

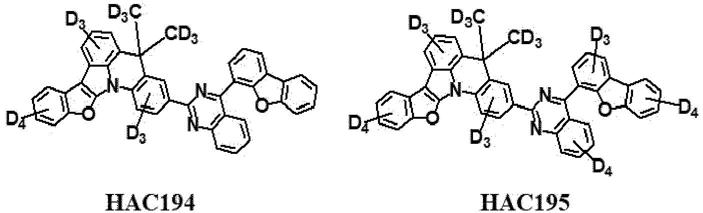

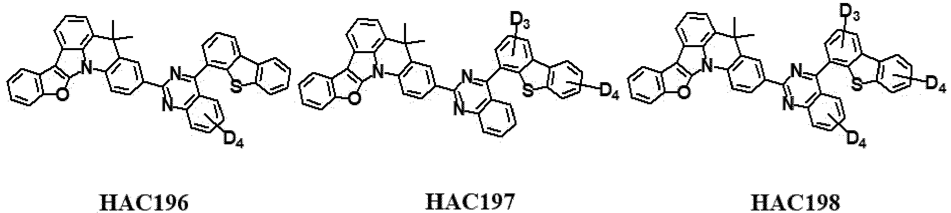

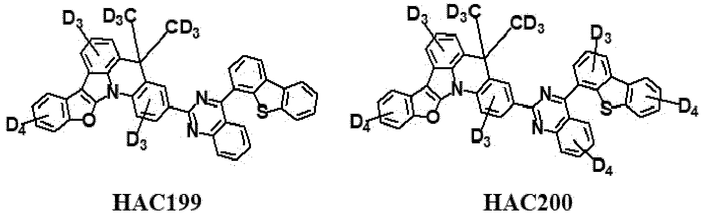

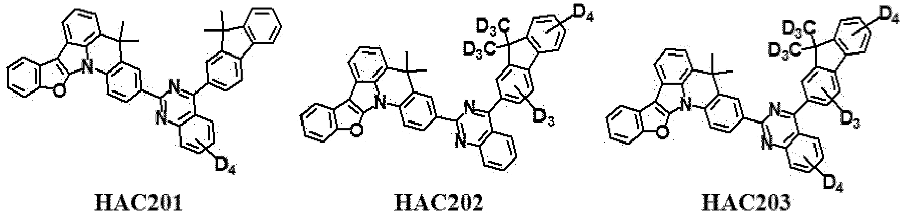

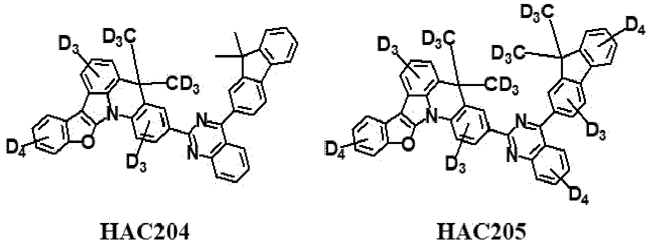

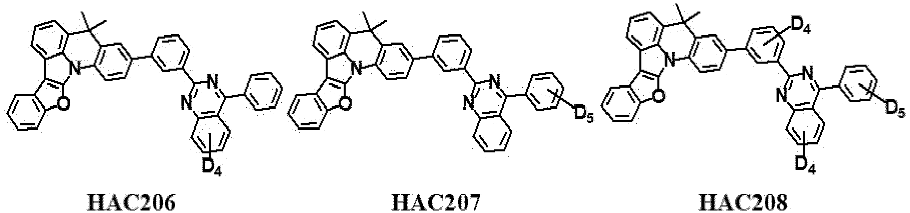

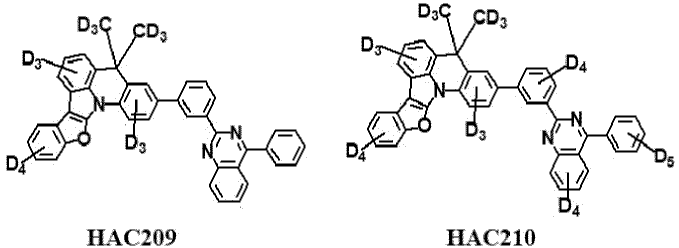

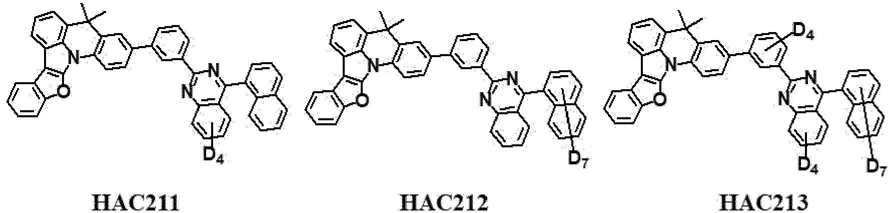

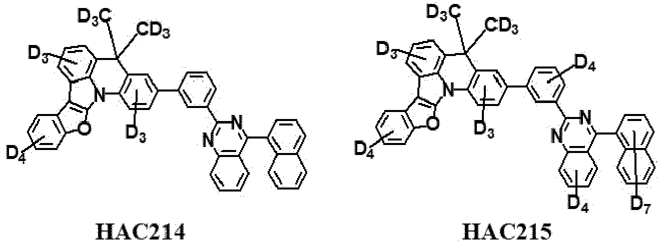

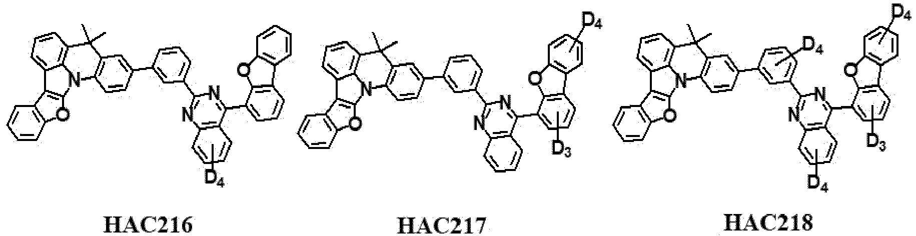

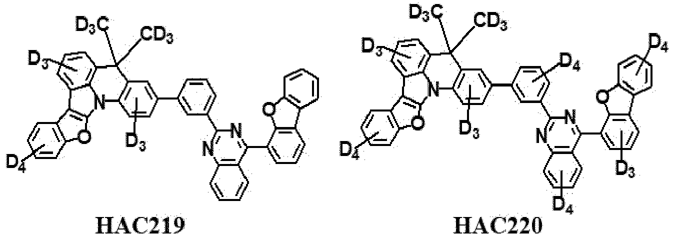

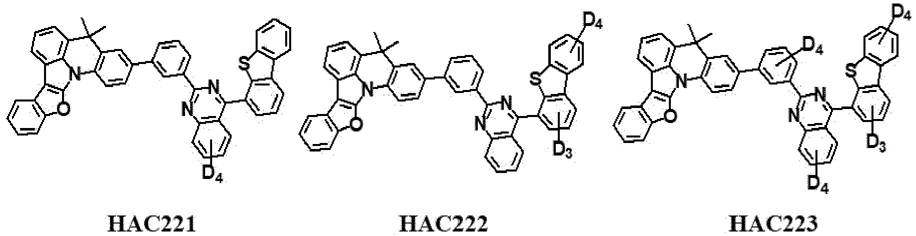

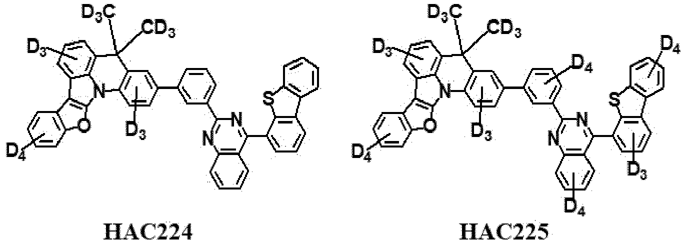

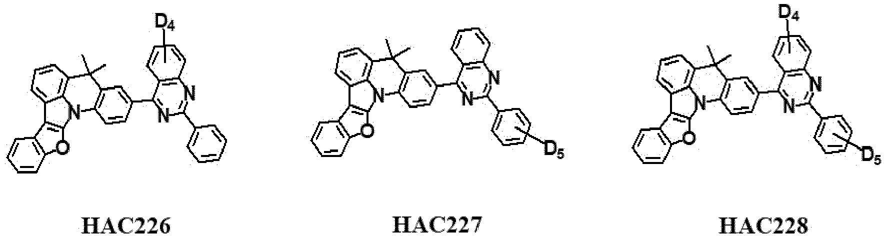

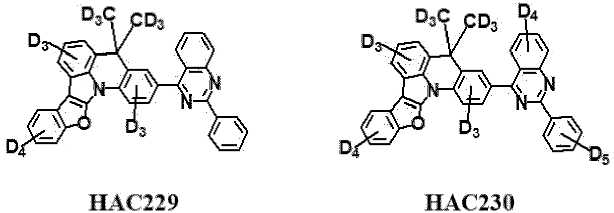

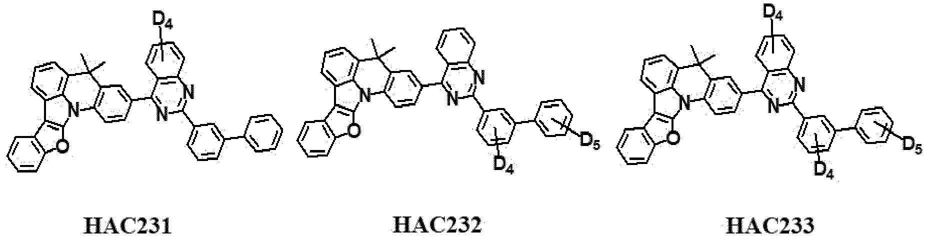

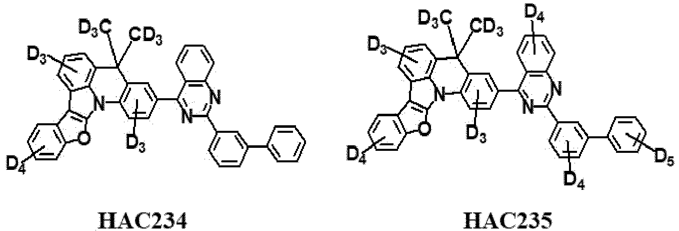

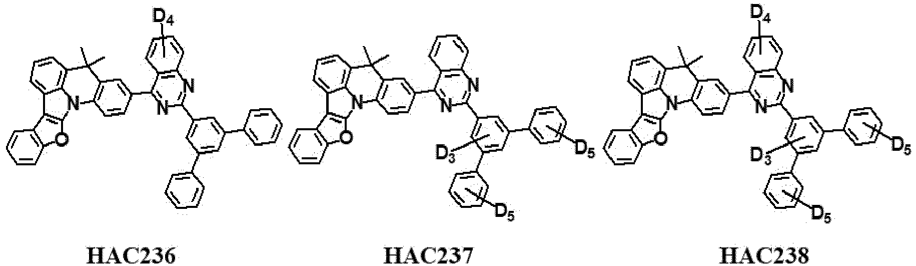

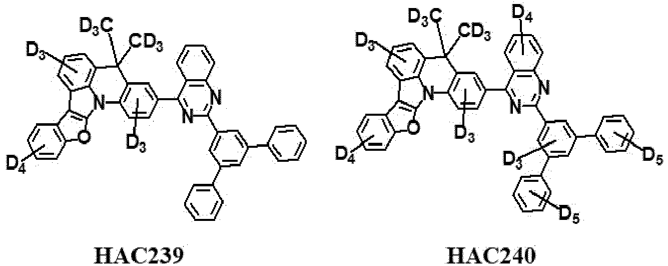

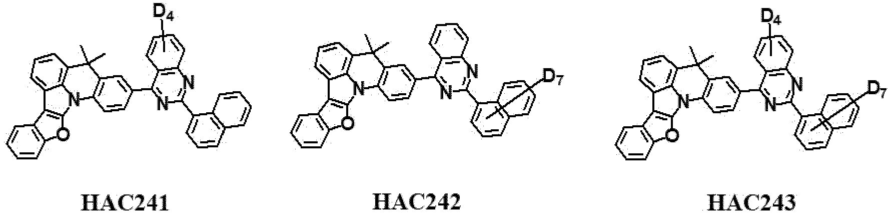

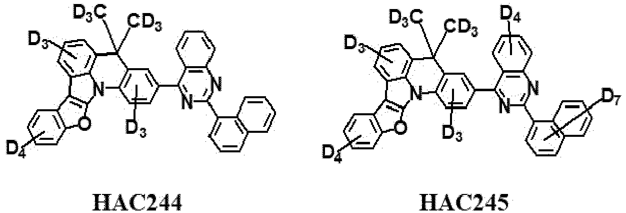

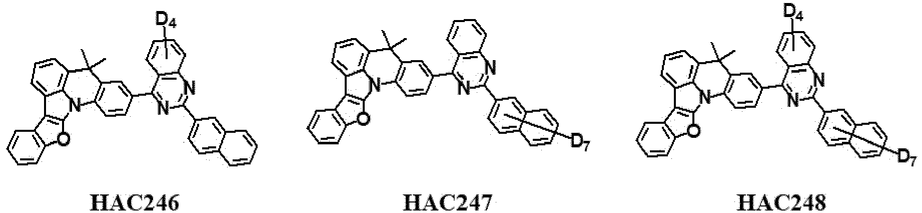

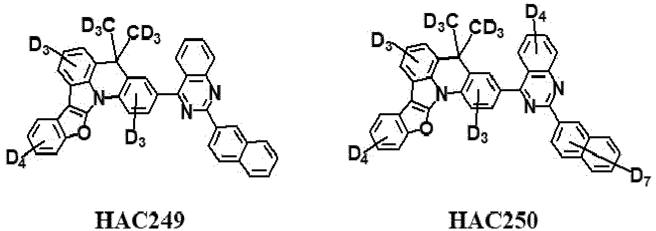

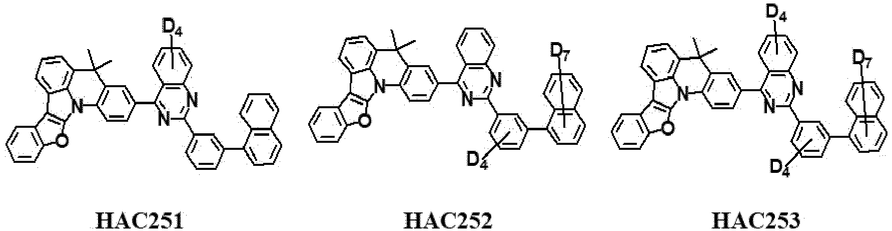

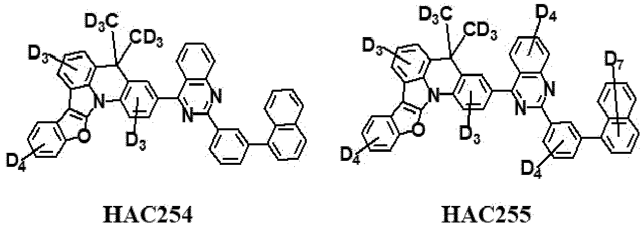

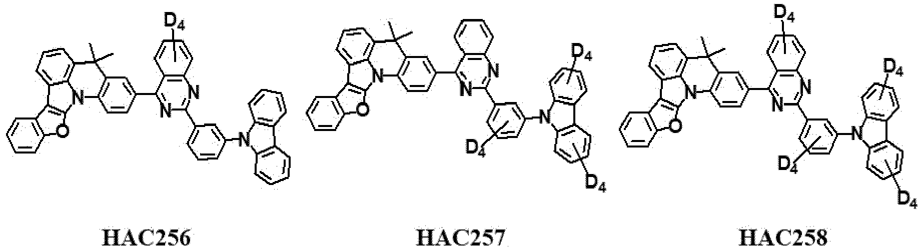

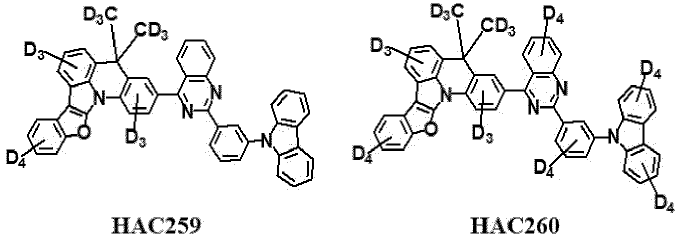

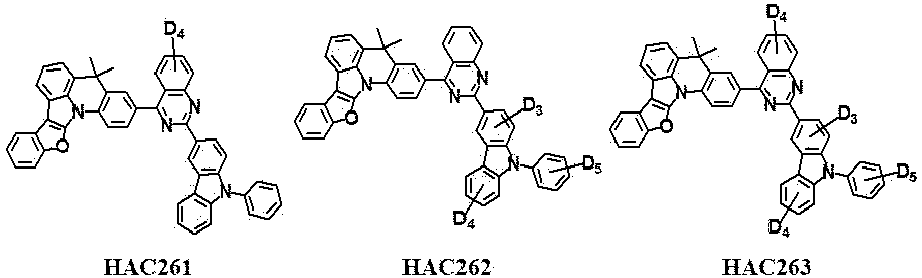

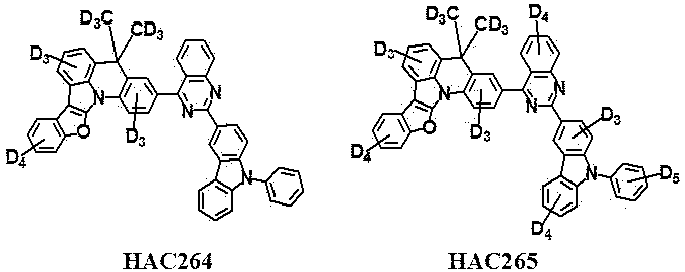

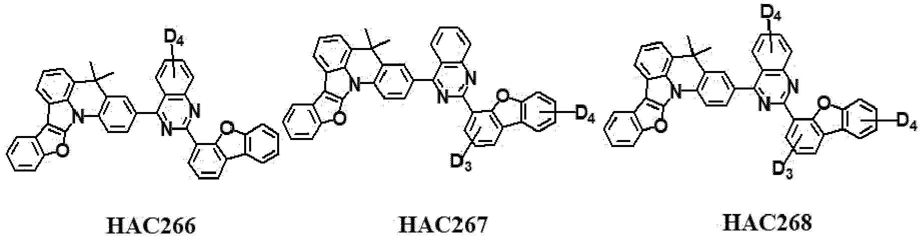

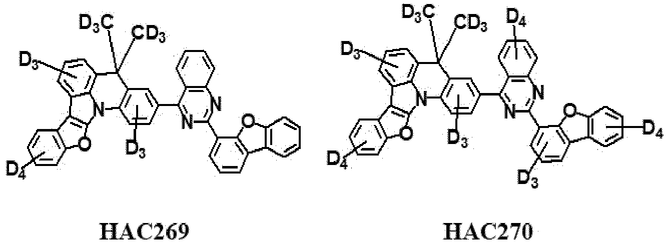

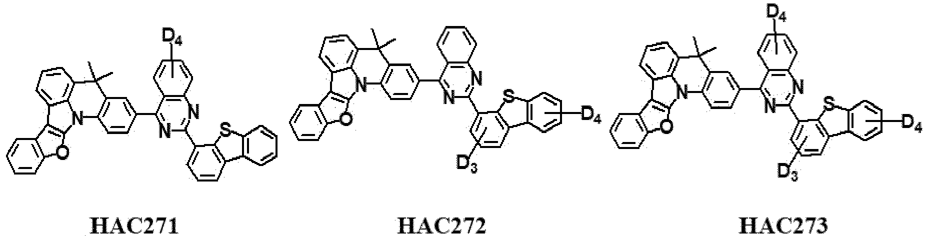

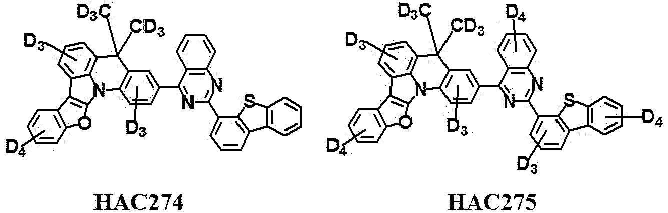

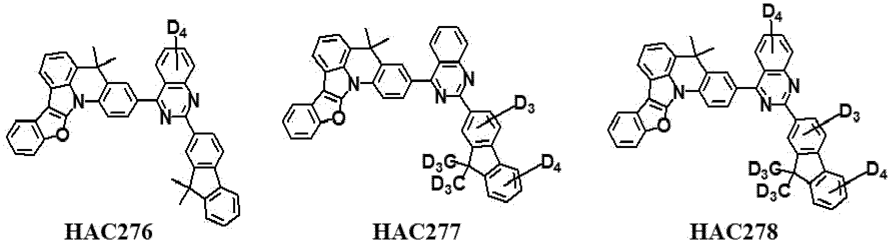

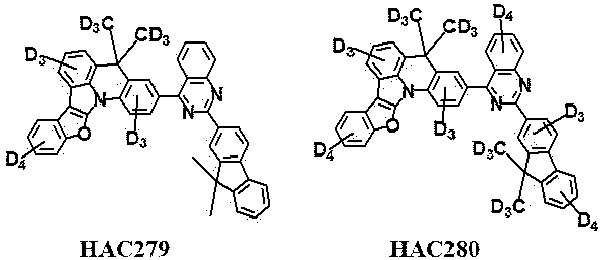

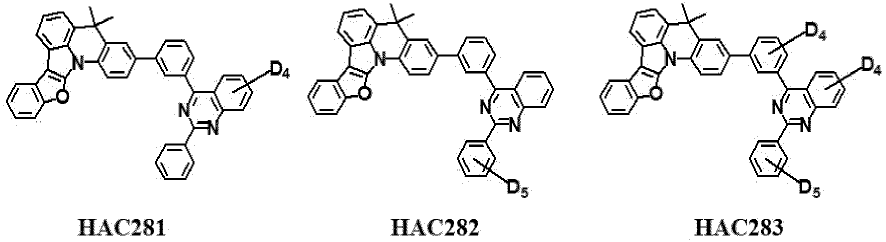

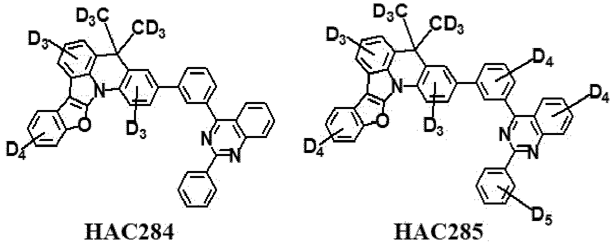

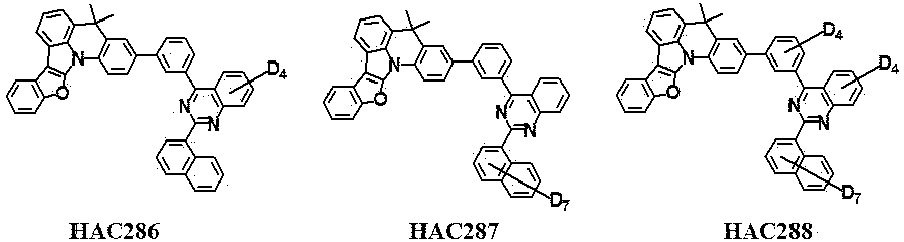

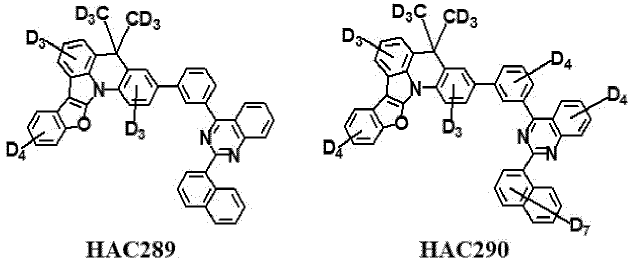

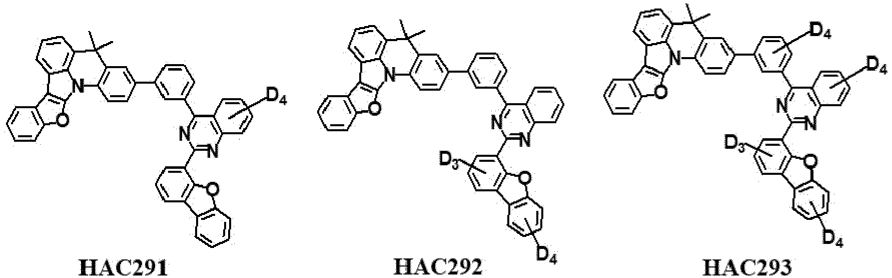

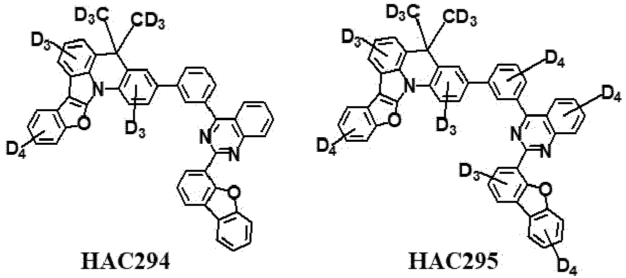

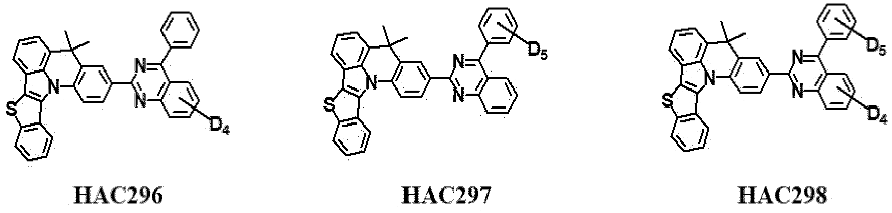

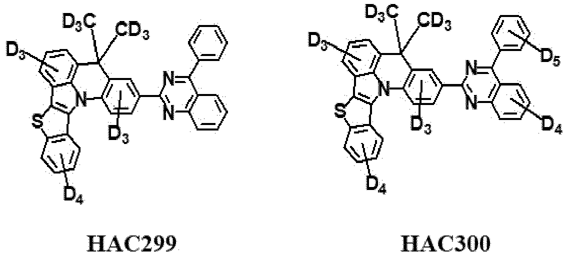

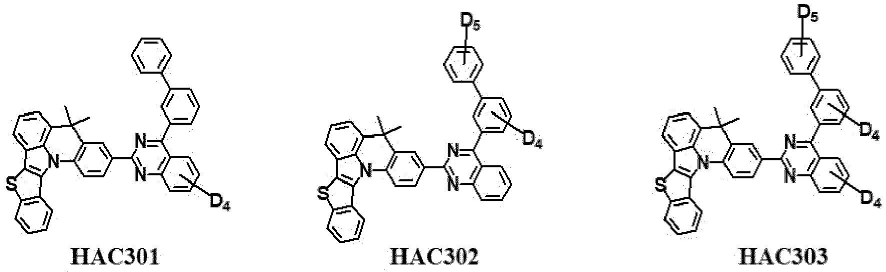

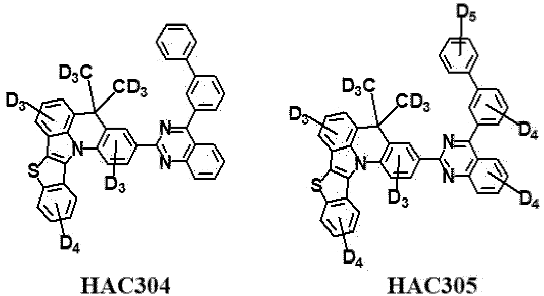

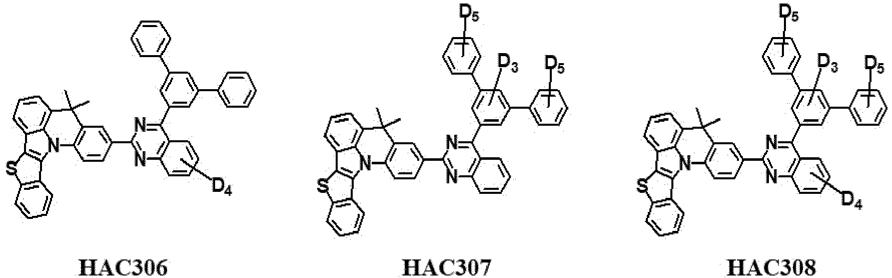

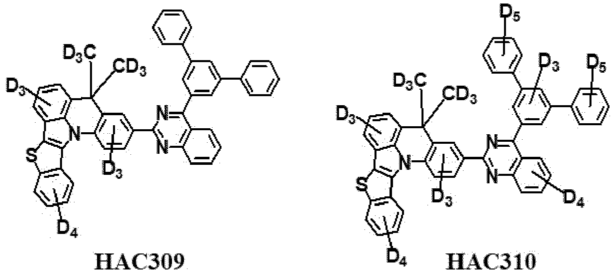

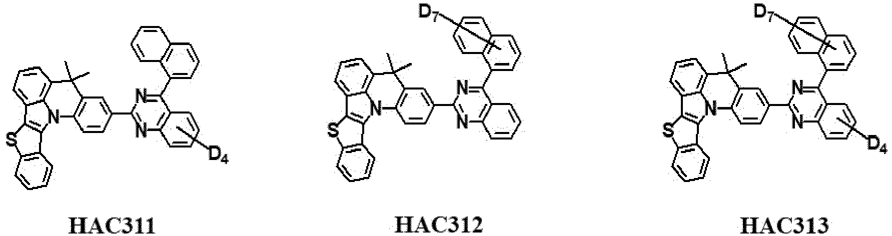

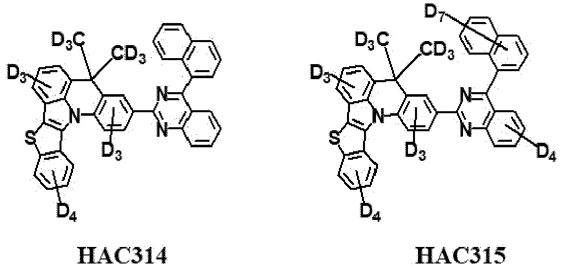

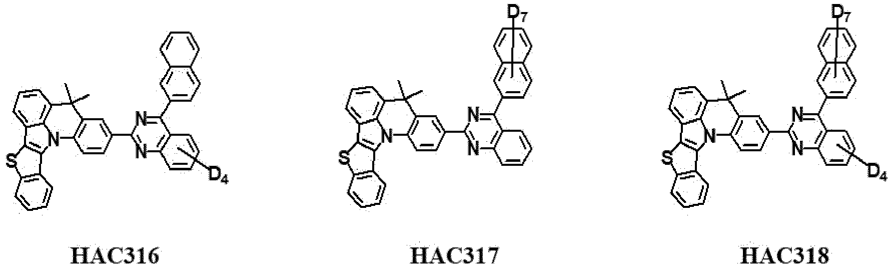

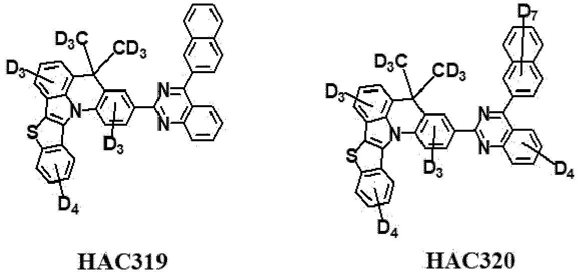

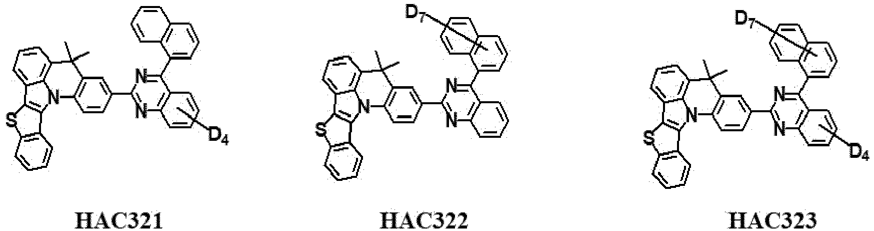

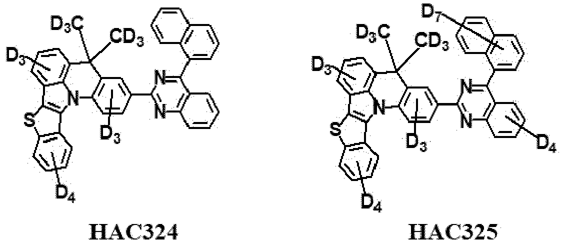

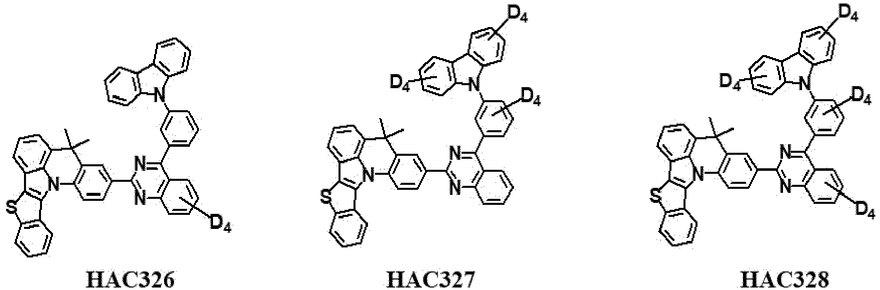

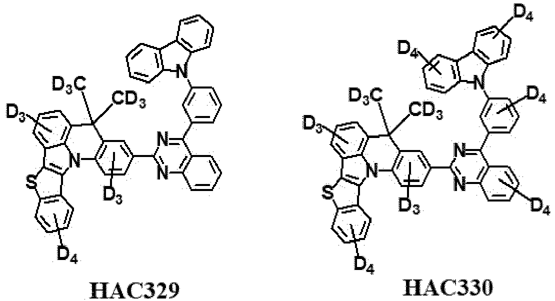

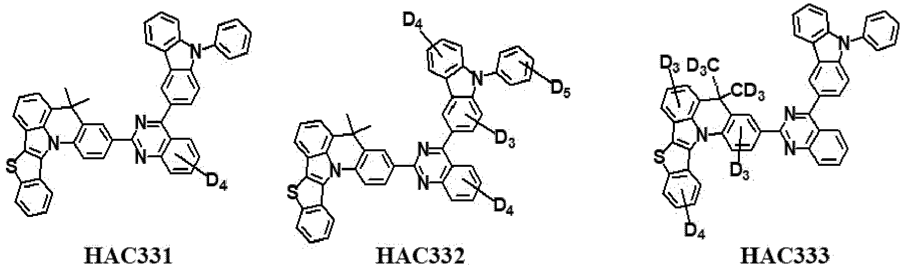

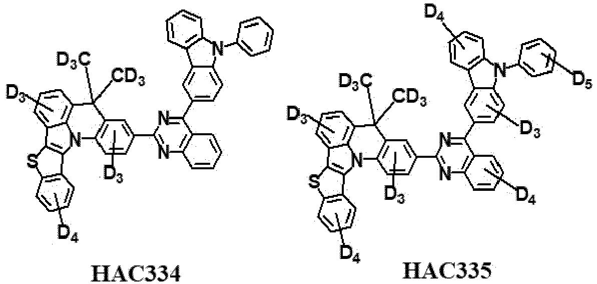

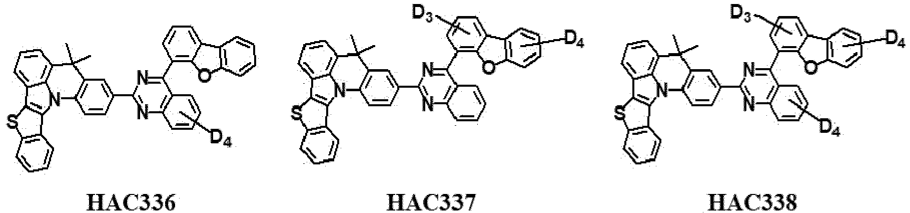

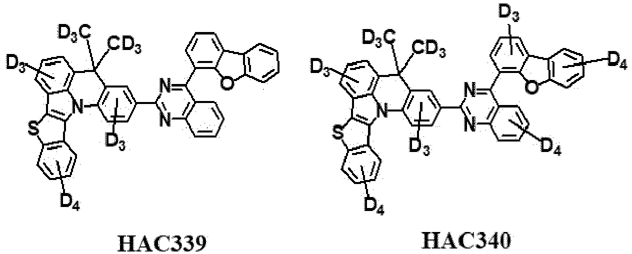

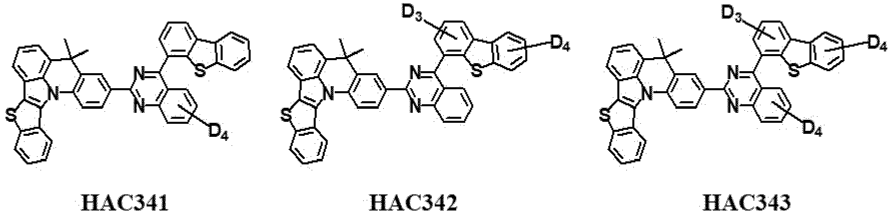

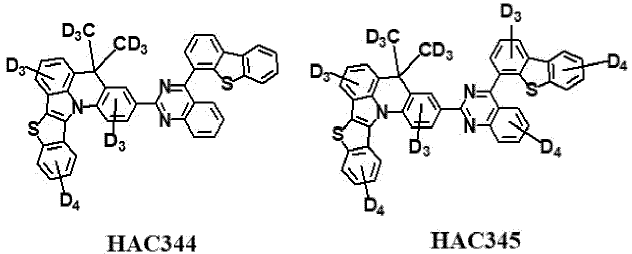

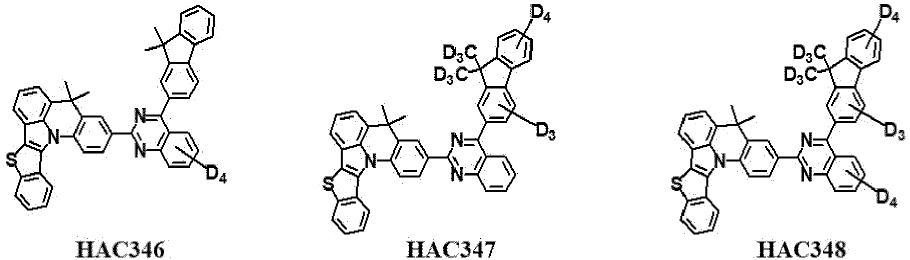

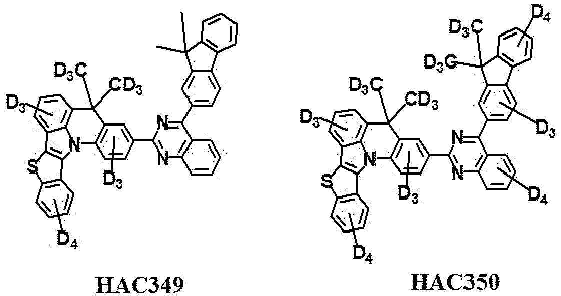

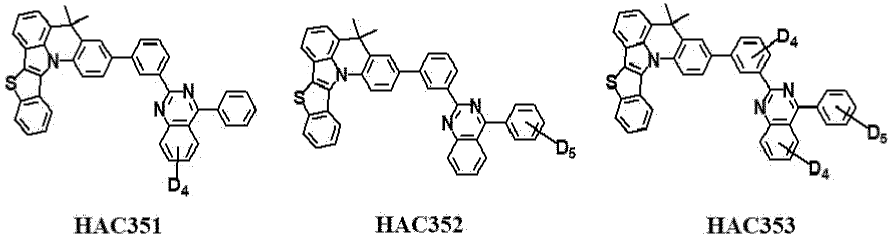

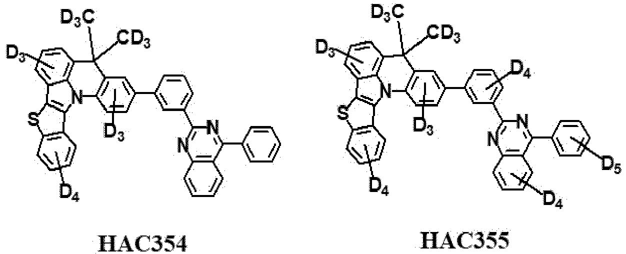

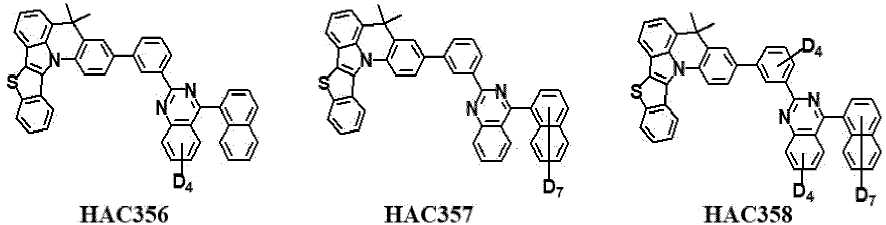

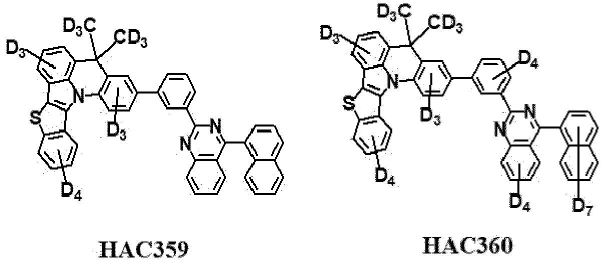

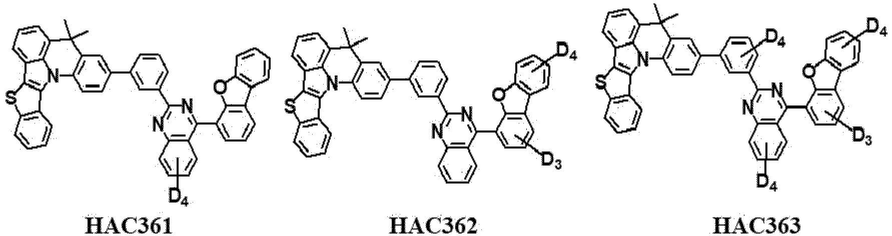

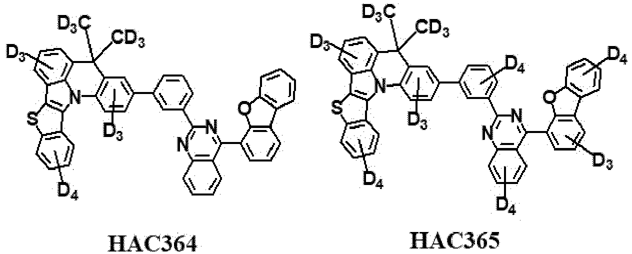

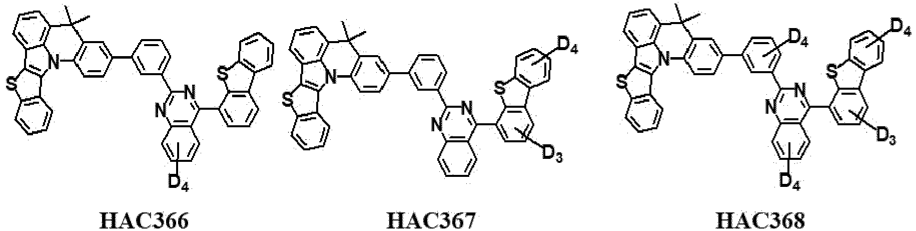

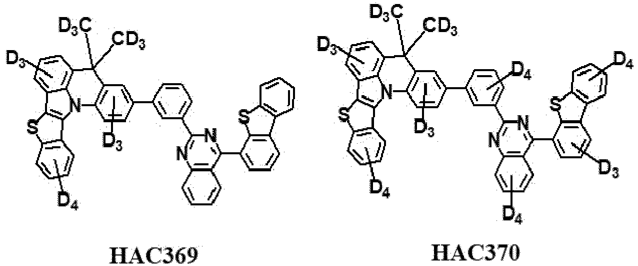

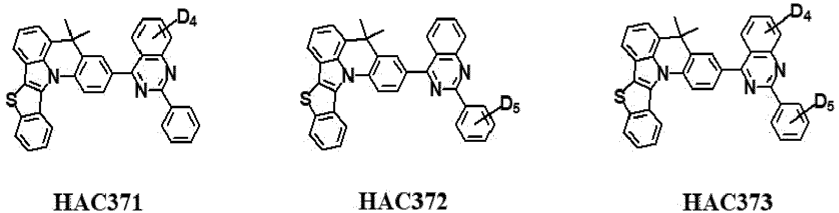

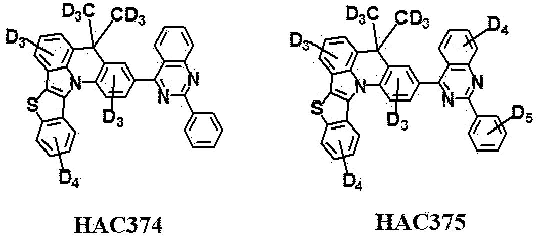

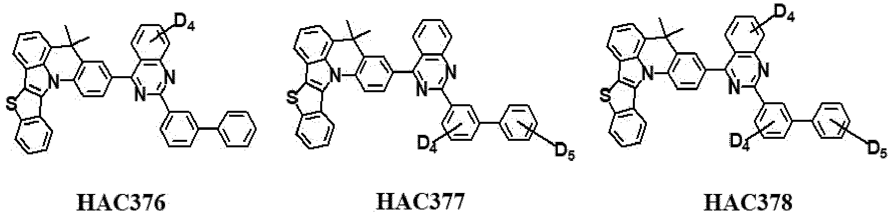

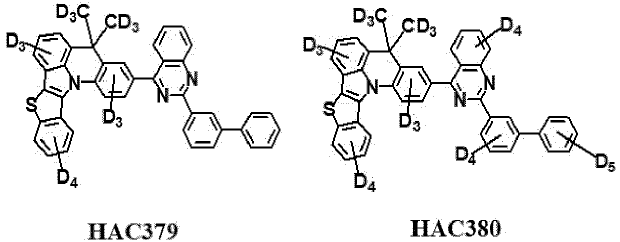

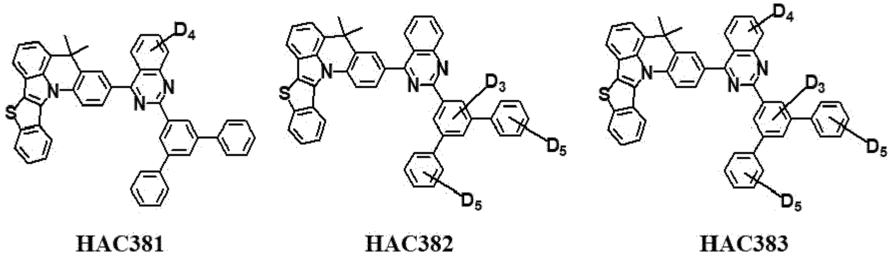

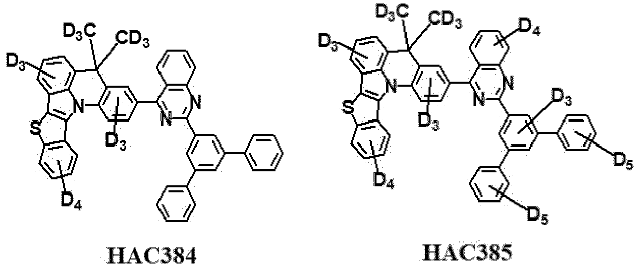

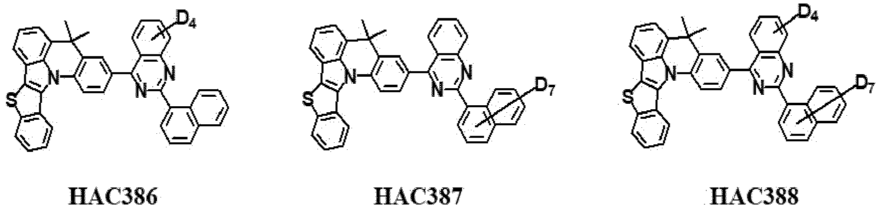

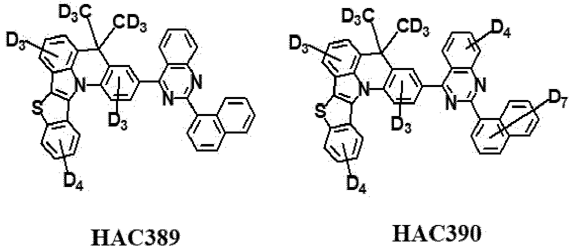

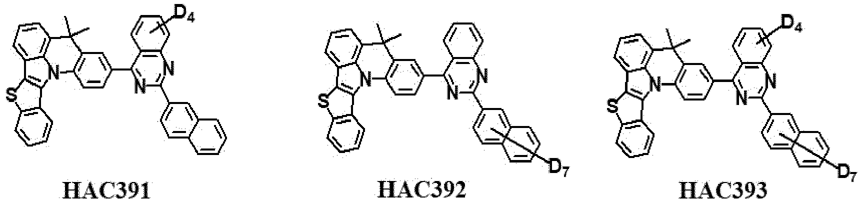

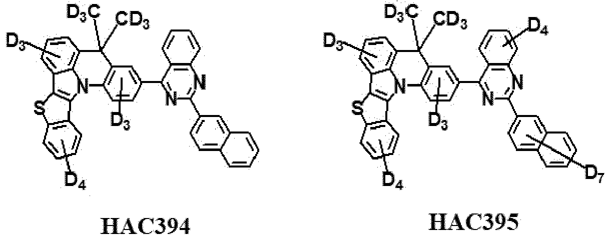

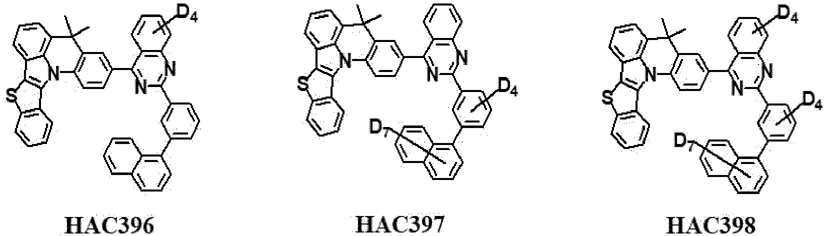

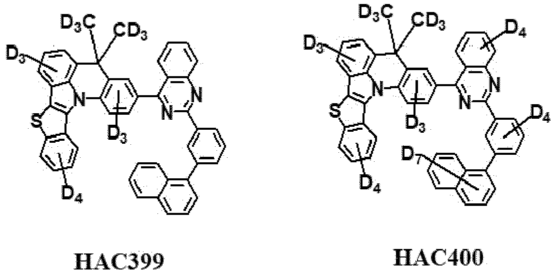

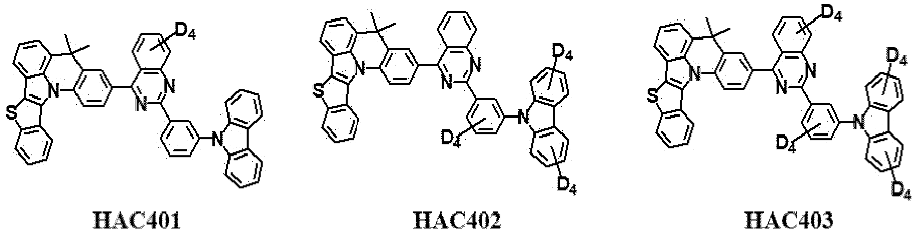

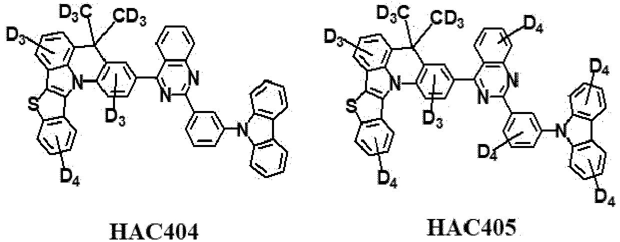

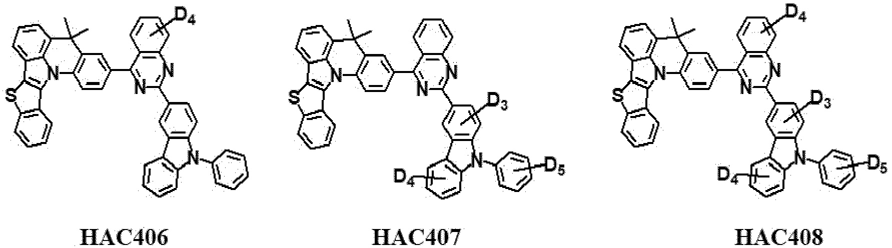

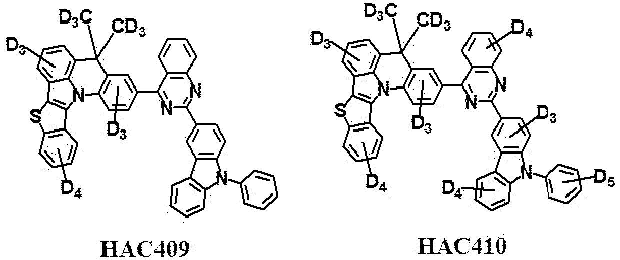

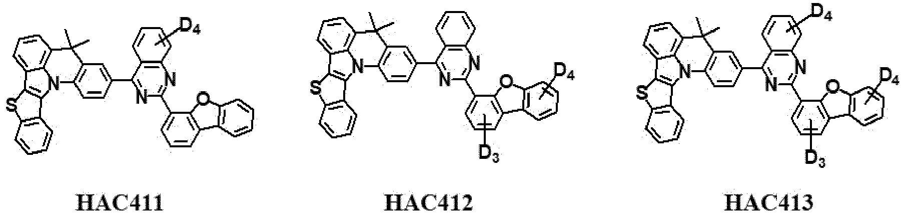

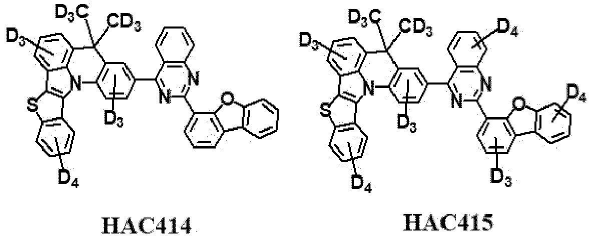

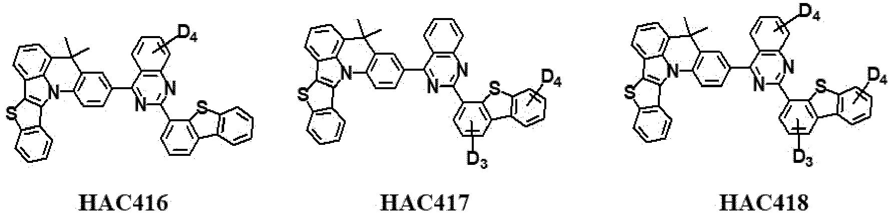

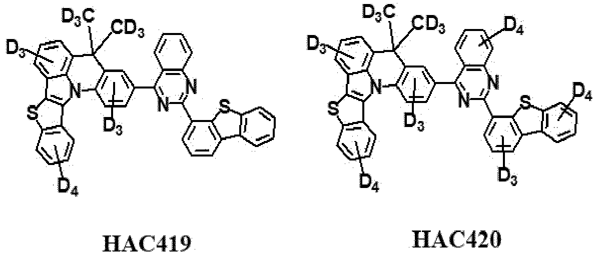

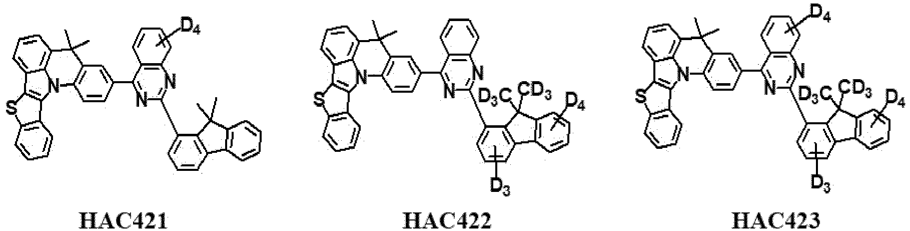

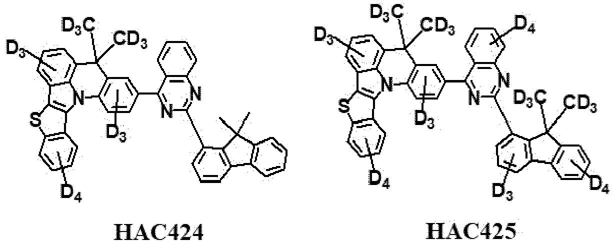

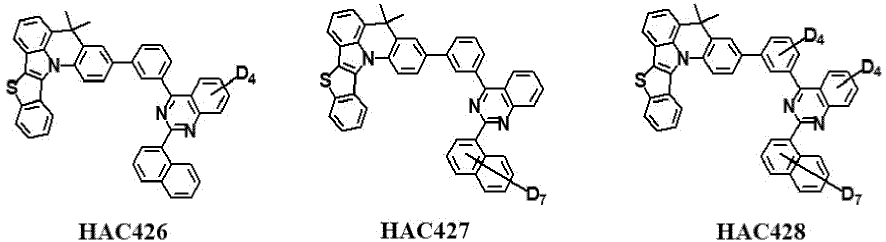

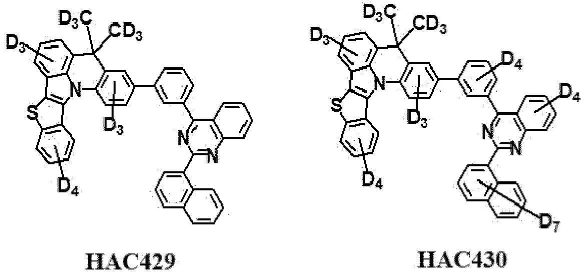

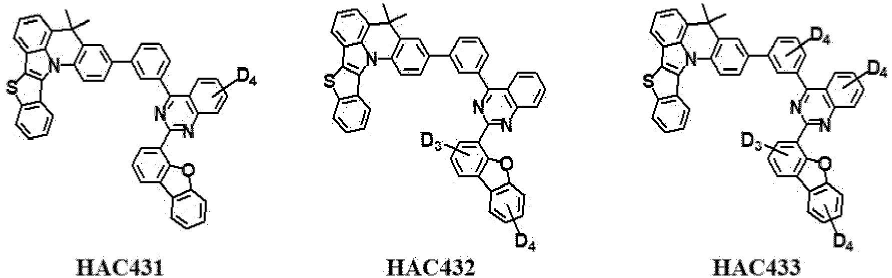

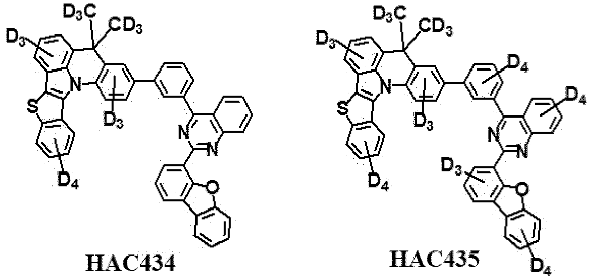

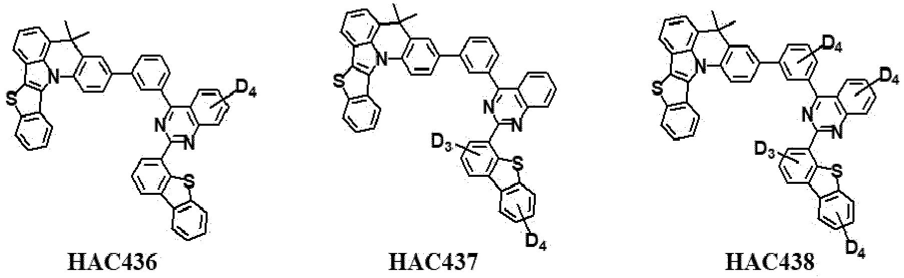

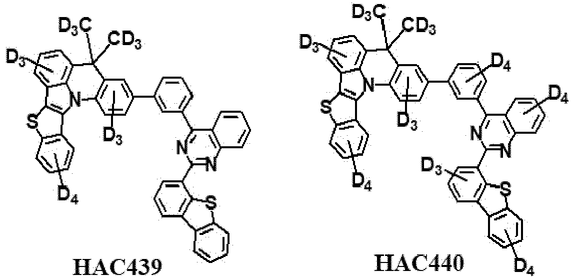

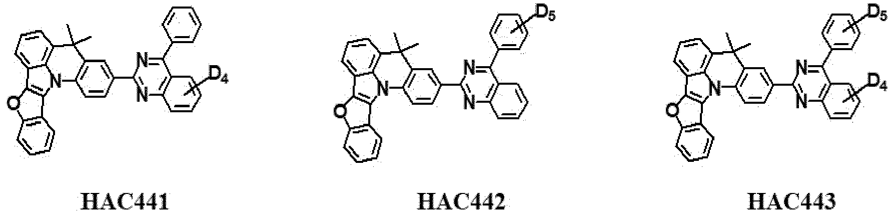

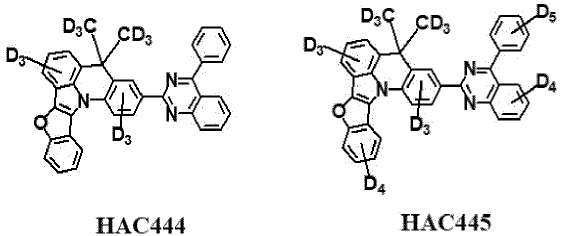

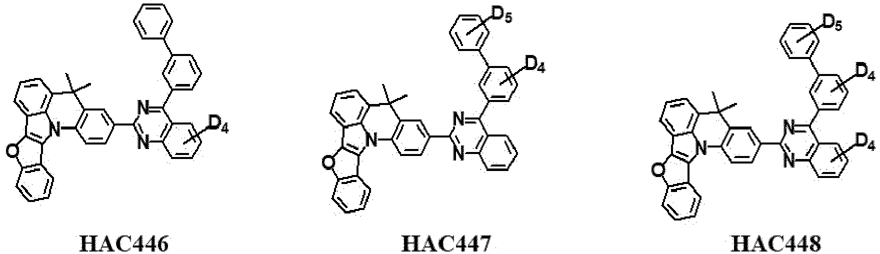

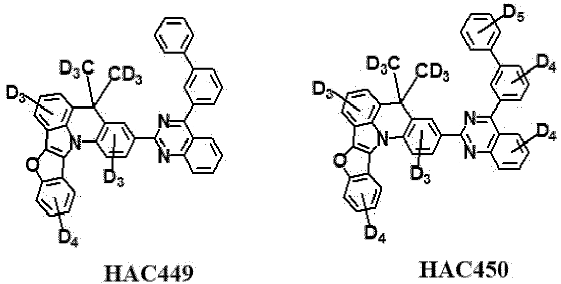

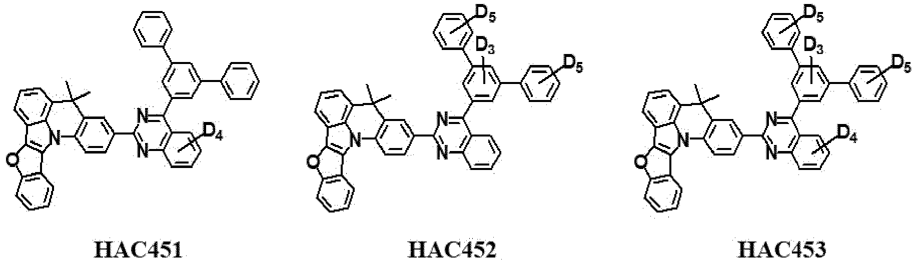

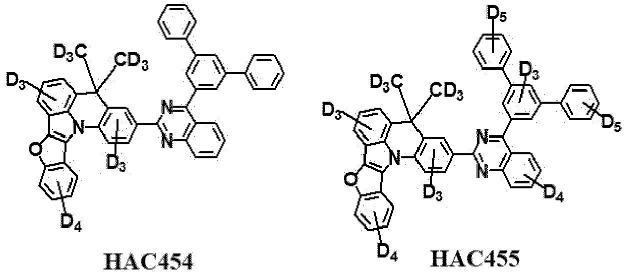

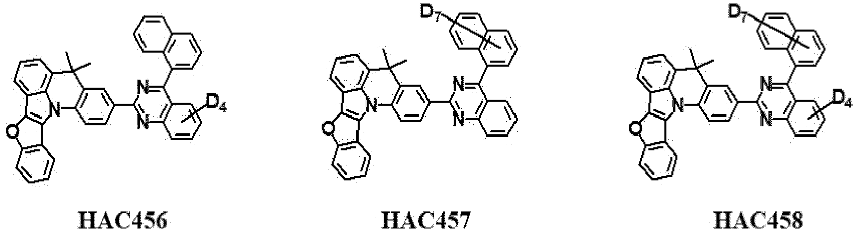

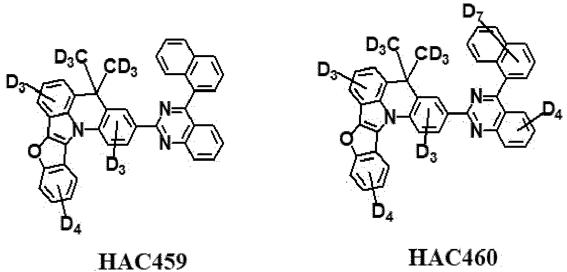

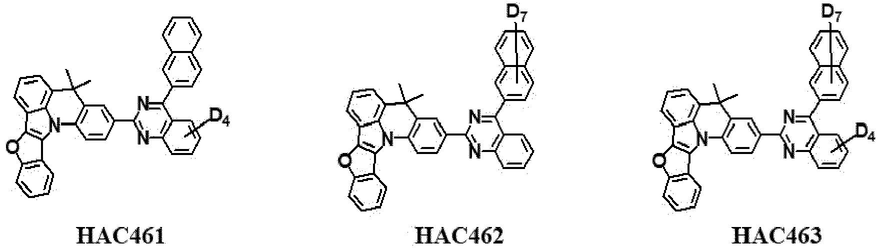

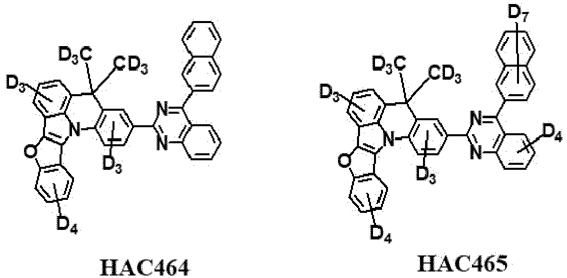

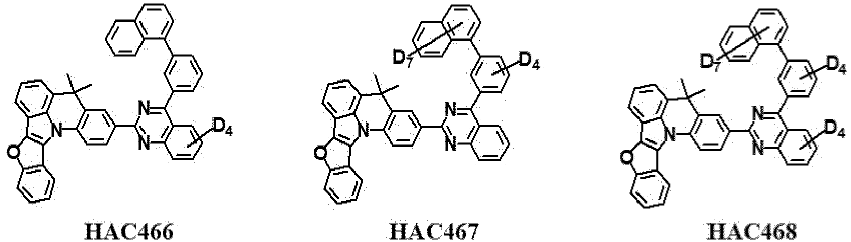

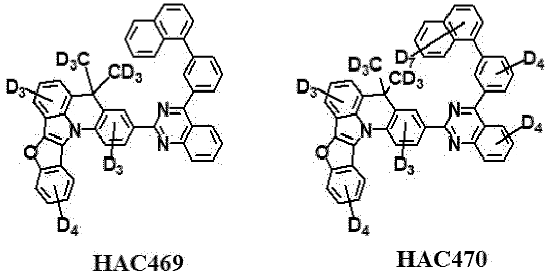

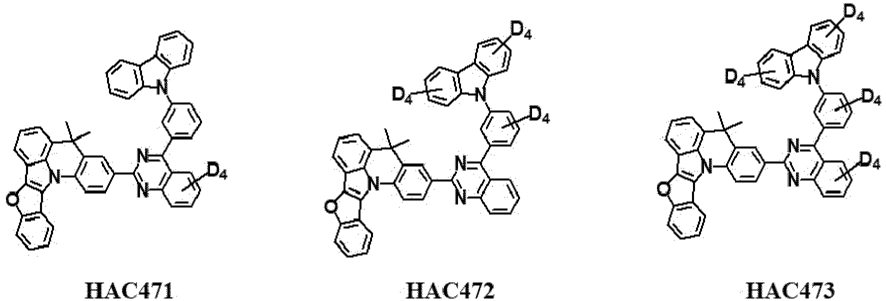

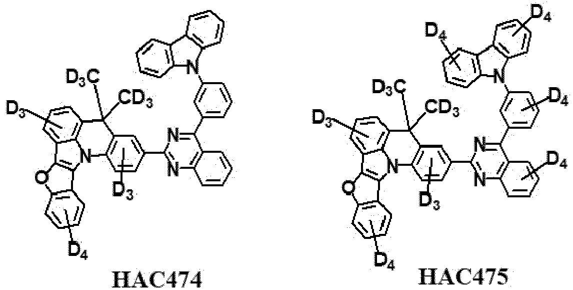

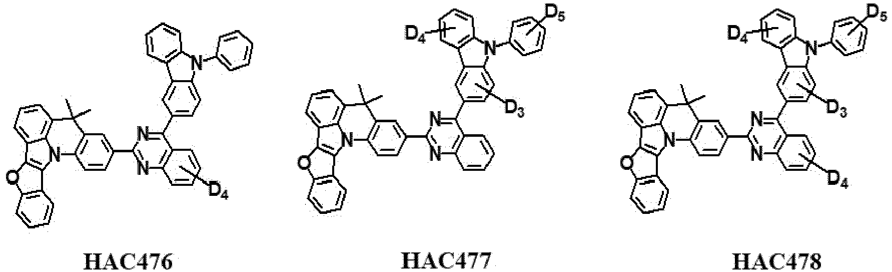

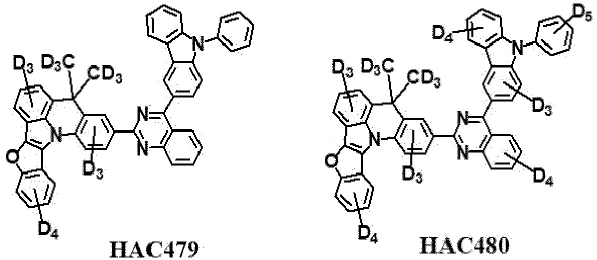

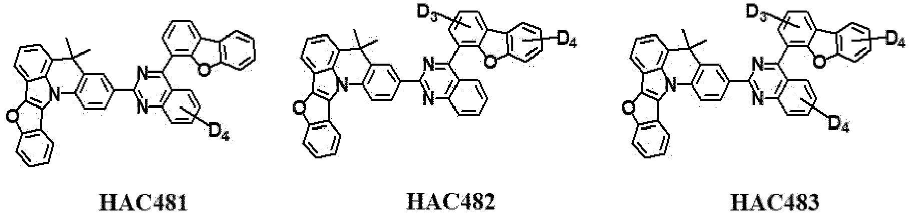

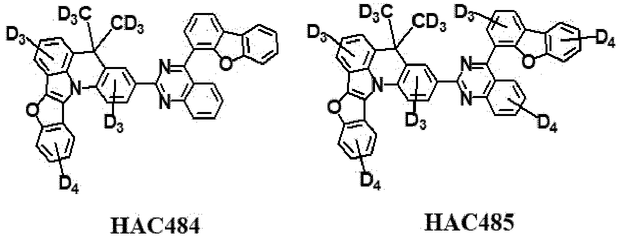

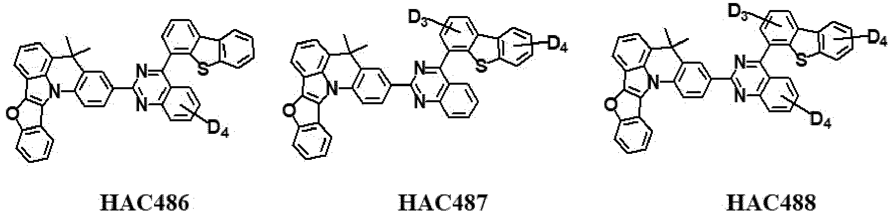

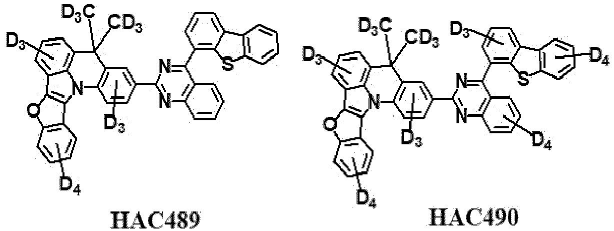

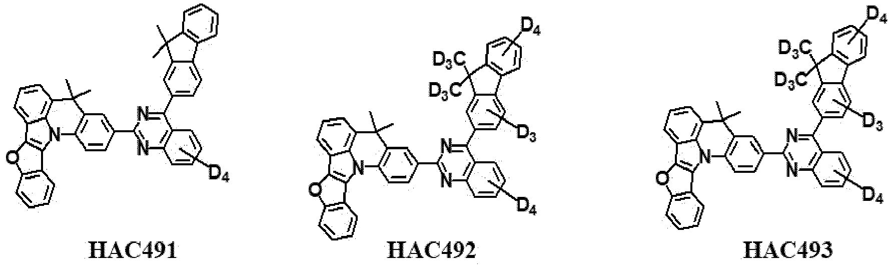

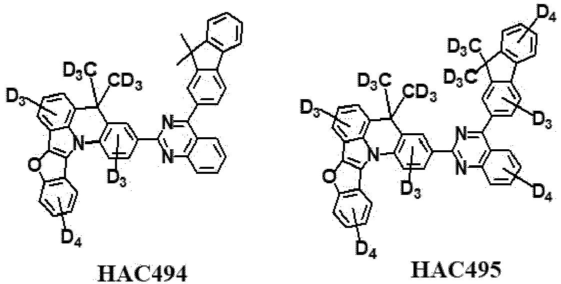

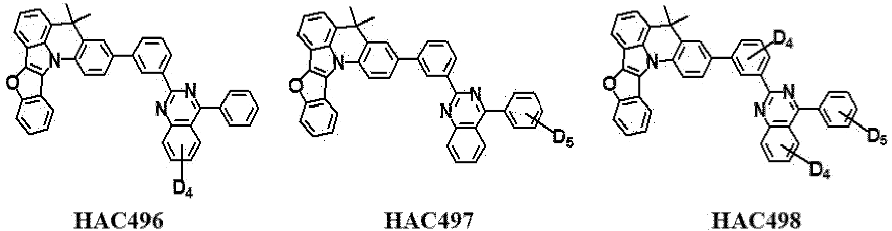

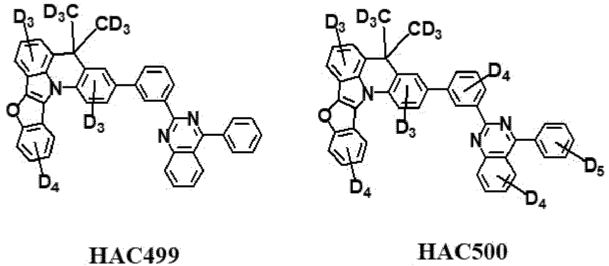

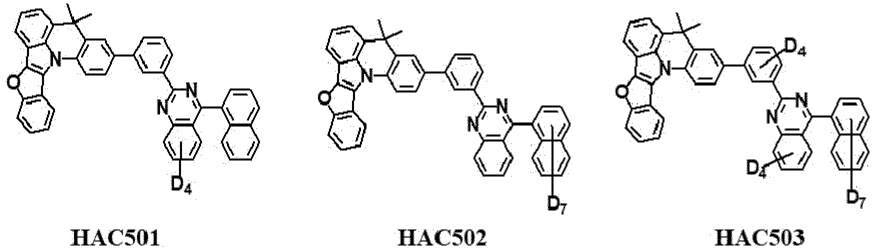

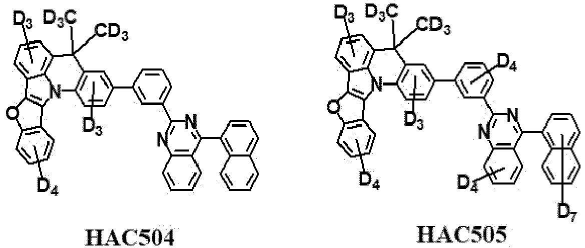

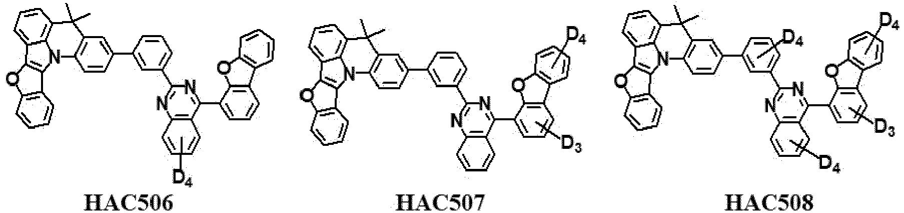

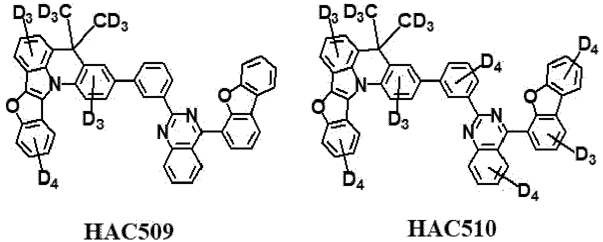

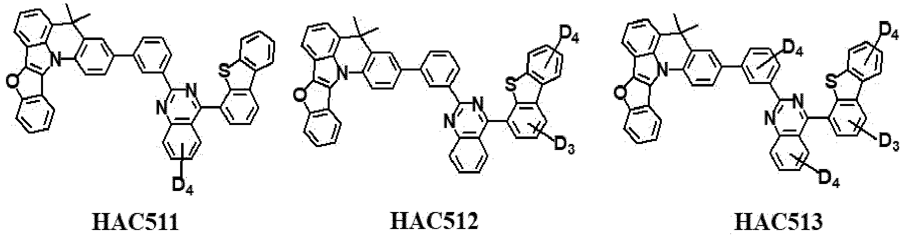

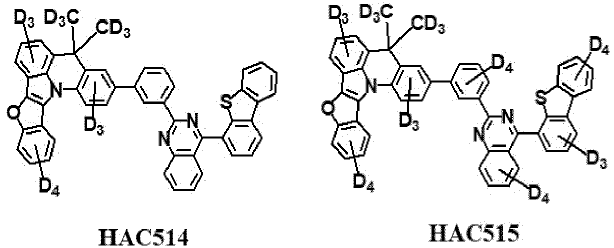

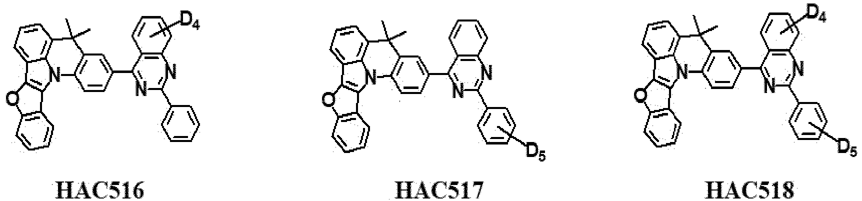

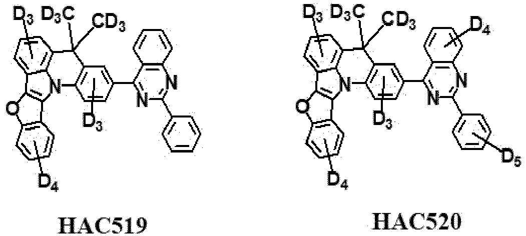

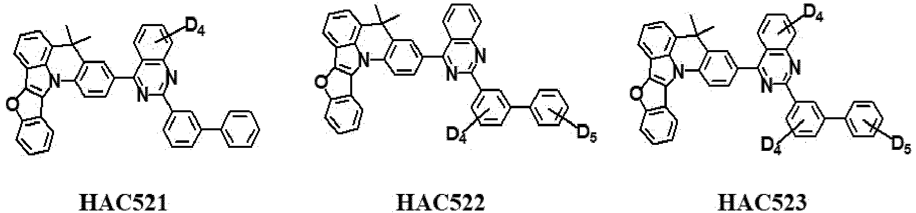

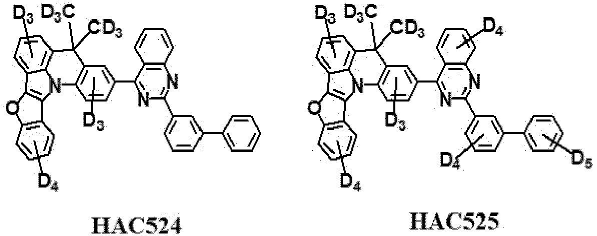

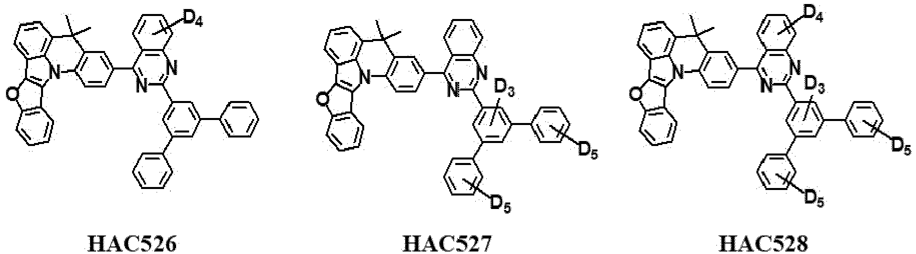

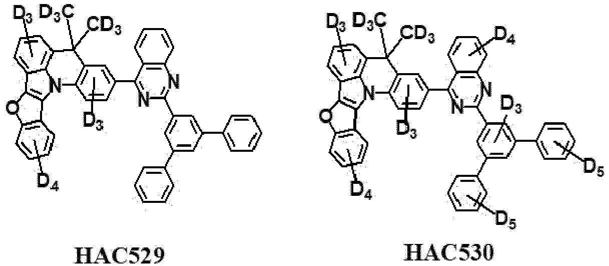

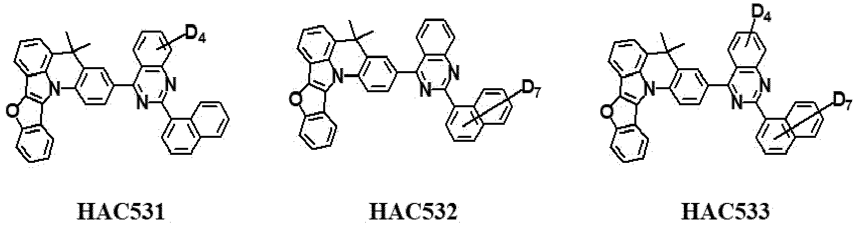

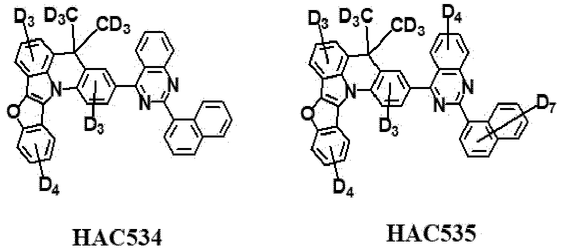

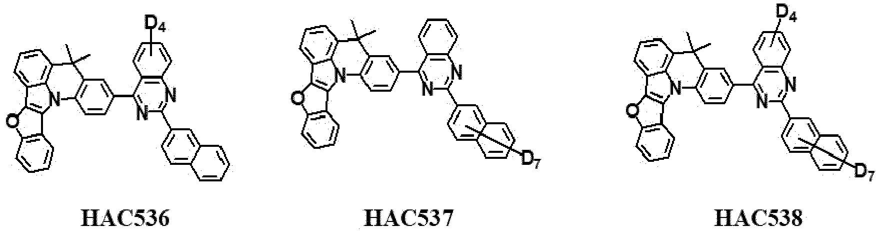

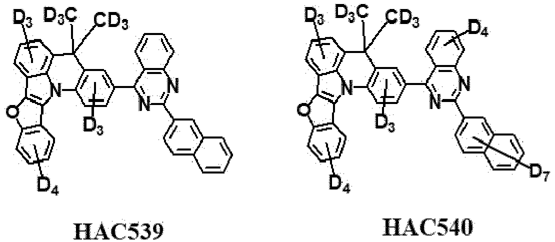

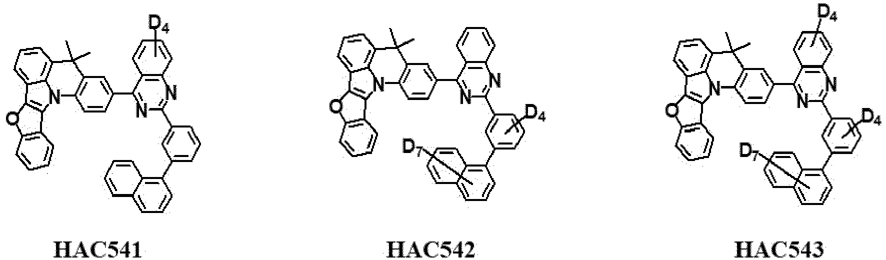

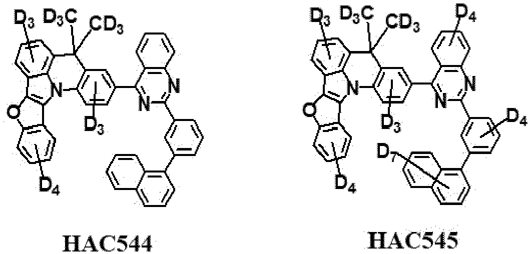

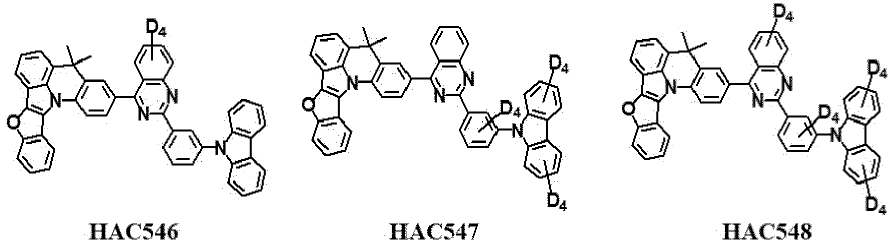

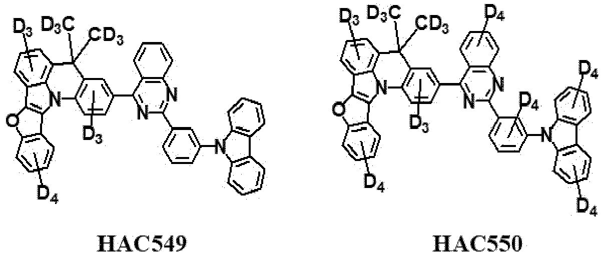

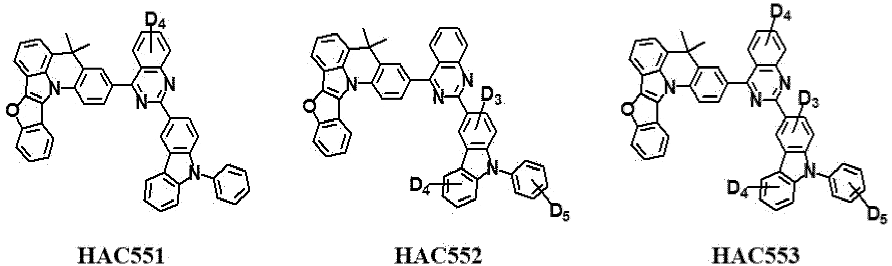

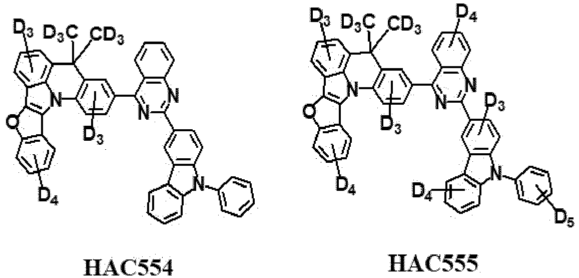

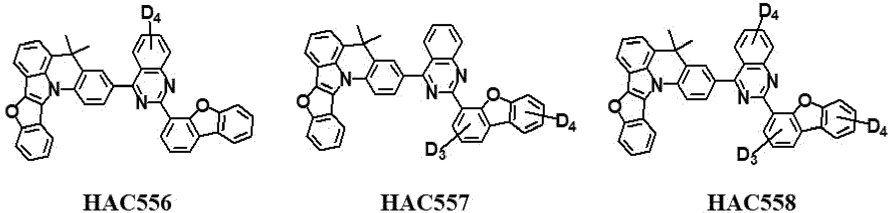

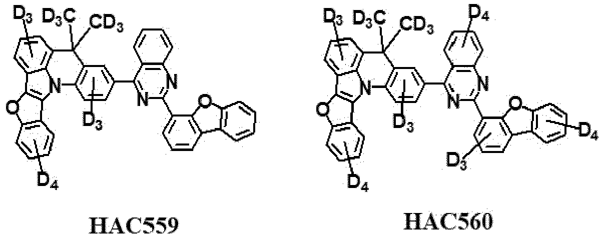

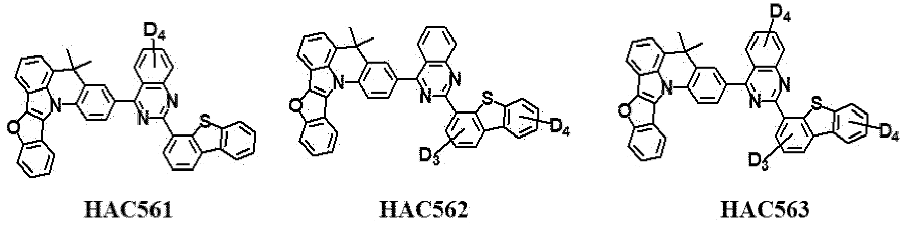

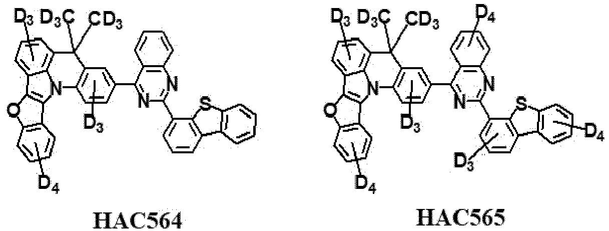

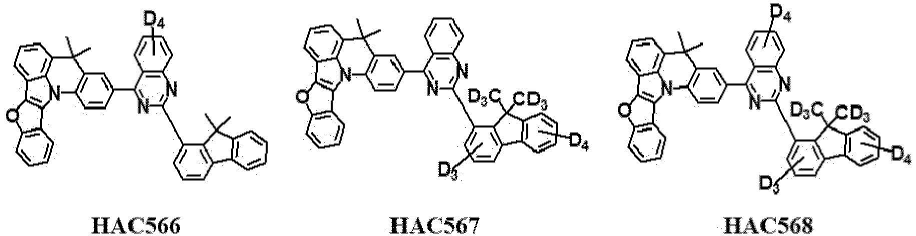

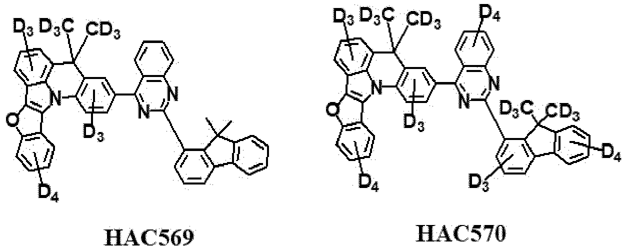

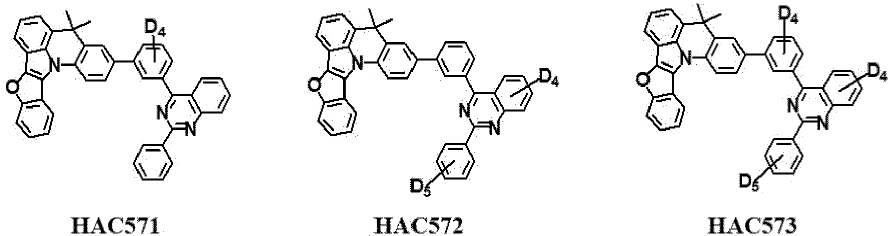

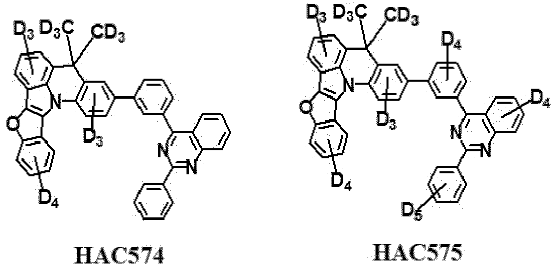

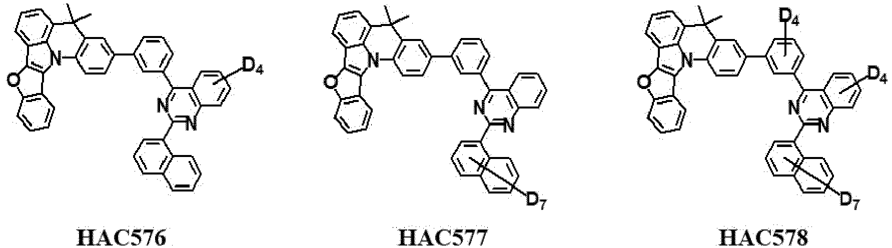

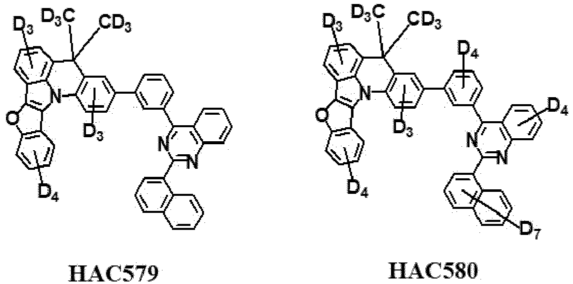

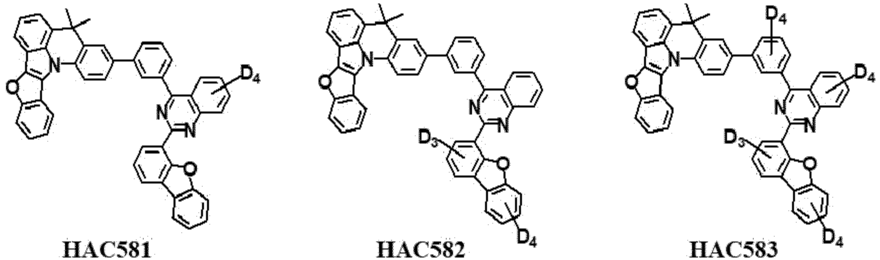

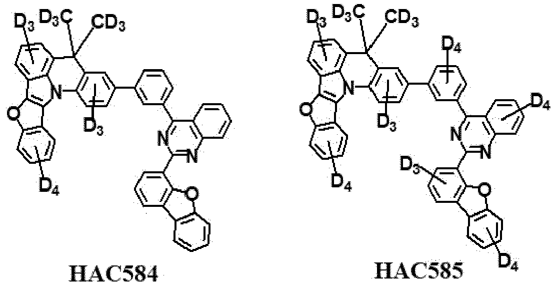

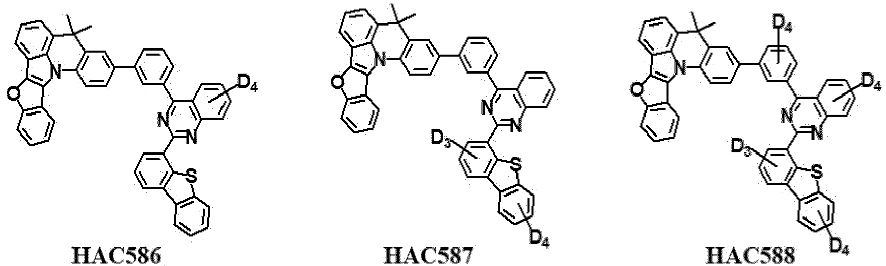

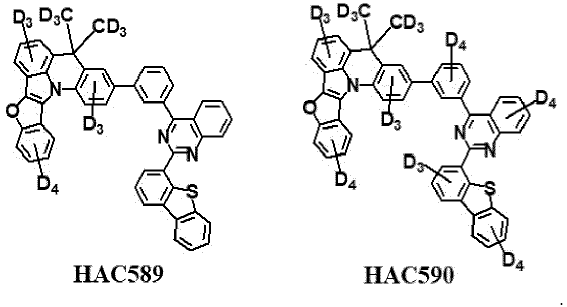

### [OLED and Organic Light Emitting Device]

The organic compound having any one of the structures of Formulae 1 to 5 may have higher triplet energy level, wide energy bandgap, and beneficial thermal resistance. The driving voltage of the OLED may be reduced. The luminous efficiency as well as the luminous lifespan of the OLED may be improved by adding the organic compound having any one of the structures of Formulae 1 to 5 into an emissive layer in the OLED. The OLED may be used in an organic light emitting device such as an organic light emitting display device or an organic light emitting illumination device.

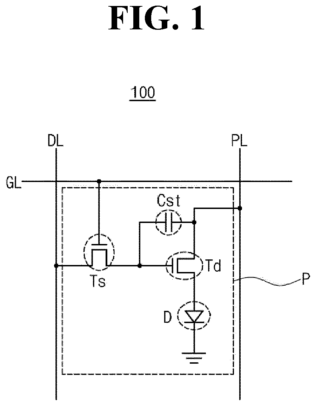

FIG. 1 illustrates a schematic circuit diagram of an organic light emitting display device in accordance with the present disclosure. As illustrated in FIG. 1, a gate line GL, a data line DL and power line PL, each of which crosses each other to define a pixel region P, in the organic light emitting display device 100. A switching thin film transistor Ts, a driving thin film transistor Td, a storage capacitor Cst and an organic light emitting diode D are disposed within the pixel region P. The pixel region P may include a red (R) pixel region, a green (G) pixel region and a blue (B) pixel region. However, embodiments of the present disclosure are not limited to such examples.

The switching thin film transistor Ts is connected to the gate line GL and the data line DL. The driving thin film transistor Td and the storage capacitor Cst are connected between the switching thin film transistor Ts and the power line PL. The organic light emitting diode D is connected to the driving thin film transistor Td. When the switching thin film transistor Ts is turned on by a gate signal applied to the gate line GL, a data signal applied to the data line DL is applied to a gate electrode of the driving thin film transistor Td and one electrode of the storage capacitor Cst through the switching thin film transistor Ts.

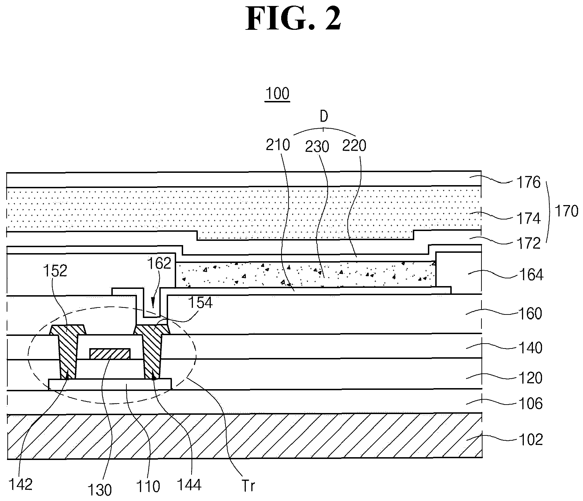

The driving thin film transistor Td is turned on by the data signal applied to the gate electrode 130 (FIG. 2) so that a current proportional to the data signal is supplied from the power line PL to the organic light emitting diode D through the driving thin film transistor Td. And then, the organic light emitting diode D emits light having a luminance proportional to the current flowing through the driving thin film transistor Td. In this case, the storage capacitor Cst is charged with a voltage proportional to the data signal so that the voltage of the gate electrode in the driving thin film transistor Td is kept constant during one frame. Therefore, the organic light emitting display device can display a desired image.

FIG. 2 illustrates a schematic cross-sectional view of an organic light emitting display device in accordance with an example embodiment of the present disclosure. As illustrated in FIG. 2, the organic light emitting display device 100 includes a substrate 102, a thin-film transistor Tr on the substrate 102, and an organic light emitting diode D connected to the thin film transistor Tr. As an example, the substrate 102 may include a red pixel region, a green pixel region and a blue pixel region and an organic light emitting diode D in each pixel region. Each of the organic light emitting diode D emits red, green or blue light, respectively, and is located correspondingly in the red pixel region, the green pixel region and the blue pixel region.

The substrate 102 may include, but is not limited to, glass, thin flexible material and/or polymer plastics. For example, the flexible material may be selected from the group of, but is not limited to, polyimide (PI), polyethersulfone (PES), polyethylenenaphthalate (PEN), polyethylene terephthalate (PET), polycarbonate (PC) and/or combinations thereof. The substrate 102, on which the thin film transistor Tr and the organic light emitting diode D are arranged, forms an array substrate.

A buffer layer 106 may be disposed on the substrate 102. The thin film transistor Tr may be disposed on the buffer layer 106. The buffer layer 106 may be omitted. A semiconductor layer 110 is disposed on the buffer layer 106. In one example embodiment, the semiconductor layer 110 may include, but is not limited to, oxide semiconductor materials. In this case, a light-shield pattern may be disposed under the semiconductor layer 110, and the light-shield pattern may prevent or reduce light from being incident toward the semiconductor layer 110, and thereby, preventing or reducing the semiconductor layer 110 from being degraded by the light. Alternatively, the semiconductor layer 110 may include polycrystalline silicon. In this case, opposite edges of the semiconductor layer 110 may be doped with impurities.

A gate insulating layer 120 including an insulating material is disposed on the semiconductor layer 110. The gate insulating layer 120 may include, but is not limited to, an inorganic insulating material such as silicon oxide (SiOₓ, wherein 0 < x ≤ 2) or silicon nitride (SiNₓ, wherein 0 < x ≤ 2).

A gate electrode 130 made of a conductive material, such as a metal, is disposed on the gate insulating layer 120 so as to correspond to a center of the semiconductor layer 110. While the gate insulating layer 120 is disposed on a whole area of the substrate 102 as shown in FIG. 2, the gate insulating layer 120 may be patterned identically as the gate electrode 130.

An interlayer insulating layer 140 including an insulating material is disposed on the gate electrode 130 with and covers an entire surface of the substrate 102. The interlayer insulating layer 140 may include, but is not limited to, an inorganic insulating material such as silicon oxide (SiOₓ) or silicon nitride (SiNₓ), or an organic insulating material such as benzocyclobutene or photo-acryl.

The interlayer insulating layer 140 has first and second semiconductor layer contact holes 142 and 144 that expose the semiconductor layer 110 or do not cover a portion of the surface the semiconductor layer 110 nearer to the opposing ends than to a center of the semiconductor layer 110. The first and second semiconductor layer contact holes 142 and 144 are disposed on opposite sides of the gate electrode 130 and spaced apart from the gate electrode 130. The first and second semiconductor layer contact holes 142 and 144 are formed within the gate insulating layer 120 in FIG. 2. Alternatively, the first and second semiconductor layer contact holes 142 and 144 may be formed only within the interlayer insulating layer 140 when the gate insulating layer 120 is patterned identically as the gate electrode 130.

A source electrode 152 and a drain electrode 154, which are made of conductive material such as a metal, are disposed on the interlayer insulating layer 140. The source electrode 152 and the drain electrode 154 are spaced apart from each other on opposing sides of the gate electrode 130. The source electrode 152 and the drain electrode 154 contact both sides of the semiconductor layer 110 through the first and second semiconductor layer contact holes 142 and 144, respectively.

The semiconductor layer 110, the gate electrode 130, the source electrode 152 and the drain electrode 154 constitute the thin film transistor Tr, which acts as a driving element. The thin film transistor Tr in FIG. 2 has a coplanar structure in which the gate electrode 130, the source electrode 152 and the drain electrode 154 are disposed on the semiconductor layer 110. Alternatively, the thin film transistor Tr may have an inverted staggered structure in which a gate electrode is disposed under a semiconductor layer and a source and drain electrodes are disposed on the semiconductor layer. In this case, the semiconductor layer may include amorphous silicon.

The gate line GL and the data line DL, which cross each other to define a pixel region P, and a switching element Ts, which is connected to the gate line GL and the data line DL, may be further formed in the pixel region P. The switching element Ts is connected to the thin film transistor Tr, which is a driving element. In addition, the power line PL is spaced apart in parallel from the gate line GL or the data line DL. The thin film transistor Tr may further include a storage capacitor Cst configured to constantly keep a voltage of the gate electrode 130 for one frame.

A passivation layer 160 is disposed on the source and drain electrodes 152 and 154. The passivation layer 160 covers the thin film transistor Tr on the whole substrate 102. The passivation layer 160 has a flat top surface and a drain contact hole 162 that exposes the drain electrode 154 or does not cover the drain electrode 154 of the thin film transistor Tr. While the drain contact hole 162 is disposed on the second semiconductor layer contact hole 144, it may be spaced apart from the second semiconductor layer contact hole 144.

The organic light emitting diode (OLED) D includes a first electrode 210 that is disposed on the passivation layer 160 and connected to the drain electrode 154 of the thin film transistor Tr. The OLED D further includes an emissive layer 230 and a second electrode 220 each of which is disposed sequentially on the first electrode 210.

The first electrode 210 is disposed in each pixel region. The first electrode 210 may be an anode and include conductive material having relatively high work function value. For example, the first electrode 210 may include, but is not limited to, a transparent conductive oxide (TCO).

In one example embodiment, when the organic light emitting display device 100 is a bottom-emission type, the first electrode 210 may have a single-layered structure of the TCO. Alternatively, when the organic light emitting display device 100 is a top-emission type, a reflective electrode or a reflective layer may be disposed under the first electrode 210. For example, the reflective electrode or the reflective layer may include, but is not limited to, silver (Ag) or aluminum-palladium-copper (APC) alloy. In the OLED D of the top-emission type, the first electrode 210 may have a triple-layered structure of ITO/Ag/ITO or ITO/APC/ITO.

In addition, a bank layer 164 is disposed on the passivation layer 160 in order to cover edges of the first electrode 210. The bank layer 164 exposes the first electrode 210 or does not cover a center of the first electrode 210 corresponding to each pixel region. The bank layer 164 may be omitted.

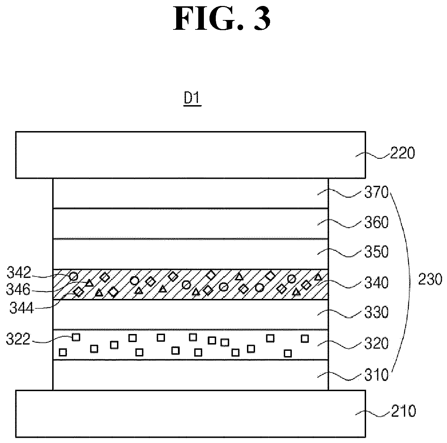

An emissive layer 230 is disposed on the first electrode 210. In one example embodiment, the emissive layer 230 may have a single-layered structure of an emitting material layer (EML). Alternatively, the emissive layer 230 may have a multiple-layered structure including a hole injection layer (HIL), a hole transport layer (HTL), an electron blocking layer (EBL), an EML, a hole blocking layer (HBL), an electron transport layer (ETL) and/or an electron injection layer (EIL) (see, FIGS. 3, 5 and 6). In one aspect, the emissive layer 230 may have a single emitting part. Alternatively, the emissive layer 230 may have multiple emitting parts to form a tandem structure.

The emissive layer 230 may include at least one host and a dopant so that the OLED D and the organic light emitting display device may lower their driving voltages and may enhance their luminous efficiency and luminous lifespan.

The second electrode 220 is disposed on the substrate 102 above which the emissive layer 230 is disposed. The second electrode 220 may be disposed on a whole display area. The second electrode 220 may include a conductive material with a relatively low work function value compared to the first electrode 210. The second electrode 220 may be a cathode. When the organic light emitting display device 100 is a top-emission type, the second electrode 220 is thin so as to have light-transmissive (semi-transmissive) property.

In addition, an encapsulation film 170 may be disposed on the second electrode 220 in order to prevent or reduce outer moisture from penetrating into the organic light emitting diode D. The encapsulation film 170 may have, but is not limited to, a laminated structure of a first inorganic insulating film 172, an organic insulating film 174 and a second inorganic insulating film 176. The encapsulation film 170 may be omitted.

A polarizing plate may be attached onto the encapsulation film to reduce reflection of external light. For example, the polarizing plate may be a circular polarizing plate. When the organic light emitting display device 100 is a bottom-emission type, the polarizer may be disposed under the substrate 102. Alternatively, when the organic light emitting display device 100 is a top-emission type, the polarizer may be disposed on the encapsulation film 170. In addition, a cover window may be attached to the encapsulation film 170 or the polarizer. In this case, the substrate 102 and the cover window may have a flexible property, thus the organic light emitting display device 100 may be a flexible display device.

Next, the OLED D is described in more detail. FIG. 3 illustrates a schematic cross-sectional view of an organic light emitting diode having a single emitting part in accordance with an example embodiment of the present disclosure. As illustrated in FIG. 3, the organic light emitting diode (OLED) D1 in accordance with the present disclosure includes first and second electrodes 210 and 220 facing each other and an emissive layer 230 disposed between the first and second electrodes 210 and 220. The organic light emitting display device 100 includes a red pixel region, a green pixel region and a blue pixel region, and the OLED D1 may be disposed in each of the pixel regions.

In an example embodiment, the emissive layer 230 includes an EML 340 disposed between the first and second electrodes 210 and 220. Also, the emissive layer 230 may include at least one of an HTL 320 disposed between the first electrode 210 and the EML 340 and an ETL 360 disposed between the second electrode 220 and the EML 340. In addition, the emissive layer 230 may further include at least one of an HIL 310 disposed between the first electrode 210 and the HTL 320 and an EIL 370 disposed between the second electrode 220 and the ETL 360. Alternatively, the emissive layer 230 may further comprise a first exciton blocking layer, *i.e.* an EBL 330 disposed between the HTL 320 and the EML 340 and/or a second exciton blocking layer, *i.e.* a HBL 350 disposed between the EML 340 and the ETL 360.

The first electrode 210 may be an anode that provides a hole into the EML 340. The first electrode 210 may include a conductive material having a relatively high work function value, for example, a transparent conductive oxide (TCO). In one example embodiment, the first electrode 210 may include, but is not limited to, indium tin oxide (ITO), indium zinc oxide (IZO), indium tin zinc oxide (ITZO), tin oxide (SnO), zinc oxide (ZnO), indium cerium oxide (ICO), aluminum doped zinc oxide (AZO), and/or the like.

The second electrode 220 may be a cathode that provides an electron into the EML 340. The second electrode 220 may include a conductive material having a relatively low work function values. As an example, the second electrode 220 may include, but is not limited to, aluminum (Al), magnesium (Mg), calcium (Ca), silver (Ag), alloy thereof and/or combination thereof such as aluminum-magnesium alloy (Al-Mg). In an example embodiment, each of the first electrode 210 and the second electrode 220 may independently have a thickness of, but is not limited to, about 500 to about 2000 Å.

The EML 340 includes a first host 342 and a dopant 346 in which substantial light emission occurs, and optionally, a second host 344. The first host 342 may include the organic compound having any one of the structures of Formulae 1 to 5. As an example, the first host 342 may be an n-type host having beneficial electron affinity property.

Optionally, the EML 340 may further include the second host 344. The second host 344 may be any p-type host having a relatively strong hole affinity property. For example, the second host 344 may include, but is not limited to, an aromatic or heteroaromatic amine-based organic compound, a carbazole-based organic compound and/or a spiro-bifluorene-based organic compound.

In one example embodiment, the second host 344 may include an aromatic or heteroaromatic amine-based organic compound selected from, but is not limited to, N,N'-bis(1-naphthyl)-1,1'-biphenyl-4,4"-diamine; NPB; NPD), Tris(4-carbazoyl-9-yl-phenyl)amine (TCTA), 1,3,5-tris[4-(diphenylamino)phenyl]benzene (TDAPB), N-(biphenyl-4-yl)-9,9-dimethyl-N-(4-(9-phenyl-9H-carbazol-3-yl)phenyl)-9H-fluoren-2-amine, N,N'-diphenyl-N,N'-di[4-(N,N-diphenyl-amino)phenyl]benzidine (NPNPB) and/or combination thereof.

In another example embodiment, the second host 344 may include, but is not limited to, 1,3-Bis(carbazol-9-yl)benzene (mCP), 3,3-Di(9H-carbazol-9-yl)biphenyl (mCBP), 9-(3-(9H-carbazol-9-yl)phenyl)-9H-carbazole-3-carbonitrile (mCP-CN), CBP-CN, 9-(3-(9H-Carbazol-9-yl)phenyl)-3-(diphenylphosphoryl)-9H-carbazole (mCPPO1) 3,5-Di(9H-carbazol-9-yl)biphenyl (Ph-mCP), 9-(9-phenyl-9H-carbazol-6-yl)-9H-carbazole (CCP), 9-(4-(9H-carbazol-9-yl)phenyl)-9H-3,9'-bicarbazole, 9-(3-(9H-carbazol-9-yl)phenyl)-9H-3,9'-bicarbazole, 9-(6-(9H-carbazol-9-yl)pyridin-3-yl)-9H-3,9'-bicarbazole, 9,9'-Diphenyl-9H,9'H-3,3'-bicarbazole (BCzPh), 1,3,5-Tris(carbazole-9-yl)benzene (TCP), 4,4'-Bis(carbazole-9-yl)-2,2'-dimethylbipheyl (CDBP), 2,7-Bis(carbazole-9-yl)-9,9-dimethylfluorene (DMFL-CBP), 2,2',7,7'-Tetrakis(carbazole-9-yl)-9,9-spirofluorene (Spiro-CBP), 3,6-Bis(carbazole-9-yl)-9-(2-ethyl-hexyl)-9H-carbazole (TCz1) and/or combination thereof.

In yet another example embodiment, the second host 344 may include a spiro-bifluorene-based organic compound having a structure represented by Formula 6: 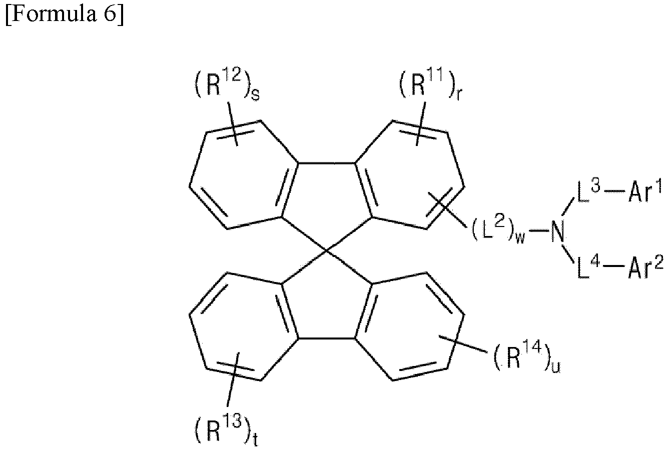
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring, wherein each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4;
each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring;
r is 3 and each of s, t, and u is 4 when each of R¹¹ to R¹⁴ is a protium, and r is 0, 1, 2 or 3, and each of s, t, and u is independently 0, 1, 2, 3 or 4 when each of R¹¹ to R¹⁴ is not a protium; and
w is 0, 1, 2 or 3.

For example, each of R¹¹ to R¹⁴ in Formula 6 may be independently selected from a protium, a deuterium, and an unsubstituted or substituted C₁-C₂₀ alkyl, and each of Ar₁ and Ar₂ in Formula 6 may independently be an unsubstituted or substituted C₆-C₃₀ aryl. As an example, each of Ar₁ and Ar₂ may independently include, but is not limited to, phenyl and fluorenyl, each of which may be independently unsubstituted or substituted with at least one of an unsubstituted or substituted C₁-C₁₀ alkyl, an unsubstituted or substituted C₆-C₂₀ aryl *(e.g.,* phenyl and/or naphthyl) and an unsubstituted or substituted C₃-C₂₀ heteroaryl.

In one example embodiment, at least one of Ar₁ and Ar² in Formula 6 may include, but is not limited to, a polycyclic aromatic group and/or a polycyclic heteroaromatic group. For example, the spiro-bifluorene-based organic compound as the second host 344 may have a structure represented by Formula 7 or Formula 8: 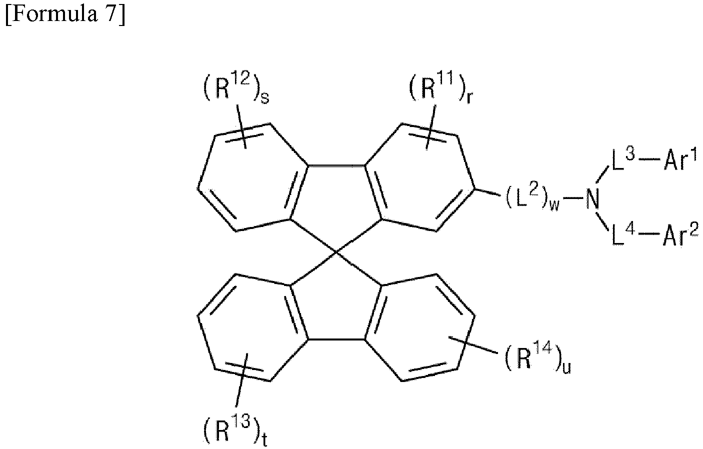 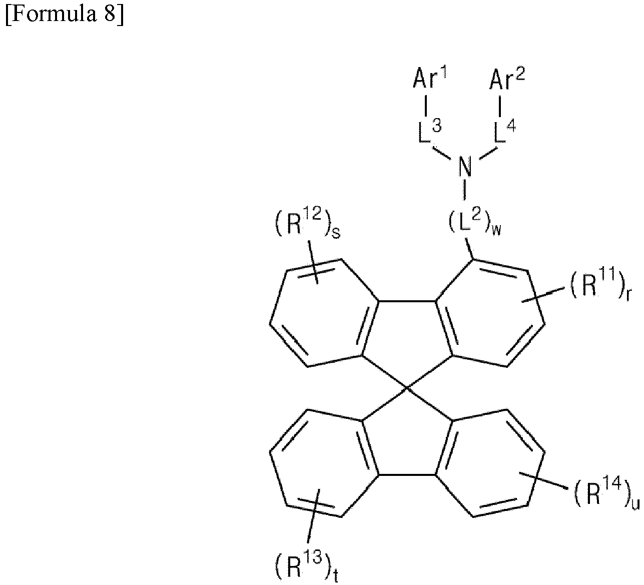
wherein in the Formulae 7 and 8,
each of R¹¹ to R¹⁴, Ar¹, Ar², L² to L⁴, r, s, t, u, and w is as defined in the Formula 6.

More particularly, the spiro-bifluorene-based organic compound as the second host 344 may include at least one, or may be selected from, but is not limited to, the following spiro-bifluorene-based organic compounds of Formula 9:

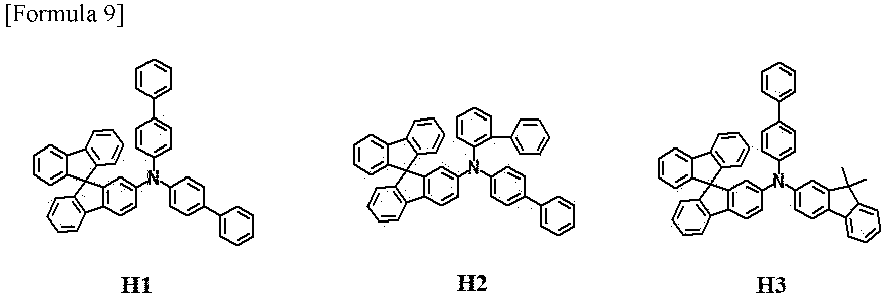

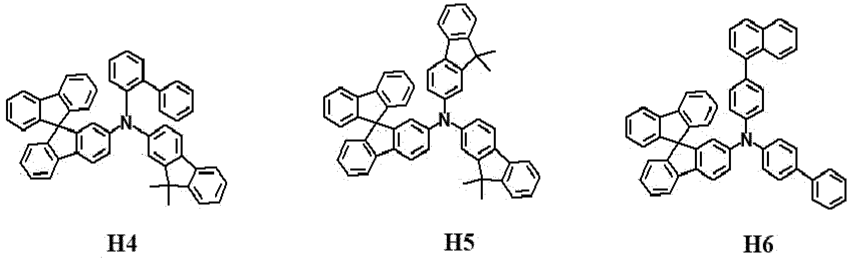

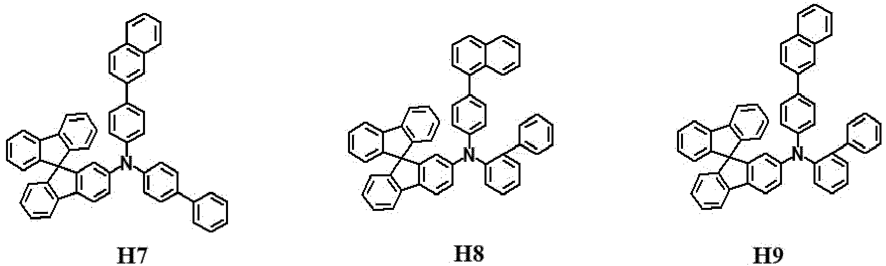

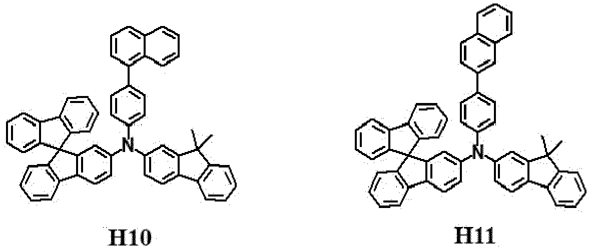

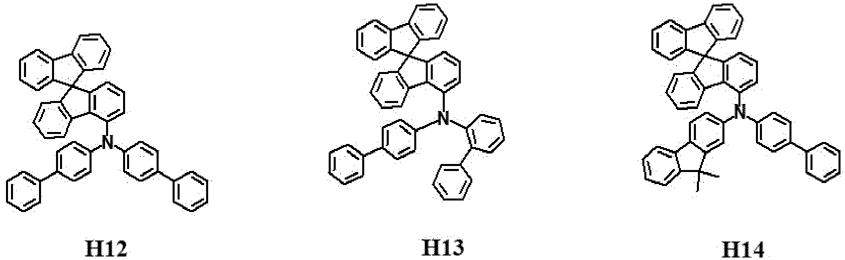

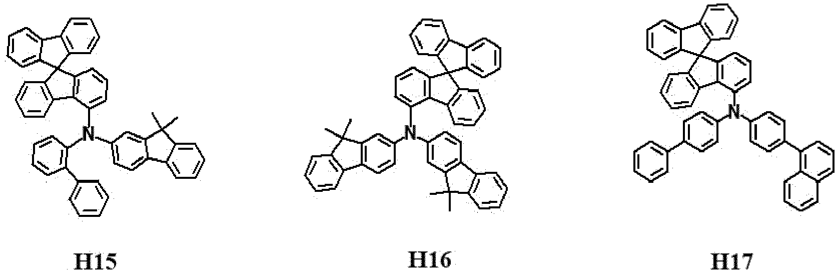

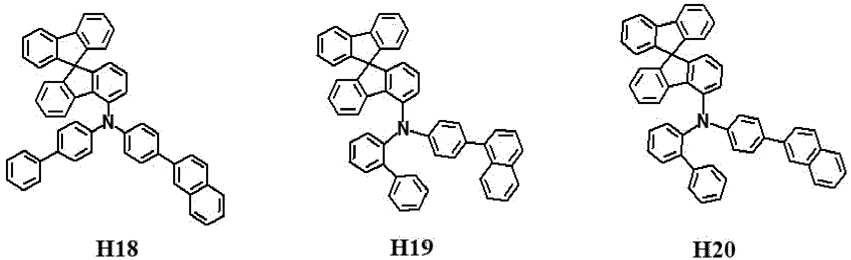

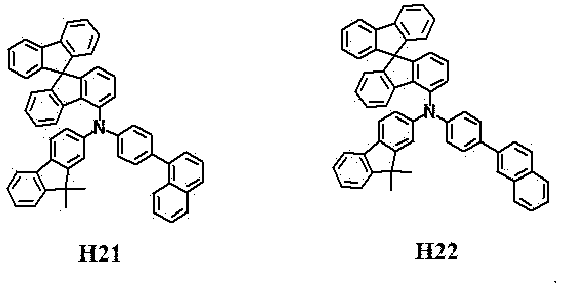

The spiro-bifluorene-based organic compound having any one of the structures of Formulae 6 to 9 may have beneficial hole transportation property and good electron blocking property owing to high LUMO energy level. Accordingly, when the spiro-bifluorene-based organic compound having any one of the structures of Formulae 6 to 9 is used as the second host 344, the EBL 330 of the emissive layer 230 may be omitted.

In addition, the EML 340 may include the dopant 346. For example, the dopant 346 may include a blue dopant, a green dopant and/or a red dopant.

The blue dopant may include at least one of blue phosphorescent material, blue fluorescent material, and blue delayed fluorescent material. In one example embodiment, the blue phosphorescent and fluorescent materials may include, but is not limited to, perylene, 4,4'-Bis[4-(di-p-tolylamino)styryl]biphenyl (DPAVBi), 4-(Di-p-tolylamino)-4-4'-[(di-p-tolylamino)styryl]stilbene (DPAVB), 4,4'-Bis[4-(diphenylamino)styryl]biphenyl (BDAVBi), 2,7-Bis(4-diphenylamino)styryl)-9,9-spirofluorene (spiro-DPVBi), [1,4-bis[2-[4-[N,N-di(p-tolyl)amino]phenyl]vinyl] benzene (DSB), 1-4-di-[4-(N,N-diphenyl)amino]styryl-benzene (DSA), 2,5,8,11-Tetra-tert-butylperylene (TBPe), Bis(2-hydroxylphenyl)-pyridine)beryllium (Bepp₂), 9-(9-Phenylcarbazole-3-yl)-10-(naphthalene-1-yl)anthracene (PCAN), mer-Tris(1-phenyl-3-methylimidazolin-2-ylidene-C,C(2)'iridium(III) (mer-Ir(pmi)₃), fac-Tris(1,3-diphenyl-benzimidazolin-2-ylidene-C,C(2)'iridium(III) (fac-Ir(dpbic)₃), Bis(3,4,5-trifluoro-2-(2-pyridyl)phenyl-(2-carboxypyridyl)iridium(III) (Ir(tfpd)₂pic), tris(2-(4,6-difluorophenyl)pyridine))iridium(III) (Ir(Fppy)₃), Bis[2-(4,6-difluorophenyl)pyridinato-C²,N](picolinato)iridium(III) (FIrpic) and/or combination thereof.

In another example embodiment, the blue delayed fluorescent material may include, but is not limited to, 10-(4-(diphenylphosphoryl)phenyl)-10H-phenoxazine (SPXZPO), 4,4'-(phenylphosphoryl)bis(4,1-phenylene))bis(10H-phenoxazine (DPXZPO), 10,10',10"-(4,4',4"-phosphoryltris(benzene-4,1-diyl))tris(10H-phenoxazine) (TPXZPO), 9,9'-(5-(4,6-diphenyl-1,3,5-triazin-2-yl)-1,3-phenylene)bis(9H-carbazole) (DcZTrz), 9,9',9",9‴-((6-phenyl-1,3,5-triazin-2,4-diyl)bis(benzene-5,3,1-triyl))tetrakis(9H-carbazole) (DDczTrz), 2,7-bis(9,9-dimethylacridin-10(9H)-yl)-9,9-dimethyl-9H-thioxanthene-10,10-dioxide (DMTDAc), 9,9'-(4,4' -sulfonylbis(4,1-phenylene))bis(3,6-dimethoxyl-9H-carbazole) (DMOC-DPS), 10,10'-(4,4'-Sulfonylbis(4,1-phenylene))bis(9,9-dimethyl-9,10-dihydroacridine (DMAC-DPS), 4,6-di(9H-carbazol-9-yl)isophthalonitrile (DczIPN), 2,3,4,6-tetra(9H-carbazol-9-yl)-5-fluorobenzonitrile (4CzFCN) and/or combination thereof.

The green dopant may include at least one of green phosphorescent material, green fluorescent material, and green delayed fluorescent material. In one example embodiment, the green phosphorescent material may include, but is not limited to, Tris[2-phenylpyridine]iridium (Ir(ppy)₃), Tris[2-tolylpyridine]iridium (Ir(tpy)₃), Tris[2-(1-cyclohexenyl)pyridine]iridium (Ir(chpy)₃), Tris[2-(3-methyl-1-cyclohexenyl)pyridine]iridium (Ir(mchpy)₃) and/or combination thereof.

In another example embodiment, the green delayed fluorescent material may include, but is not limited to, 2-biphenyl-4,6-bis(12-phenylindolo[2,3-a]carbazol-11-yl)-1,3,5-triazine (PIC-TRZ), 2,4,5,6-tetra(9H-carbazol-9-yl)isophthalonitrile (4CzIPN), 3,4,5,6-tetra(9H-carbazol-9-yl)isophthalonitrile (4CzPN), 2,3,4,6-tetra(9H-carbazol-9-yl)-5-fluorobenzonitrile (4CzFCN), 4,5-bis(5H-benzofuro[3,2-c]carbazol-5-yl)phthalonitrile (BFCz-2CN), 4,5-bis(5H-benzo[4,5]thieno[3,2-c]carbazol-5-yl)phthalonitrile (BTCz-2CN), 9,9',9"-(5-(4,6-diphenyl-1,3,5-triazin-2-yl)benzene-1,2,3-triyl)tris(9H-carbazole)(TcZTrz) and/or combination thereof.

The red dopant may include at least one of red phosphorescent material, red fluorescent material, and red delayed fluorescent material. In one example embodiment, the red phosphorescent material may include, but is not limited to, [Bis(2-(4,6-dimethyl)phenylquinoline)](2,2,6,6-tetramethylheptane-3,5-dionate)iridium(III), Bis[2-(4-n-hexylphenyl)quinoline](acetylacetonate)iridium(III) (Hex-Ir(phq)₂(acac)), Tris[2-(4-n-hexylphenyl)quinoline]iridium(III) (Hex-Ir(phq)₃), Tris[2-phenyl-4-methylquinoline]iridium(III) (Ir(Mphq)₃), Bis(2-phenylquinoline)(2,2,6,6-tetramethylheptene-3,5-dionate)iridium(III) (Ir(dpm)PQ₂), Bis(phenylisoquinoline)(2,2,6,6-tetramethylheptene-3,5-dionate)iridium(III) (Ir(dpm)(piq)₂), Bis[(4-n-hexylphenyl)isoquinoline](acetylacetonate)iridium(III) (Hex-Ir(piq)₂(acac)), Tris[2-(4-n-hexylphenyl)quinoline]iridium(III) (Hex-Ir(piq)₃), Tris(2-(3-methylphenyl)-7-methyl-quinolato)iridium (Ir(dmpq)₃), Bis[2-(2-methylphenyl)-7-methylquinoline](acetylacetonate)iridium(III) (Ir(dmpq)₂(acac)), Bis[2-(2-methylphenyl)-7-methylquinoline](acetylacetonate)iridium(III) (Ir(drmpq)₂(acac), Bis[2-(3,5-dimethylphenyl)-4-methylquinoline](acetylacetonate)iridium(III) (Ir(mphmq)₂(acac)), Tris(dibenzoylmethane)mono(1,10-phenanthroline)europium(III) (Eu(dbm)₃(phen)) and/or combination thereof.

In another example embodiment, the red delayed fluorescent material may include, but is not limited to, 1,3-bis[4-(10H-phenoxazin-10-yl)benzoyl]benzene (mPx2BBP), 2,3,5,6-tetrakis(3,6-diphenylcarbazol-9-yl)-1,4-dicyanobenzene (4CzTPN-Ph), 10,10'-(sulfonylbis(4,1-phenylene))bis(5-phenyl-5,10-dihydrophenazine) (PPZ-DPS), 5,10-bis(4-(benzo[d]thiazol-2-yl)phenyl)-5,10-dihydrophenazine (DHPZ-2BTZ), 5,10-bis(4-(4,6-diphenyl-1,3,5-triazin-2-yl)phenyl)-5,10-dihydrophenazine (DHPZ-2TRZ), 7,10-bis(4-(diphenylamino)phenyl)-2,3-dicyanopyrazino phenanathrene (TPA-DCPP) and/or combination thereof.

The content of the host including the first host 342 and the second host 344 in the EML 340 may be, but is not limited to, about 50 to about 90 wt%, for example, about 80 to about 95 wt%, based on a total weight of the components in the EML 340. The content of the dopant 346 in the EML 340 may be, but is not limited to, about 1 to 10 wt%, for example, about 5 to 20 wt%, based on a total weight of the components in the EML 340. When the EML 340 includes the first and second hosts 342 and 344, the first host 342 and the second host 344 may be mixed, but is not limited to, with a weight ratio between about 4:1 and about 1:4, for example, about 3:1 and about 1:3. As an example, the EML 340 may have a thickness of, but is not limited to, about 300 to about 500 Å.

The HIL 310 is disposed between the first electrode 210 and the HTL 320 and may improve an interface property between the inorganic first electrode 210 and the organic HTL 320. In one example embodiment, the HIL 310 may include, but is not limited to, 4,4',4"-Tris(3-methylphenylamino)triphenylamine (MTDATA), 4,4',4"-Tris(N,N-diphenyl-amino)triphenylamine (NATA), 4,4',4"-Tris(N-(naphthalene-1-yl)-N-phenyl-amino)triphenylamine (1T-NATA), 4,4',4"-Tris(N-(naphthalene-2-yl)-N-phenyl-amino)triphenylamine (2T-NATA), Copper phthalocyanine (CuPc), TCTA, NPB, 1,4,5,8,9,11-Hexaazatriphenylenehexacarbonitrile (Dipyrazino[2,3-f:2'3'-h]quinoxaline-2,3,6,7,10,11-hexacarbonitrile; HAT-CN), 1,3,5-tris[4-(diphenylamino)phenyl]benzene (TDAPB), poly(3,4-ethylenedioxythiphene)polystyrene sulfonate (PEDOT/PSS), 2,3,5,6-tetrafluoro-7,7,8,8-tetracyanoquinodimethane (F4TCNQ), N-(biphenyl-4-yl)-9,9-dimethyl-N-(4-(9-phenyl-9H-carbazol-3-yl)phenyl)-9H-fluoren-2-amine, N,N'-diphenyl-N,N'-di[4-(N,N'-diphenyl-amino)phenyl]benzidine (NPNPB) and/or combination thereof.

Alternatively, the HIL 310 may include a hole injection host and a hole injection dopant. In this case, the content of the hole injection dopant in the HIL 310 may be, but is not limited to, about 1 to about 25 wt%, based on a total weight of the components in the HIL 310. The hole injection host may include, but is not limited to, the hole injection material and hole transportation material as described below, for example, a tertiary amine-based material. The hole injection material may include, but is not limited to, a material having a radialene structure.

As an example, the HIL 310 may have a thickness of, but is not limited to, about 100 to about 300 Å. The HIL 310 may be omitted in compliance of the OLED D1 property.

The HTL 320 is disposed adjacent to the EML 340 between the first electrode 210 and the EML 340, and includes a hole transporting material 322.

In one example embodiment, the hole transporting material 322 may include, but is not limited to, N,N'-Diphenyl-N,N'-bis(3-methylphenyl)-1,1'-biphenyl-4,4'-diamine (TPD), NPB(NPD), 4,4'-bis(N-carbazolyl)-1,1'-biphenyl (CBP), Poly[N,N'-bis(4-butylpnehyl)-N,N'-bis(phenyl)-benzidine] (Poly-TPD), Poly [(9,9-dioctylfluorenyl-2,7-diyl)-co-(4,4'-(N-(4-sec-butylphenyl)diphenylamine))] (TFB), Di-[4-(N,N-di-p-tolyl-amino)-phenyl]cyclohexane (TAPC), 3,5-Di(9H-carbazol-9-yl)-N,N-diphenylaniline (DCDPA), N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine, N-([1,1'-Biphenyl]-4-yl)-9,9-dimethyl-N-(4-(9-phenl-9H-carbazol-3-yl)phenyl)-9H-fluoren-2-amine and/or combinations thereof.

In another example embodiment, the hole transporting material 322 may include the spiro-bifluorene-based organic compound having any one of the structures of Formulae 6 to 9. The spiro-bifluorene-based organic compound may have beneficial hole transportation property as well as good electron blocking affinity. When the hole transporting material 322 having any one of the structures of Formulae 6 to 9 is added to the HTL 320, holes may be injected into the EML 340 rapidly and the EBL 330 may be omitted.

The ETL 360 and the EIL 370 may be laminated sequentially between the EML 340 and the second electrode 220. The ETL 360 includes a material having high electron mobility so as to provide electrons stably with the EML 340 by fast electron transportation.

In one example embodiment, the ETL 360 may include, but is not limited to, at least one of oxadiazole-based compounds, triazole-based compounds, phenanthroline-based compounds, benzoxazole-based compounds, benzothiazole-based compounds, benzimidazole-based compounds, triazine-based compounds, and/or the like.

As an example, the ETL 360 may include, but is not limited to, tris-(8-hydroxyquinolinato) aluminum (Alq₃), Bis(2-methyl-8-quinolinolato-N1,O8)-(1,1'-biphenyl-4-olato)aluminum (BAlq), lithium quinolate (Liq), 2-biphenyl-4-yl-5-(4-t-butylphenyl)-1,3,4-oxadiazole (PBD), spiro-PBD, 1,3,5-Tris(N-phenylbenzimidazol-2-yl)benzene (TPBi), 4,7-diphenyl-1,10-phenanthroline (Bphen), 2,9-Bis(naphthalene-2-yl)4,7-diphenyl-1,10-phenanthroline (NBphen), 2,9-Dimethyl-4,7-diphenyl-1,10-phenanthroline (BCP), 3-(4-Biphenyl)-4-phenyl-5-tert-butylphenyl-1,2,4-triazole (TAZ), 4-(Naphthalen-1-yl)-3,5-diphenyl-4H-1,2,4-triazole (NTAZ), 1,3,5-Tri(p-pyrid-3-yl-phenyl)benzene (TpPyPB), 2,4,6-Tris(3'-(pyridin-3-yl)biphenyl-3-yl)1,3,5-triazine (TmPPPyTz), Poly[9,9-bis(3'-(N,N-dimethyl)-N-ethylammonium)-propyl)-2,7-fluorene]-alt-2,7-(9,9-dioctylfluorene)] (PFNBr), tris(phenylquinoxaline) (TPQ), diphenyl-4-triphenysilyl-phenylphosphine oxide (TSPO1), 2-[4-(9,10-di-2-naphthalen-2-yl-2-anthracen-2-yl)phenyl] 1-phenyl-1H-benzimidazole (ZADN) and/or combination thereof.

The EIL 370 is disposed between the second electrode 220 and the ETL 360, and may improve physical properties of the second electrode 220 and therefore, may enhance the lifetime of the OLED D1. In one example embodiment, the EIL 370 may include, but is not limited to, an alkali metal halide or an alkaline earth metal halide such as LiF, CsF, NaF, BaF₂ and/or the like, and/or an organic salt compound such as Liq, lithium benzoate, sodium stearate, and/or the like. Alternatively, the EIL 370 may be omitted. Each of the ETL 360 and the EIL 370 may independently have a thickness, but is not limited to, about 10 to about 100 Å. Alternatively, the EIL 370 may be omitted.

In an alternative aspect, the electron transporting material and the electron injection material may be admixed to form a single ETL-EIL. The electron transporting material and the electron injection material may be admixed with, but is not limited to, about 4:1 to about 1:4 by weight, for example, about 2:1 to about 1:2 by weight.

When holes are transferred to the second electrode 220 via the EML 340 and/or electrons are transferred to the first electrode 210 via the EML 340, the OLED D1 may have short lifetime and reduced luminous efficiency. In order to prevent or reduce these phenomena, the OLED D1 in accordance with this aspect of the present disclosure may have at least one exciton blocking layer adjacent to the EML 340.

For example, the OLED D1 may include the EBL 330 between the HTL 320 and the EML 340 so as to control and prevent or reduce electron transfers. In one example embodiment, the EBL 330 may include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2-amine, TAPC, MTDATA, mCP, mCBP, CuPc, N,N'-bis[4-[bis(3-methylphenyl)amino]phenyl]-N,N'-diphenyl-[1,1'-biphenyl]-4,4'-diamine (DNTPD), TDAPB, DCDPA, 2,8-bis(9-phenyl-9H-carbazol-3-yl)dibenzo[b,d]thiophene and/or combination thereof.

In addition, the OLED D1 may further include the HBL 350 as a second exciton blocking layer between the EML 340 and the ETL 360 so that holes cannot be transferred from the EML 340 to the ETL 360. In one example embodiment, the HBL 350 may include, but is not limited to, at least one of oxadiazole-based compounds, triazole-based compounds, phenanthroline-based compounds, benzoxazole-based compounds, benzothiazole-based compounds, benzimidazole-based compounds, and triazine-based compounds each of which may be used in the ETL 360.

For example, the HBL 350 may include a compound having a relatively low HOMO energy level compared to the luminescent materials in EML 340. The HBL 350 may include, but is not limited to, Alq₃, BAlq, Liq, PBD, spiro-PBD, BCP, Bis-4,5-(3,5-di-3-pyridylphenyl)-2-methylpyrimidine (B3PYMPM), bis[2-(diphenylphosphino)phenyl]ether oxide (DPEPO), 9-(6-(9H-carbazol-9-yl)pyridine-3-yl)-9H-3,9'-bicarbazole, TSPO1 and/or combination thereof. Alternatively, the EBL 330 and/or the HBL 350 may be omitted.

The organic compound having any one of the structures of Formulae 1 to 5 may have high triple energy level, wide energy band gap, beneficial thermal stability, and good electron affinity property. When the organic compound having any one of the structures of Formulae 1 to 5 is applied as the first host 342 in the EML 340, electrons may be injected into the dopant 346 from the first host 342.

Optionally, when the second host 344 having beneficial hole affinity property is used together with the first host and/or the hole transporting material 322 is introduced into the HTL 320, holes and electrons may be injected into the EML 340 in balance. Holes and electrons may be provided into the dopant 346 of the EML 340 and light may be emitted uniformly in whole area of the EML 340. It may be possible to implement the OLED D1 that reduces its driving voltage and maximizes its luminous efficiency as well as luminous lifespan.

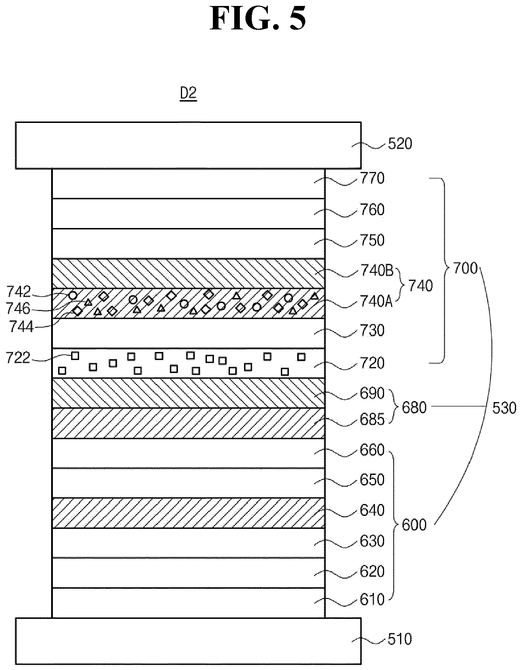

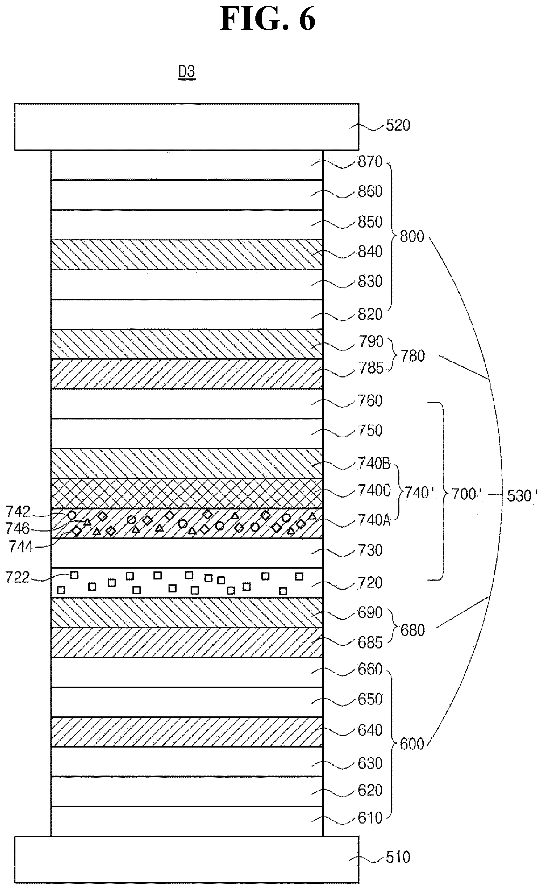

In the above example first aspect, the OLED and the organic light emitting display device include a single emitting part. Alternatively, the OLED may include multiple emitting parts (see, *e.g.,* FIGS. 5 and 6) among which at least one includes the first host 342 and the dopant 346, and optionally, the second host 344.

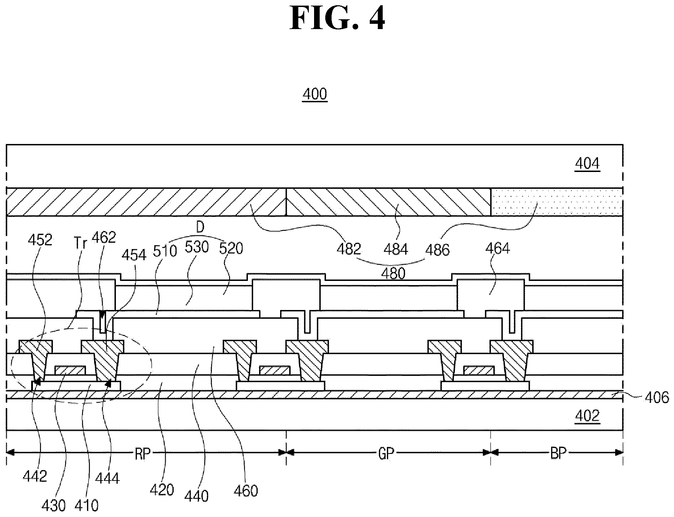

In another example embodiment, an organic light emitting display device can implement full-color including white color. FIG. 4 illustrates a schematic cross-sectional view of an organic light emitting display device in accordance with another example embodiment of the present disclosure.

As illustrated in FIG. 4, the organic light emitting display device 400 comprises a first substrate 402 that defines each of a red pixel region RP, a green pixel region GP and a blue pixel region BP, a second substrate 404 facing the first substrate 402, a thin film transistor Tr on the first substrate 402, an OLED D disposed between the first and second substrates 402 and 404 and emitting white (W) light and a color filter layer 480 disposed between the OLED D and the second substrate 404.

Each of the first and second substrates 402 and 404 may include, but is not limited to, glass, flexible material and/or polymer plastics. For example, each of the first and second substrates 402 and 404 may be made of PI, PES, PEN, PET, PC and/or combinations thereof. The first substrate 402, on which a thin film transistor Tr and the OLED D are arranged, forms an array substrate.

A buffer layer 406 may be disposed on the first substrate 402. The thin film transistor Tr is disposed on the buffer layer 406 correspondingly to each of the red pixel region RP, the green pixel region GP and the blue pixel region BP. The buffer layer 406 may be omitted.

A semiconductor layer 410 is disposed on the buffer layer 406. The semiconductor layer 410 may be made of or include oxide semiconductor material or polycrystalline silicon.

A gate insulating layer 420 including an insulating material, for example, inorganic insulating material such as silicon oxide (SiOₓ, wherein 0 < x ≤ 2) or silicon nitride (SiNₓ, wherein 0 < x ≤ 2) is disposed on the semiconductor layer 410.

A gate electrode 430 made of a conductive material such as a metal is disposed on the gate insulating layer 420 so as to correspond to a center of the semiconductor layer 410. An interlayer insulating layer 440 including an insulating material, for example, inorganic insulating material such as SiOₓ or SiNₓ, or an organic insulating material such as benzocyclobutene or photo-acryl, is disposed on the gate electrode 430.

The interlayer insulating layer 440 has first and second semiconductor layer contact holes 442 and 444 that expose the semiconductor layer 410 or do not cover a portion of the surface of the semiconductor layer 410 nearer to the opposing ends than to a center of the semiconductor layer 410. The first and second semiconductor layer contact holes 442 and 444 are disposed on opposite sides of the gate electrode 430 with spacing apart from the gate electrode 430.

A source electrode 452 and a drain electrode 454, which are made of or include a conductive material such as a metal, are disposed on the interlayer insulating layer 440. The source electrode 452 and the drain electrode 454 are spaced apart from each other with respect to the gate electrode 430. The source electrode 452 and the drain electrode 454 contact both sides of the semiconductor layer 410 through the first and second semiconductor layer contact holes 442 and 444, respectively.

The semiconductor layer 410, the gate electrode 430, the source electrode 452 and the drain electrode 454 constitute the thin film transistor Tr, which acts as a driving element.

Although not shown in FIG. 4, the gate line GL and the data line DL, which cross each other to define the pixel region P, and a switching element Ts, which is connected to the gate line GL and the data line DL, may be further formed in the pixel region P. The switching element Ts is connected to the thin film transistor Tr, which is a driving element. In addition, the power line PL is spaced apart in parallel from the gate line GL or the data line DL, and the thin film transistor Tr may further include the storage capacitor Cst configured to constantly keep a voltage of the gate electrode 430 for one frame.

A passivation layer 460 is disposed on the source and drain electrodes 452 and 454 and covers the thin film transistor Tr on the whole first substrate 402. The passivation layer 460 has a drain contact hole 462 that exposes the drain electrode 454 or does not cover the drain electrode 454 of the thin film transistor Tr.

The OLED D is located on the passivation layer 460. The OLED D includes a first electrode 510 that is connected to the drain electrode 454 of the thin film transistor Tr, a second electrode 520 facing the first electrode 510 and an emissive layer 530 disposed between the first and second electrodes 510 and 520.

The first electrode 510 formed for each pixel region RP, GP or BP may be an anode and may include a conductive material having relatively high work function value. For example, the first electrode 510 may include, but is not limited to, ITO, IZO, ITZO, SnO, ZnO, ICO, AZO, and/or the like. Alternatively, a reflective electrode or a reflective layer may be disposed under the first electrode 510. For example, the reflective electrode or the reflective layer may include, but is not limited to, Ag or APC alloy.

A bank layer 464 is disposed on the passivation layer 460 in order to cover edges of the first electrode 510. The bank layer 464 exposes or does not cover a center of the first electrode 510 corresponding to each of the red pixel RP, the green pixel GP and the blue pixel BP. The bank layer 464 may be omitted.

An emissive layer 530 that may include emitting parts is disposed on the first electrode 510. As illustrated in FIGS. 5 and 6, the emissive layer 530 may include multiple emitting parts 600, 700, 700', and 800 and at least one charge generation layer 680 and 780. Each of the emitting parts 600, 700, 700' and 800 includes at least one emitting material layer and may further include an HIL, an HTL, an EBL, an HBL, an ETL and/or an EIL.

The second electrode 520 may be disposed on the first substrate 402 above which the emissive layer 530 may be disposed. The second electrode 520 may be disposed on a whole display area, and may include a conductive material with a relatively low work function value compared to the first electrode 510, and may be a cathode. For example, the second electrode 520 may include, but is not limited to, Al, Mg, Ca, Ag, alloy thereof, and/or combinations thereof such as Al-Mg.

Since the light emitted from the emissive layer 530 is incident to the color filter layer 480 through the second electrode 520 in the organic light emitting display device 400 in accordance with the second embodiment of the present disclosure, the second electrode 520 has a thin thickness so that the light may be transmitted.

The color filter layer 480 is disposed on the OLED D and includes a red color filter pattern 482, a green color filter pattern 484 and a blue color filter pattern 486 each of which is disposed correspondingly to the red pixel RP, the green pixel GP and the blue pixel BP, respectively. Although not shown in FIG. 4, the color filter layer 480 may be attached to the OLED D through an adhesive layer. Alternatively, the color filter layer 480 may be disposed directly on the OLED D.

In addition, an encapsulation film may be disposed on the second electrode 520 in order to prevent or reduce outer moisture from penetrating into the OLED D. The encapsulation film may have, but is not limited to, a laminated structure including a first inorganic insulating film, an organic insulating film and a second inorganic insulating film (176 in FIG. 2). In addition, a polarizing plate may be attached onto the second substrate 404 to reduce reflection of external light. For example, the polarizing plate may be a circular polarizing plate.

In FIG. 4, the light emitted from the OLED D is transmitted through the second electrode 520 and the color filter layer 480 is disposed on the OLED D. Alternatively, the light emitted from the OLED D is transmitted through the first electrode 510 and the color filter layer 480 may be disposed between the OLED D and the first substrate 402. In addition, a color conversion layer may be formed or disposed between the OLED D and the color filter layer 480. The color conversion layer may include a red color conversion layer, a green color conversion layer and a blue color conversion layer each of which is disposed correspondingly to each pixel (RP, GP and BP), respectively, so as to convert the white (W) color light to each of a red, green and blue color lights, respectively. Alternatively, the organic light emitting display device 400 may comprise the color conversion film instead of the color filter layer 480.

As described above, the white (W) color light emitted from the OLED D is transmitted through the red color filter pattern 482, the green color filter pattern 484 and the blue color filter pattern 486 each of which is disposed correspondingly to the red pixel region RP, the green pixel region GP and the blue pixel region BP, respectively, so that red, green and blue color lights are displayed in the red pixel region RP, the green pixel region GP and the blue pixel region BP, respectively.

FIG. 5 illustrates a schematic cross-sectional view of an organic light emitting diode having a tandem structure of two emitting parts in accordance with another example embodiment of the present disclosure. As illustrated in FIG. 5, the OLED D2 in accordance with the example embodiment of the present disclosure includes first and second electrodes 510 and 520 and an emissive layer 530 disposed between the first and second electrodes 510 and 520. The emissive layer 530 includes a first emitting part 600 disposed between the first and second electrodes 510 and 520, a second emitting part 700 disposed between the first emitting part 600 and the second electrode 520 and a charge generation layer (CGL) 680 disposed between the first and second emitting parts 600 and 700.

The first electrode 510 may be an anode and may include a conductive material having relatively high work function value such as TCO. For example, the first electrode 510 may include, but is not limited to, ITO, IZO, ITZO, SnO, ZnO, ICO, AZO, and/or the like. The second electrode 520 may be a cathode and may include a conductive material with a relatively low work function value. For example, the second electrode 520 may include, but is not limited to, Al, Mg, Ca, Ag, alloy thereof and/or combination thereof such as Al-Mg.

The first emitting part 600 comprise a first EML (EML1) 640. The first emitting part 600 may further include at least one of an HIL 610 disposed between the first electrode 510 and the EML1 640, a first HTL (HTL1) 620 disposed between the HIL 610 and the EML1 640, a first ETL (ETL1) 660 disposed between the EML1 640 and the CGL 680. Alternatively, the first emitting part 600 may further include a first EBL (EBL1) 630 disposed between the HTL1 620 and the EML1 640 and/or a first HBL (HBL1) 650 disposed between the EML1 640 and the ETL1 660.

The second emitting part 700 includes a second EML (EML2) 740. The second emitting part 700 may further include at least one of a second HTL (HTL2) 720 disposed between the CGL 680 and the EML2 740, a second ETL (ETL2) 760 disposed between the second electrode 520 and the EML2 740 and an EIL 770 disposed between the second electrode 520 and the ETL2 760. Alternatively, the second emitting part 700 may further include a second EBL (EBL2) 730 disposed between the HTL2 720 and the EML2 740 and/or a second HBL (HBL2) 750 disposed between the EML2 740 and the ETL2 760.

At least one of the EML1 640 and the EML2 740 may include a first host 742, and optionally, a second host 744. For example, one of the EML1 640 and the EML2 740 emits blue color and the other of the EML1 640 and the EML2 740 emits red, green and/or yellow green color so that the OLED D2 can realize white (W) emission. Hereinafter, the example embodiment of OLED D2 where the EML 1 640 emits blue color and the EML2 740 emits green, red and/or yellow green color will be described in detail.

The HIL 610 is disposed between the first electrode 510 and the HTL1 620 and may improve an interface property between the inorganic first electrode 510 and the organic HTL1 620. In one example embodiment, the HIL 610 may include, but is not limited to, MTDATA, NATA, 1T-NATA, 2T-NATA, CuPc, TCTA, NPB (NPD), HAT-CN, TDAPB, PEDOT/PSS, F4TCNQ, N-(biphenyl-4-yl)-9,9-dimethyl-N-(4-(9-phenyl-9H-carbazol-3-yl)phenyl)-9H-fluoren-2-amine, NPNPB and/or combination thereof. Alternatively, the HIL 610 may include the hole injection host and the hole injection dopant. The HIL 610 may be omitted in compliance of the OLED D2 property.

Each of the HTL1 620 and the HTL2 720 may facilitate hole transportation in each of the first emitting part 600 and the second emitting part 700, respectively. In one example embodiment, each of the HTL1 620 and the HTL2 720 may independently include, but is not limited to, TPD, NPB, CBP, Poly-TPD, TFB, TAPC, DCDPA, N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine, N-([1,1'-Biphenyl]-4-yl)-9,9-dimethyl-N-(4-(9-phenl-9H-carbazol-3-yl)phenyl)-9H-fluoren-2-amine and/or combination thereof.

In another example embodiment, each of the HTL1 620 and the HTL2 720 may independently include the hole transporting material 722 that may be the spiro-bifluorene-based organic compound having any one of the structures of Formulae 6 to 9.

Each of the ETL1 660 and the ETL2 760 may facilitate electron transportation in each of the first emitting part 600 and the second emitting part 700, respectively. As an example, each of the ETL1 660 and the ETL2 760 may independently include, but is not limited to, at least one of oxadiazole-based compounds, triazole-based compounds, phenanthroline-based compounds, benzoxazole-based compounds, benzothiazole-based compounds, benzimidazole-based compounds, triazine-based compounds, and/or the like.

As an example, each of the ETL1 660 and the ETL2 760 may independently include, but is not limited to, Alq₃, BAlq, Liq, PBD, spiro-PBD, TPBi, Bphen, NBphen, BCP, TAZ, NTAZ, TpPyPB, TmPPPyTz, PFNBr, TPQ, TSPO1, ZADN and/or combination thereof.

The EIL 770 is disposed between the second electrode 520 and the ETL2 760, and may improve physical properties of the second electrode 520 and therefore, may enhance the lifetime of the OLED D2. In one example embodiment, the EIL 770 may include, but is not limited to, an alkali metal halide or an alkaline earth metal halide such as LiF, CsF, NaF, BaF₂ and/or the like, and/or an organic salt compound such as Liq, lithium benzoate, sodium stearate, and/or the like.

Each of the EBL1 630 and the EBL2 730 may independently include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2-amine, TAPC, MTDATA, mCP, mCBP, CuPc, DNTPD, TDAPB, DCDPA, 2,8-bis(9-phenyl-9H-carbazol-3-yl)dibenzo[b,d]thiophene and/or combination thereof, respectively.

Each of the HBL1 650 and the HBL2 750 may independently include, but is not limited to, at least one of oxadiazole-based compounds, triazole-based compounds, phenanthroline-based compounds, benzoxazole-based compounds, benzothiazole-based compounds, benzimidazole-based compounds, and triazine-based compounds each of which may be used in the ETL1 660 and the ETL2 760. For example, each of the HBL1 650 and the HBL2 750 may independently include, but is not limited to, Alq₃, BAlq, Liq, PBD, spiro-PBD, BCP, B3PYMPM, DPEPO, 9-(6-(9H-carbazol-9-yl)pyridine-3-yl)-9H-3,9'-bicarbazole, TSPO1 and/or combination thereof, respectively.

The CGL 680 is disposed between the first emitting part 600 and the second emitting part 700. The CGL 680 includes an N-type CGL (N-CGL) 685 disposed adjacent to the first emitting part 600 and a P-type CGL (P-CGL) 690 disposed adjacent to the second emitting part 700. The N-CGL 685 injects electrons to the EML1 640 of the first emitting part 600 and the P-CGL 690 injects holes to the EML2 740 of the second emitting part 700.

The N-CGL 685 may be an organic layer doped with an alkali metal such as Li, Na, K and Cs and/or an alkaline earth metal such as Mg, Sr, Ba and Ra. The host in the N-CGL 685 may include, but is not limited to, Bphen and/or MTDATA. The content of the alkali metal or the alkaline earth metal in the N-CGL 685 may be between about 0.01 wt% and about 30 wt%, based on a total weight of the components in N-CGL 685.

The P-CGL 690 may include, but is not limited to, an inorganic material selected from the group consisting of WOₓ, MoOₓ, V₂O₅ and/or combinations thereof and/or an organic material selected from the group consisting of NPD, HAT-CN, F4TCNQ, TPD, N,N,N',N'-tetranaphthalenyl-benzidine (TNB), TCTA, N,N'-dioctyl-3,4,9,10-perylenedicarboximide (PTCDI-C8) and/or combinations thereof.

The EML1 640 may be a blue EML. In this case, the EML1 640 may be a blue EML, a sky-blue EML or a deep-blue EML. The EML1 640 may include a first host and/or a second host and a blue dopant.

For example, the first host may include the organic compound having any one of the structures of Formulae 1 to 5. The second host may include the spiro-bifluorene-based organic compound having any one of the structures of Formulae 6 to 9 and/or a blue host. As an example, the blue host may include, but is not limited to, mCP, mCBP, mCP-CN, CBP-CN, mCPPO1 Ph-mCP, TSPO1, 9-(3'-(9H-carbazol-9-yl)-[1,1'-biphenyl]-3-yl)-9H-pyrido[2,3-b]indole (CzBPCb), Bis(2-methylphenyl)diphenylsilane (UGH-1), 1,4-Bis(triphenylsilyl)benzene (UGH-2), 1,3-Bis(triphenylsilyl)benzene (UGH-3), 9,9-Spirobifluoren-2-yl-diphenyl-phosphine oxide (SPPO1), 9,9'-(5-(Triphenylsilyl)-1,3-phenylene)bis(9H-carbazole) (SimCP) and/or combination thereof. The blue dopant may include at least one of the blue phosphorescent material, blue fluorescent material, and the blue delayed fluorescent material.

The EML2 740 may include a first layer (lower EML) 740A disposed between the EBL2 730 and the HBL2 750 and a second layer (upper EML) 740B disposed between the first layer 740A and the HBL2 750. One of the first layer 740A and the second layer 740B may emit red color and the other of the first layer 740A and the second layer 740B may emit green color. Hereinafter, an example embodiment of the EML2 740 where the first layer 740A emits a red color and the second layer 740B emits a green color will be described in detail.

The first layer may include a first host 742 and a red dopant 746, and optionally, a second host 744. The first host 742 may include the organic compound having any one of the structures of Formulae 1 to 5. In one example embodiment, the second host 744 may include the spiro-bifluorene-based organic compound having any one of the structures of Formulae 6 to 9 and/or a red host.

The red host may include, but is not limited to, mCP-CN, CBP, mCBP, mCP, DPEPO, 2,8-bis(diphenylphosphoryl)dibenzothiophene (PPT), 1,3,5-Tri[(3-pyridyl)-phen-3-yl]benzene (TmPyPB), 2,6-Di(9H-carbazol-9-yl)pyridine (PYD-2Cz), 2,8-di(9H-carbazol-9-yl)dibenzothiophene (DCzDBT), 3',5'-Di(carbazol-9-yl)-[1,1'-biphenyl]-3,5-dicarbonitrile (DCzTPA), 4'-(9H-carbazol-9-yl)biphenyl-3,5-dicarbonitrile(4'-(9H-carbazol-9-yl)biphenyl-3,5-dicarbonitrile (pCzB-2CN), 3'-(9H-carbazol-9-yl)biphenyl-3,5-dicarbonitrile (mCzB-2CN), TSPO1, CCP, 4-(3-(triphenylen-2-yl)phenyl)dibenzo[b,d]thiophene, 9-(4-(9H-carbazol-9-yl)phenyl)-9H-3,9'-bicarbazole, 9-(3-(9H-carbazol-9-yl)phenyl)-9H-3,9'-bicarbazole, 9-(6-(9H-carbazol-9-yl)pyridin-3-yl)-9H-3,9'-bicarbazole), BCzPh, TCP, TCTA, CDBP, DMFL-CBP, Spiro-CBP, TCz1 and/or combination thereof. The red dopant may include at least one of red phosphorescent material, red fluorescent material and red delayed fluorescent material.

The second layer 740B may include a first host and/or a second host and a green dopant. As an example, the first host may include the organic compound having any one of the structures of Formulae 1 to 5. The second host may include the spiro-bifluorene-based organic compound having the structure of Formulae 6 to 9 and/or a green host. The green host may be, but is not limited to, identical to the red host. The green dopant may include at least one of green phosphorescent material, green fluorescent material, and green delayed fluorescent material.

As an example, the content of the host including the first and second host in each of the first layer 740A and the second layer 740B may be, but is not limited to, between about 50 and about 99 wt%, for example, about 80 and about 95 wt%, based on a total weight of the component in the first layer 740A or the second layer 740B. The content of the dopant in each of the first layer 740A and the second layer 740B may be, but is not limited to, between about 1 and about 50 wt%, for example, about 5 and about 20 wt%, based on a total weight of the components in the first layer 740A or the second layer 740B. In addition, when the first and second layers 740A and 740B include the first host and second host, the first host and the second host may be admixed, but is not limited to, with a weight ratio from about 4:1 to about 1:4, for example, about 3:1 to about 1:3.

Alternatively, the EML2 740 may further include a third layer (740C in FIG. 6) of yellow green EML disposed between the first layer 740A of the red EML and the second layer 740B of the green EML

The OLED D2 in accordance with this example embodiment has a tandem structure. At least one EML includes the first host 742 that may have beneficial hole transportation property. Alternatively, the driving voltage of the OLED D2 may be further reduced and the luminous efficiency and luminous lifespan of the OLED D2 may be further improved by using the spiro-bifluorene-based organic compound as the second host and/or the hole transporting material 722.

The OLED may have three or more emitting parts to form a tandem structure. FIG. 6 illustrates a schematic cross-sectional view of an organic light emitting diode in accordance with yet another example embodiment of the present disclosure. As illustrated in FIG. 6, the OLED D3 includes first and second electrodes 510 and 520 facing each other and an emissive layer 530' disposed between the first and second electrodes 510 and 520. The emissive layer 530' includes a first emitting part 600 disposed between the first and second electrodes 510 and 520, a second emitting part 700' disposed between the first emitting part 600 and the second electrode 520, a third emitting part 800 disposed between the second emitting part 700' and the second electrode 520, a first charge generation layer (CGL1) 680 disposed between the first and second emitting parts 600 and 700', and a second charge generation layer (CGL2) 780 disposed between the second and third emitting parts 700' and 800.

The first emitting part 600 includes a first EML (EML1) 640. The first emitting part 600 may further include at least one of an HIL 610 disposed between the first electrode 510 and the EML1 640, a first HTL (HTL1) 620 disposed between the HIL 610 and the EML1 640, a first ETL (ETL1) 660 disposed between the EML1 640 and the CGL 680. Alternatively, the first emitting part 600 may further comprise a first EBL (EBL1) 630 disposed between the HTL1 620 and the EML1 640 and/or a first HBL (HBL1) 650 disposed between the EML1 640 and the ETL1 660.

The second emitting part 700' comprise a second EML (EML2) 740'. The second emitting part 700' may further include at least one of a second HTL (HTL2) 720 disposed between the CGL1 680 and the EML2 740' and a second ETL (ETL2) 760 disposed between the second electrode 520 and the EML2 740'. Alternatively, the second emitting part 700' may further include a second EBL (EBL2) 730 disposed between the HTL2 720 and the EML2 740' and/or a second HBL (HBL2) 750 disposed between the EML2 740' and the ETL2 760.

The third emitting part 800 includes a third EML (EML3) 840. The third emitting part 800 may further comprise at least one of a third HTL (HTL3) 820 disposed between the CGL2 780 and the EML3 840, a third ETL (ETL3) 860 disposed between the second electrode 520 and the EML3 840 and an EIL 870 disposed between the second electrode 520 and the ETL3 860. Alternatively, the third emitting part 800 may further comprise a third EBL (EBL3) 830 disposed between the HTL3 820 and the EML3 840 and/or a third HBL (HBL3) 850 disposed between the EML3 840 and the ETL3 860.

At least one of the EML1 640, the EML2 740' and the EML3 840 may include the first host 742 and optionally the second host 744. For example, at least one of the EML1 640, the EML2 740' and the EML3 840 may emit blue color and another of the EML1 640, the EML2 740' and the EML3 840 may emit green, red and/or yellow green color so that the OLED D3 can realize white emission. Hereinafter, an example embodiment of the OLED where the EML1 640 and the EML2 840 emit blue color and the EML2 740' emits green, red and/or yellow green color will be described in detail.

Each of the HTL1 620, the HTL2 720, and the HTL3 820 facilitates hole transportation in each of the first emitting part 600, the second emitting part 700', and the third emitting part 800, respectively. In one example embodiment, each of the HTL1 620, the HTL2 720 and the HTL3 820 may independently include, but is not limited to, TPD, NPB, CBP, Poly-TPD, TFB, TAPC, DCDPA, N-(biphenyl-4-yl)-9,9-dimethyl-N-(4-(9-phenyl-9H-carbazol-3-yl)phenyl)-9H-fluoren-2-amine, N-(biphenyl-4-yl)-N-(4-(9-phenyl-9H-carbazol-3-yl)phenyl)biphenyl-4-amine, N-([1,1'-Biphenyl]-4-yl)-9,9-dimethyl-N-(4-(9-phenl-9H-carbazol-3-yl)phenyl)-9H-fluoren-2-amine and/or combination thereof.

In another example embodiment, each of the HTL1 620, the HTL2 720, and the HTL3 820 may independently include the hole transporting material 722 that may be the spiro-bifluorene-based organic compound having any one of the structures of Formulae 6 to 9.

Each of the ETL1 660, the ETL2 760, and the ETL3 860 facilitates electron transportation in each of the first emitting part 600, the second emitting part 700' and the third emitting part 800, respectively. As an example, each of the ETL1 660, the ETL2 760, and the ELT3 860 may independently include, but is not limited to, at least one of oxadiazole-based compounds, triazole-based compounds, phenanthroline-based compounds, benzoxazole-based compounds, benzothiazole-based compounds, benzimidazole-based compounds, triazine-based compounds, and/or the like.

More particularly, each of the ETL1 660, the ETL2 760, and the ETL3 860 may independently include, but is not limited to, Alq₃, BAlq, Liq, PBD, spiro-PBD, TPBi, Bphen, NBphen, BCP, TAZ, NTAZ, TpPyPB, TmPPPyTz, PFNBr, TPQ, TSPO1, ZADN and/or combination thereof.

Each of the EBL1 630, the EBL2 730, and the EBL3 830 may independently include, but is not limited to, TCTA, Tris[4-(diethylamino)phenyl]amine, N-(biphenyl-4-yl)-9,9-dimethyl-N-(4-(9-phenyl-9H-carbazol-3-yl)phenyl)-9H-fluorene-2-amine, TAPC, MTDATA, mCP, mCBP, CuPc, DNTPD, TDAPB, DCDPA, 2,8-bis(9-phenyl-9H-carbazol-3-yl)dibenzo[b,d]thiophene and/or combination thereof, respectively.

Each of the HBL1 650, the HBL2 750, and the HBL3 850 may independently include, but is not limited to at least one of oxadiazole-based compounds, triazole-based compounds, phenanthroline-based compounds, benzoxazole-based compounds, benzothiazole-based compounds, benzimidazole-based compounds, and triazine-based compounds each of which may be used in the ETL1 660, the ETL2 760, and the ETL3 860. More particularly, each of the HBL1 650, the HBL2 750, and the HBL3 850 may independently include, but is not limited to, Alq₃, BAlq, Liq, PBD, spiro-PBD, BCP, B3PYMPM, DPEPO, 9-(6-(9H-carbazol-9-yl)pyridine-3-yl)-9H-3,9'-bicarbazole, TSPO1 and/or combination thereof, respectively.

The CGL1 680 is disposed between the first emitting part 600 and the second emitting part 700' and the CGL2 780 is disposed between the second emitting part 700' and the third emitting part 800. The CGL1 680 includes a first N-type CGL (N-CGL1) 685 disposed adjacent to the first emitting part 600 and a first P-type CGL (P-CGL1) 690 disposed adjacent to the second emitting part 700'. The CGL2 780 includes a second N-type CGL (N-CGL2) 785 disposed adjacent to the second emitting part 700' and a second P-type CGL (P-CGL2) 790 disposed adjacent to the third emitting part 800. Each of the N-CGL1 685 and the N-CGL2 785 injects electrons to the EML1 640 of the first emitting part 600 and the EML2 740' of the second emitting part 700', respectively, and each of the P-CGL1 690 and the P-CGL2 790 injects holes to the EML2 740' of the second emitting part 700' and the EML3 840 of the third emitting part 800, respectively.

Each of the EML1 640 and the EML3 840 may be independently a blue EML. In this case, each of the EML1 640 and the EML3 840 may be independently a blue EML, a sky-blue EML or a deep-blue EML. Each of the EML1 640 and the EML3 840 may independently include a first host and/or a second host and a blue dopant.

For example, the first host may include the organic compound having any one of the structures of Formulae 1 to 5. The second host may include the spiro-bifluorene-based organic compound having any one of the structures of Formulae 6 to 9 and/or a blue host. For example, the blue dopant may include at least one of the blue phosphorescent material, blue fluorescent material, and the blue delayed fluorescent material. Alternatively, the blue dopant in the EML1 640 may be identical to or different from the blue dopant in the EML3 840 in terms of color and/or luminous efficiency.

The EML2 740' may include a first layer 740A, a third layer (middle EML) 740C and a second layer 740B disposed sequentially between the EBL2 730 and the HBL2 750. One of the first layer 740A and the second layer 740B may emit red color and the other of the first layer 740A and the second layer 740B may emit green color. Hereinafter, an example embodiment of the EML2 740' where the first layer 740A emits a red color and the second layer EML 740B emits a green color will be described in detail.

The first layer 740A may include the first host 742 and the red dopant 746, and optionally, the second host 744. The first host 742 may include the organic compound having any one of the structures of Formulae 1 to 5. In one example embodiment, the second host 744 may include the spiro-bifluorene-based organic compound having any one of the structures of Formulae 6 to 9 and/or a red host. The red dopant may include at least one of red phosphorescent material, red fluorescent material, and red delayed fluorescent material.

The second layer 740B may include a first host and/or a second host and a green dopant. As an example, the first host may include the organic compound having any one of the structures of Formulae 1 to 5. The second host may include the spiro-bifluorene-based organic compound having the structure of Formulae 6 to 9 and/or a green host. For example, the green dopant may include at least one of green phosphorescent material, green fluorescent material, and green delayed fluorescent material.

The third layer 740C may be a yellow green EML and may include a yellow green host and a yellow green dopant. As an example, the yellow green host may be, but is not limited to, the red host. The yellow green dopant may include at least one of yellow green phosphorescent materials, yellow green fluorescent material, and yellow green delayed fluorescent material. The third layer 740C may be omitted.

As an example, the content of the host including the first and second host in each of the first layer 740A, the second layer 740B, and the third layer 740C may be, but is not limited to, between about 50 and about 99 wt%, for example, about 80 and about 95 wt%, based on a total weight the components in each of the first layer 740A, the second layer 740B, and the third layer 740C, respectively. The content of the dopant in each of the first layer 740A, the second layer 740B and the third layer 740C may be, but is not limited to, between about 1 and about 50 wt%, for example, about 5 and about 20 wt%, based on a total weight of the components in each of the first layer 740A, the second layer 740B, and the third layer 740C, respectively. In addition, when the first to third layers 740A, 740B, and 740C include the first host and second host, the first host and the second host may be admixed, but is not limited to, with a weight ratio from about 4:1 to about 1:4, for example, about 3:1 to about 1:3.

### Synthesis Example 1: Synthesis of Compound HAC1

### (1) Synthesis of Intermediate A-1

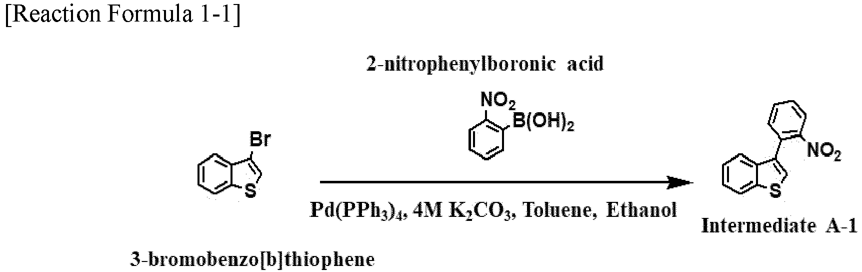

3-bromobenzo[b]thiophene (42.6 g), 2-nitrophenylboric acid (40 g), Tetrakis(triphenylphosphine)palladium(0) (Pd(PPh₃)₄, 4.6 g), toluene (250 mL), ethanol (25mL) and 4M K₂CO₃ aqueous solution (100 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate A-1 (36.7 g, yield: 72%).

### (2) Synthesis of Intermediate A-2

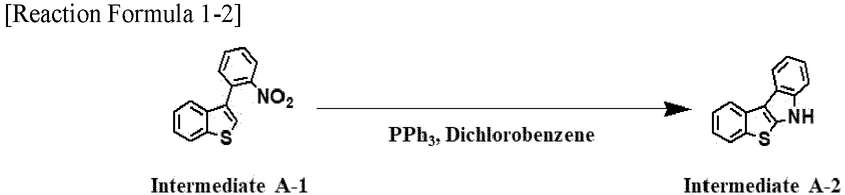

The Intermediate A-1 (36.5 g), triphenylphsophine (PPh₃, 107 g) and dichlorobenzene (250 mL) were added to a round bottom flask, and then the solution was refluxed with stirring for 8 hours. After the reaction was completed, the reaction solution was concentrated under reduced pressure, and then washed with methanol to give the Intermediate A-2 (22.3 g, yield: 61%).

### (3) Synthesis of Intermediate A-3

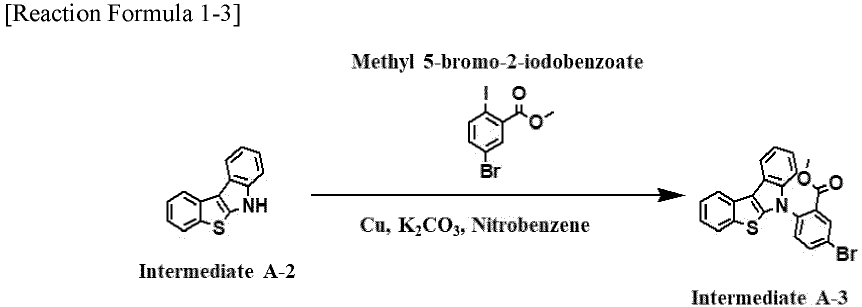

The Intermediate A-2 (22.0 g), methyl 5-bromo-2-iodobenzene (37 g), Cu powder (1.3 g), potassium carbonate (27 g) and nitrobenzene (200 mL) were added to a round bottom flask under nitrogen atmosphere, and then the solution was refluxed with stirring for 24 hours. After the reaction was completed, the reaction solution was cooled, washed with ethyl acetate/distilled water, and then separated into layers to recover an organic layer. The organic layer was distilled under reduced pressure to obtain a crude product. The crude product was separated by column to give the Intermediate A-3 (27.1 g, yield: 63%).

### (4) Synthesis of Intermediate A-4

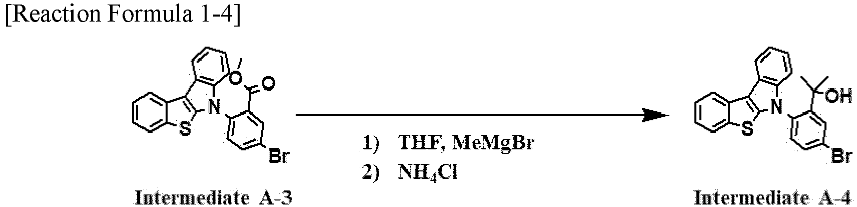

The Intermediate A-3 (27.0 g) and anhydrous tetrahydrofuran (THF, 100 mL) were added to a round bottom flask under nitrogen atmosphere, and then the solution was stirred. MeMgBr 3M solution (52 mL) was added to the solution slowly, and then the solution was stirred for 6 hours at room temperature. After the reaction was completed, 10% NH₄Cl aqueous solution was added to the reaction solution, the solution was stirred for 1 hour, and then separated into layers using ethyl acetate to recover an organic layer. The organic layer was distilled under reduced pressure to give the Intermediate A-4 (18.9 g, yield: 70%).

### (5) Synthesis of Intermediate A-5

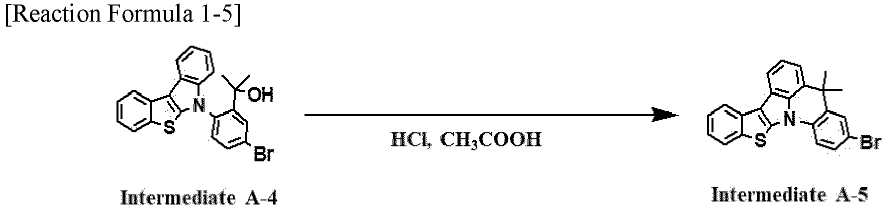

The Intermediate A-4 (18.5 g), 37% chloric acid aqueous solution (10 mL) and acetic acid (10 mL) were added to a round bottom flask, and then the solution was refluxed with stirring and heating for 2 hours. After the reaction was completed, the reaction solution was poured into distilled water to precipitate the crude product, which was separated from the mixture by filtration. The precipitate was dissolved in methylene chloride and dried with MgSO₄, filtered and distilled under reduced pressure. Re-precipitation was performed using ethanol to give the Intermediate A-5 (15 g, yield: 85%).

### (6) Synthesis of Intermediate A-6

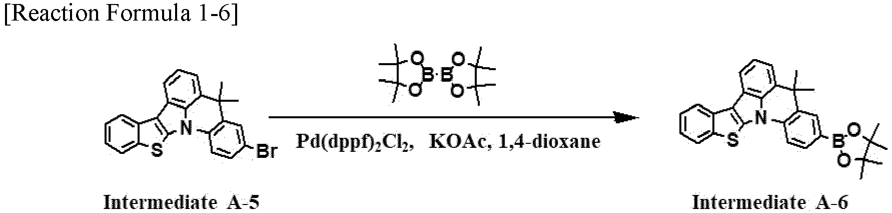

The Intermediate A-5 (9 g), Bis(pinacolato)diboron (8.2 g), potassium acetate (4.2 g), [1,1'-Bis(diphenylphosphino)ferrocene]dichloropalladium (Pd(dppf)₂Cl₂, 0.3 g) and 1,4-dioxane (100 mL) were added to a round bottom flask, and then the solution was refluxed with stirring for 12 hours. After the reaction was completed, the reaction solution was cooled, filtered using Celite 545 under reduced pressure, and then Celite 545 was washed with chloroform. The filtrate was concentrated under reduced pressure and re-crystallization was performed using ethyl acetate to give the Intermediate A-6 (7.4 g, yield: 72%).

### (7) Synthesis of Intermediate C-1

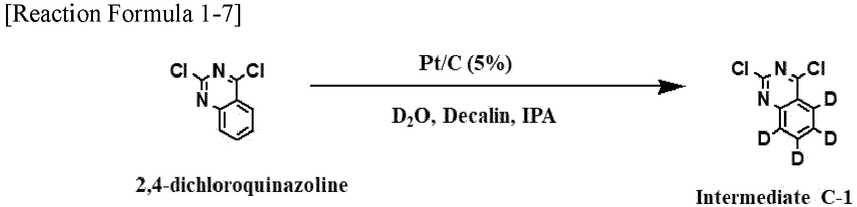

2,4-dichloroquinazoline (20 g), D₂O (100 mL), isopropyl alcohol (IPA, 10 mL) and Decalin (decahydronaphthalene, 200 mL) were added to a round bottom flask and 5% Pt/C (2.2 g) was added to the solution. The solution was stirred at 100°C in a high-pressure reactor for 24 hours, and then the solution was cooled to room temperature. Dichloromethane was added to the solution to separate an organic layer from an aqueous layer. The organic layer was dried with magnesium sulfate, and then the filtrate was concentrated. IPA was added to the concentrated filtrate to precipitate the Intermediate C-1, and the precipitate was separated from the mixture by filtration to give the Intermediate C-1 (17 g).

### (8) Synthesis of Intermediate C-2

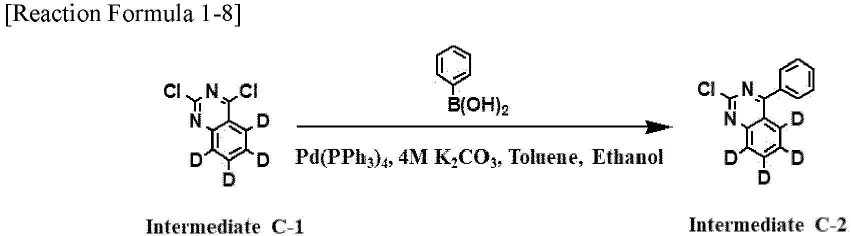

The Intermediate C-1 (10 g), phenylboronic acid (7.4 g), Pd(PPh₃)₄ (1.1 g), toluene (70 mL), ethanol (7 mL) and 4M K₂CO₃ aqueous solution (25 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate C-2 (8.8 g, yield: 70%).

### (9) Synthesis of Compound HAC1

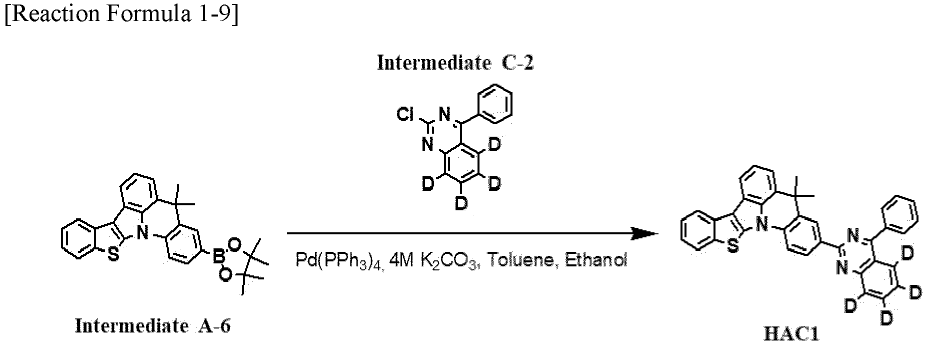

The Intermediate A-6 (5 g), the Intermediate C-2 (4.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC1 (4.2 g, yield: 72%).

### Synthesis Example 2: Synthesis of Compound HAC2

### (1) Synthesis of Intermediate C-3

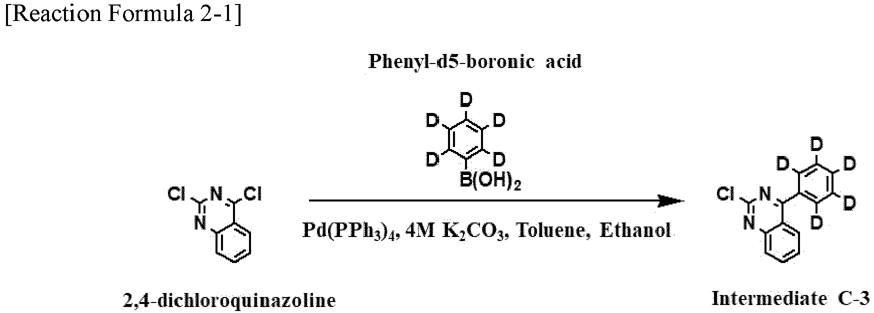

2,4-dichloroquinazoline (10 g), phenyl-d5-boronic acid (6.3 g), Pd(PPh₃)₄ (1.0 g), toluene (150 mL), ethanol (15 mL) and 4M K₂CO₃ aqueous solution (50 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate C-3 (7.4 g, yield: 72%).

### (2) Synthesis of Compound HAC2

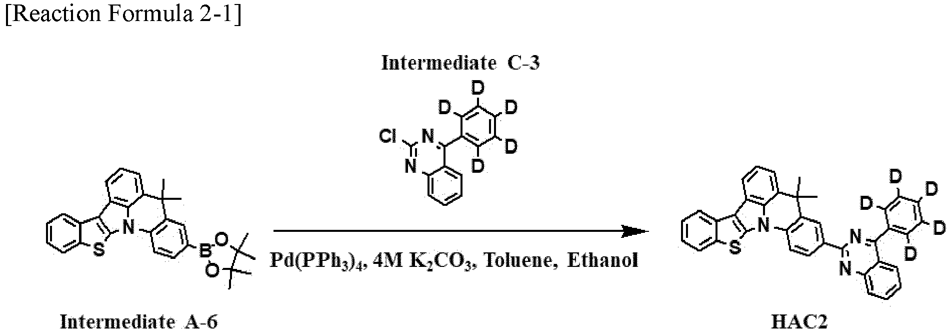

The Intermediate A-6 (5 g), the Intermediate C-3 (3.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC2 (4.1 g, yield: 72%).

### Synthesis Example 3: Synthesis of Compound HAC3

### (1) Synthesis of Intermediate C-4

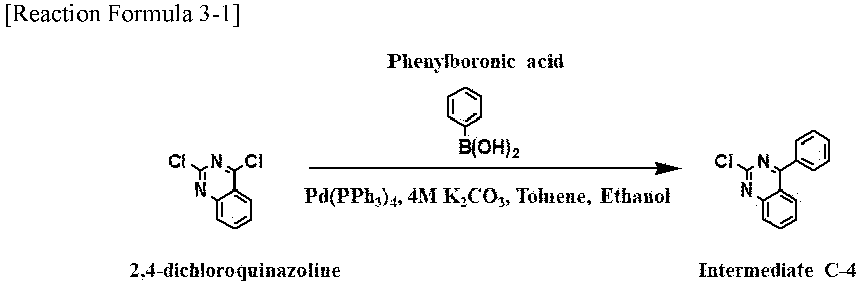

2,4-dichloroquinazoline (20 g), phenyl-d5-boronic acid (14.7 g), Pd(PPh₃)₄ (2.3 g), toluene (150 mL), ethanol (15 mL) and 4M K₂CO₃ aqueous solution (50 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate C-4 (17.4 g, yield: 72%).

### (2) Synthesis of Intermediate C-5

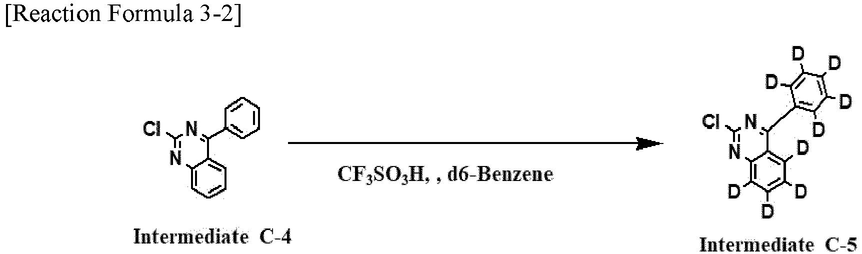

The Intermediate C-4 (10 g), triflic acid (trifluoromethane sulfonic acid, 30.5 g) and D6-benzene were added to a round bottom flask, and then the solution was refluxed with stirring at 70°C. After the reaction was completed, dichloromethane and distilled water were added to the solution to quench the reaction, and then the solution was separated into layers to recover an organic layer. The organic layer was distilled under reduced pressure and filtered through silica gel. The filtrate was concentrated again and re-crystallization was performed using methanol to give the Intermediate C-5 (9.7 g, yield: 95%).

### (3) Synthesis of Compound HAC3

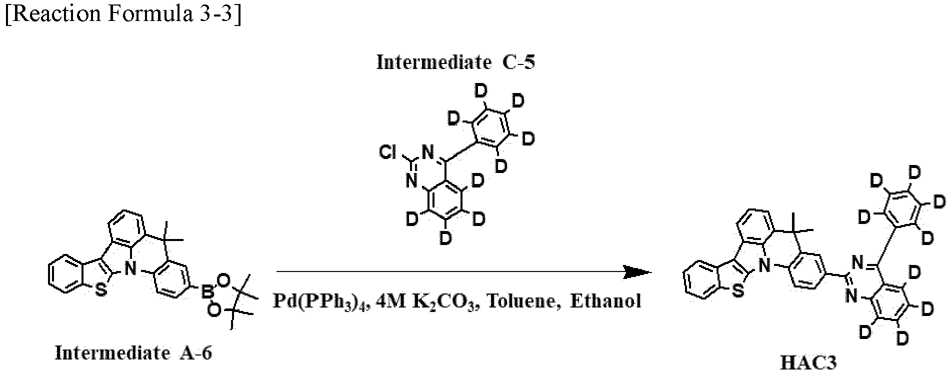

The Intermediate A-6 (5 g), the Intermediate C-5 (3.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC3 (4.2 g, yield: 72%).

### Synthesis Example 4: Synthesis of Compound HAC4

### (1) Synthesis of Intermediate A-7

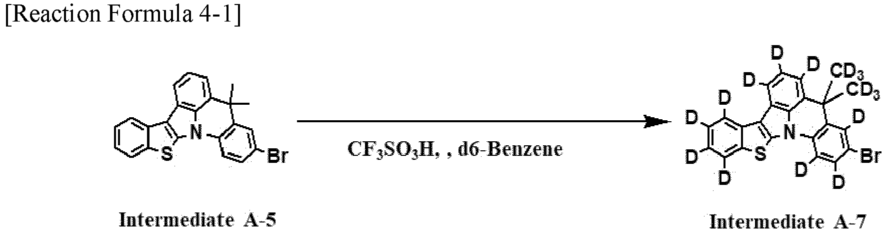

The Intermediate A-5 (6 g), triflic acid (10.8 g) and D6-benzene were added to a round bottom flask, and then the solution was refluxed with stirring at 70°C. After the reaction was completed, dichloromethane and distilled water were added to the solution to quench the reaction, and then the solution was separated into layers to recover an organic layer. The organic layer was distilled under reduced pressure and filtered through silica gel. The filtrate was concentrated again and re-crystallization was performed using methanol to give the Intermediate A-7 (5.9 g, yield: 95%).

### (2) Synthesis of Intermediate A-8

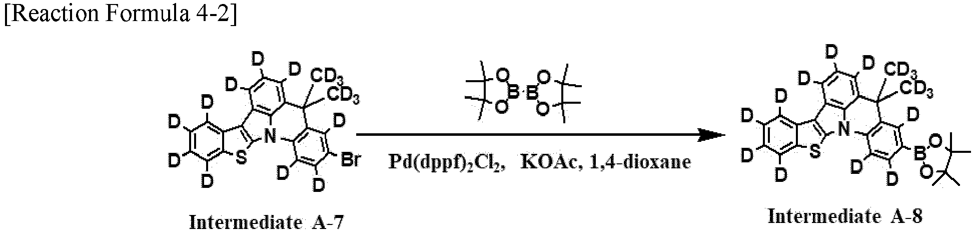

The Intermediate A-7 (5.5 g), Bis(pinacolato)diboron (4.8 g), potassium acetate (2.5 g), Pd(dppf)₂Cl₂ (0.2 g) and 1,4-dioxane (70 mL) were added to a round bottom flask, and then the solution was refluxed with stirring for 12 hours. After the reaction was completed, the reaction solution was cooled, filtered using Celite 545 under reduced pressure, and then Celite 545 was washed with chloroform. The filtrate was concentrated under reduced pressure and re-crystallization was performed using ethyl acetate to give the Intermediate A-8 (4.4 g, yield: 72%).

### (3) Synthesis of Compound HAC4

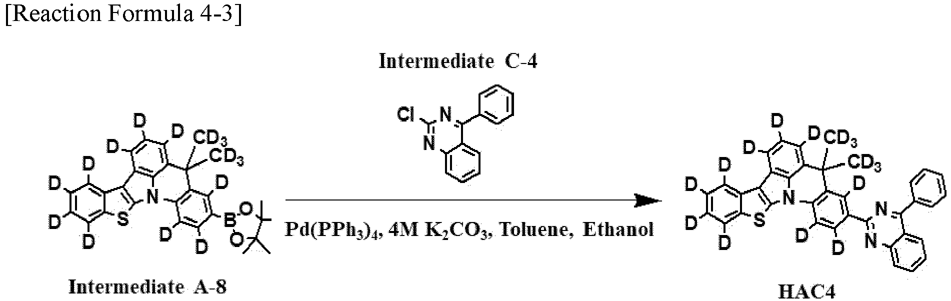

The Intermediate A-8 (5 g), the Intermediate C-4 (3.0 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC4 (4.2 g, yield: 72%).

### Synthesis Example 5: Synthesis of Compound HAC5

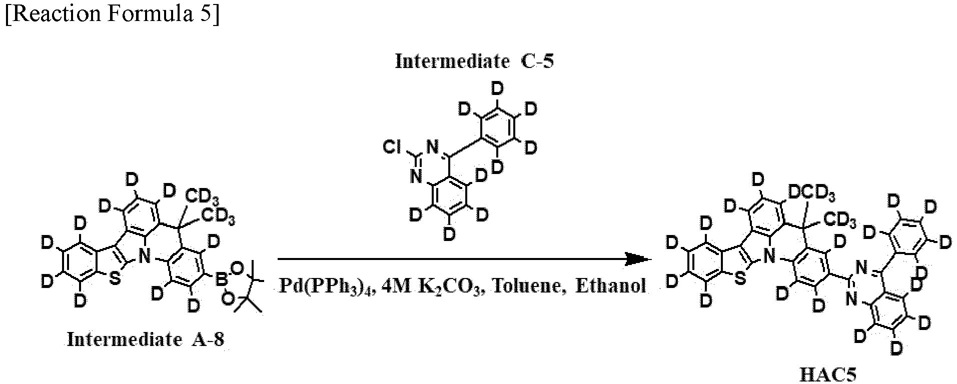

The Intermediate A-8 (5 g), the Intermediate C-5 (3.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC5 (4.2 g, yield: 72%).

### Comparative Synthesis Example 1: Synthesis of Compound HAC A

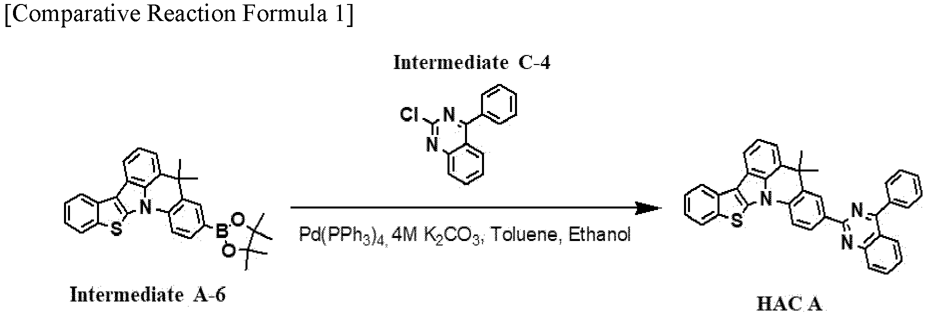

The Intermediate A-6 (5 g), the Intermediate C-4 (3.0 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC A (4.1 g, yield: 72%).

### Synthesis Example 6: Synthesis of Compound HAC296

### (1) Synthesis of Intermediate B-1

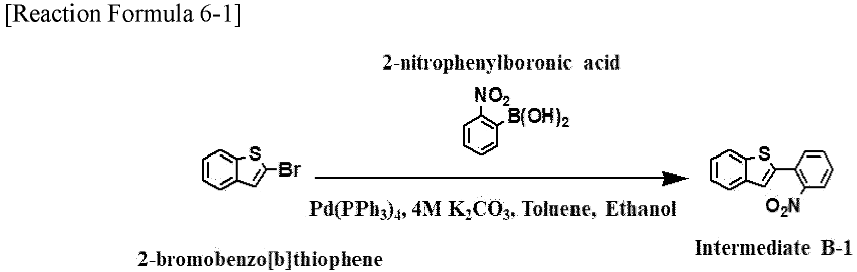

2-bromobenzo[b]thiophene (42.6 g), 2-nitrophenylboric acid (42.6 g), Pd(PPh₃)₄ (4.6 g), toluene (250 mL), ethanol (25mL) and 4M K₂CO₃ aqueous solution (100 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate B-1 (36.7 g, yield: 72%).

### (2) Synthesis of Intermediate B-2

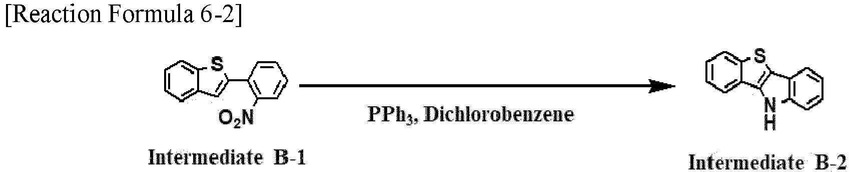

The Intermediate B-1 (36.5 g), PPh₃ (107 g) and dichlorobenzene (250 mL) were added to a round bottom flask, and then the solution was refluxed with stirring for 8 hours. After the reaction was completed, the reaction solution was concentrated under reduced pressure, and then washed with methanol to give the Intermediate B-2 (22.3 g, yield: 61%).

### (3) Synthesis of Intermediate B-3

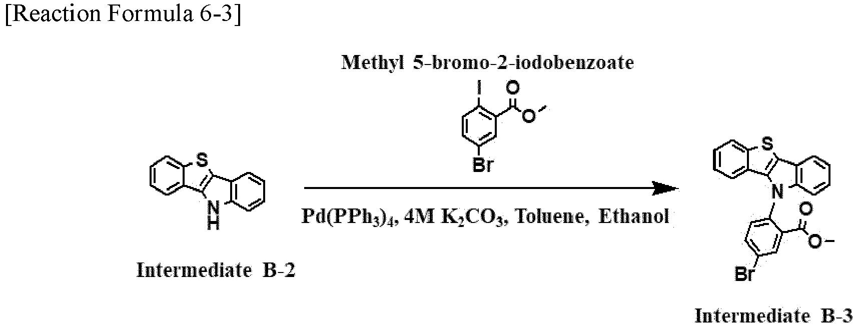

The Intermediate B-2 (22.0 g), methyl 5-bromo-2-iodobenzene (37 g), Cu powder (1.3 g), potassium carbonate (27 g) and nitrobenzene (200 mL) were added to a round bottom flask under nitrogen atmosphere, and then the solution was refluxed with stirring for 24 hours. After the reaction was completed, the reaction solution was cooled, washed with ethyl acetate/distilled water, and then separated into layers to recover an organic layer. The organic layer was distilled under reduced pressure to obtain a crude product. The crude product was separated by column to give the Intermediate B-3 (27.1 g, yield: 63%).

### (4) Synthesis of Intermediate B-4

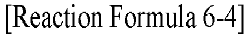

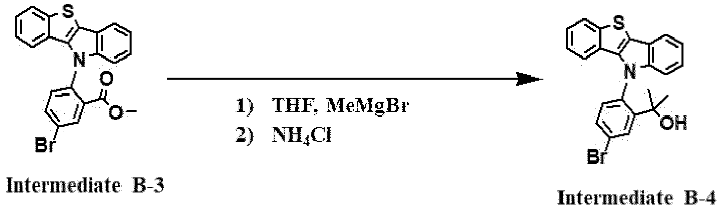

The Intermediate B-3 (27.0 g) and anhydrous THF (100 mL) were added to a round bottom flask under nitrogen atmosphere, and then the solution was stirred. MeMgBr 3M solution (52 mL) was added to the solution slowly, and then the solution was stirred for 6 hours at room temperature. After the reaction was completed, 10% NH₄Cl aqueous solution was added to the reaction solution, the solution was stirred for 1 hour, and then separated into layers using ethyl acetate to recover an organic layer. The organic layer was distilled under reduced pressure to give the Intermediate B-4 (18.9 g, yield: 70%).

### (5) Synthesis of Intermediate B-5

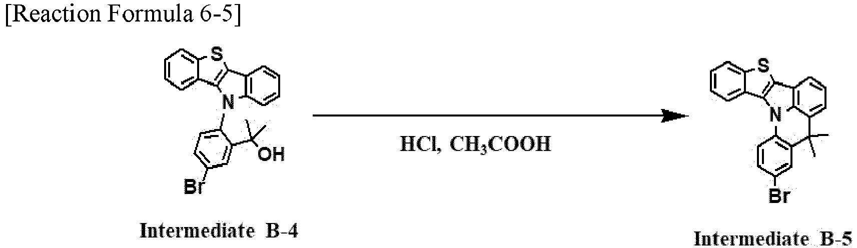

The Intermediate B-4 (18.5 g), 37% chloric acid aqueous solution (10 mL) and acetic acid (10 mL) were added to a round bottom flask, and then the solution was refluxed with stirring and heating for 2 hours. After the reaction was completed, the reaction solution was poured into distilled water to precipitate the crude product, which was separated from the mixture by filtration. The precipitate was dissolved in methylene chloride and dried with MgSO₄, filtered and distilled under reduced pressure. Re-precipitation was performed using ethanol to give the Intermediate B-5 (15 g, yield: 85%).

### (6) Synthesis of Intermediate B-6

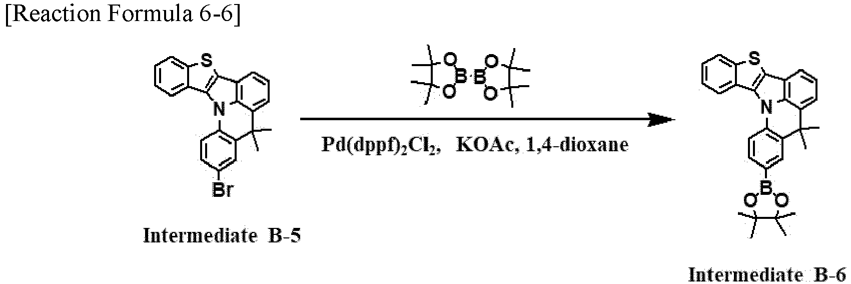

The Intermediate B-5 (9 g), Bis(pinacolato)diboron (8.2 g), potassium acetate (4.2 g), (Pd(dppf)₂Cl₂ (0.3 g) and 1,4-dioxane (100 mL) were added to a round bottom flask, and then the solution was refluxed with stirring for 12 hours. After the reaction was completed, the reaction solution was cooled, filtered using Celite 545 under reduced pressure, and then Celite 545 was washed with chloroform. The filtrate was concentrated under reduced pressure and re-crystallization was performed using ethyl acetate to give the Intermediate B-6 (7.4 g, yield: 72%).

### (7) Synthesis of Compound HAC296

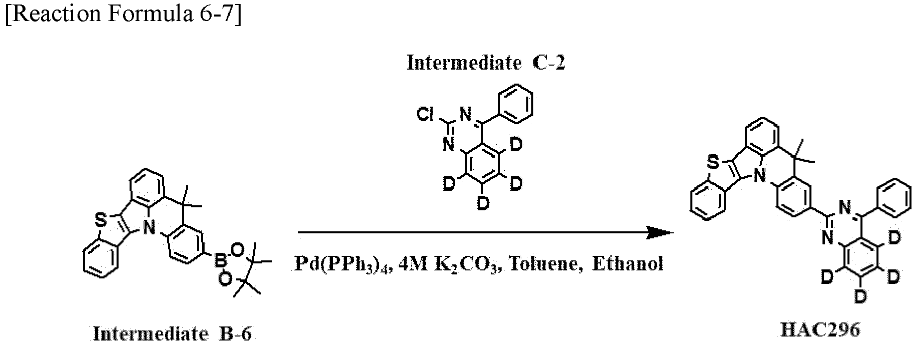

The Intermediate B-6 (5 g), the Intermediate C-2 (3.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC296 (4.3 g, yield: 72%).

### Synthesis Example 7: Synthesis of Compound HAC297

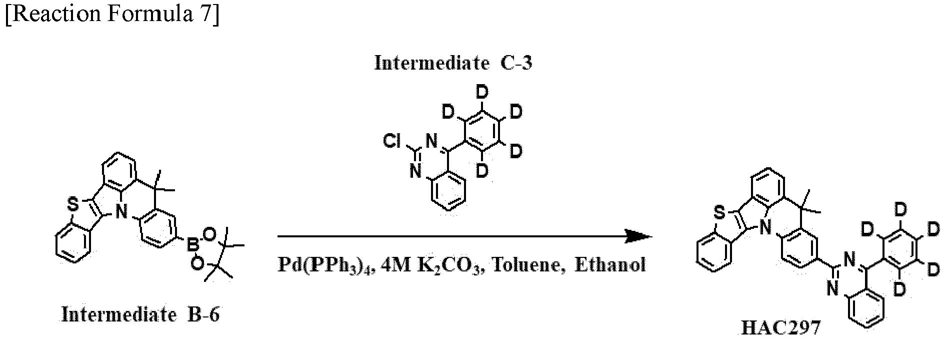

The Intermediate B-6 (5 g), the Intermediate C-3 (3.2 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC297 (4.2 g, yield: 72%).

### Synthesis Example 8: Synthesis of Compound HAC298

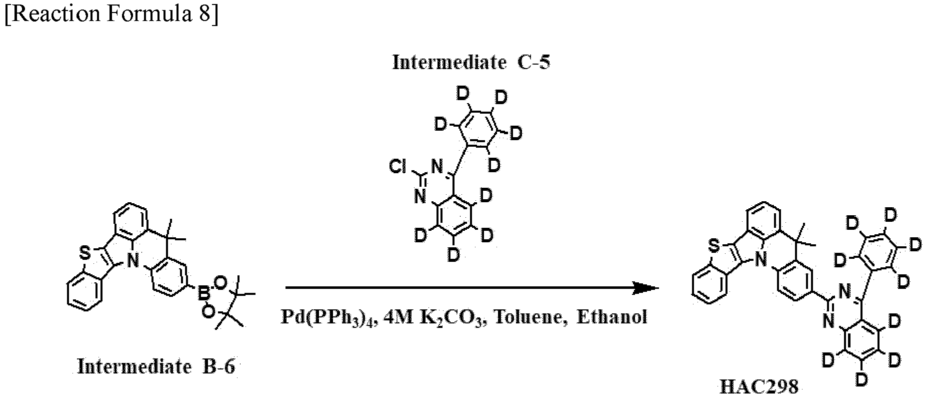

The Intermediate B-6 (5 g), the Intermediate C-5 (3.2 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC298 (4.3 g, yield: 72%).

### Synthesis Example 9: Synthesis of Compound HAC299

### (1) Synthesis of Intermediate B-7

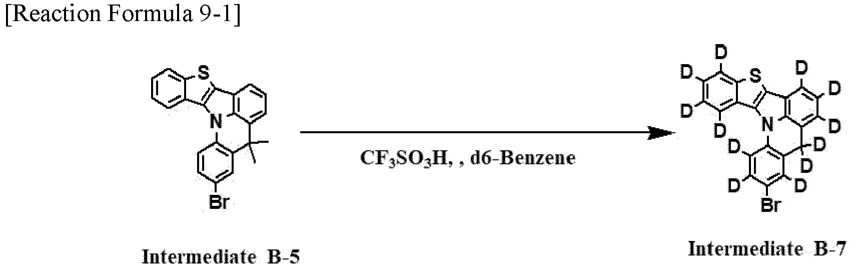

The Intermediate B-5 (6 g), triflic acid (10.8 g) and D6-benzene were added to a round bottom flask, and then the solution was refluxed with stirring at 70°C. After the reaction was completed, dichloromethane and distilled water were added to the solution to quench the reaction, and then the solution was separated into layers to recover an organic layer. The organic layer was distilled under reduced pressure and filtered through silica gel. The filtrate was concentrated again and re-crystallization was performed using methanol to give the Intermediate B-7 (5.9 g, yield: 95%).

### (2) Synthesis of Intermediate B-8

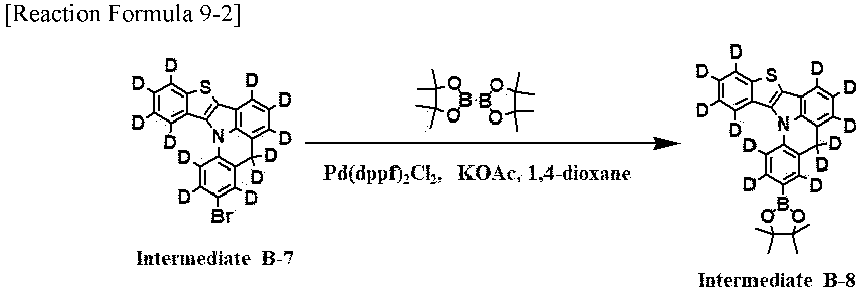

The Intermediate B-7 (5.5 g), Bis(pinacolato)diboron (4.8 g), potassium acetate (2.5 g), Pd(dppf)₂Cl₂ (0.2 g) and 1,4-dioxane (70 mL) were added to a round bottom flask, and then the solution was refluxed with stirring for 12 hours. After the reaction was completed, the reaction solution was cooled, filtered using Celite 545 under reduced pressure, and then Celite 545 was washed with chloroform. The filtrate was concentrated under reduced pressure and re-crystallization was performed using ethyl acetate to give the Intermediate B-8 (4.4 g, yield: 72%).

### (3) Synthesis of Compound HAC299

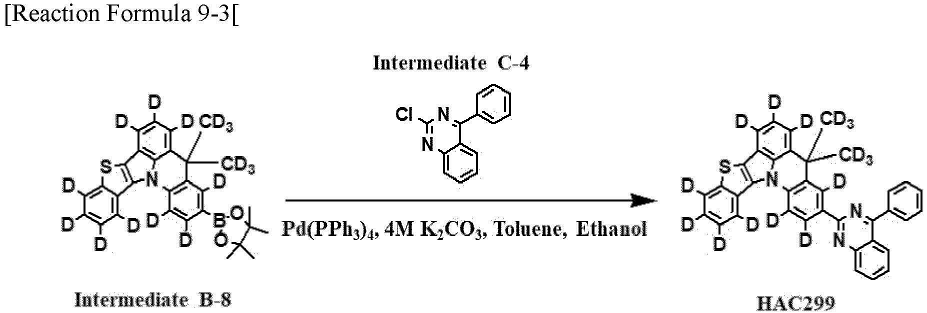

The Intermediate B-8 (5 g), the Intermediate C-4 (3.0 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC299 (4.2 g, yield: 72%).

### Synthesis Example 10: Synthesis of Compound HAC300

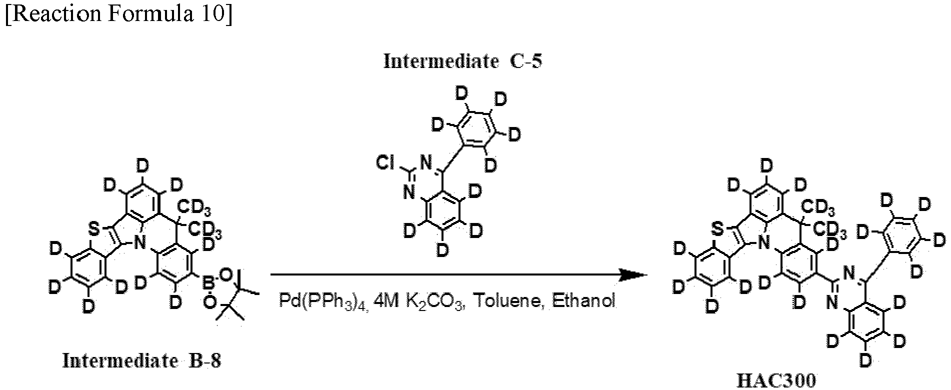

The Intermediate B-8 (5 g), the Intermediate C-5 (3.2 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC300 (4.2 g, yield: 72%).

### Comparative Synthesis Example 2: Synthesis of Compound HAC B

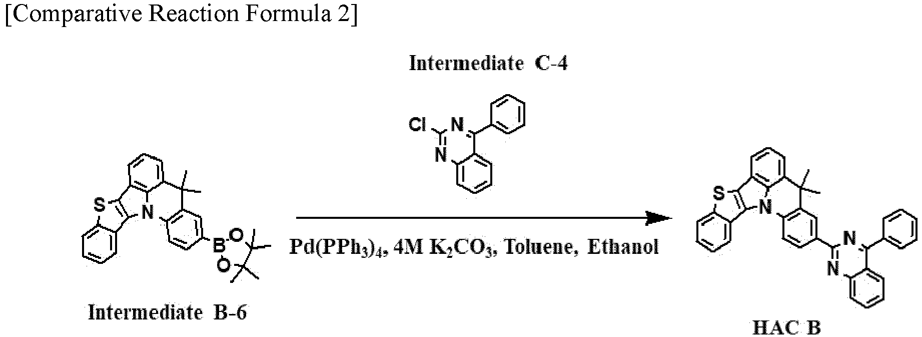

The Intermediate B-6 (5 g), the Intermediate C-4 (3.0 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC B (4.2 g, yield: 72%).

### Synthesis Example 11: Synthesis of Compound HAC41

### (1) Synthesis of Intermediate D-1

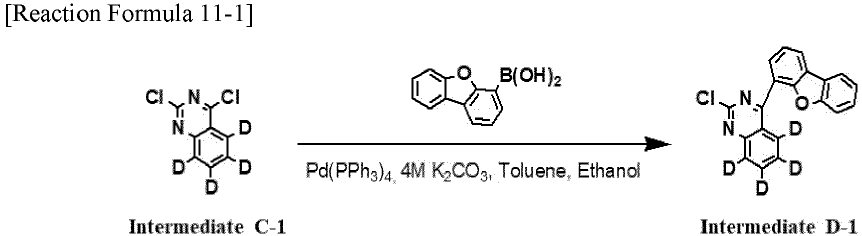

The Intermediate C-1 (10 g), 4-(Dibenzofuranyl)boronic acid (13 g), Pd(PPh₃)₄ (2.4 g), toluene (150 mL), ethanol (15 mL) and 4M K₂CO₃ aqueous solution (50 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate D-1 (11.8 g, yield: 72%).

### (2) Synthesis of Compound HAC41

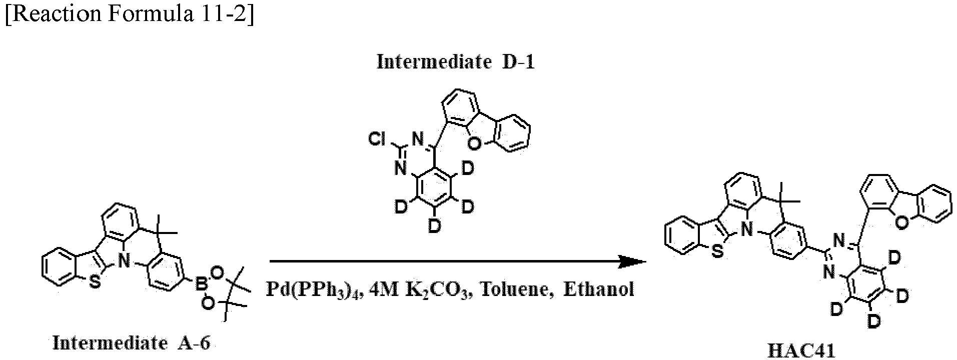

The Intermediate A-6 (5 g), the Intermediate D-1 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC41 (4.9 g, yield: 72%).

### Synthesis Example 12: Synthesis of Compound HAC42

### (1) Synthesis of Intermediate D-2

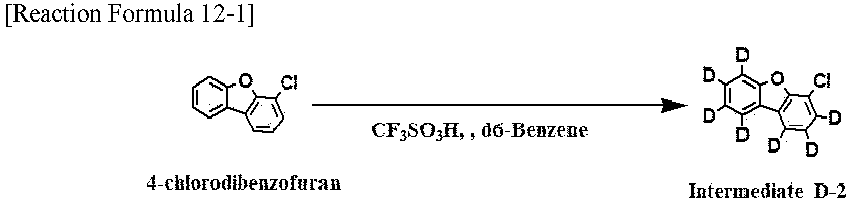

4-chlorodibenzofuran (10 g), triflic acid (37.0 g) and D6-benzene were added to a round bottom flask, and then the solution was refluxed with stirring at 70°C. After the reaction was completed, dichloromethane and distilled water were added to the solution to quench the reaction, and then the solution was separated into layers to recover an organic layer. The organic layer was distilled under reduced pressure and filtered through silica gel. The filtrate was concentrated again and re-crystallization was performed using methanol to give the Intermediate D-2 (9.8 g, yield: 95%).

### (2) Synthesis of Intermediate D-3

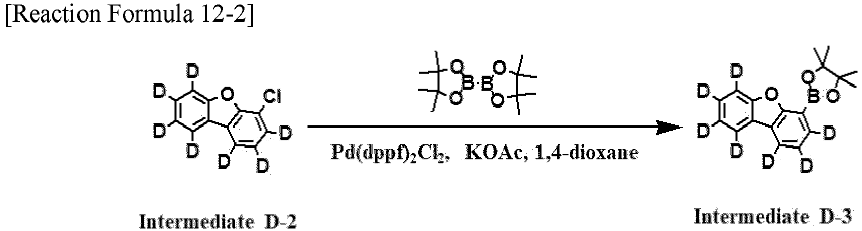

The Intermediate D-2 (9.5 g), Bis(pinacolato)diboron (17.3 g), potassium acetate (8.9 g), Pd(dppf)₂Cl₂ (0.7 g) and 1,4-dioxane (120 mL) were added to a round bottom flask, and then the solution was refluxed with stirring for 12 hours. After the reaction was completed, the reaction solution was cooled, filtered using Celite 545 under reduced pressure, and then Celite 545 was washed with chloroform. The filtrate was concentrated under reduced pressure and re-crystallization was performed using ethyl acetate to give the Intermediate D-3 (9.8 g, yield: 72%).

### (3) Synthesis of Intermediate D-4

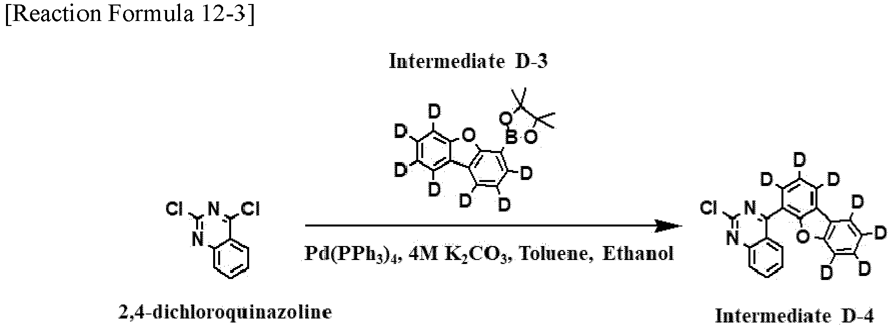

2,4-dichloroquinazoline (5.5 g), the Intermediate D-3 (9.2 g), Pd(PPh₃)₄ (0.6 g), toluene (100 mL), ethanol (10 mL) and 4M K₂CO₃ aqueous solution (13 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate D-4 (6.7 g, yield: 72%).

### (4) Synthesis of Compound HAC42

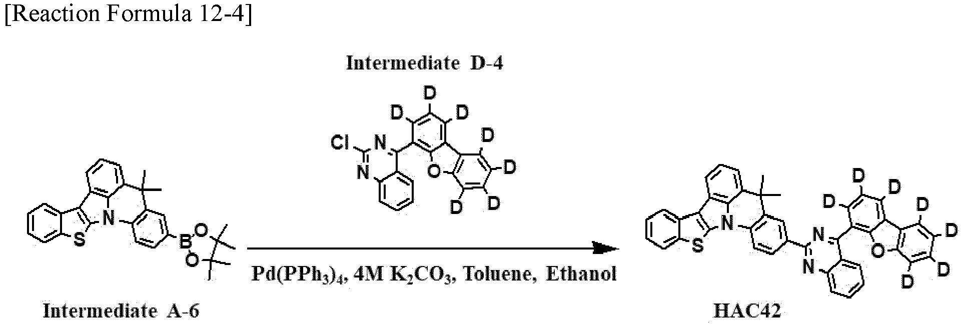

The Intermediate A-6 (5 g), the Intermediate D-4 (4.2 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC42 (4.8 g, yield: 72%).

### Synthesis Example 13: Synthesis of Compound HAC43

### (1) Synthesis of Intermediate D-5

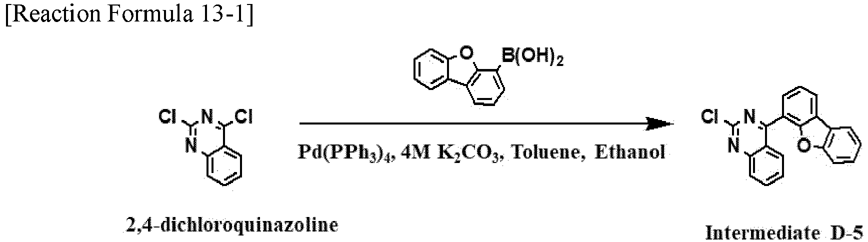

2,4-dichloroquinazoline (20 g), 4-(Dibenzofuranyl)boronic acid (25.6 g), Pd(PPh₃)₄ (2.3 g), toluene (150 mL), ethanol (15 mL) and 4M K₂CO₃ aqueous solution (50 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Intermediate D-5 (17.4 g, yield: 72%).

### (2) Synthesis of Intermediate D-6

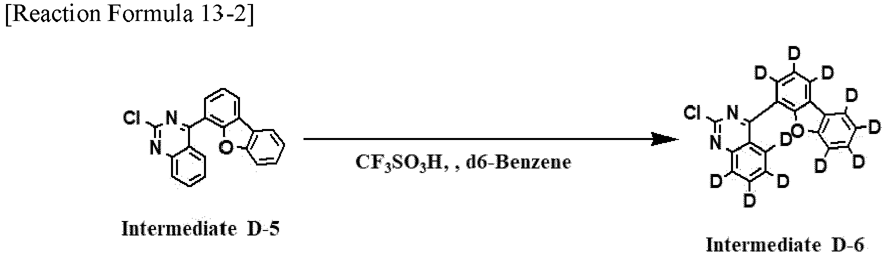

The Intermediate D-5 (10 g), triflic acid (22.7 g) and D6-benzene were added to a round bottom flask, and then the solution was refluxed with stirring at 70°C. After the reaction was completed, dichloromethane and distilled water were added to the solution to quench the reaction, and then the solution was separated into layers to recover an organic layer. The organic layer was distilled under reduced pressure and filtered through silica gel. The filtrate was concentrated again and re-crystallization was performed using methanol to give the Intermediate D-6 (9.8 g, yield: 95%).

### (3) Synthesis of Compound HAC43

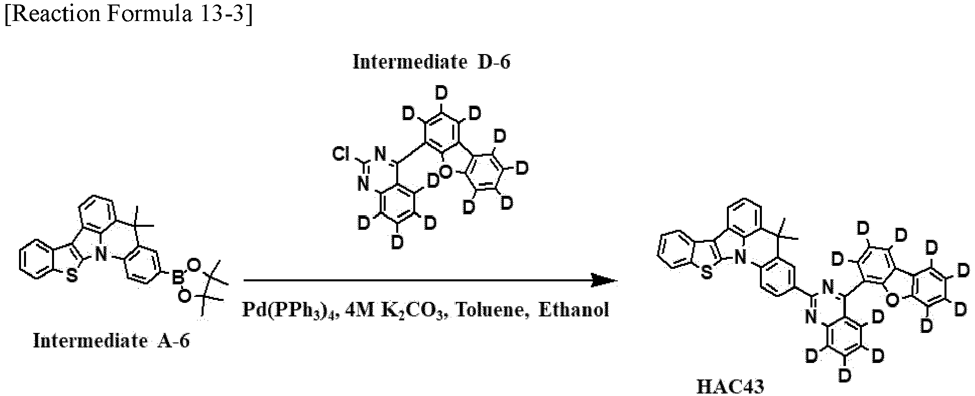

The Intermediate A-6 (5 g), the Intermediate D-6 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC43 (4.9 g, yield: 72%).

### Synthesis Example 14: Synthesis of Compound HAC448

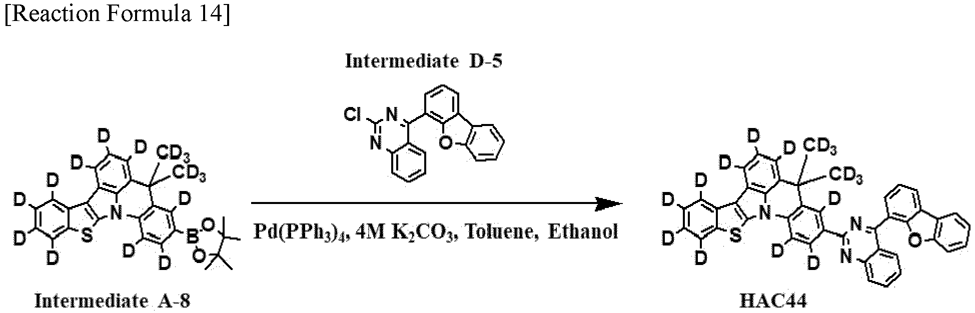

The Intermediate A-8 (5 g), the Intermediate D-5 (4.1 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC44 (4.9 g, yield: 72%).

### Synthesis Example 15: Synthesis of Compound HAC45

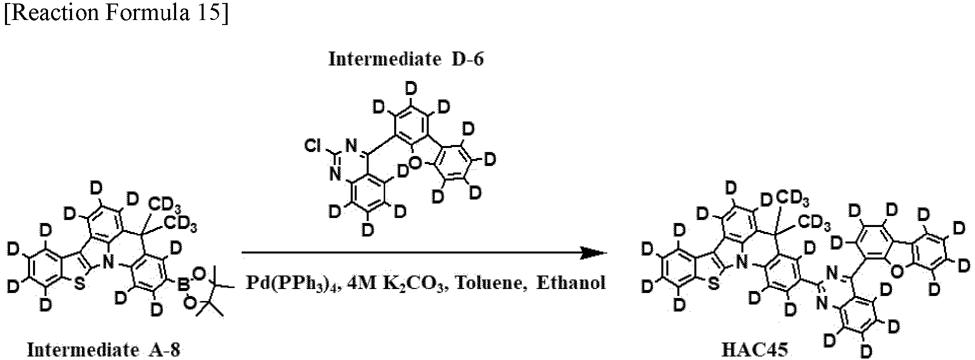

The Intermediate A-8 (5 g), the Intermediate D-6 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC45 (4.9 g, yield: 72%).

### Comparative Synthesis Example 3: Synthesis of Compound HAC C

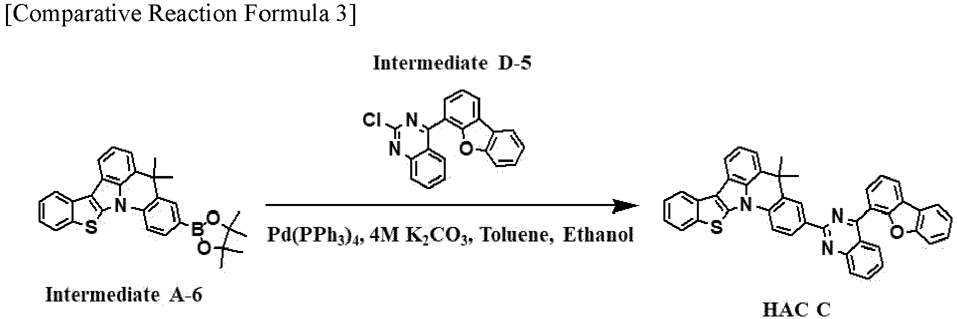

The Intermediate A-6 (5 g), the Intermediate D-5 (4.2 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC C (4.8 g, yield: 72%).

### Synthesis Example 16: Synthesis of Compound HAC336

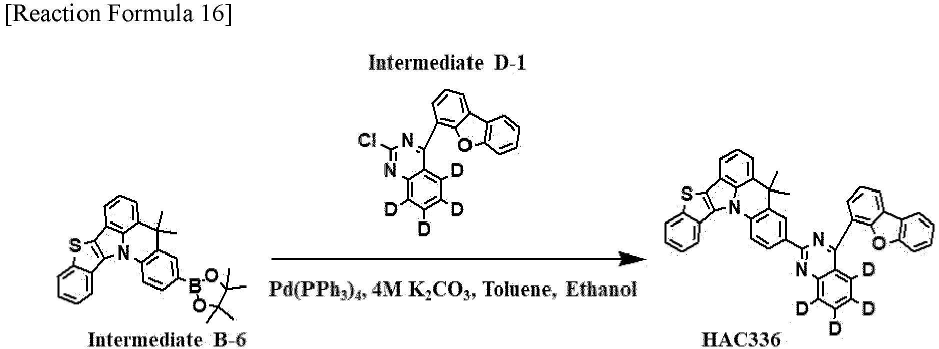

The Intermediate B-6 (5 g), the Intermediate D-1 (4.4 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC336 (5.0 g, yield: 72%).

### Synthesis Example 17: Synthesis of Compound HAC337

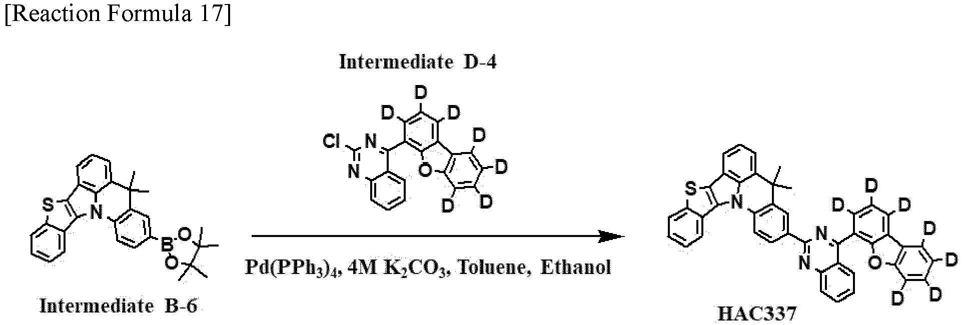

The Intermediate B-6 (5 g), the Intermediate D-4 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC337 (4.9 g, yield: 72%).

### Synthesis Example 18: Synthesis of Compound HAC338

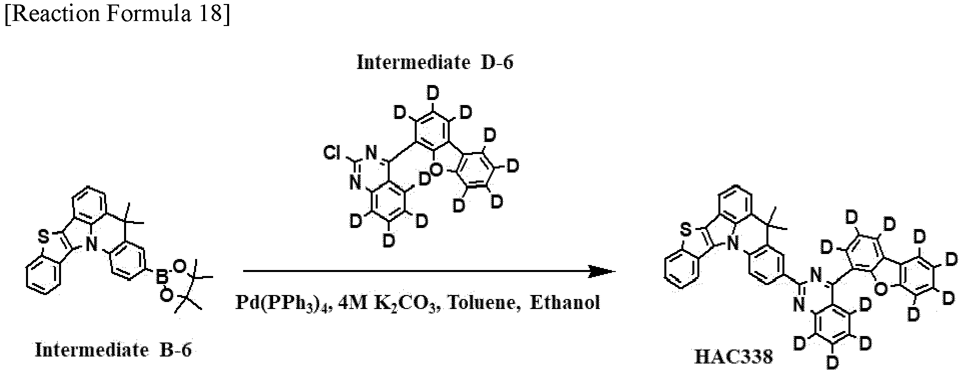

The Intermediate B-6 (5 g), the Intermediate D-6 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC338 (4.9 g, yield: 72%).

### Synthesis Example 19: Synthesis of Compound HAC339

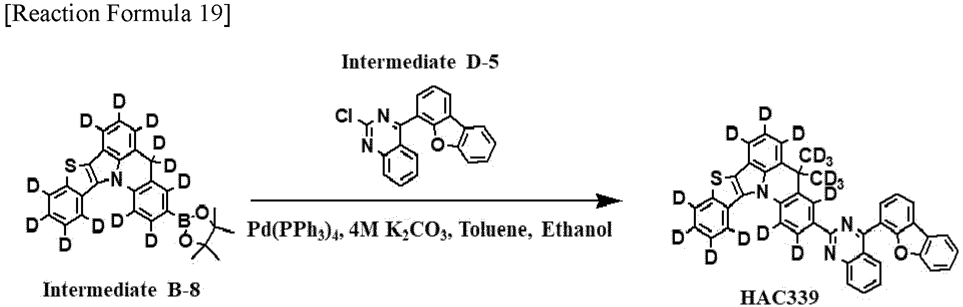

The Intermediate B-8 (5 g), the Intermediate D-5 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC339 (4.9 g, yield: 72%).

### Synthesis Example 20: Synthesis of Compound HAC340

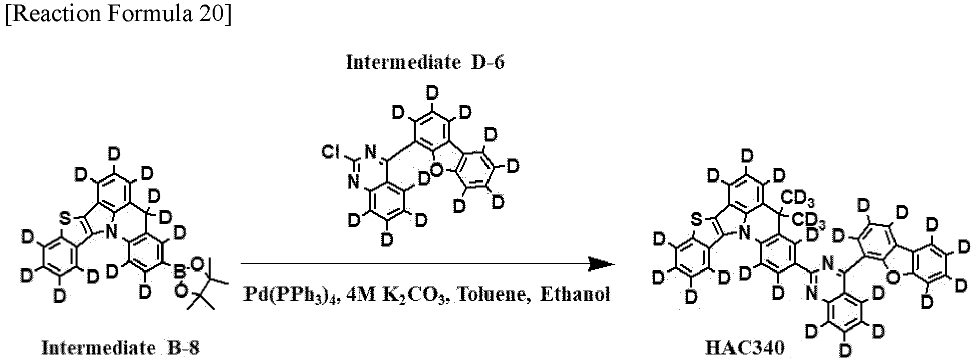

The Intermediate B-8 (5 g), the Intermediate D-6 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC340 (4.9 g, yield: 72%).

### Comparative Synthesis Example 4: Synthesis of Compound HAC D

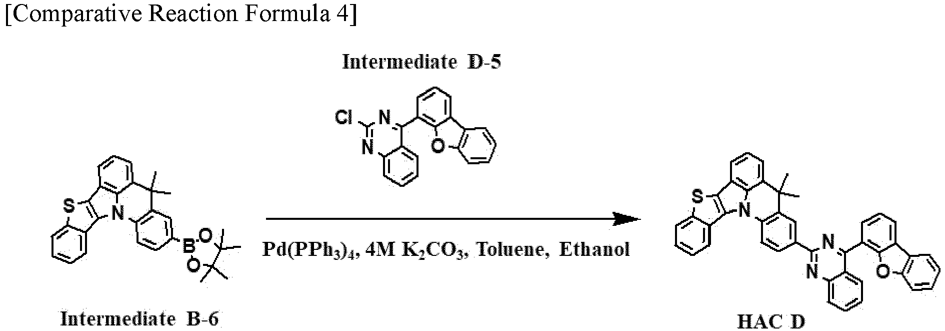

The Intermediate B-6 (5 g), the Intermediate D-5 (4.3 g), Pd(PPh₃)₄ (0.2 g), toluene (70 mL), ethanol (7 mL) and 4M K₂CO₃ aqueous solution (5 mL) were added to a round bottom flask, and then the solution was refluxed with stirring for 12 hours. After the reaction was completed, the reaction solution was separated into layers to recover an organic layer, and then the organic layer was filtered through silica gel to remove impurities. The solution was concentrated under reduced pressure, and the resulting crude product was separated by column to give the Compound HAC D (4.9 g, yield: 72%).

### Example 1 (Ex.1): Fabrication of OLED

An organic light emitting diode was fabricated by adding the Compound HAC1 synthesized in Synthesis Example 1 as the first host and Bis[2-(2-methylphenyl)-7-methylquinoline](acetylacetonate)iridium (III) (Ir(drmpq)₂(acac) as the dopant into an emitting material layer (EML). A glass substrate onto which ITO (1000-1500 Å thickness) was coated as a thin film was washed and ultrasonically cleaned by solvent such as isopropyl alcohol, acetone and dried at 100°C oven. The substrate was transferred to a vacuum chamber for depositing an emissive layer. Subsequently, an emissive layer and a cathode were deposited by evaporation from a heating boat under about 5-7 x 10⁻⁷ Torr in the following order:

A hole injection layer (HIL) (HAT-CN, 50 Å thickness); a hole transport layer (HTL) (NPB, 1000 Å thickness); an EML {HAC1 (90~99 wt%), Ir(drmpq)₂(acac) (1~10 wt%), 300~500 Å thickness]; an electron transport layer (ETL) (ZADN, 130~350 Å thickness); an electron injection layer (EIL) (LiF, 10∼30 Å thickness); and a cathode (Al, 1000 Å thickness).

The structures of the compounds used in the HIL, HTL, dopant and ETL are illustrated in the following:

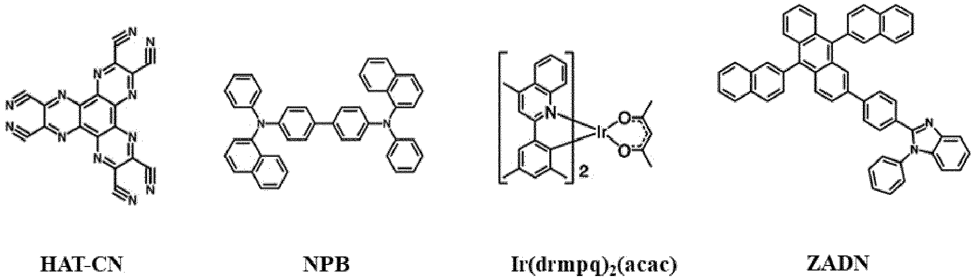

### Examples 2-20 (Ex. 2-20): Fabrication of OLED

An OLED was fabricated using the same procedure and the same material as Example 1, except that each of the Compounds HAC2-HAC5, HAC41-HAC45, HAC296-HAC300 and HAC336-HAC340 synthesized in Synthesis Examples 2-20 was used as the host in the EML for Examples 2-20, respectively, instead of the Compound HAC1.

### Comparative Examples 1-5 (Ref. 1-5): Fabrication of OLED

An OLED was fabricated using the same procedure and the same material as Example 1, except that each of the below 10,10'-[5-(-(6-[1,1'-biphenyl]-4-yl-2-phenyl-4-pyrimidinyl)-1,3-phenylene]bis[9,10-dihydro-9,9-dimethyl-acridine] (DMAC-BPP, for Ref. 1), HAC A (for Ref. 2), HAC B (for Ref. 3), HAC C (for Ref. 4) and HAC D (for Ref. 5) was used as the host in the EML instead of the Compound HAC1.

### [Host in Ref. 1]

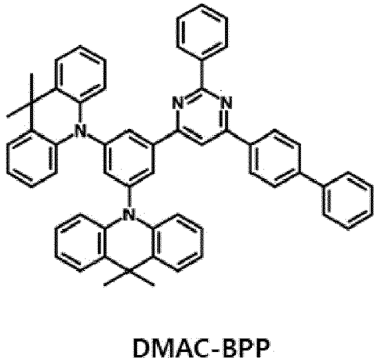

### Experimental Example 1: Measurement of Luminous Properties of OLEDs

Each of the OLEDs, having a 9 mm² of emission area, fabricated in Examples 1 to 20 and Comparative Examples 1 to 5 was connected to an external power source and then luminous properties for all the OLEDs were evaluated using a constant current source (KEITHLEY) and a photometer PR650 at room temperature. In particular, driving voltage (V, the difference based on the driving voltage measured in the device of Ref. 1), External quantum efficiency (EQE, a relative value compared to the EQE in the device of Ref. 1) and time period (LT₉₅, a relative value compared to the LT₉₅ in the device of Ref. 1) at which the luminance was reduced to 95% from initial luminance were measured at a current density of 10 mA/cm². The measurement results are indicated in the following Table 1.

**Table 1: Luminous Properties of OLED**

| Sample | HTL | EML Host | Voltage (ΔV) | EQE | LT₉₅ |
|---|---|---|---|---|---|
| Ref. 1 | NPB | DMAC-BPP | 0.00 | 100% | 100% |
| Ref. 2 | NPB | HAC A | -0.04 | 106% | 116% |
| Ref. 3 | NPB | HAC B | -0.05 | 106% | 116% |
| Ref. 4 | NPB | HAC C | -0.10 | 108% | 115% |
| Ref. 5 | NPB | HAC D | -0.10 | 107% | 115% |
| Ex. 1 | NPB | HAC1 | -0.04 | 106% | 117% |
| Ex. 2 | NPB | HAC2 | -0.04 | 106% | 116% |
| Ex. 3 | NPB | HAC3 | -0.02 | 105% | 126% |
| Ex. 4 | NPB | HAC4 | -0.04 | 106% | 141% |
| Ex. 5 | NPB | HAC5 | -0.01 | 104% | 144% |
| Ex. 6 | NPB | HAC41 | -0.09 | 108% | 116% |
| Ex. 7 | NPB | HAC42 | -0.10 | 108% | 115% |
| Ex. 8 | NPB | HAC43 | -0.08 | 107% | 123% |
| Ex. 9 | NPB | HAC44 | -0.10 | 108% | 140% |
| Ex. 10 | NPB | HAC45 | -0.07 | 106% | 143% |
| Ex. 116 | NPB | HAC296 | -0.04 | 106% | 117% |
| Ex. 12 | NPB | HAC297 | -0.05 | 106% | 116% |
| Ex. 13 | NPB | HAC298 | -0.03 | 105% | 123% |
| Ex. 14 | NPB | HAC299 | -0.05 | 106% | 140% |
| Ex. 15 | NPB | HAC300 | -0.02 | 104% | 144% |
| Ex. 16 | NPB | HAC336 | -0.09 | 107% | 116% |
| Ex. 17 | NPB | HAC337 | -0.10 | 107% | 115% |
| Ex. 18 | NPB | HAC338 | -0.08 | 106% | 123% |
| Ex. 19 | NPB | HAC339 | -0.10 | 107% | 141% |
| Ex. 20 | NPB | HAC340 | -0.07 | 105% | 145% |

### Example 21 (Ex. 21): Fabrication of OLED

An OLED was fabricated using the same procedure and the same material as Example 1, except that Compound H4 in Formula 9 was used as a hole transporting material in the HTL instead of NPB.

### Examples 22-40 (Ex. 22-40): Fabrication of OLED

An OLED was fabricated using the same procedure and the same material as Example 21, except that each of the Compounds HAC2-HAC5, HAC41-HAC45, HAC296-HAC300 and HAC336-HAC340 was used as the host in the EML for Examples 22-40, respectively, instead of the Compound HAC1.

### Comparative Examples 6-10 (Ref. 6-10): Fabrication of OLED

An OLED was fabricated using the same procedure and the same material as Example 21, except that each of DMAC-BPP (for Ref. 6), HAC A (for Ref. 7), HAC B (for Ref. 8), HAC C (for Ref. 9) and HAC D (for Ref. 10) was used as the host in the EML instead of the Compound HAC1.

### Experimental Example 2: Measurement of Luminous Properties OLED

Optical properties for each of the OLED fabricated in Examples 21 to 40 and Comparative Examples 6-10 were measured using the same procedures as Experimental Example 1. In particular, driving voltage (V, the difference based on the driving voltage measured in the device of Ref. 1), External quantum efficiency (EQE, a relative value compared to the EQE in the device of Ref. 1) and time period (LT₉₅, a relative value compared to the LT₉₅ in the device of Ref. 1) were measured at a current density of 10 mA/cm². The measurement results are indicated in the following Table 2.

**Table 2: Luminous Properties of OLED**

| Sample | HTL | EML Host | Voltage (ΔV) | EQE | LT₉₅ |
|---|---|---|---|---|---|
| Ref. 1 | NPB | DMAC-BPP | 0.00 | 100% | 100% |
| Ref. 6 | H4 | DMAC-BPP | -0.31 | 115% | 108% |
| Ref. 7 | H4 | HAC A | -0.32 | 119% | 122% |
| Ref. 8 | H4 | HAC B | -0.33 | 119% | 122% |
| Ref. 9 | H4 | HAC C | -0.38 | 120% | 121% |
| Ref. 19 | H4 | HAC D | -0.39 | 120% | 121% |
| Ex. 21 | H4 | HAC1 | -0.32 | 119% | 123% |
| Ex. 22 | H4 | HAC2 | -0.32 | 119% | 122% |
| Ex. 23 | H4 | HAC3 | -0.29 | 117% | 127% |
| Ex. 24 | H4 | HAC4 | -0.32 | 119% | 147% |
| Ex. 25 | H4 | HAC5 | -0.28 | 116% | 151% |
| Ex. 26 | H4 | HAC41 | -0.37 | 120% | 122% |
| Ex. 27 | H4 | HAC42 | -0.32 | 119% | 122% |
| Ex. 28 | H4 | HAC43 | -0.30 | 117% | 126% |
| Ex. 29 | H4 | HAC44 | -0.32 | 119% | 147% |
| Ex. 30 | H4 | HAC45 | -0.28 | 116% | 150% |
| Ex. 31 | H4 | HAC296 | -0.32 | 119% | 123% |
| Ex. 32 | H4 | HAC297 | -0.33 | 119% | 122% |
| Ex. 33 | H4 | HAC298 | -0.31 | 117% | 126% |
| Ex. 34 | H4 | HAC299 | -0.33 | 119% | 145% |
| Ex. 35 | H4 | HAC300 | -0.30 | 116% | 149% |
| Ex. 36 | H4 | HAC336 | -0.38 | 120% | 122% |
| Ex. 37 | H4 | HAC337 | -0.39 | 120% | 121% |
| Ex. 38 | H4 | HAC338 | -0.37 | 118% | 127% |
| Ex. 39 | H4 | HAC339 | -0.39 | 120% | 148% |
| Ex. 40 | H4 | HAC340 | -0.36 | 117% | 152% |

### Example 41 (Ex. 41): Fabrication of OLED

An OLED was fabricated using the same procedure and the same material as Example 1, except that Compound H14 in Formula 9 was used as a hole transporting material in the HTL instead of NPB.

### Examples 42-60 (Ex. 42-60): Fabrication of OLED

An OLED was fabricated using the same procedure and the same material as Example 41, except that each of the Compounds HAC2-HAC5, HAC41-HAC45, HAC296-HAC300 and HAC336-HAC340 was used as the host in the EML for Examples 42-60, respectively, instead of the Compound HAC1.

### Comparative Examples 11-15 (Ref. 11-15): Fabrication of OLED

An OLED was fabricated using the same procedure and the same material as Example 41, except that each of DMAC-BPP (for Ref. 11), HAC A (for Ref. 12), HAC B (for Ref. 13), HAC C (for Ref. 14) and HAC D (for Ref. 15) was used as the host in the EML instead of the Compound HAC1.

### Experimental Example 3: Measurement of Luminous Properties OLED

Optical properties for each of the OLED fabricated in Examples 41 to 60 and Comparative Examples 11-15 were measured using the same procedures as Experimental Example 1. In particular, driving voltage (V, the difference based on the driving voltage measured in the device of Ref. 1), External quantum efficiency (EQE, a relative value compared to the EQE in the device of Ref. 1) and time period (LT₉₅, a relative value compared to the LT₉₅ in the device of Ref. 1) were measured at a current density of 10 mA/cm². The measurement results are indicated in the following Table 3.

**Table 3: Luminous Properties of OLED**

| Sample | HTL | EML Host | Voltage (ΔV) | EQE | LT₉₅ |
|---|---|---|---|---|---|
| Ref. 1 | NPB | DMAC-BPP | 0.00 | 100% | 100% |
| Ref. 11 | H14 | DMAC-BPP | -0.17 | 108% | 112% |
| Ref. 12 | H14 | HAC A | -0.16 | 115% | 128% |
| Ref. 13 | H14 | HAC B | -0.15 | 115% | 128% |
| Ref. 14 | H14 | HAC C | -0.22 | 117% | 127% |
| Ref. 15 | H14 | HAC D | -0.20 | 116% | 127% |
| Ex. 41 | H14 | HAC1 | -0.14 | 115% | 129% |
| Ex. 42 | H14 | HAC2 | -0.16 | 115% | 129% |
| Ex. 43 | H14 | HAC3 | -0.14 | 114% | 136% |
| Ex. 44 | H14 | HAC4 | -0.16 | 115% | 147% |
| Ex. 45 | H14 | HAC5 | -0.13 | 113% | 151% |
| Ex. 46 | H14 | HAC41 | -0.20 | 117% | 128% |
| Ex. 47 | H14 | HAC42 | -0.22 | 117% | 127% |
| Ex. 48 | H14 | HAC43 | -0.20 | 116% | 135% |
| Ex. 49 | H14 | HAC44 | -0.22 | 117% | 152% |
| Ex. 50 | H14 | HAC45 | -0.19 | 115% | 157% |
| Ex. 51 | H14 | HAC296 | -0.14 | 115% | 129% |
| Ex. 52 | H14 | HAC297 | -0.15 | 115% | 128% |
| Ex. 53 | H14 | HAC298 | -0.13 | 114% | 134% |
| Ex. 54 | H14 | HAC299 | -0.15 | 115% | 153% |
| Ex. 55 | H14 | HAC300 | -0.12 | 113% | 157% |
| Ex. 56 | H14 | HAC336 | -0.19 | 116% | 128% |
| Ex. 57 | H14 | HAC337 | -0.20 | 116% | 127% |
| Ex. 58 | H14 | HAC338 | -0.18 | 115% | 135% |
| Ex. 59 | H14 | HAC339 | -0.20 | 116% | 152% |
| Ex. 60 | H14 | HAC340 | -0.17 | 114% | 156% |

### Example 61 (Ex. 61): Fabrication of OLED

An OLED was fabricated using the same procedure and the same material as Example 1, except that the Compound HAC1 as the first host (electron host, e-host) and NPB as the second host (hole host, h-host) was admixed with a ratio in a range of 3:7-7:3 by weight and the red dopant Ir(drmpq)₂(acac) was doped at an amount in a range of 1-10 wt% in the EML.

### Examples 62-80 (Ex. 62-80): Fabrication of OLED

An OLED was fabricated using the same procedure and the same material as Example 61, except that each of the Compounds HAC2-HAC5, HAC41-HAC45, HAC296-HAC300 and HAC336-HAC340 was used as the first host (e-host) in the EML for Examples 62-80, respectively, instead of the Compound HAC1.

### Comparative Examples 16-20 (Ref. 16-20): Fabrication of OLED

An OLED was fabricated using the same procedure and the same material as Example 61, except that each of the below 4,6-Bis(3,5-di(pyridine-3-yl)phenyl)-2-methylpyrimidine (B3PYMPM, for Ref. 16), HAC A (for Ref. 17), HAC B (for Ref. 18), HAC C (for Ref. 19) and HAC D (for Ref. 20) was used as the first host (e-host) in the EML instead of the Compound HAC1.

### [Second host in Ref. 16]

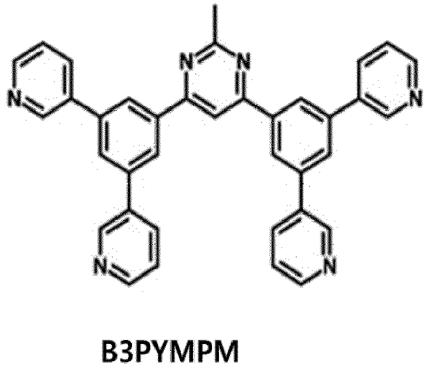

### Experimental Example 4: Measurement of Luminous Properties OLED

Optical properties for each of the OLED fabricated in Examples 61 to 80 and Comparative Examples 16-20 were measured using the same procedures as Experimental Example 1. In particular, driving voltage (V, the difference based on the driving voltage measured in the device of Ref. 16), External quantum efficiency (EQE, a relative value compared to the EQE in the device of Ref. 16) and time period (LT₉₅, a relative value compared to the LT₉₅ in the device of Ref. 16) were measured at a current density of 10 mA/cm². The measurement results are indicated in the following Table 4.

**Table 4: Luminous Properties of OLED**

| Sample | HTL | EML h-Host | EML e-host | Voltage (ΔV) | EQE | LT₉₅ |
|---|---|---|---|---|---|---|
| Ref. 16 | NPB | NPB | B3PYMPM | 0.00 | 100% | 100% |
| Ref. 17 | NPB | NPB | HAC A | -0.06 | 108% | 120% |
| Ref. 18 | NPB | NPB | HAC B | -0.04 | 108% | 118% |
| Ref. 19 | NPB | NPB | HAC C | -0.11 | 110% | 116% |
| Ref. 20 | NPB | NPB | HAC D | -0.10 | 110% | 114% |
| Ex. 61 | NPB | NPB | HAC1 | -0.06 | 108% | 120% |
| Ex. 62 | NPB | NPB | HAC2 | -0.06 | 108% | 121% |
| Ex. 63 | NPB | NPB | HAC3 | -0.05 | 107% | 128% |
| Ex. 64 | NPB | NPB | HAC4 | -0.06 | 108% | 146% |
| Ex. 65 | NPB | NPB | HAC5 | -0.05 | 107% | 149% |
| Ex. 66 | NPB | NPB | HAC296 | -0.04 | 108% | 118% |
| Ex. 67 | NPB | NPB | HAC297 | -0.04 | 108% | 119% |
| Ex. 68 | NPB | NPB | HAC298 | -0.03 | 107% | 127% |
| Ex. 69 | NPB | NPB | HAC299 | -0.04 | 108% | 144% |
| Ex. 70 | NPB | NPB | HAC300 | -0.02 | 106% | 147% |
| Ex. 71 | NPB | NPB | HAC41 | -0.11 | 110% | 116% |
| Ex. 72 | NPB | NPB | HAC42 | -0.11 | 110% | 118% |
| Ex. 73 | NPB | NPB | HAC43 | -0.10 | 109% | 125% |
| Ex. 74 | NPB | NPB | HAC44 | -0.11 | 110% | 144% |
| Ex. 75 | NPB | NPB | HAC45 | -0.09 | 108% | 149% |
| Ex. 76 | NPB | NPB | HAC336 | -0.10 | 110% | 114% |
| Ex. 77 | NPB | NPB | HAC337 | -0.10 | 110% | 115% |
| Ex. 78 | NPB | NPB | HAC338 | -0.09 | 108% | 123% |
| Ex. 79 | NPB | NPB | HAC339 | -0.10 | 110% | 139% |
| Ex. 80 | NPB | NPB | HAC340 | -0.08 | 107% | 142% |

### Example 81 (Ex. 81): Fabrication of OLED

An OLED was fabricated using the same procedure and the same material as Example 61, except that Compound H4 in Formula 9 was used as a hole transporting material in the HTL and as a second host in the EML instead of NPB.

### Examples 82-100 (Ex. 82-100): Fabrication of OLED

An OLED was fabricated using the same procedure and the same material as Example 81, except that each of the Compounds HAC2-HAC5, HAC41-HAC45, HAC296-HAC300 and HAC336-HAC340 was used as the host in the EML for Examples 82-100, respectively, instead of the Compound HAC1.

### Comparative Examples 21-25 (Ref. 21-25): Fabrication of OLED

An OLED was fabricated using the same procedure and the same material as Example 81, except that each of B3PYMPM (for Ref. 21), HAC A (for Ref. 22), HAC B (for Ref. 23), HAC C (for Ref. 24) and HAC D (for Ref. 25) was used as the host in the EML instead of the Compound HAC1.

### Experimental Example 5: Measurement of Luminous Properties OLED

Optical properties for each of the OLED fabricated in Examples 81 to 100 and Comparative Examples 21-25 were measured using the same procedures as Experimental Example 1. In particular, driving voltage (V, the difference based on the driving voltage measured in the device of Ref. 16), External quantum efficiency (EQE, a relative value compared to the EQE in the device of Ref. 16) and time period (LT₉₅, a relative value compared to the LT₉₅ in the device of Ref. 16) were measured at a current density of 10 mA/cm². The measurement results are indicated in the following Table 5.

**Table 5: Luminous Properties of OLED**

| Sample | HTL | EML h-Host | EML e-host | Voltage (ΔV) | EQE | LT₉₅ |
|---|---|---|---|---|---|---|
| Ref. 16 | NPB | NPB | B3PYMPM | 0.00 | 100% | 100% |
| Ref. 21 | H4 | H4 | B3PYMPM | -0.32 | 117% | 113% |
| Ref. 22 | H4 | H4 | HAC A | -0.40 | 125% | 134% |
| Ref. 23 | H4 | H4 | HAC B | -0.38 | 126% | 132% |
| Ref. 24 | H4 | H4 | HAC C | -0.46 | 127% | 130% |
| Ref. 25 | H4 | H4 | HAC D | -0.49 | 128% | 128% |
| Ex. 81 | H4 | H4 | HAC1 | -0.40 | 125% | 134% |
| Ex. 82 | H4 | H4 | HAC2 | -0.40 | 125% | 135% |
| Ex. 83 | H4 | H4 | HAC3 | -0.38 | 124% | 143% |
| Ex. 84 | H4 | H4 | HAC4 | -0.40 | 125% | 161% |
| Ex. 85 | H4 | H4 | HAC5 | -0.36 | 123% | 165% |
| Ex. 86 | H4 | H4 | HAC41 | -0.46 | 127% | 130% |
| Ex. 87 | H4 | H4 | HAC42 | -0.46 | 127% | 130% |
| Ex. 88 | H4 | H4 | HAC43 | -0.44 | 125% | 138% |
| Ex. 89 | H4 | H4 | HAC44 | -0.46 | 127% | 155% |
| Ex. 90 | H4 | H4 | HAC45 | -0.42 | 124% | 159% |
| Ex. 91 | H4 | H4 | HAC296 | -0.38 | 126% | 132% |
| Ex. 92 | H4 | H4 | HAC297 | -0.38 | 126% | 133% |
| Ex. 93 | H4 | H4 | HAC298 | -0.37 | 125% | 141% |
| Ex. 94 | H4 | H4 | HAC299 | -0.38 | 126% | 157% |
| Ex. 95 | H4 | H4 | HAC300 | -0.36 | 124% | 161% |
| Ex. 96 | H4 | H4 | HAC336 | -0.49 | 128% | 128% |
| Ex. 97 | H4 | H4 | HAC337 | -0.49 | 128% | 129% |
| Ex. 98 | H4 | H4 | HAC338 | -0.48 | 127% | 138% |
| Ex. 99 | H4 | H4 | HAC339 | -0.49 | 128% | 165% |
| Ex. 100 | H4 | H4 | HAC340 | -0.47 | 126% | 171% |

### Example 101 (Ex. 101): Fabrication of OLED

An OLED was fabricated using the same procedure and the same material as Example 61, except that Compound H14 in Formula 9 was used as a hole transporting material in the HTL and as a second host in the EML instead of NPB.

### Examples 102-120 (Ex. 102-120): Fabrication of OLED

An OLED was fabricated using the same procedure and the same material as Example 101, except that each of the Compounds HAC2-HAC5, HAC41-HAC45, HAC296-HAC300 and HAC336-HAC340 was used as the host in the EML for Examples 102-120, respectively, instead of the Compound HAC 1.

### Comparative Examples 26-30 (Ref. 26-30): Fabrication of OLED

An OLED was fabricated using the same procedure and the same material as Example 101, except that each of B3PYMPM (for Ref. 26), HAC A (for Ref. 27), HAC B (for Ref. 28), HAC C (for Ref. 29) and HAC D (for Ref. 30) was used as the host in the EML instead of the Compound HAC1.

### Experimental Example 6: Measurement of Luminous Properties OLED

Optical properties for each of the OLED fabricated in Examples 101 to 120 and Comparative Examples 26-30 were measured using the same procedures as Experimental Example 1. In particular, driving voltage (V, the difference based on the driving voltage measured in the device of Ref. 16), External quantum efficiency (EQE, a relative value compared to the EQE in the device of Ref. 16) and time period (LT₉₅, a relative value compared to the LT₉₅ in the device of Ref. 16) were measured at a current density of 10 mA/cm². The measurement results are indicated in the following Table 6.

**Table 6: Luminous Properties of OLED**

| Sample | HTL | EML h-Host | EML e-host | Voltage (ΔV) | EQE) | LT₉₅ |
|---|---|---|---|---|---|---|
| Ref. 16 | NPB | NPB | B3PYMPM | 0.00 | 100% | 100% |
| Ref. 26 | H14 | H14 | B3PYMPM | -0.19 | 110% | 123% |
| Ref. 27 | H14 | H14 | HAC A | -0.25 | 118% | 142% |
| Ref. 28 | H14 | H14 | HAC B | -0.23 | 118% | 140% |
| Ref. 29 | H14 | H14 | HAC C | -0.30 | 120% | 138% |
| Ref. 30 | H14 | H14 | HAC D | -0.29 | 120% | 136% |
| Ex. 101 | H14 | H14 | HAC1 | -0.25 | 118% | 142% |
| Ex. 102 | H14 | H14 | HAC2 | -0.25 | 118% | 144% |
| Ex. 103 | H14 | H14 | HAC3 | -0.23 | 117% | 151% |
| Ex. 104 | H14 | H14 | HAC4 | -0.25 | 118% | 171% |
| Ex. 105 | H14 | H14 | HAC5 | -0.22 | 115% | 175% |
| Ex. 106 | H14 | H14 | HAC41 | -0.30 | 120% | 138% |
| Ex. 107 | H14 | H14 | HAC42 | -0.30 | 120% | 139% |
| Ex. 108 | H14 | H14 | HAC43 | -0.28 | 118% | 144% |
| Ex. 109 | H14 | H14 | HAC44 | -0.30 | 120% | 174% |
| Ex. 110 | H14 | H14 | HAC45 | -0.29 | 118% | 178% |
| Ex. 111 | H14 | H14 | HAC296 | -0.23 | 118% | 140% |
| Ex. 112 | H14 | H14 | HAC297 | -0.23 | 118% | 141% |
| Ex. 113 | H14 | H14 | HAC298 | -0.21 | 117% | 149% |
| Ex. 114 | H14 | H14 | HAC299 | -0.23 | 118% | 165% |
| Ex. 115 | H14 | H14 | HAC300 | -0.20 | 116% | 169% |
| Ex. 116 | H14 | H14 | HAC336 | -0.29 | 120% | 136% |
| Ex. 117 | H14 | H14 | HAC337 | -0.29 | 120% | 138% |
| Ex. 118 | H14 | H14 | HAC338 | -0.27 | 119% | 146% |
| Ex. 119 | H14 | H14 | HAC339 | -0.29 | 120% | 165% |
| Ex. 120 | H14 | H14 | HAC340 | -0.26 | 118% | 170% |

### Example 121 (Ex. 121): Fabrication of OLED

An OLED was fabricated using the same procedure and the same material as Example 61, except that except that Compound H4 in Formula 9 was used as a hole transporting material in the HTL instead of NPB and Compound H14 in Formula 14 was used as the second host in the EML instead of NPB.

### Examples 122-140 (Ex. 122-140): Fabrication of OLED

An OLED was fabricated using the same procedure and the same material as Example 121, except that each of the Compounds HAC2-HAC5, HAC41-HAC45, HAC296-HAC300 and HAC336-HAC340 was used as the host in the EML for Examples 122-140, respectively, instead of the Compound HAC 1.

### Comparative Examples 31-35 (Ref. 31-34): Fabrication of OLED

An OLED was fabricated using the same procedure and the same material as Example 121, except that each HAC A (for Ref. 31), HAC B (for Ref. 32), HAC C (for Ref. 33) and HAC D (for Ref. 34) was used as the host in the EML instead of the Compound HAC1.

### Experimental Example 7: Measurement of Luminous Properties OLED

Optical properties for each of the OLED fabricated in Examples 121 to 140 and Comparative Examples 31-34 were measured using the same procedures as Experimental Example 1. In particular, driving voltage (V, the difference based on the driving voltage measured in the device of Ref. 16), External quantum efficiency (EQE, a relative value compared to the EQE in the device of Ref. 16) and time period (LT₉₅, a relative value compared to the LT₉₅ in the device of Ref. 16) were measured at a current density of 10 mA/cm². The measurement results are indicated in the following Table 7.

**Table 7: Luminous Properties of OLED**

| Sample | HTL | EML h-Host | EML e-host | Voltage (ΔV) | EQE | LT₉₅ |
|---|---|---|---|---|---|---|
| Ref. 16 | NPB | NPB | B3PYMPM | 0.00 | 100% | 100% |
| Ref. 31 | H4 | H14 | HAC A | -0.32 | 122% | 137% |
| Ref. 32 | H4 | H14 | HAC B | -0.30 | 124% | 136% |
| Ref. 33 | H4 | H14 | HAC C | -0.38 | 123% | 134% |
| Ref. 34 | H4 | H14 | HAC D | -0.39 | 124% | 134% |
| Ex. 121 | H4 | H14 | HAC1 | -0.32 | 122% | 137% |
| Ex. 122 | H4 | H14 | HAC2 | -0.32 | 122% | 139% |
| Ex. 123 | H4 | H14 | HAC3 | -0.30 | 121% | 146% |
| Ex. 124 | H4 | H14 | HAC4 | -0.32 | 122% | 171% |
| Ex. 125 | H4 | H14 | HAC5 | -0.29 | 120% | 174% |
| Ex. 126 | H4 | H14 | HAC41 | -0.38 | 123% | 134% |
| Ex. 127 | H4 | H14 | HAC42 | -0.38 | 123% | 136% |
| Ex. 128 | H4 | H14 | HAC43 | -0.36 | 122% | 142% |
| Ex. 129 | H4 | H14 | HAC44 | -0.38 | 123% | 161% |
| Ex. 130 | H4 | H14 | HAC45 | -0.35 | 121% | 165% |
| Ex. 131 | H4 | H14 | HAC296 | -0.30 | 124% | 136% |
| Ex. 132 | H4 | H14 | HAC297 | -0.30 | 124% | 136% |
| Ex. 133 | H4 | H14 | HAC298 | -0.28 | 123% | 142% |
| Ex. 134 | H4 | H14 | HAC299 | -0.30 | 124% | 161% |
| Ex. 135 | H4 | H14 | HAC300 | -0.27 | 122% | 168% |
| Ex. 136 | H4 | H14 | HAC336 | -0.39 | 124% | 134% |
| Ex. 137 | H4 | H14 | HAC337 | -0.39 | 124% | 134% |
| Ex. 138 | H4 | H14 | HAC338 | -0.37 | 123% | 139% |
| Ex. 139 | H4 | H14 | HAC339 | -0.39 | 124% | 158% |
| Ex. 140 | H4 | H14 | HAC340 | -0.36 | 122% | 161% |

In summary, as indicated in Tables 1-7, compared to the OLEDs in which DMAC-BPP or B3PYMPM was used as the host in the EML, the OLEDs fabricated in Examples in which organic compounds substituted with deuterium at least one partial moiety were used as the host in the EML had reduced driving voltage and improved luminous efficiency as well as the luminous lifespan. In addition, when the spiro-bifluorene-based organic compounds in Formula 9 were added to an emissive layer as a hole transporting material in the HTL and/or a hole host in the EML, the driving voltage was further reduced and the luminous efficiency as well as the luminous lifespan was greatly enhanced. Particularly, when the organic compound with relatively high deuterium substitution ratio was used, the luminous properties of the OLED were extremely improved.

Various embodiments provide an organic compound having a structure represented by any of Formulae 1 to 4 described herein above.

In one or more embodiments, at least one of R¹ to R⁷ is a deuterium, a deuterium-substituted C₁-C₂₀ alkyl, a deuterium-substituted C₃-C₂₀ cycloalkyl, a deuterium-substituted C₃-C₂₀ heterocycloalkyl, a deuterium-substituted C₆-C₃₀ aryl, or a deuterium-substituted C₃-C₃₀ heteroaryl.

In one or more embodiments, the organic compound includes at least one of the organic compounds HAC1 to HAC590 described herein above.

Various embodiments provide an organic light emitting diode, including: a first electrode; a second electrode facing the first electrode; and an emissive layer disposed between the first and second electrodes; wherein the emissive layer includes an organic compound having a structure represented by Formula 1 described herein above.

In one or more embodiments, the organic compound has a structure represented by Formula 2 described herein above.

In one or more embodiments, the organic compound has a structure represented by Formula 3 or 4 described herein above.

In one or more embodiments, at least one of R¹ to R⁷ is a deuterium, a deuterium-substituted C₁-C₂₀ alkyl, a deuterium-substituted C₃-C₂₀ cycloalkyl, a deuterium-substituted C₃-C₂₀ heterocycloalkyl, a deuterium-substituted C₆-C₃₀ aryl, or a deuterium-substituted C₃-C₃₀ heteroaryl.

In one or more embodiments, the emissive layer includes at least one emitting material layer, and wherein the at least one emitting material layer includes the organic compound.

In one or more embodiments, the at least one emitting material layer further includes a spiro-bifluorene-based organic compound having a structure represented by Formula 6 described herein above.

In one or more embodiments, the spiro-bifluorene-based organic compound has a structure represented by Formula 7 or Formula 8 described herein above.

In one or more embodiments, the spiro-bifluorene-based organic compound includes at least one of the spiro-bifluorene-based organic compounds H1 to H22 described herein above.

In one or more embodiments, the at least one emitting material layer includes a dopant.

In one or more embodiments, the dopant includes a red dopant.

In one or more embodiments, the emissive layer further includes at least one hole transport layer disposed between the first electrode and the at least one emitting material layer.

In one or more embodiments, the at least one hole transport layer includes a spiro-bifluorene-based organic compound having a structure represented by Formula 6 described herein above.

In one or more embodiments, the spiro-bifluorene-based organic compound has a structure represented by Formula 7 or Formula 8 described herein above.

In one or more embodiments, the spiro-bifluorene-based organic compound includes at least one of the spiro-bifluorene-based organic compounds H1 to H22 described herein above.

In one or more embodiments, the emissive layer includes: a first emitting part disposed between the first and second electrodes and includes a first emitting material layer; a second emitting part disposed between the first emitting part and the second electrode and includes a second emitting material layer; and a first charge generation layer disposed between the first emitting part and the second emitting part, wherein at least one of the first emitting material layer and the second emitting material layer includes the organic compound.

In one or more embodiments, the second emitting material layer includes: a first layer disposed between the first charge generation layer and the second electrode; and a second layer disposed between the first layer and the second electrode, wherein at least one of the first layer and the second layer includes the organic compound.

In one or more embodiments, the second emitting material layer further includes a third layer disposed between the first layer and the second layer.

In one or more embodiments, the emissive layer further includes: a third emitting part disposed between the second emitting part and the second electrode and includes a third emitting material layer, and a second charge generation layer disposed between the second emitting part and the third emitting part.

In one or more embodiments, the second emitting material layer includes: a first layer disposed between the first charge generation layer and the second electrode, and a second layer disposed between the first layer and the second electrode, wherein at least one of the first layer and the second layer includes the organic compound.

In one or more embodiments, the second emitting material layer further includes a third layer disposed between the first layer and the second layer.

Various embodiments provide an organic light emitting diode, including: a first electrode; a second electrode facing the first electrode; and an emissive layer disposed between the first and second electrodes and including: a first emitting part disposed between the first and second electrodes and including a blue emitting material layer; a second emitting part disposed between the first emitting part and the second electrode and including at least one emitting material layer; and a first charge generation layer disposed between the first emitting part and the second emitting part, wherein the at least one emitting material layer includes an organic compound having a structure represented by Formula 1 described herein above.

In one or more embodiments, the organic compound has a structure represented by Formula 2 described herein above.

In one or more embodiments, the organic compound has a structure represented by Formula 3 or 4 described herein above.

In one or more embodiments, at least one of R¹ to R⁷ is a deuterium, a deuterium-substituted C₁-C₂₀ alkyl, a deuterium-substituted C₃-C₂₀ cycloalkyl, a deuterium-substituted C₃-C₂₀ cycloalkyl, a deuterium-substituted C₆-C₃₀ aryl, or a deuterium-substituted C₃-C₃₀ heteroaryl.

In one or more embodiments, the at least one emitting material layer includes: a first layer disposed between the first charge generation layer and the second electrode, and a second layer disposed between the first layer and the second electrode, wherein at least one of the first layer and the second layer includes the organic compound.

In one or more embodiments, the first layer includes the organic compound and a red dopant, and the second layer is a green emitting material layer.

In one or more embodiments, the first layer further includes a spiro-bifluorene-based organic compound having a structure represented by Formula 6 described herein above.

In one or more embodiments, the spiro-bifluorene-based organic compound has a structure represented by Formula 7 or Formula 8 described herein above.

In one or more embodiments, the at least one emitting material layer further includes a third layer disposed between the first layer and the second layer, and wherein the third layer is a yellow green emitting material layer.

In one or more embodiments, the second emitting part further includes a hole transport layer disposed between the first charge generation layer and the at least one emitting material layer, and wherein the hole transport layer includes a spiro-bifluorene-based organic compound having a structure represented by Formula 6 described herein above.

In one or more embodiments, the spiro-bifluorene-based organic compound has a structure represented by Formula 7 or Formula 8 described herein above.

In one or more embodiments, the emissive layer further includes: a third emitting part disposed between the second emitting part and the second electrode and including a blue emitting material layer; and a second charge generation layer disposed between the second emitting part and the third emitting part.

In one or more embodiments, the at least one emitting material layer includes: a first layer disposed between the first charge generation layer and the second electrode; and a second layer disposed between the first layer and the second electrode, wherein at least one of the first layer and the second layer includes the organic compound.

In one or more embodiments, the first layer includes the organic compound and a red dopant, and the second layer is a green emitting material layer.

Various embodiments provide an organic light emitting device, including: a substrate and the organic light emitting diode according to one or more embodiments described herein disposed on the substrate.

It will be apparent to those skilled in the art that various modifications and variations can be made in the present disclosure without departing from the scope of the disclosure. Thus, it is intended that the present disclosure cover the modifications and variations of the present disclosure provided they come within the scope of the appended claims.

## Claims

1. An organic compound having a structure represented by Formula 1: 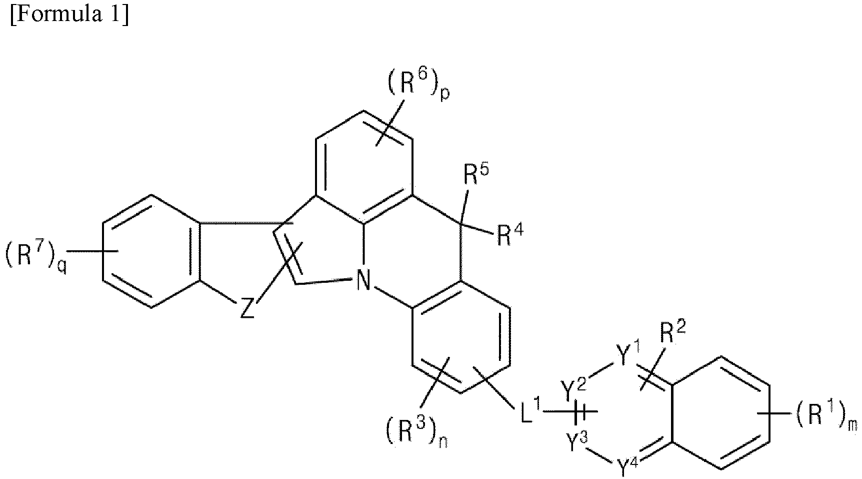
wherein in the Formula 1,
two of Y¹ to Y⁴ are nitrogen atoms, one of Y¹ to Y⁴ is a carbon atom linked to L¹, and the other of Y¹ to Y⁴ is CR²;
each of R¹ to R⁷ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
at least one of R¹ to R⁷ is a deuterium, a deuterium-substituted C₁-C₂₀ alkyl, a deuterium-substituted C₁-C₂₀ heteroalkyl, a deuterium-substituted C₂-C₂₀ alkenyl, a deuterium-substituted C₂-C₂₀ heteroalkenyl, a deuterium-substituted C₁-C₂₀ alkoxy, a deuterium-substituted C₁-C₂₀ alkyl amino, a deuterium-substituted C₃-C₂₀ alicyclic group, a deuterium-substituted C₃-C₂₀ heteroalicyclic group, a deuterium-substituted C₆-C₃₀ aromatic group, or a deuterium-substituted C₃-C₃₀ heteroaromatic group,
each R¹ is identical to or different from each other when m is 2, 3 or 4, each R³ is identical to or different from each other when n is 2 or 3, each R⁶ is identical to or different from each other when p is 2 or 3, and each R⁷ is identical to or different from each other when q is 2, 3 or 4,
each of m and q is 4 and each of n and p is 3 when each of R¹, R³, R⁶, and R⁷ is a protium,
each of m and q is independently 1, 2, 3 or 4 and each of n and p is independently 1, 2 or 3 when each of R¹, R³, R⁶, and R⁷ is not a protium;
Z is O or S; and
L¹ is a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring.

2. The organic compound of claim 1, wherein the organic compound has a structure represented by Formula 2: 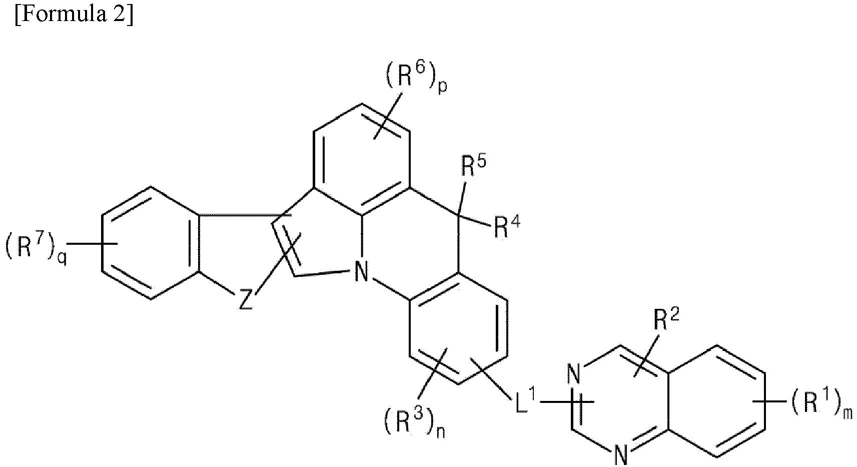
wherein in the Formula 2,
each of R¹ to R⁷, Z, L¹, m, n, p, and q is as defined in the Formula 1.

3. The organic compound of claim 1, wherein the organic compound has a structure represented by Formula 3 or Formula 4: 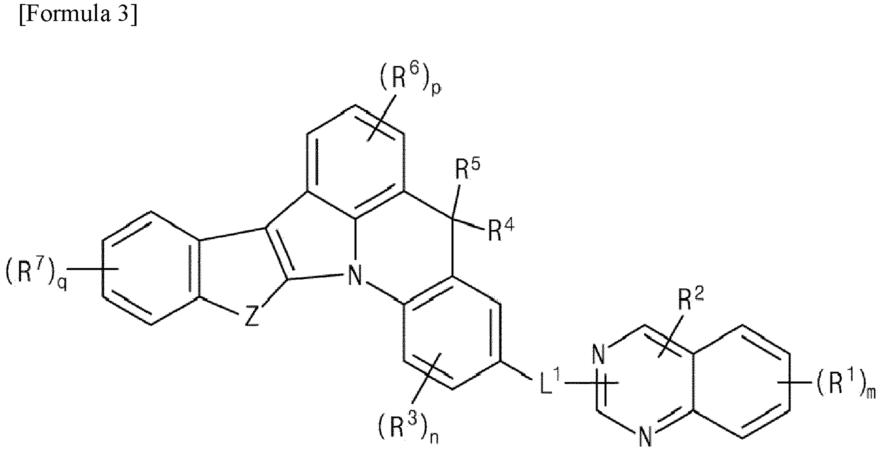 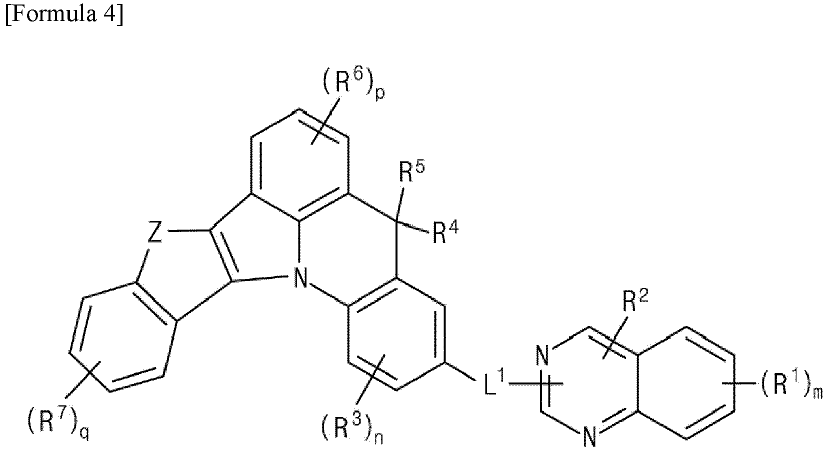
wherein in the Formulae 3 and 4,
each of R¹ to R⁷, Z, L¹, m, n, p, and q is as defined in the Formula 1.

4. The organic compound of any of claims 1 to 3, wherein at least one of R¹ to R⁷ is a deuterium, a deuterium-substituted C₁-C₂₀ alkyl, a deuterium-substituted C₃-C₂₀ cycloalkyl, a deuterium-substituted C₃-C₂₀ heterocycloalkyl, a deuterium-substituted C₆-C₃₀ aryl, or a deuterium-substituted C₃-C₃₀ heteroaryl.

5. The organic compound of claim 1, wherein the organic compound includes at least one of the following organic compounds:

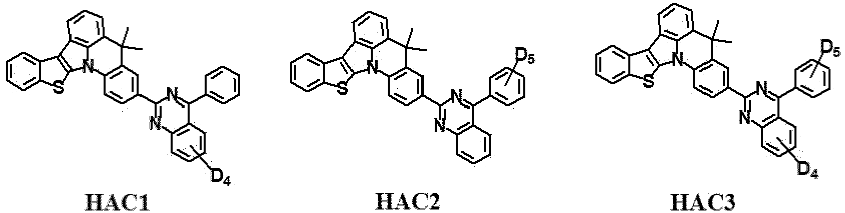

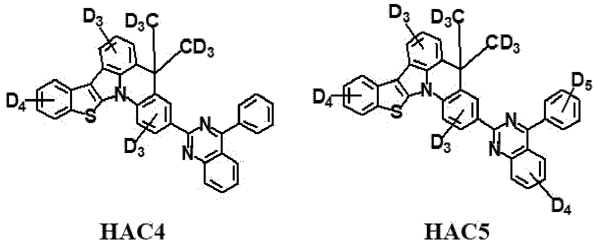

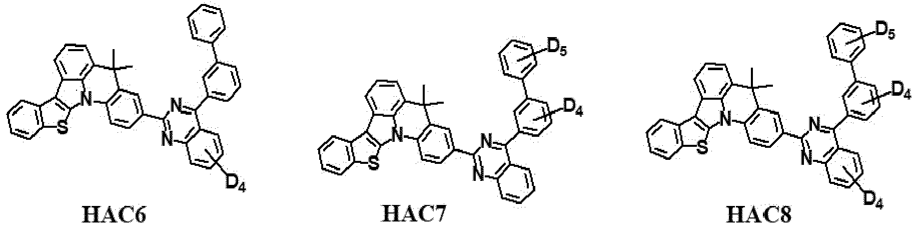

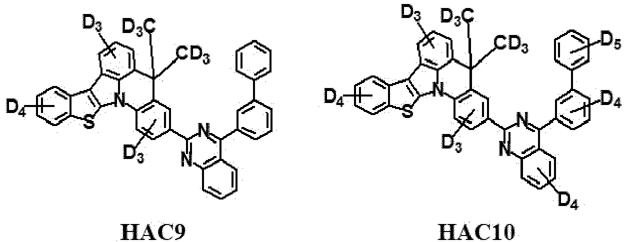

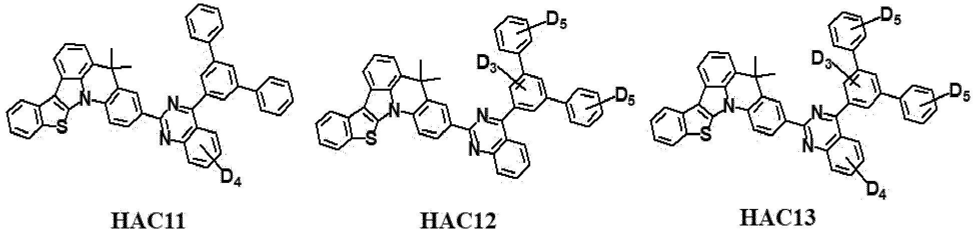

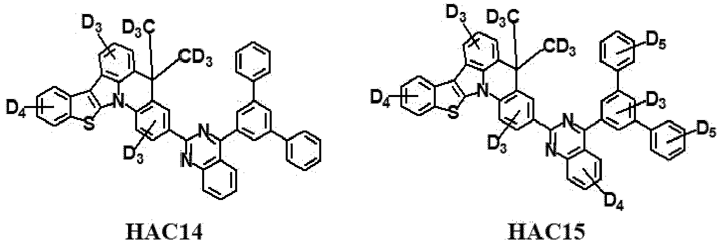

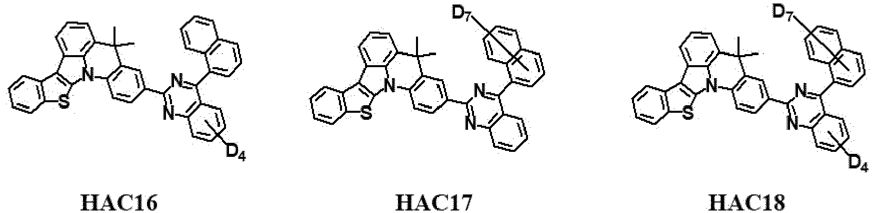

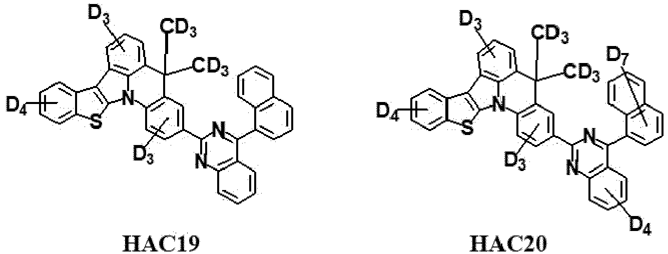

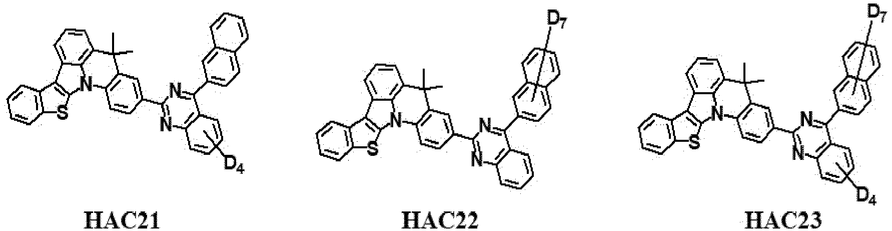

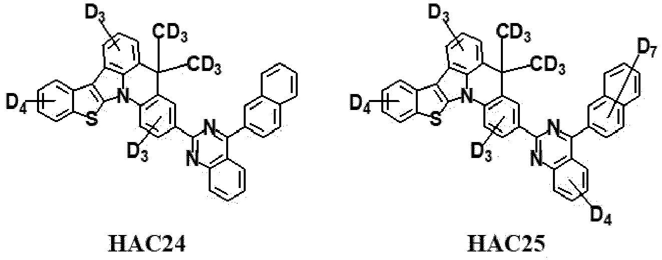

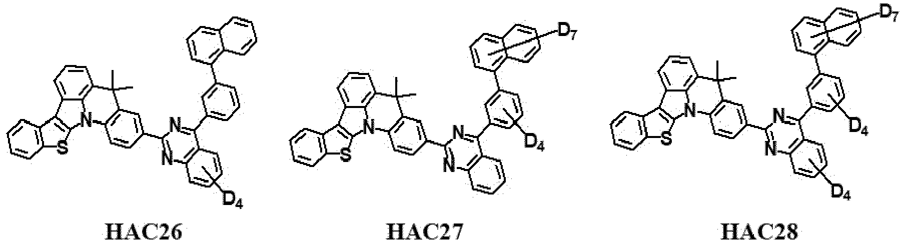

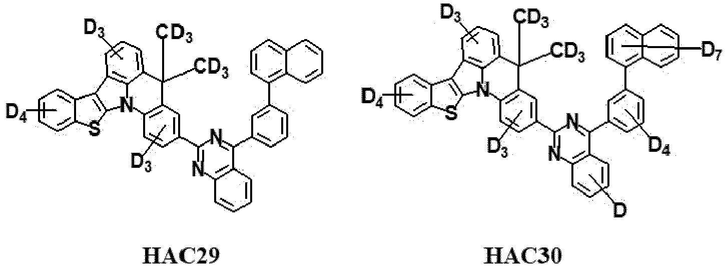

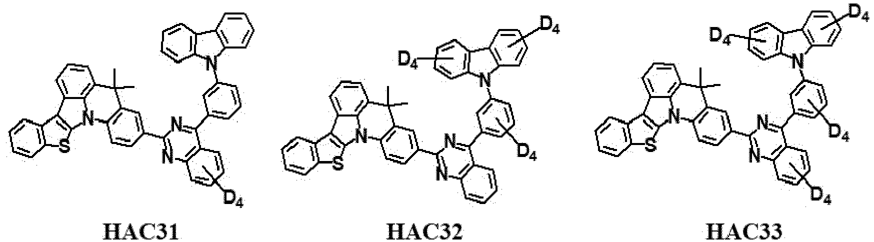

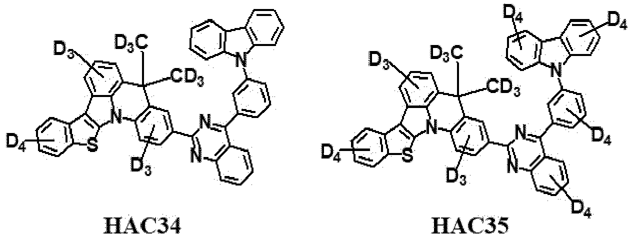

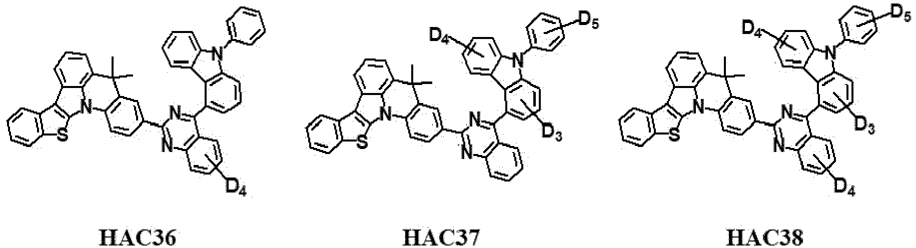

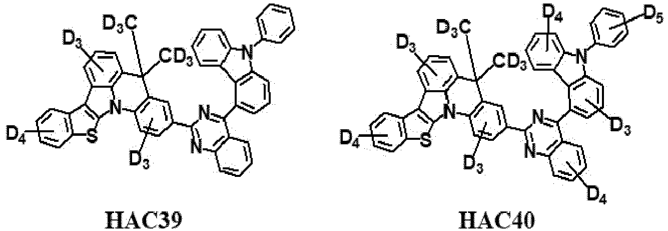

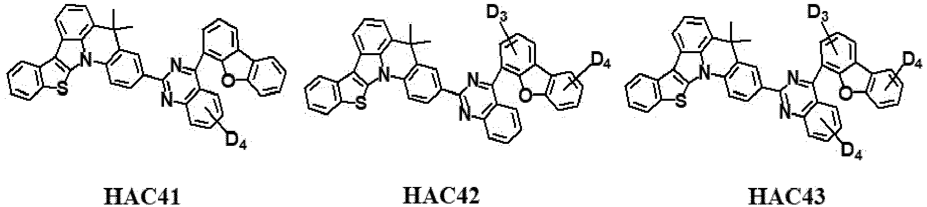

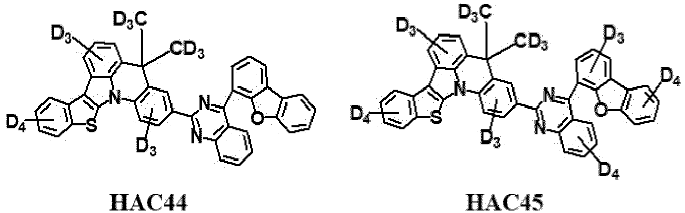

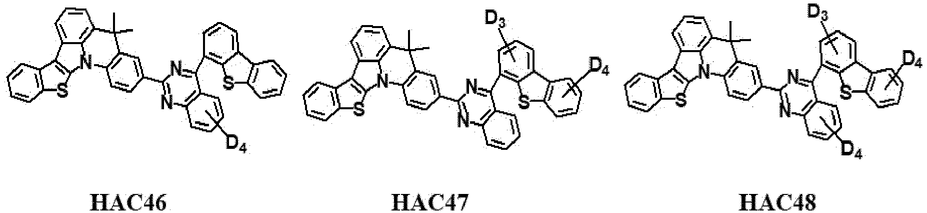

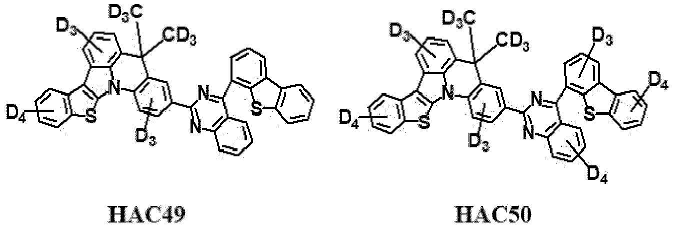

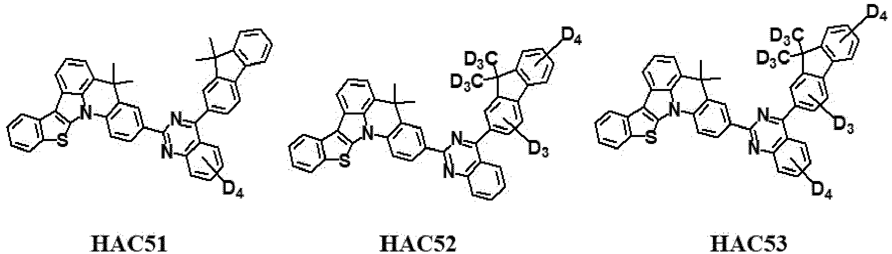

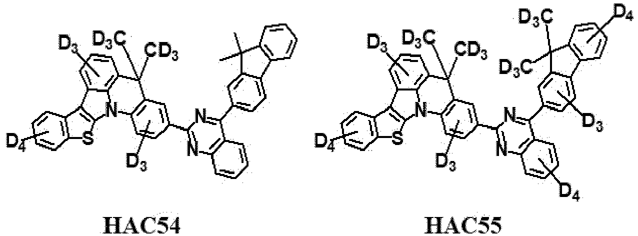

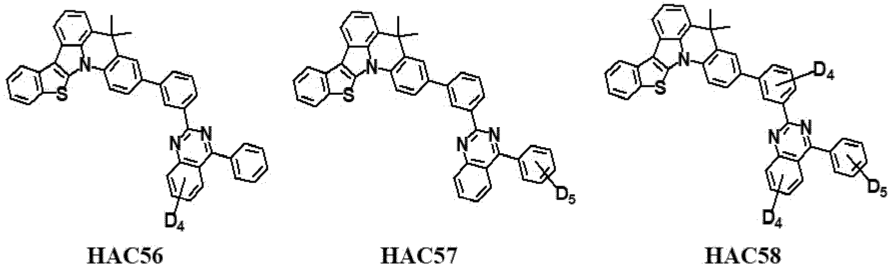

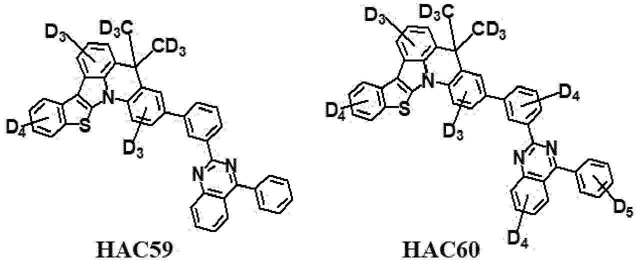

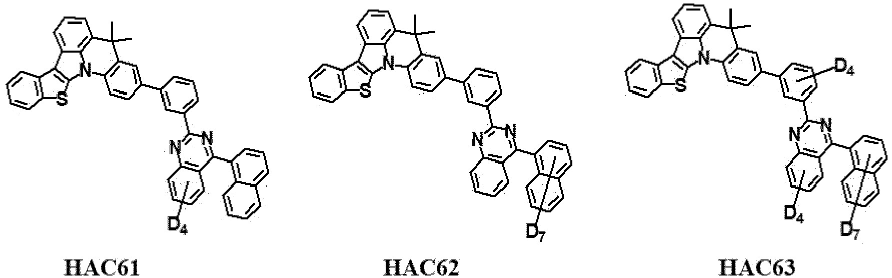

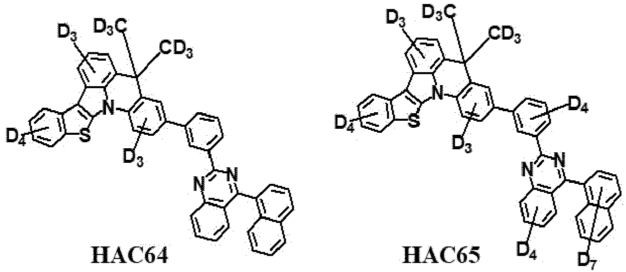

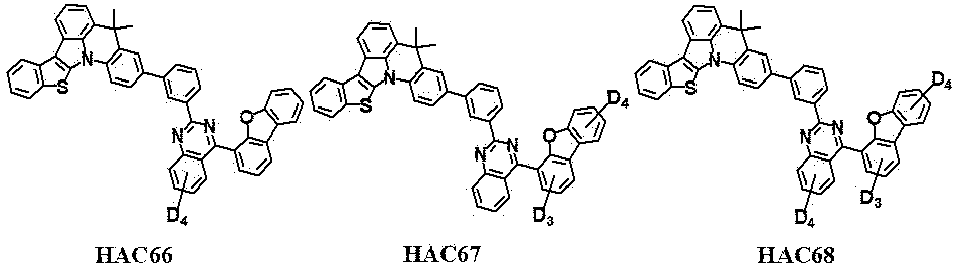

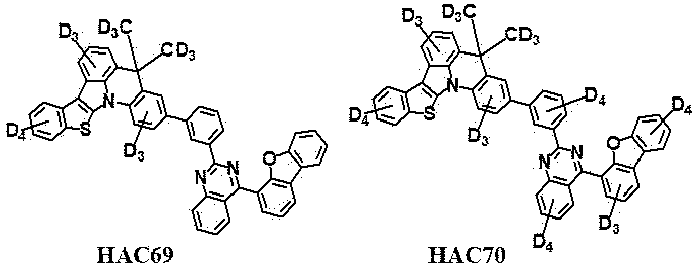

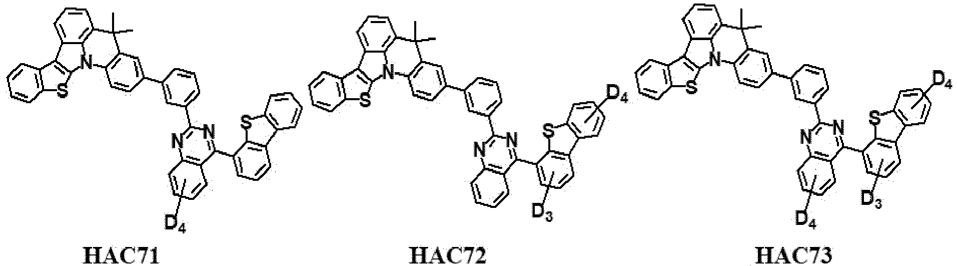

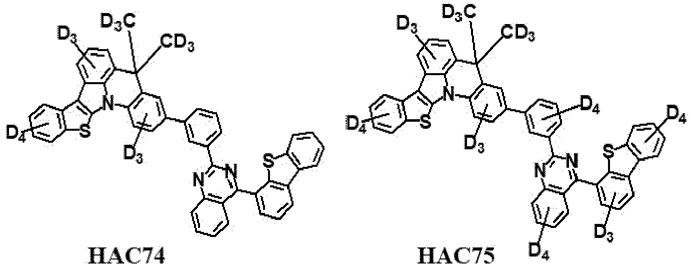

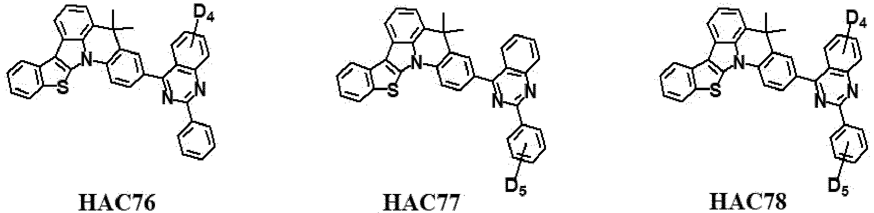

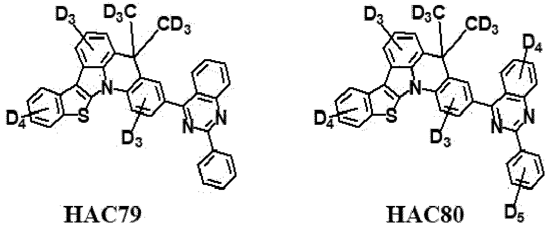

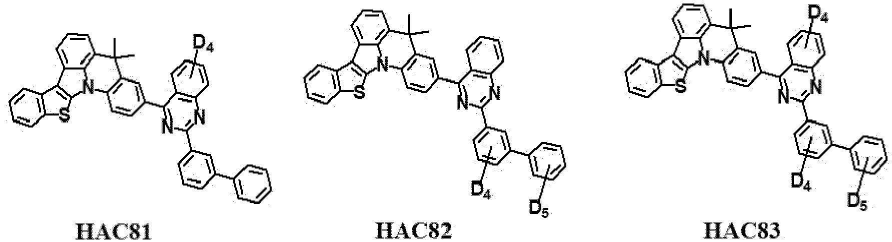

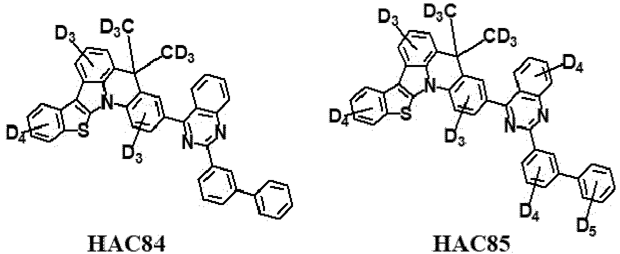

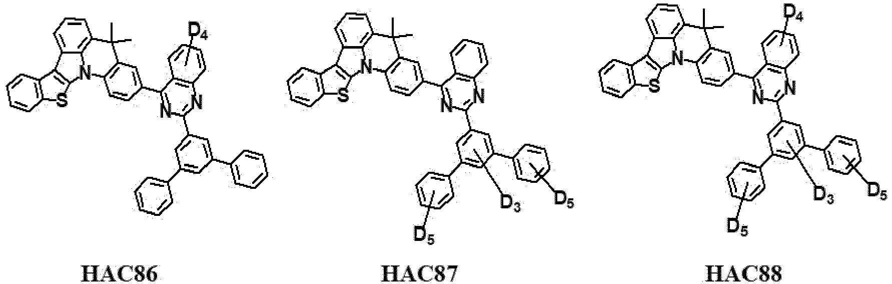

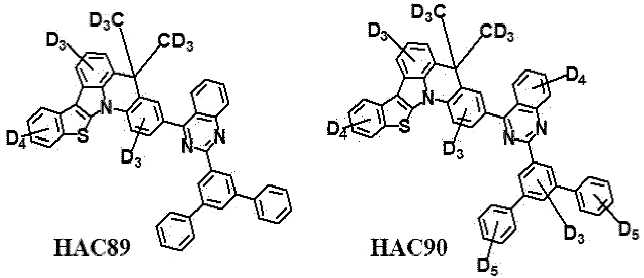

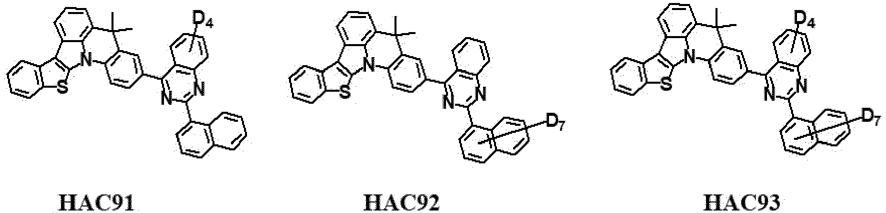

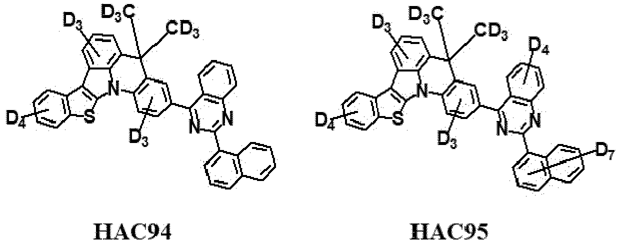

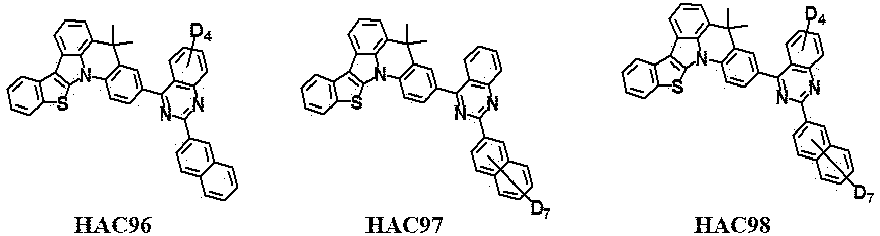

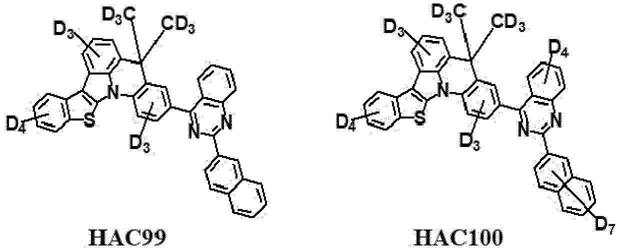

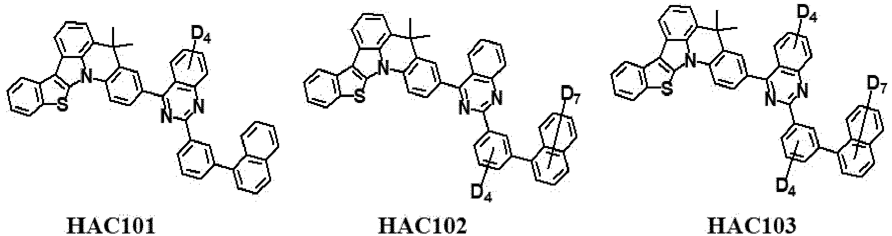

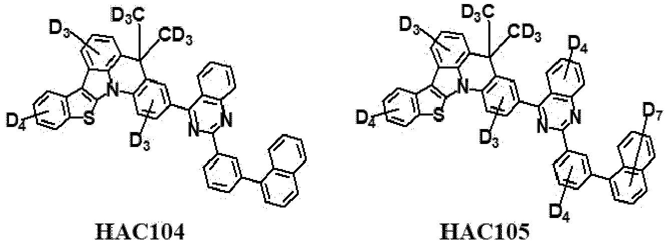

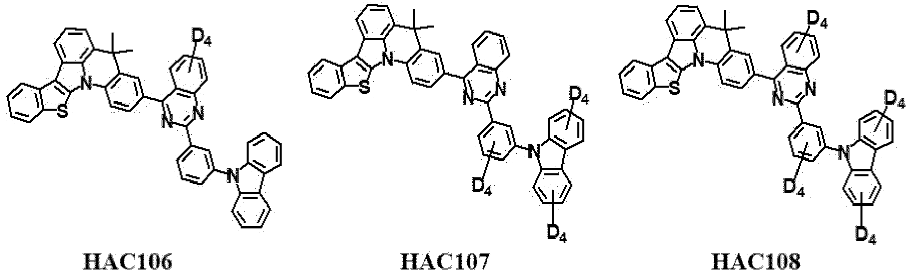

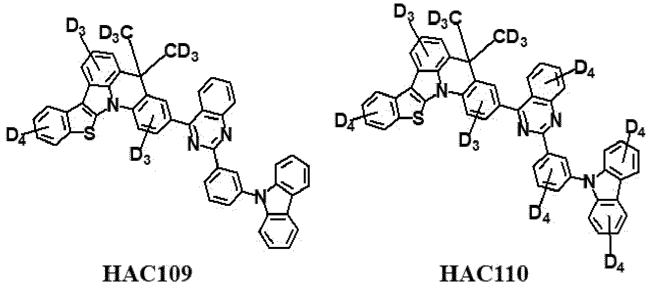

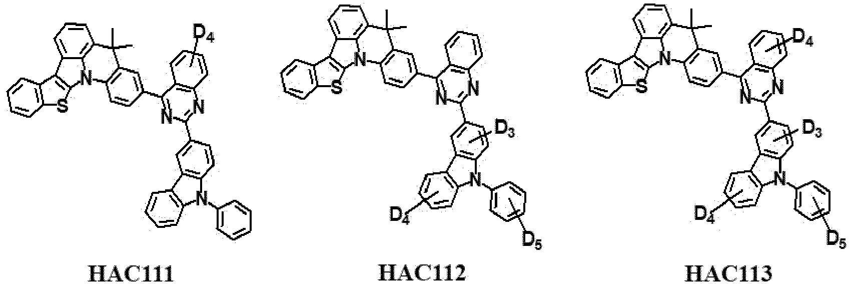

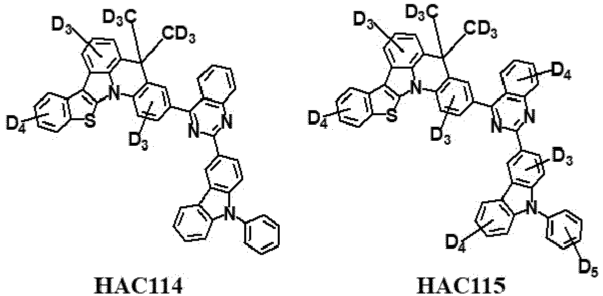

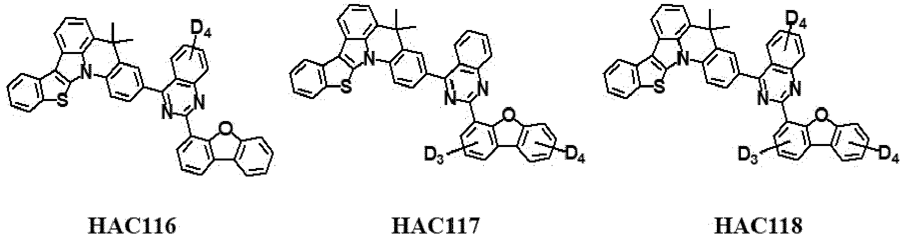

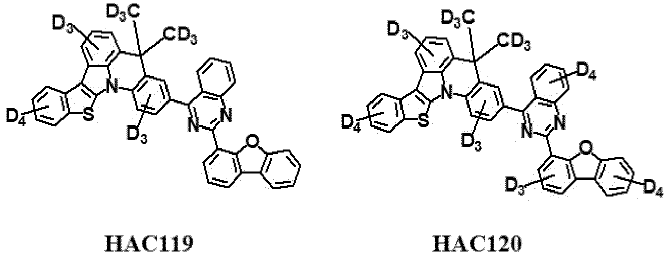

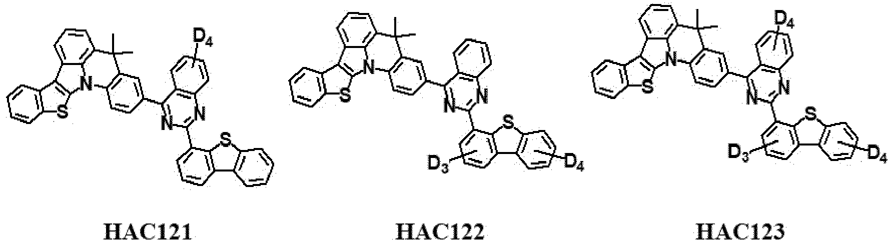

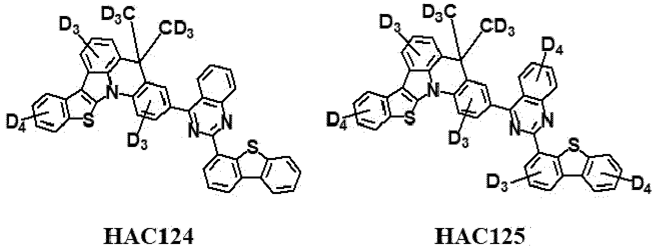

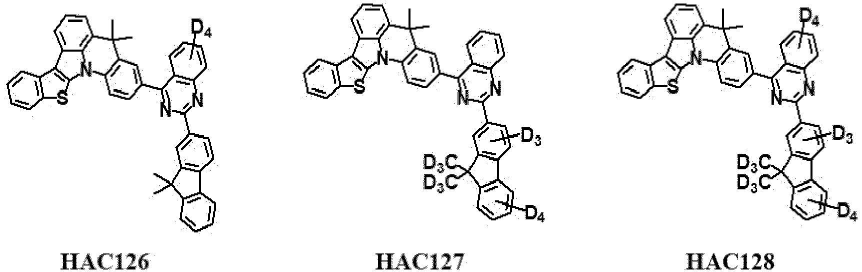

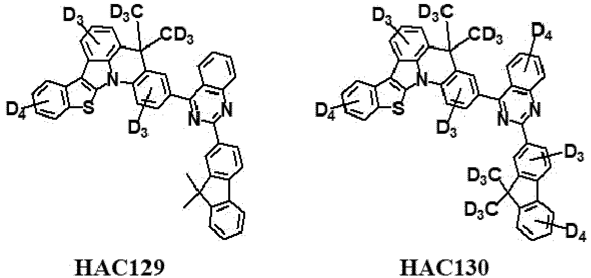

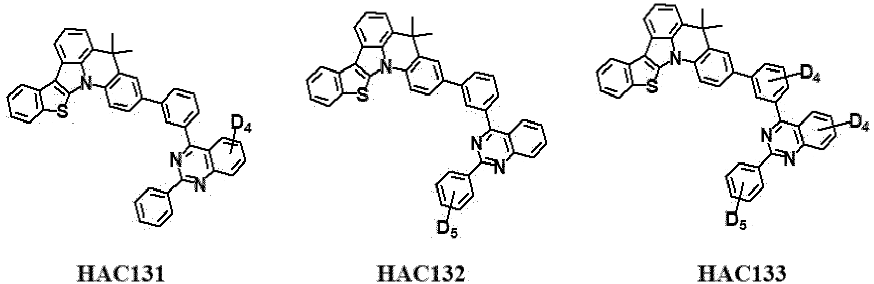

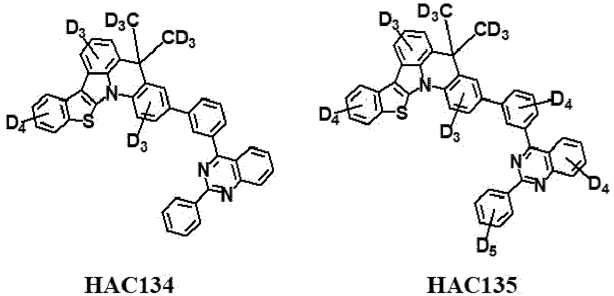

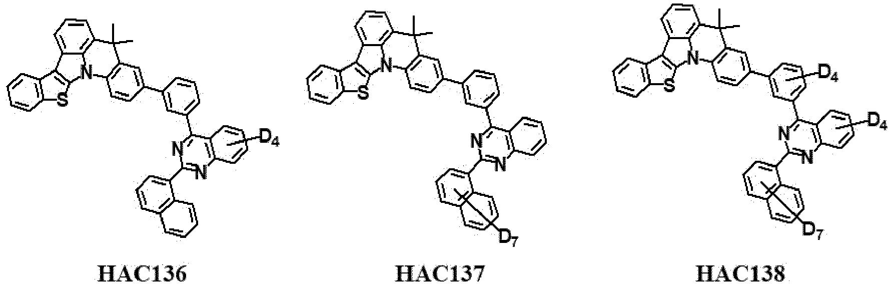

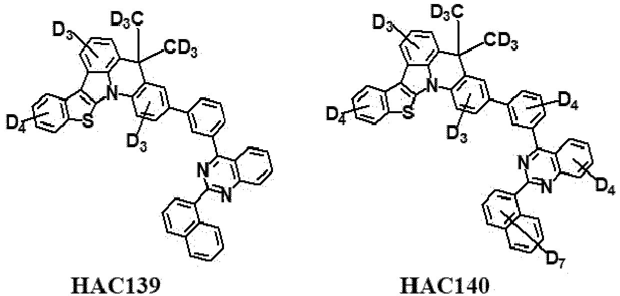

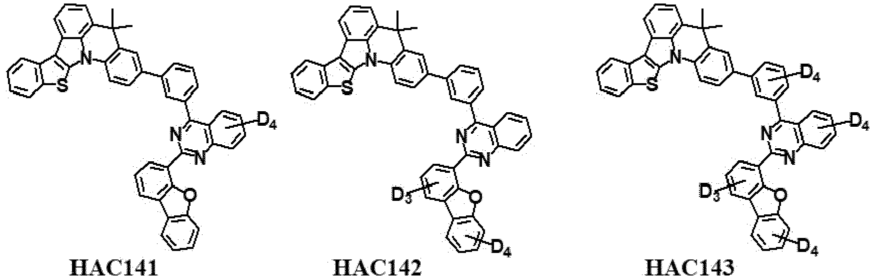

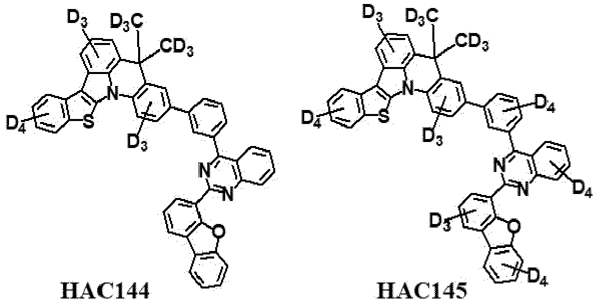

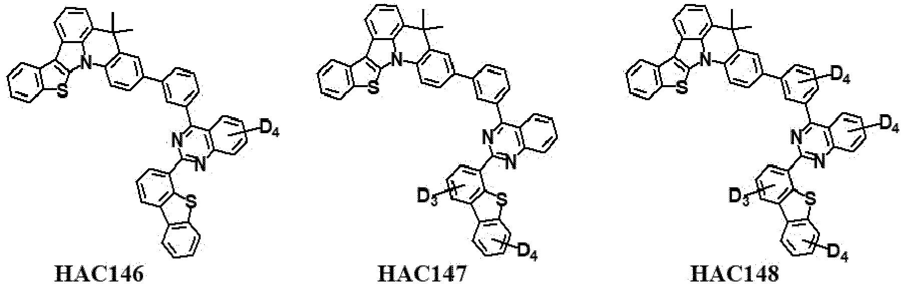

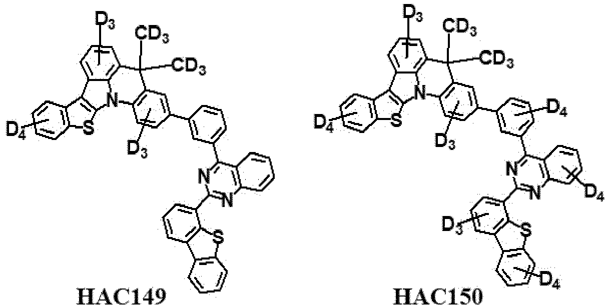

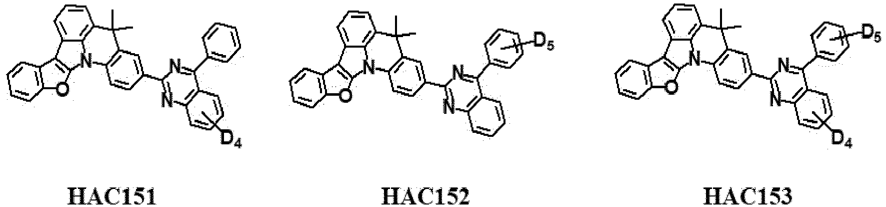

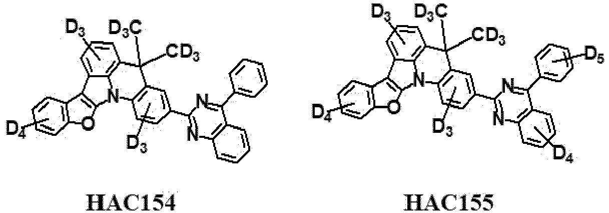

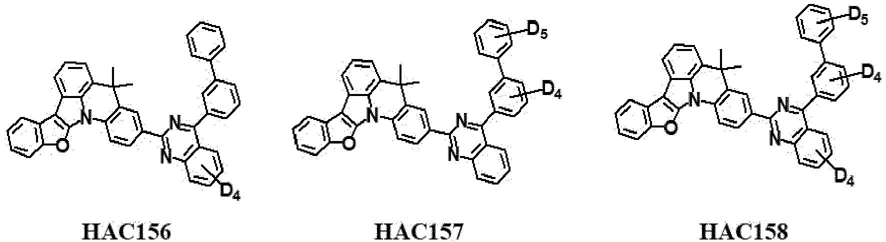

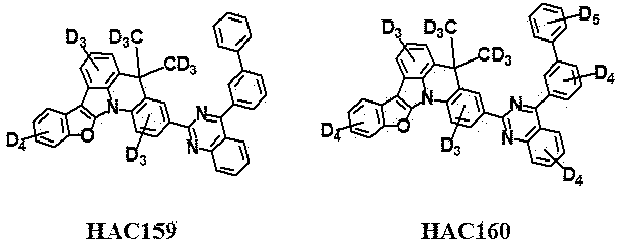

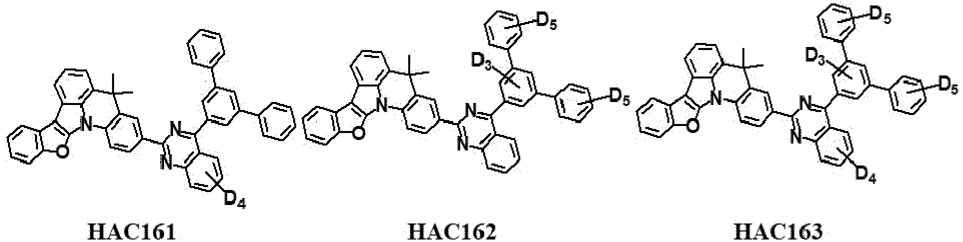

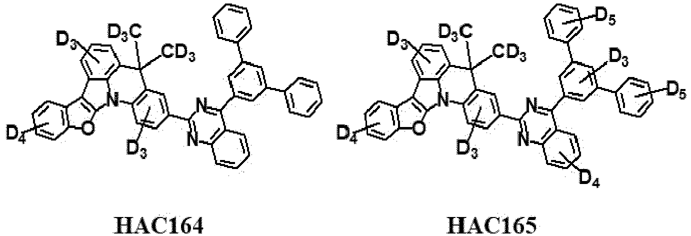

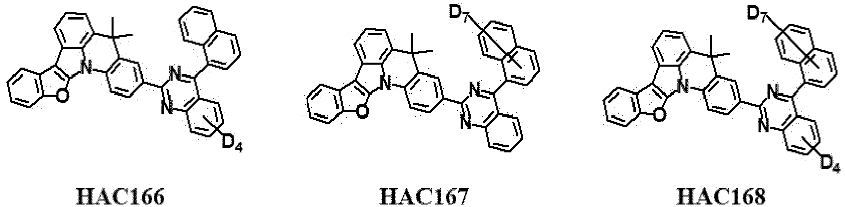

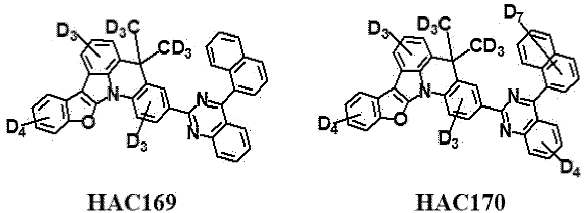

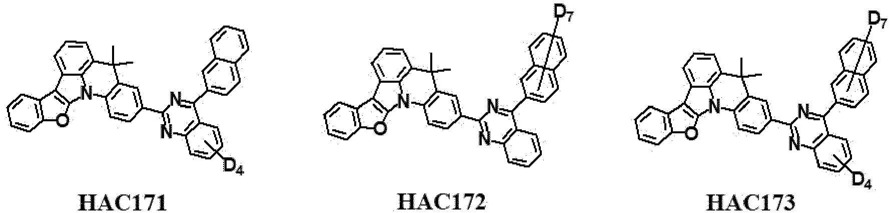

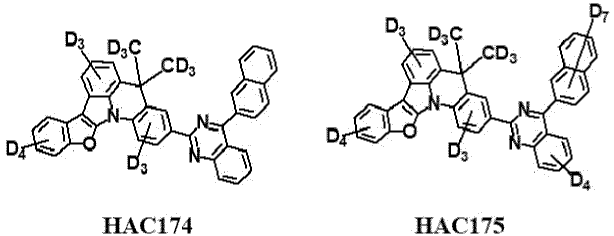

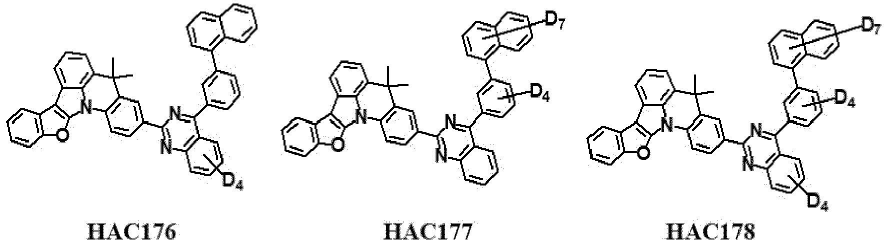

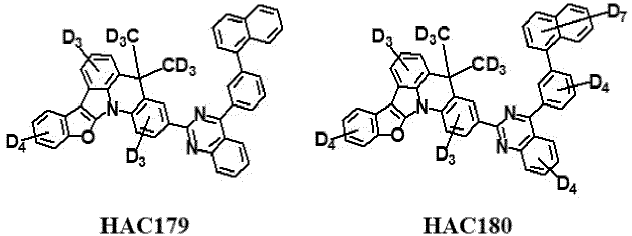

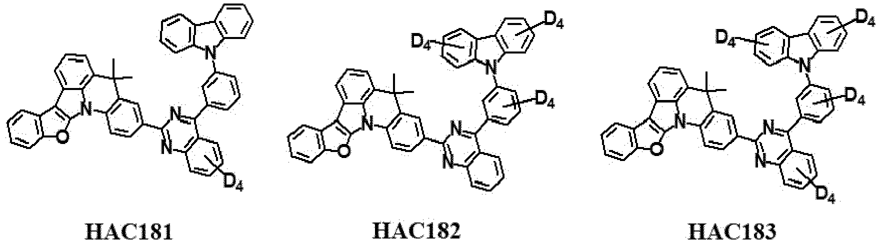

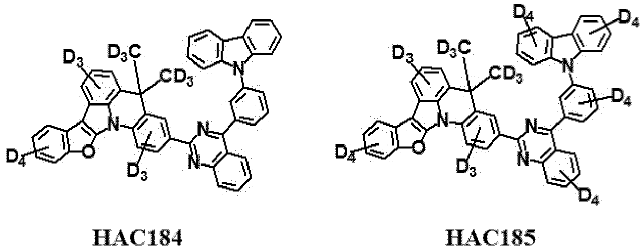

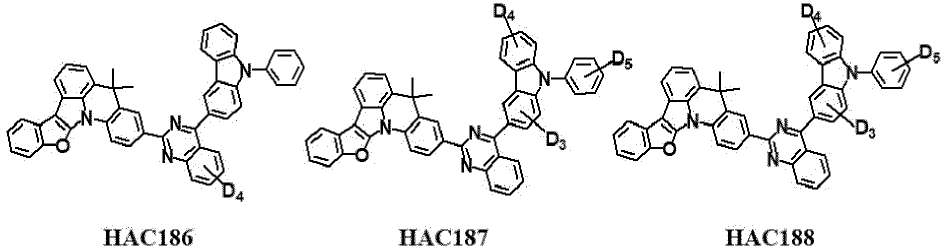

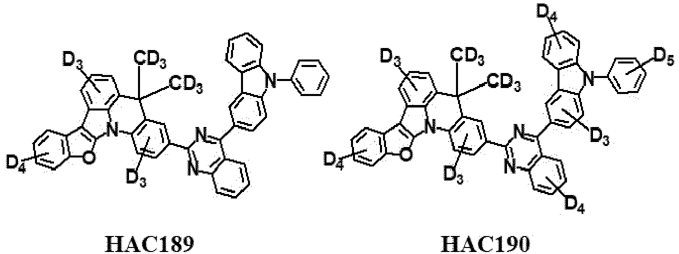

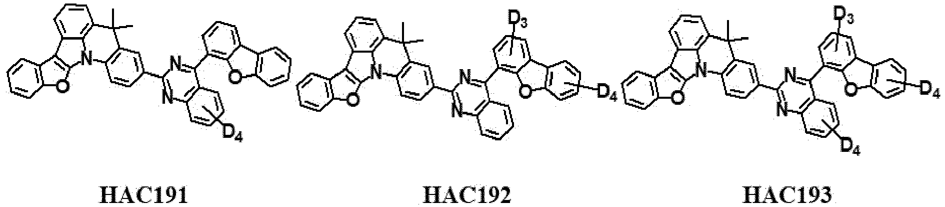

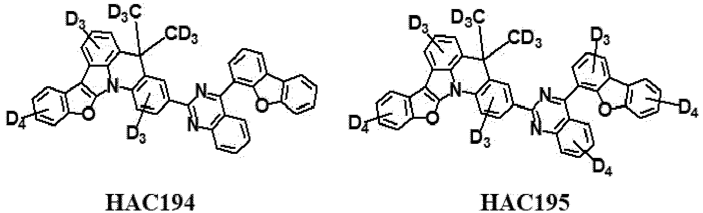

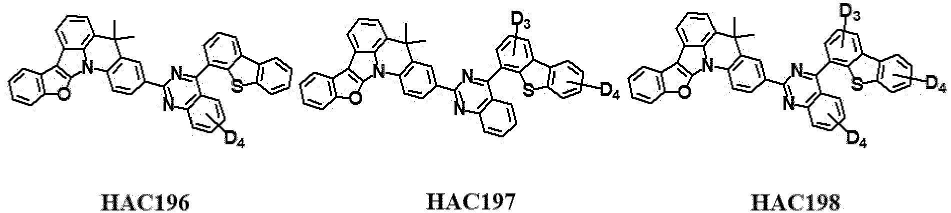

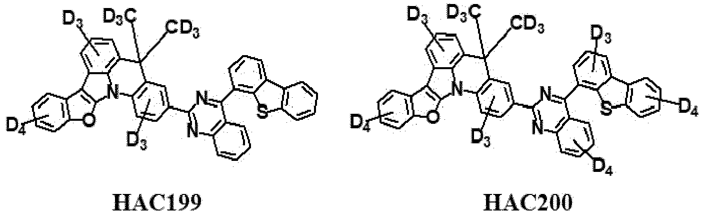

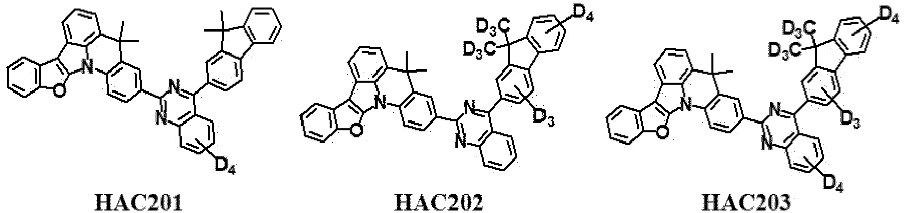

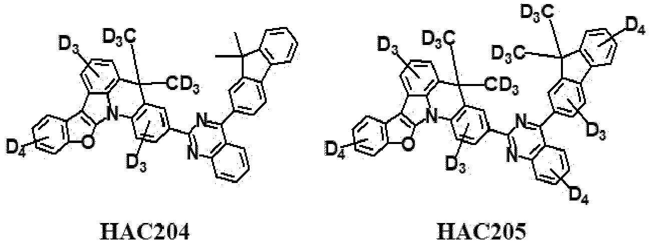

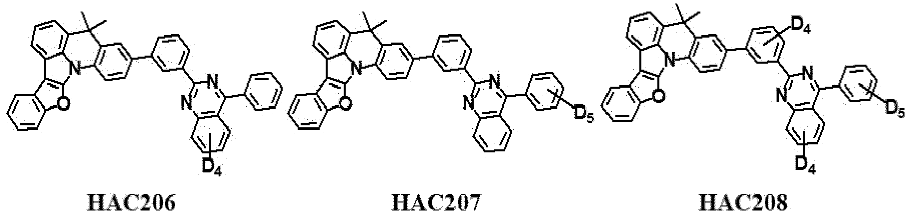

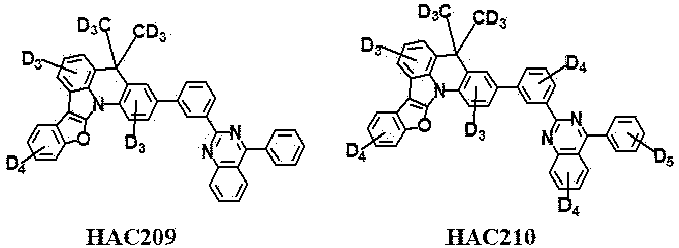

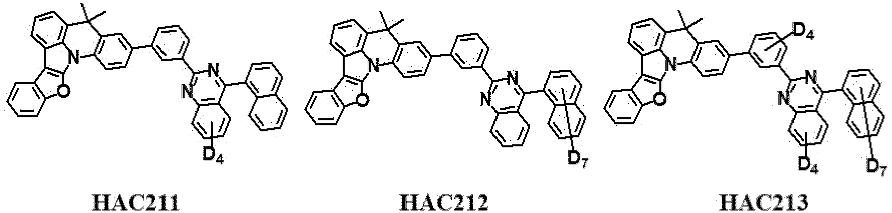

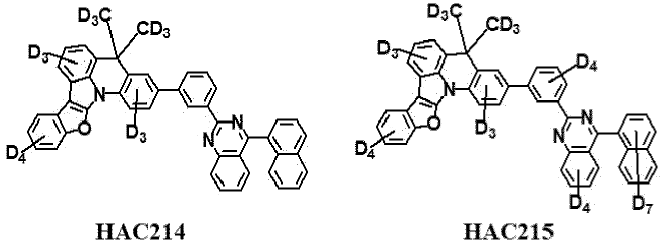

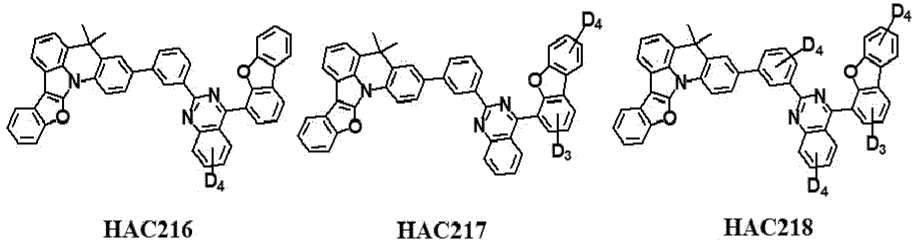

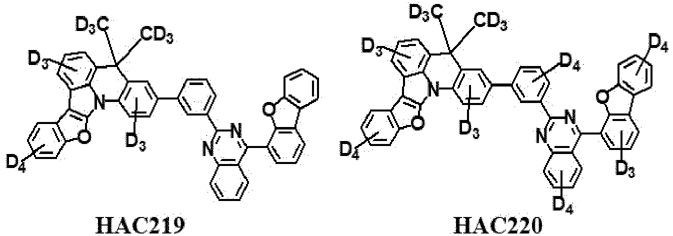

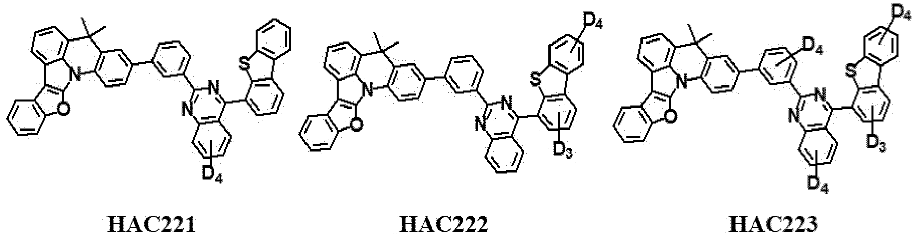

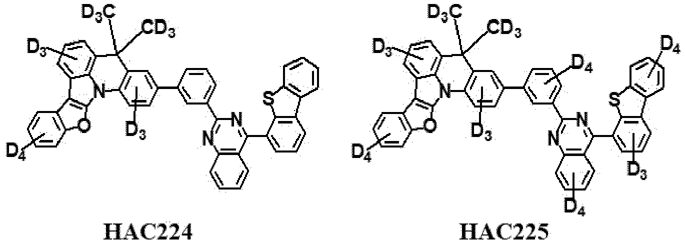

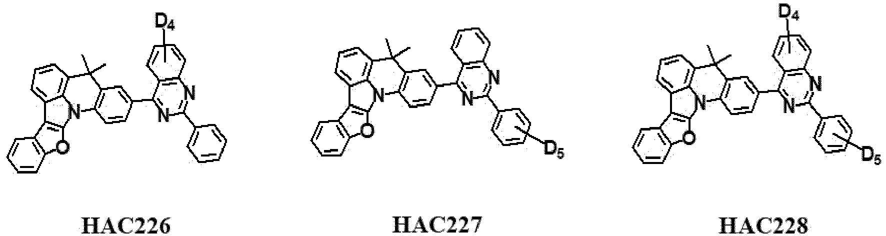

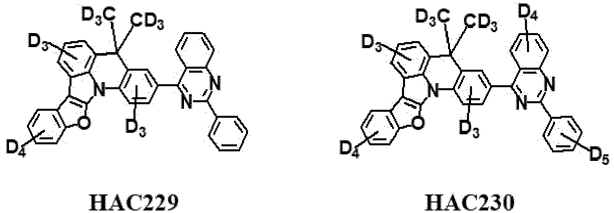

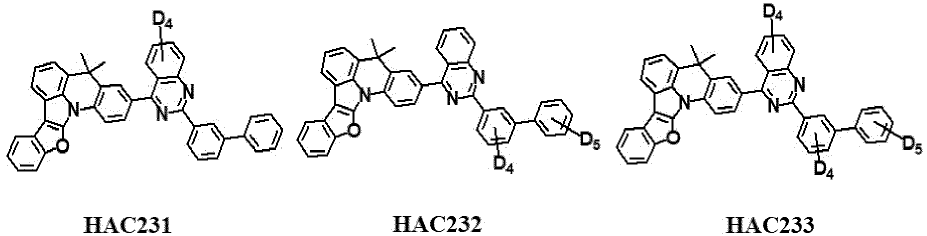

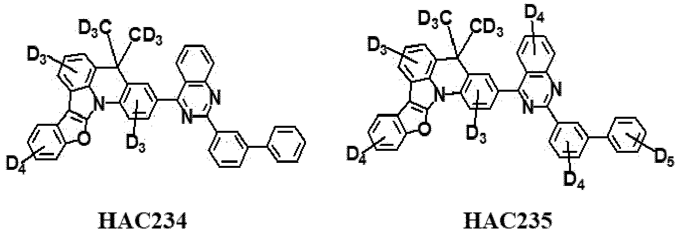

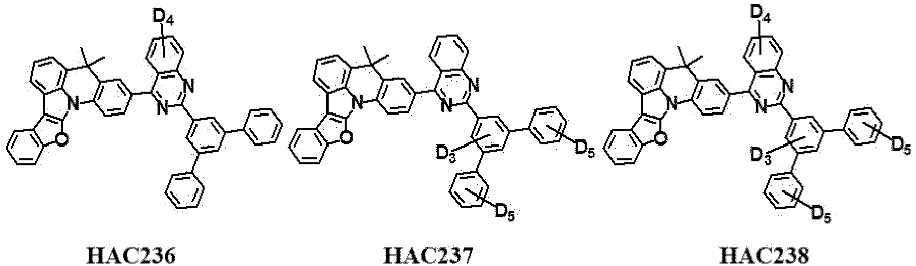

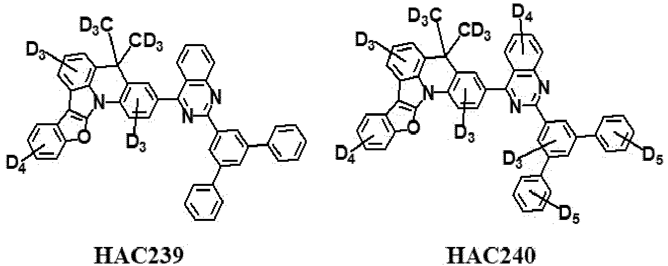

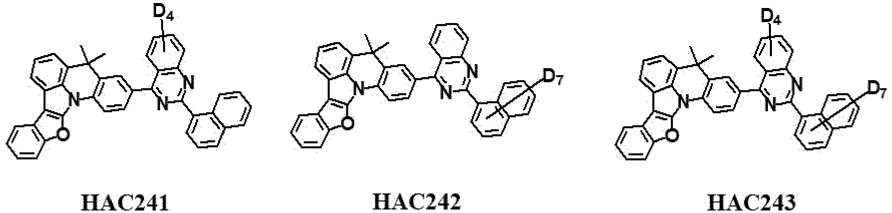

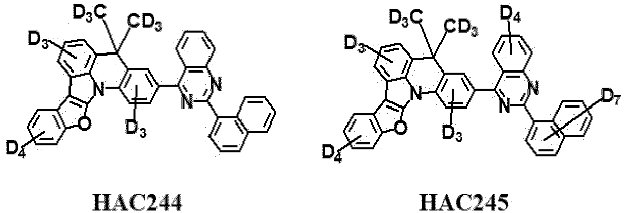

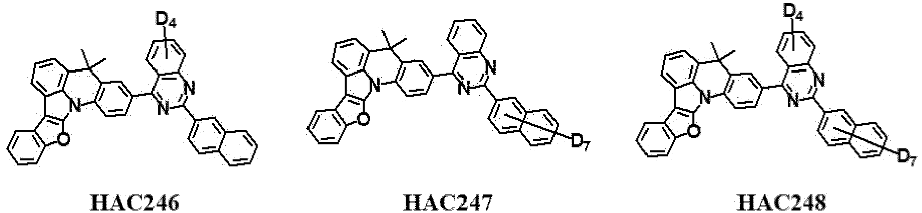

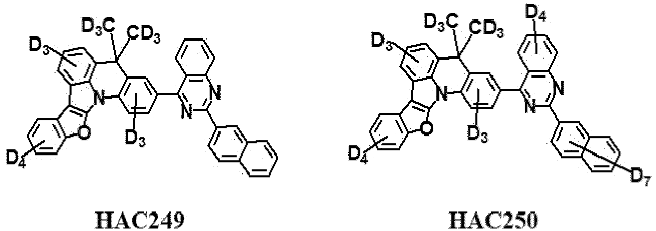

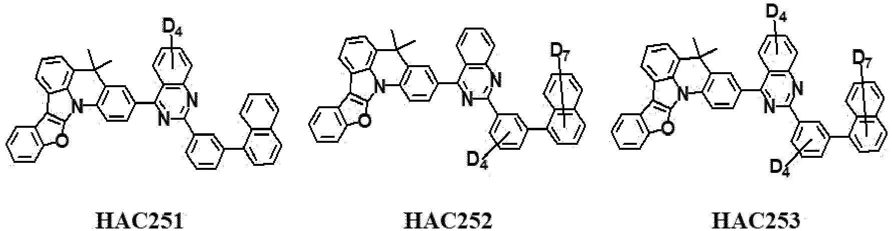

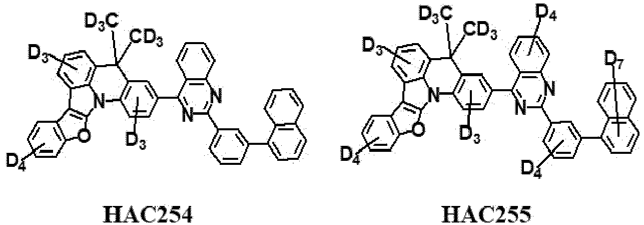

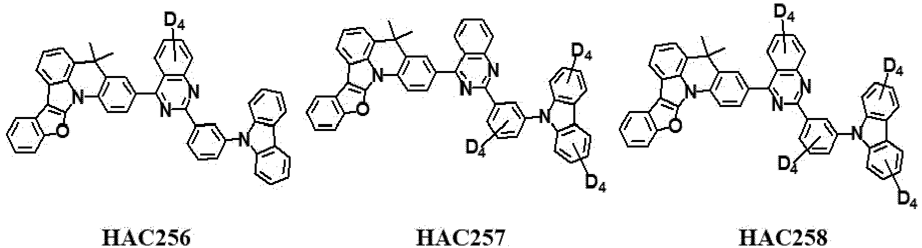

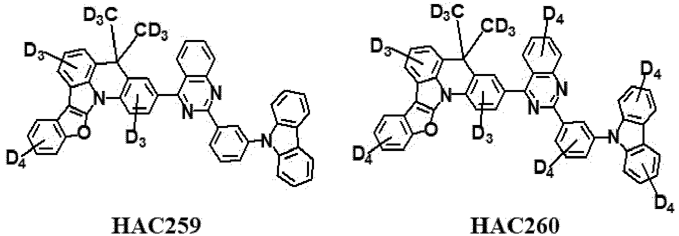

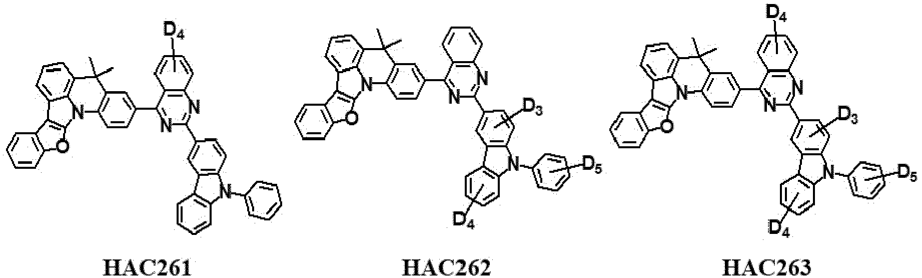

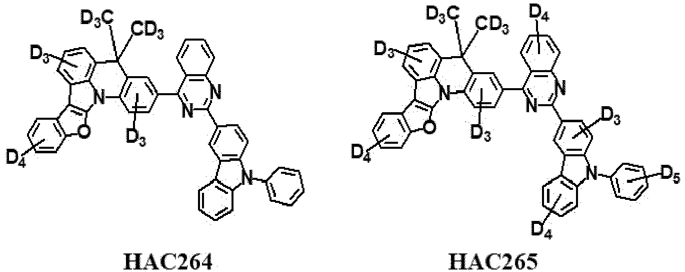

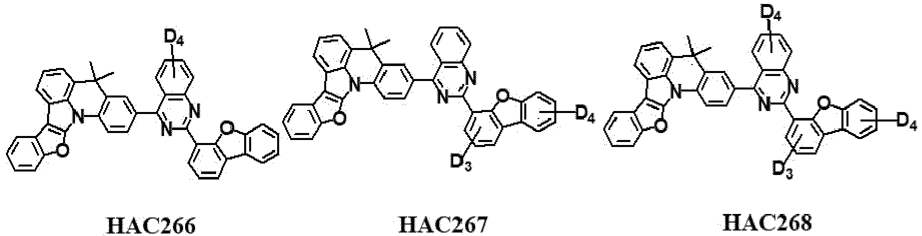

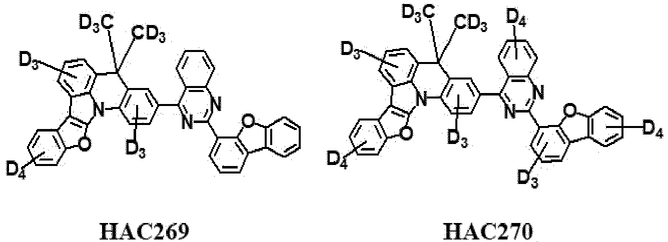

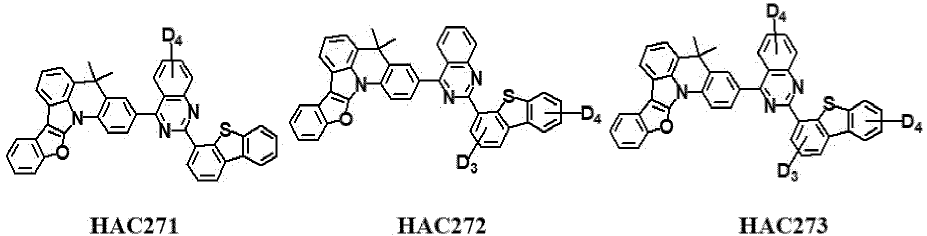

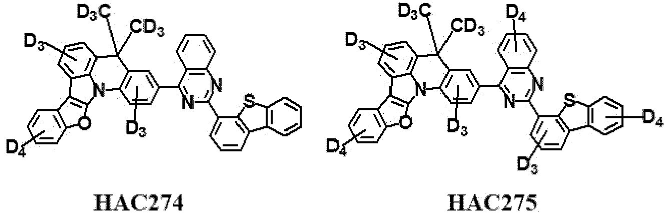

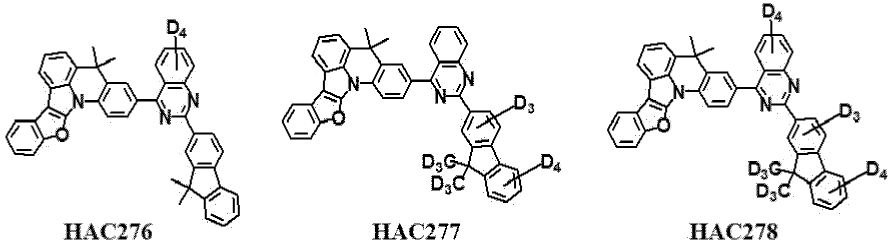

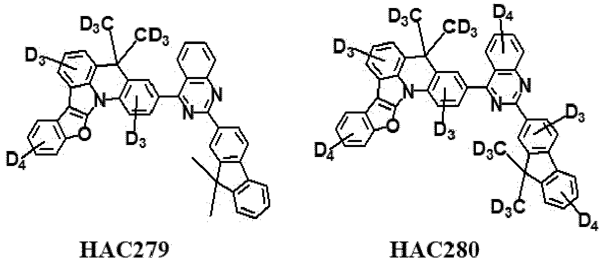

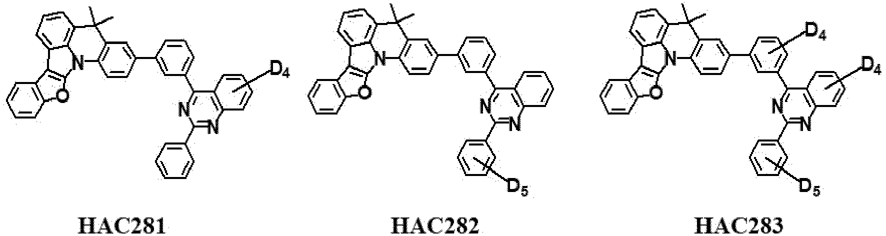

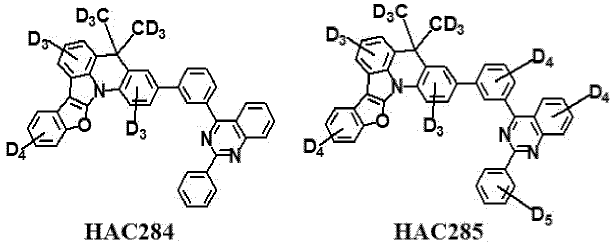

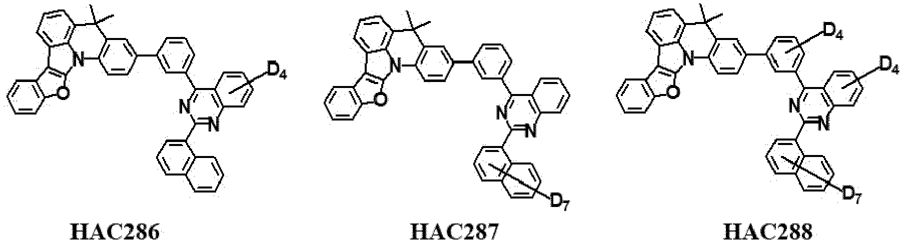

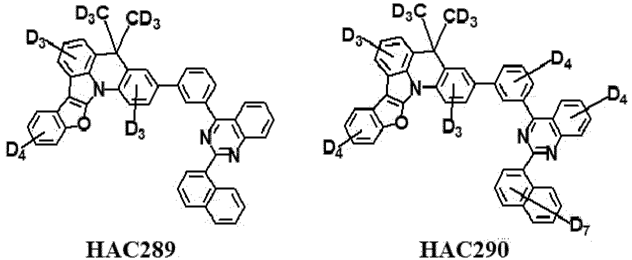

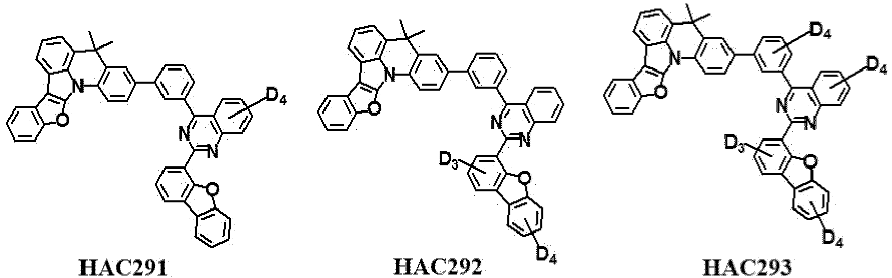

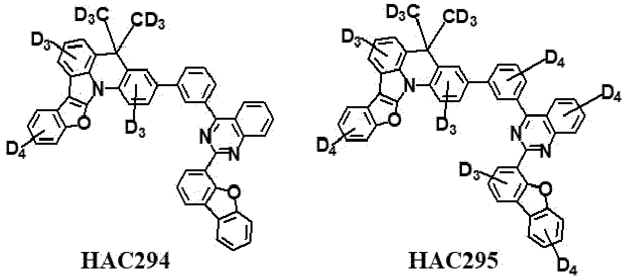

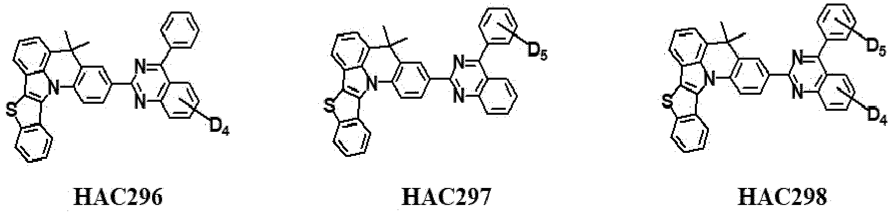

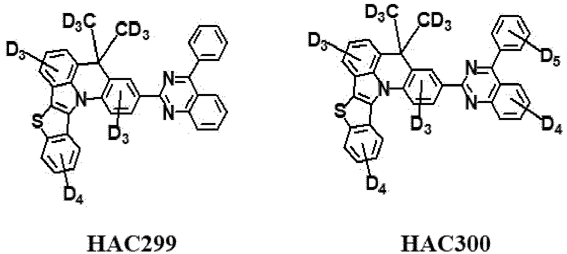

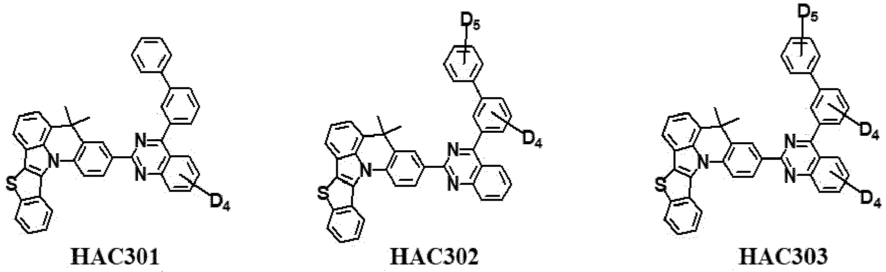

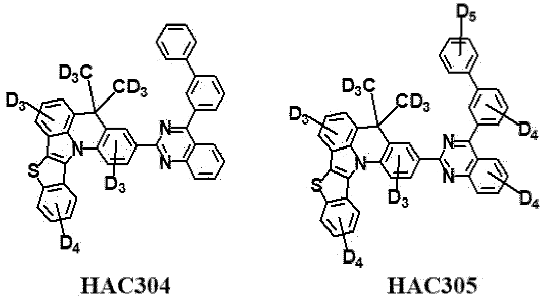

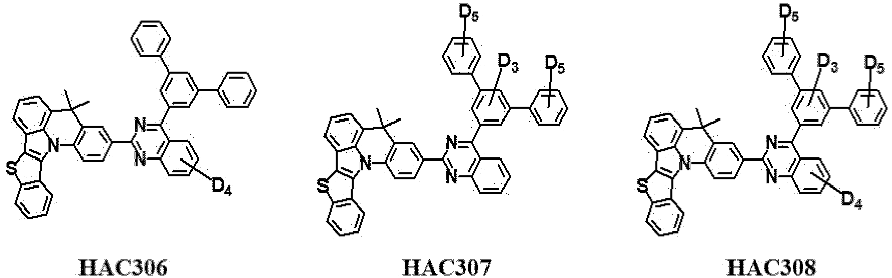

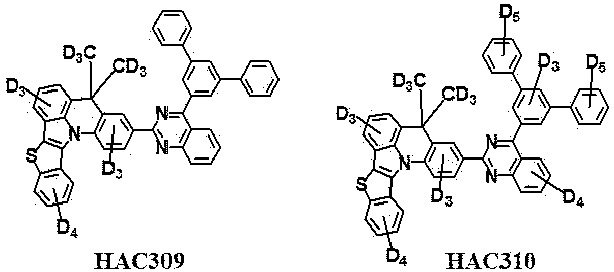

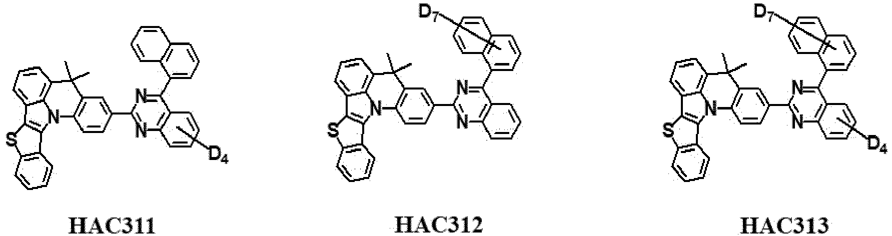

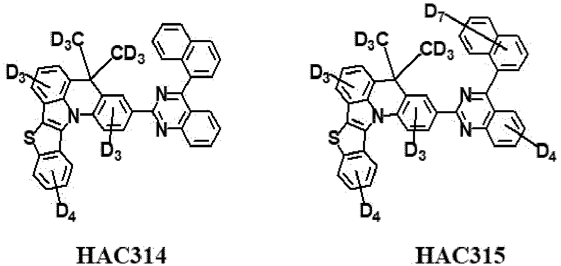

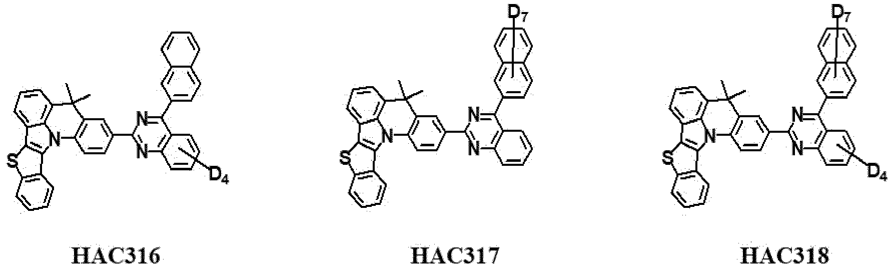

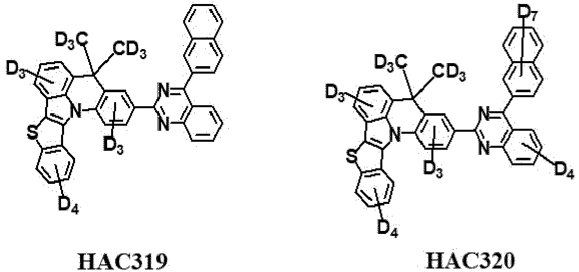

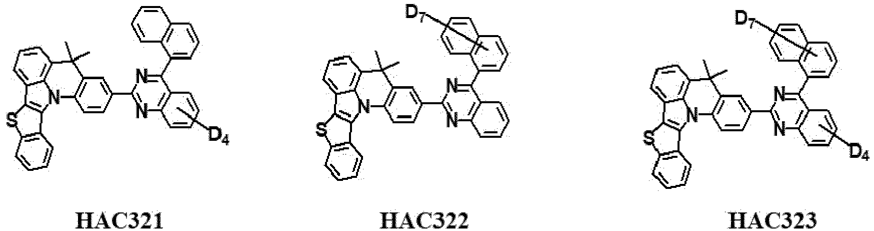

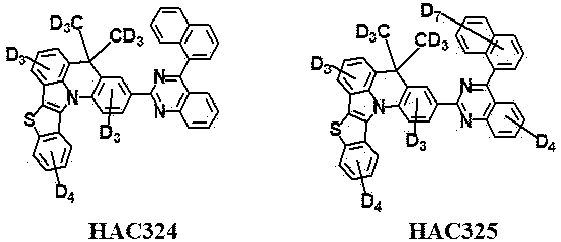

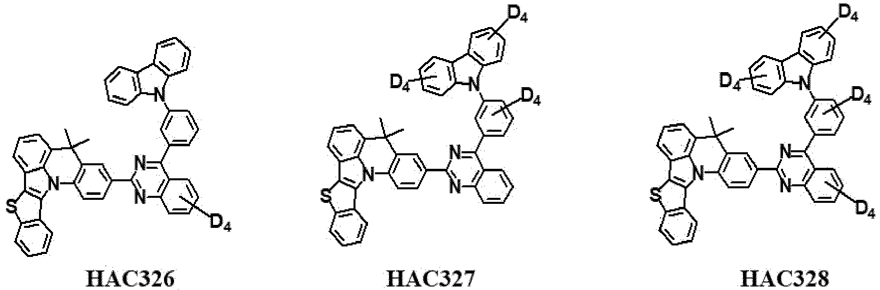

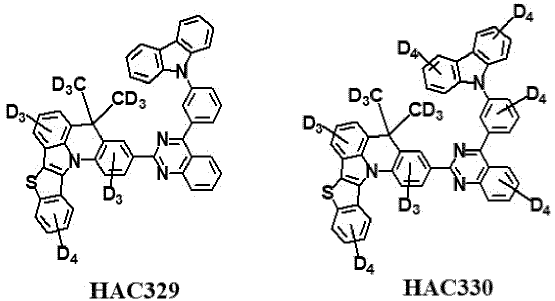

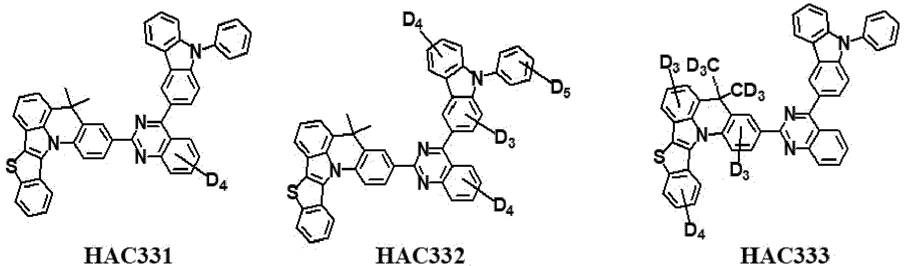

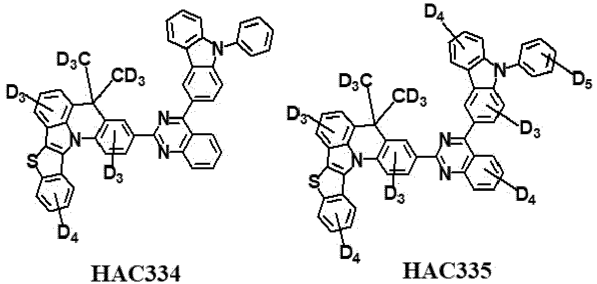

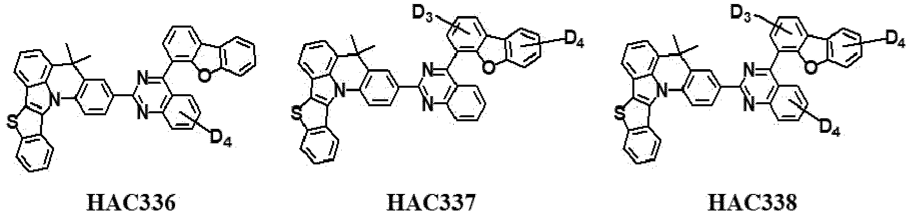

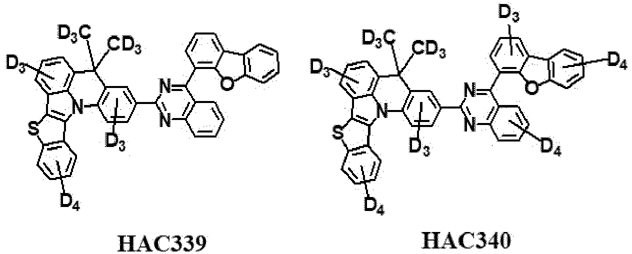

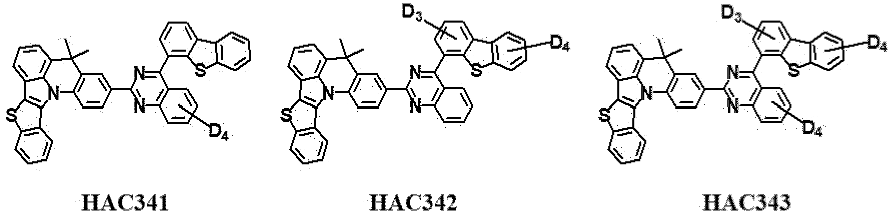

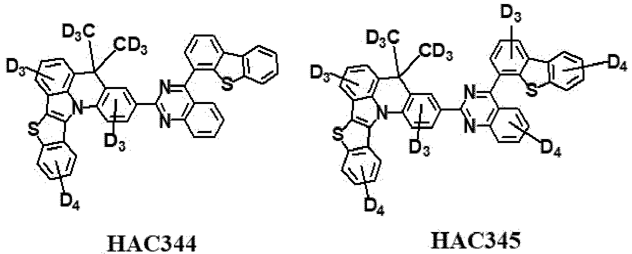

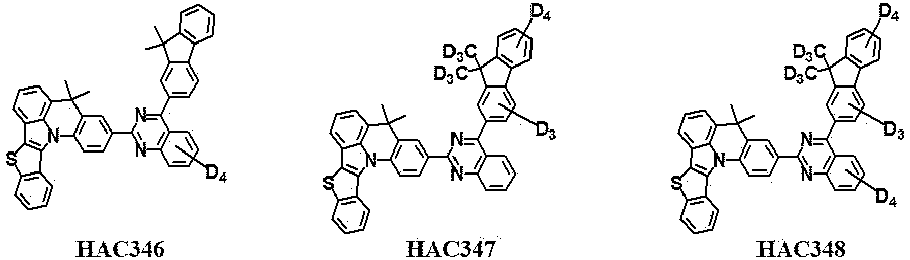

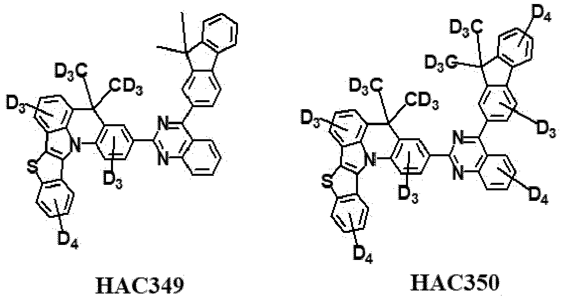

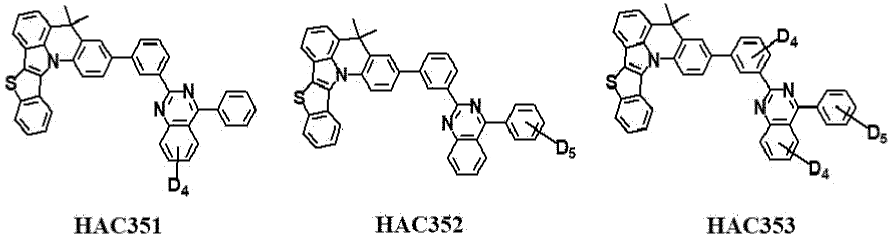

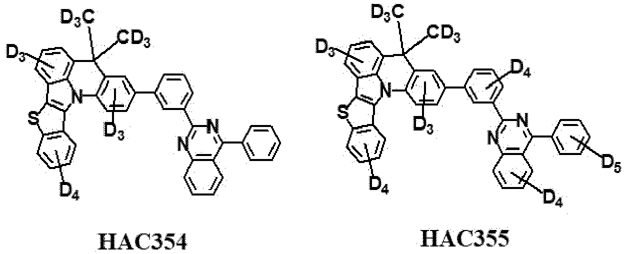

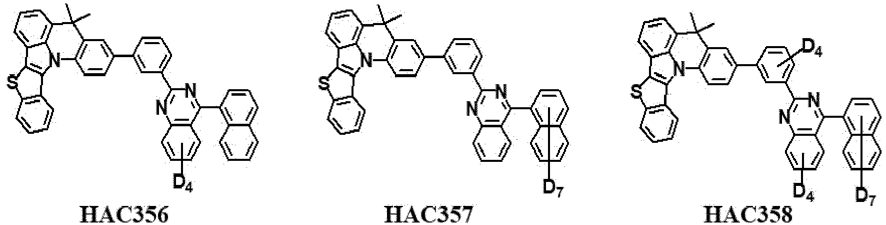

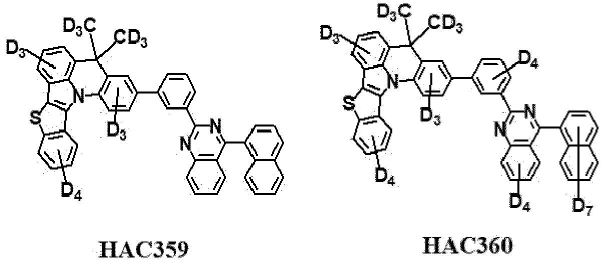

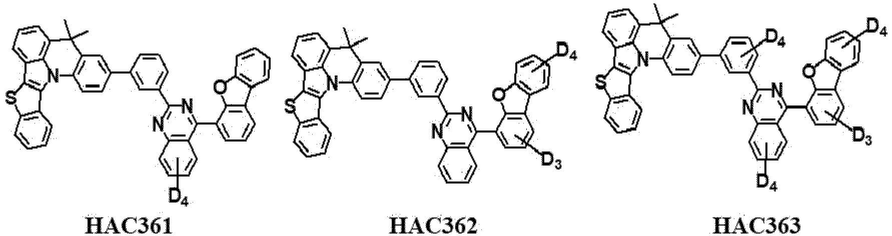

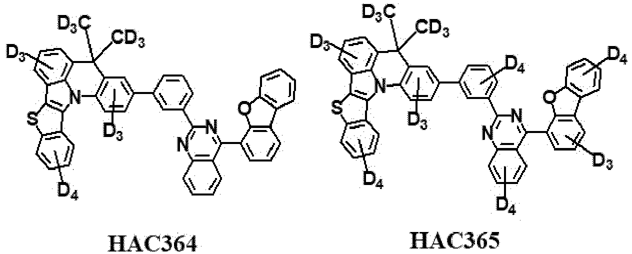

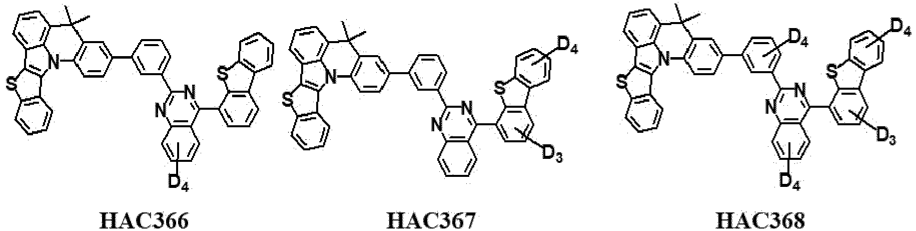

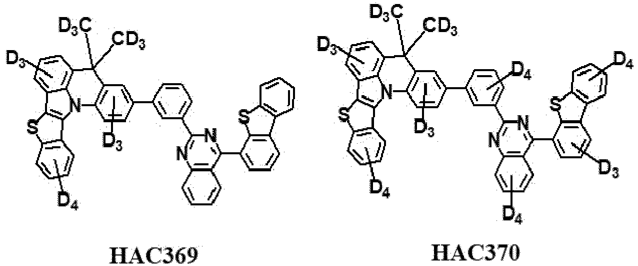

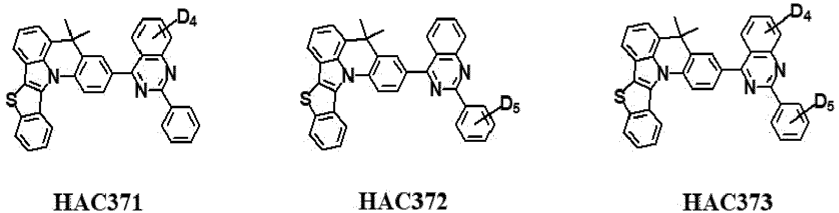

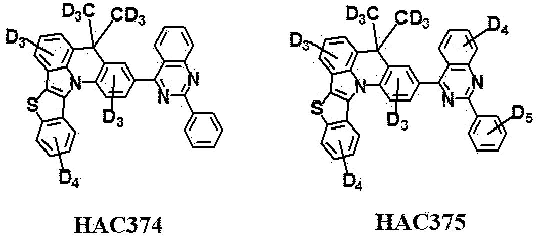

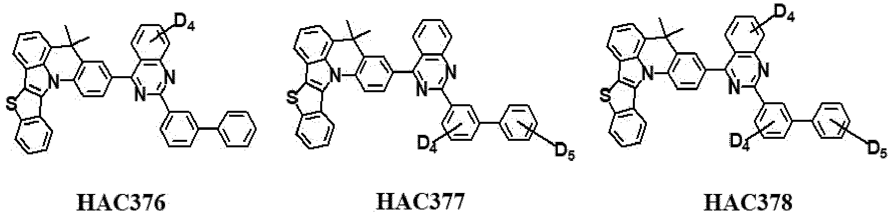

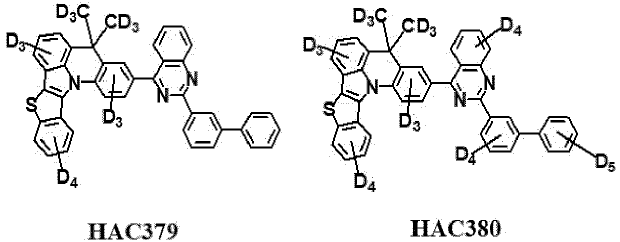

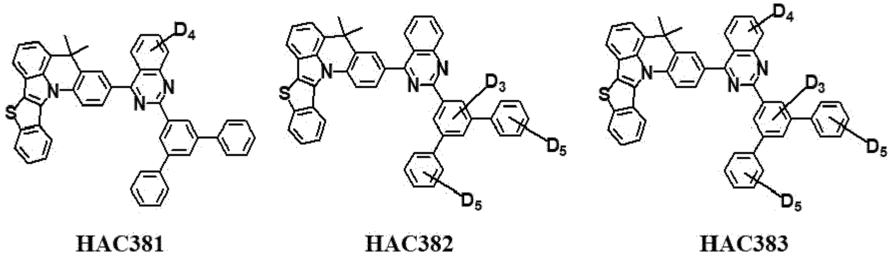

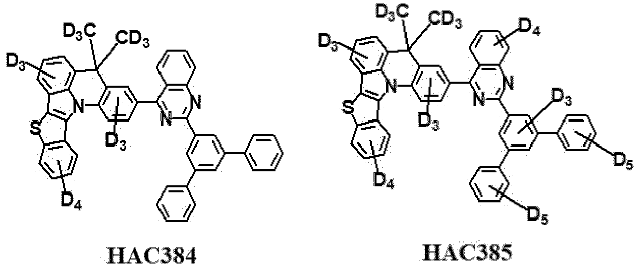

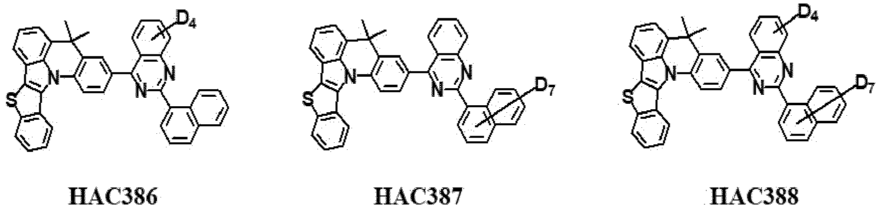

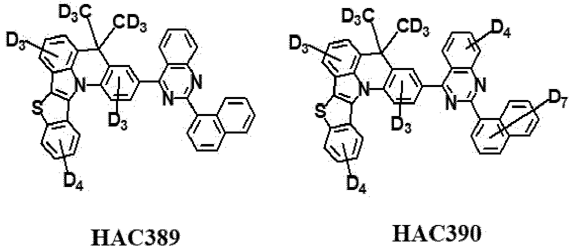

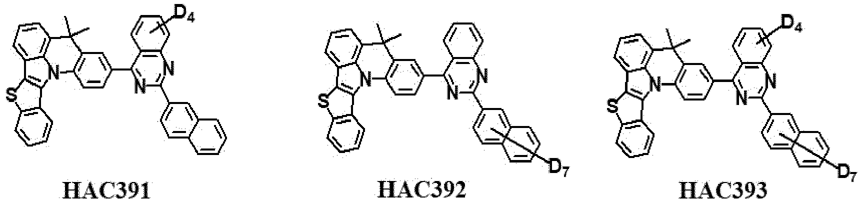

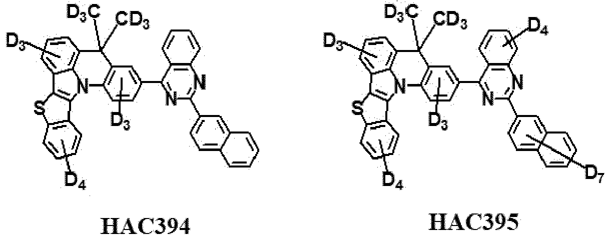

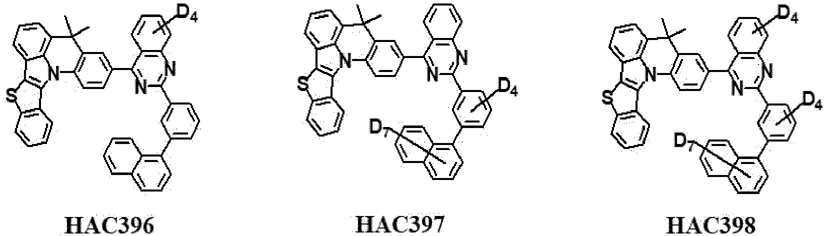

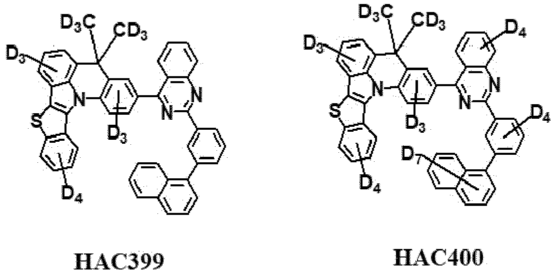

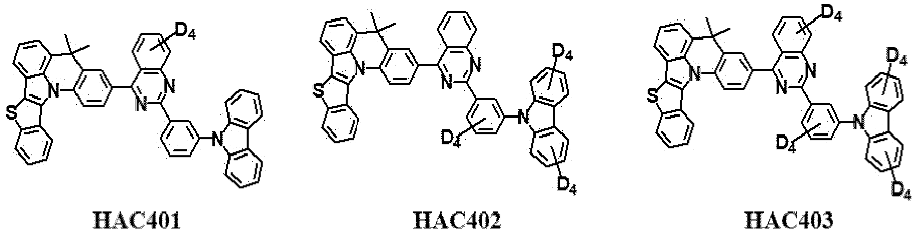

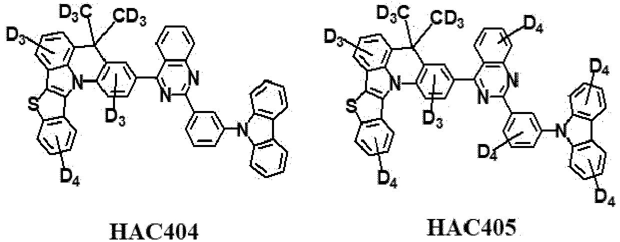

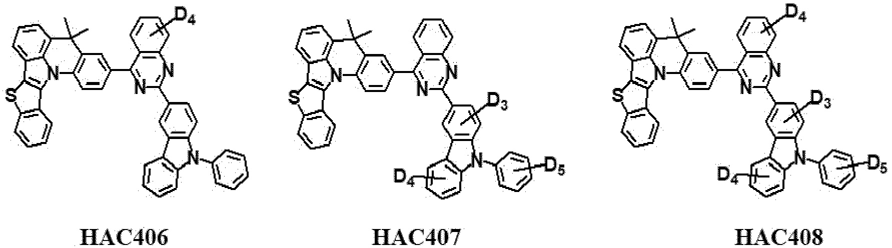

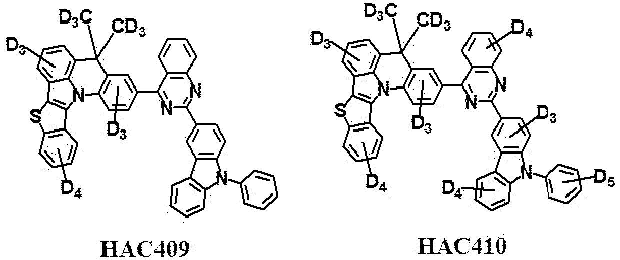

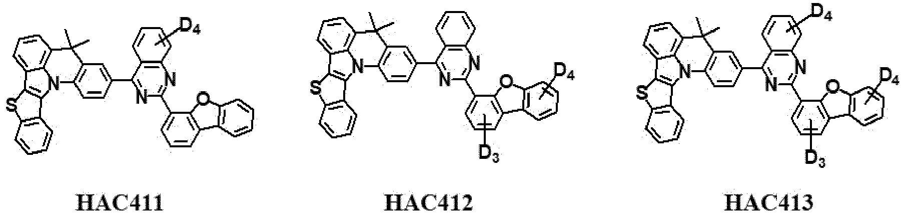

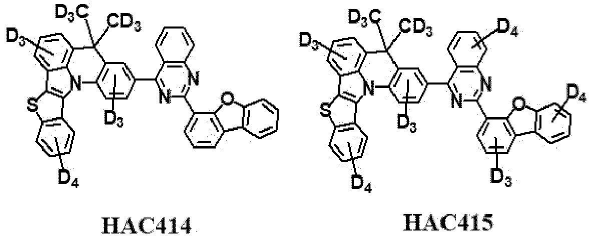

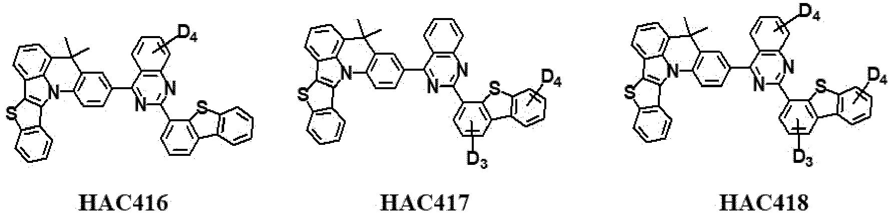

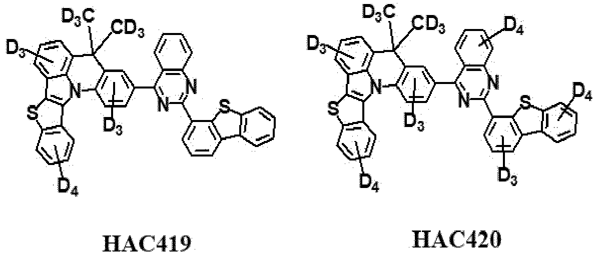

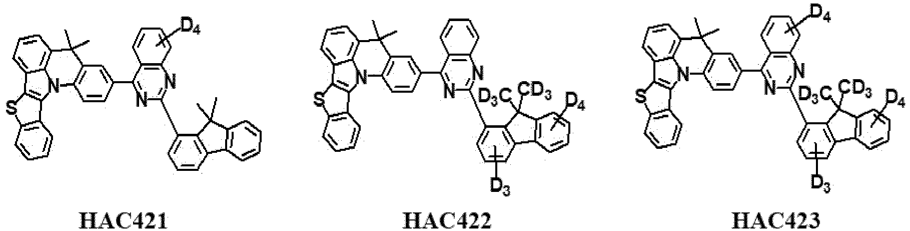

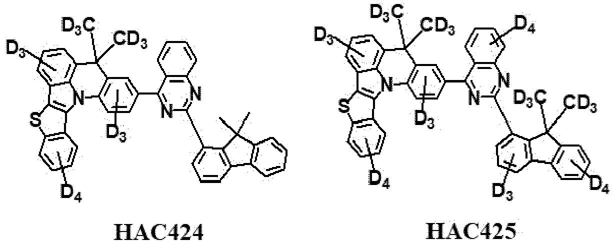

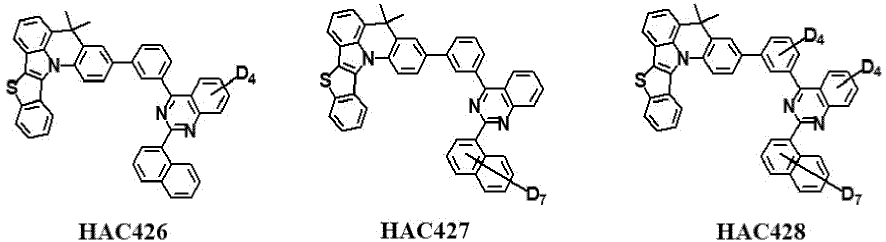

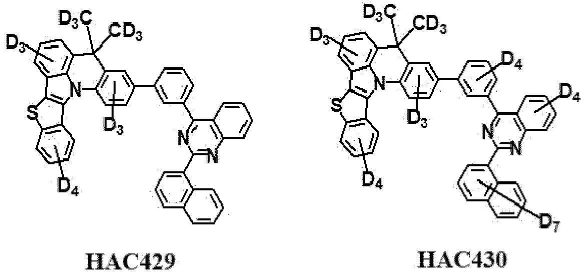

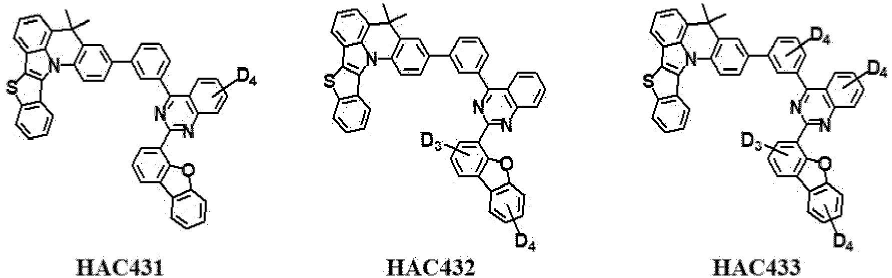

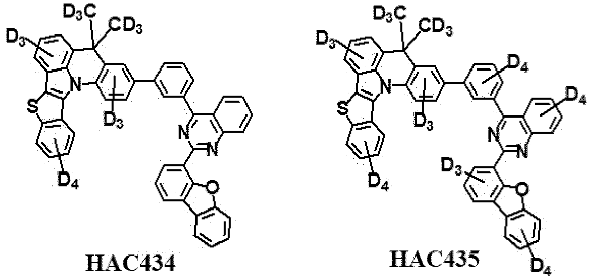

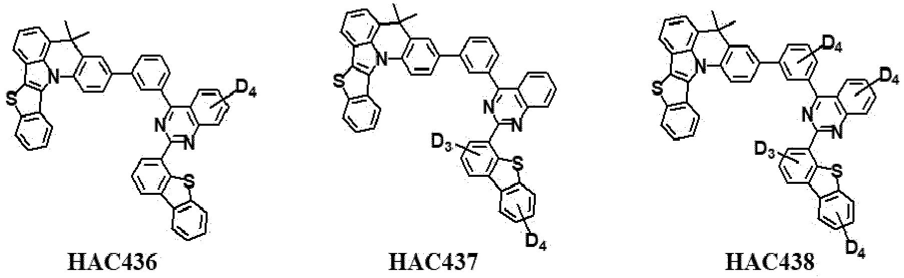

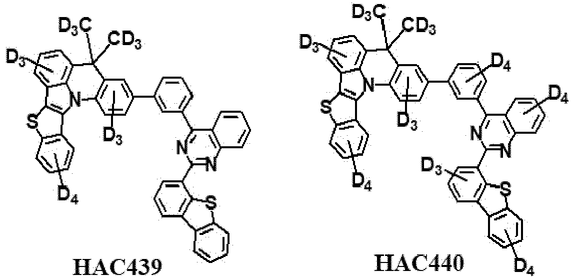

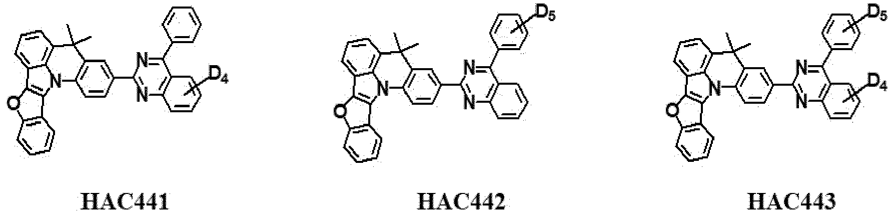

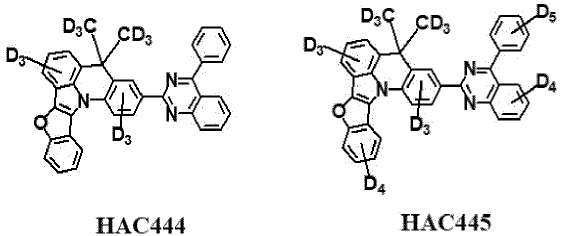

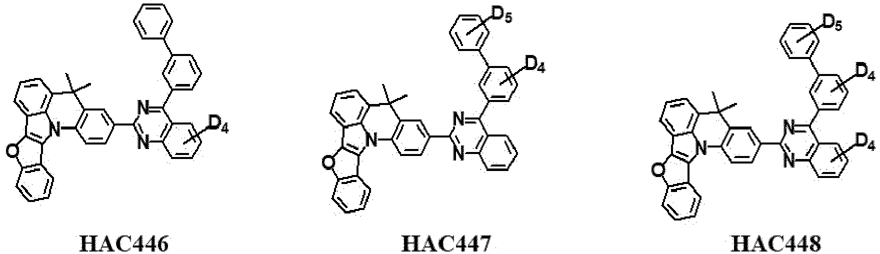

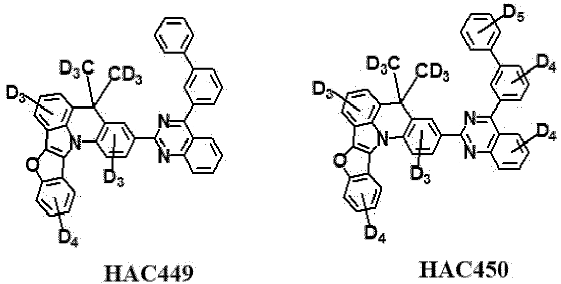

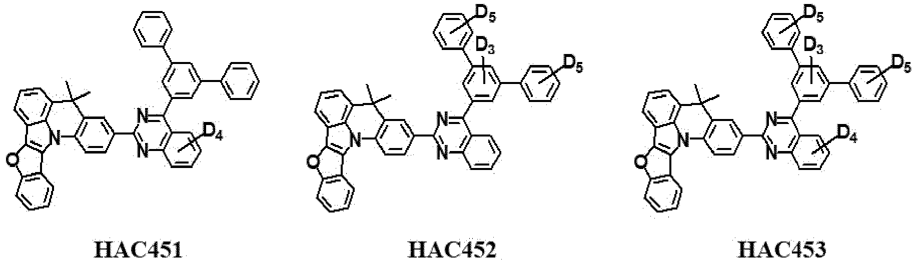

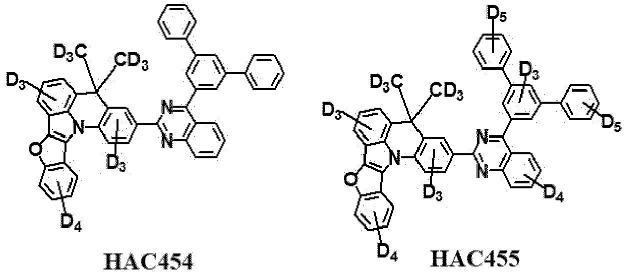

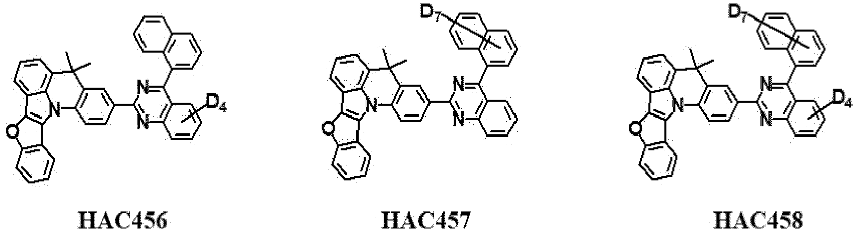

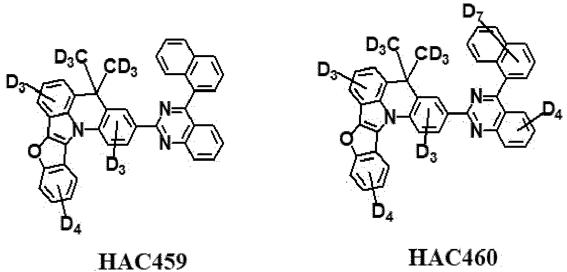

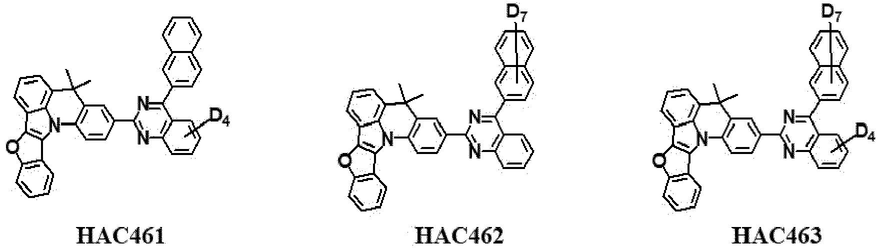

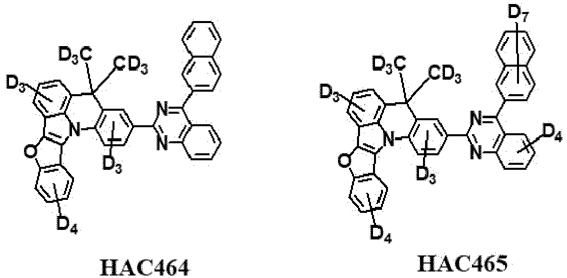

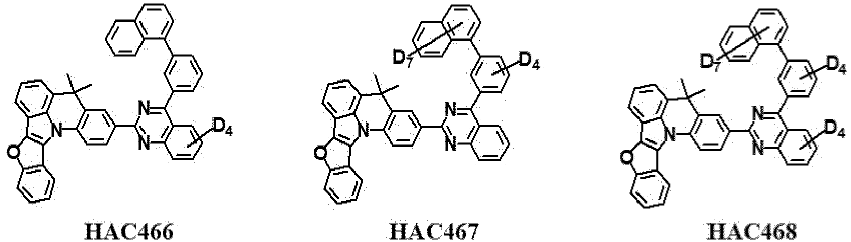

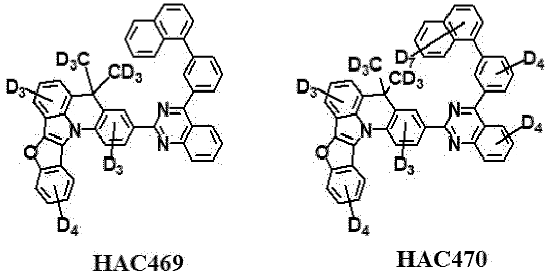

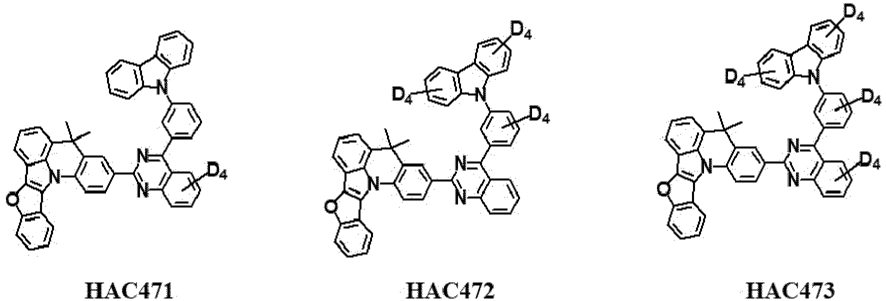

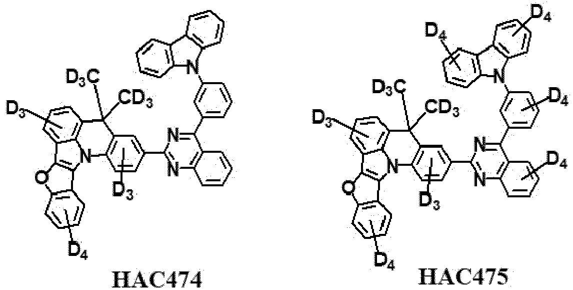

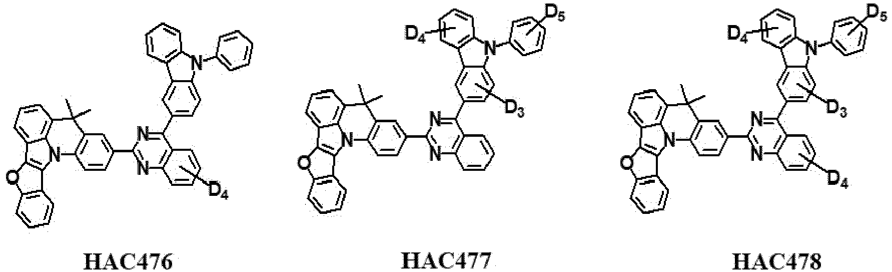

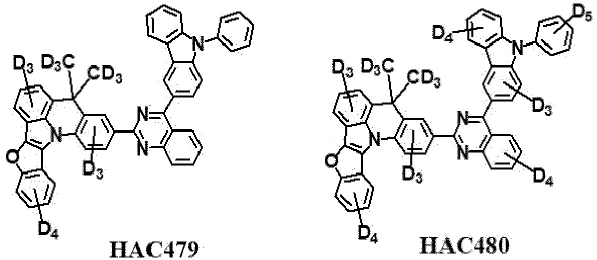

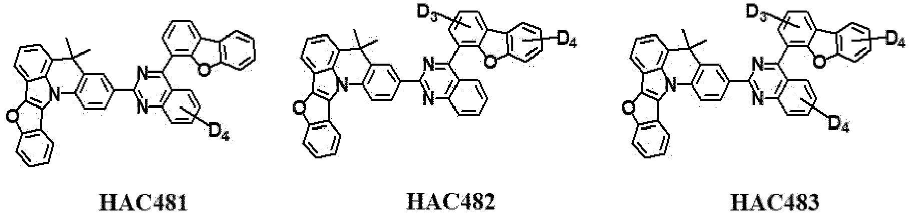

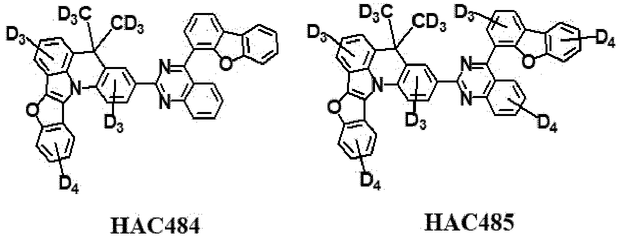

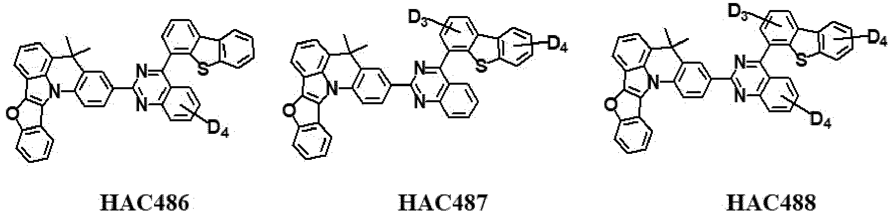

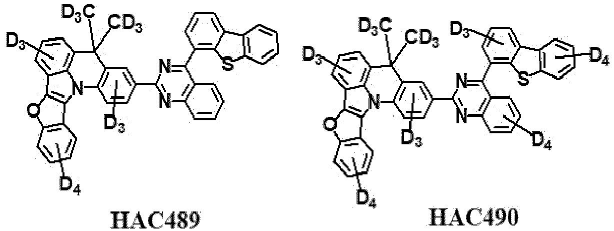

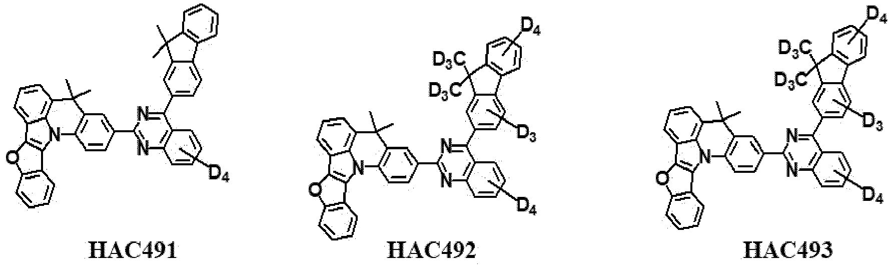

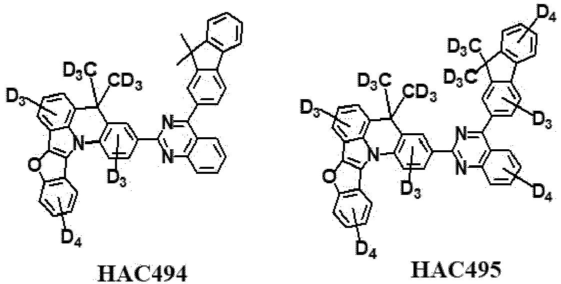

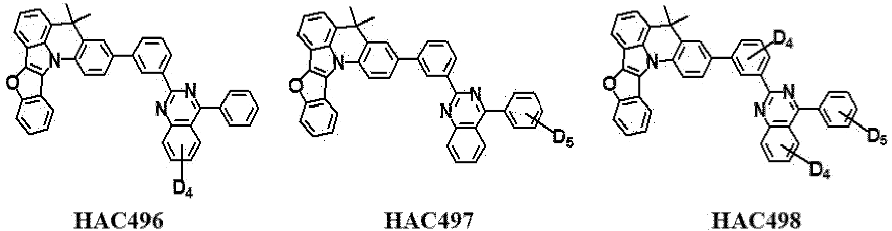

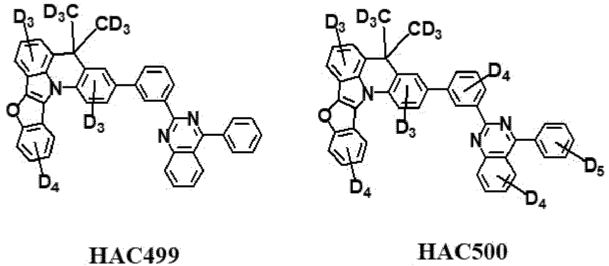

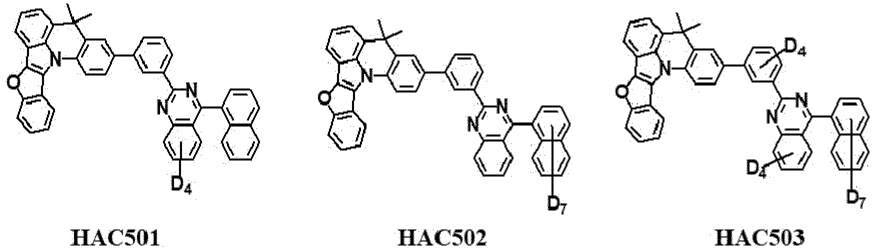

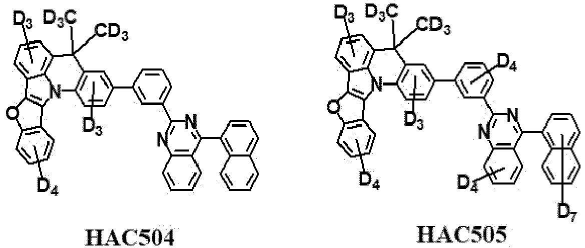

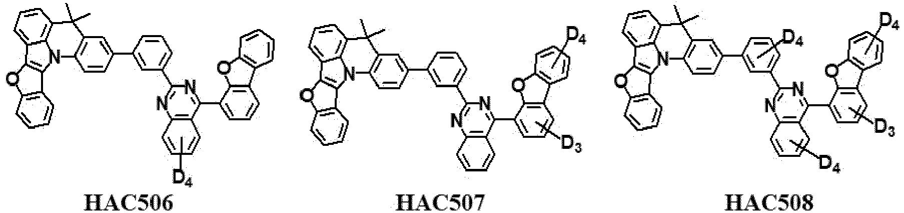

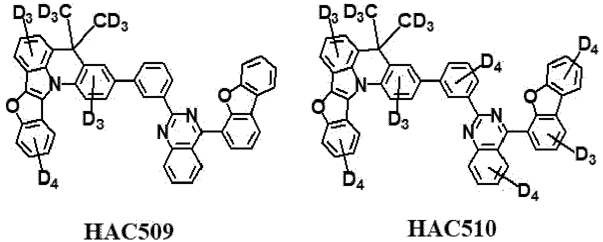

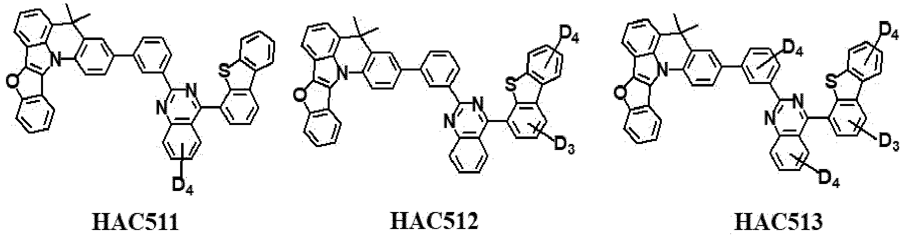

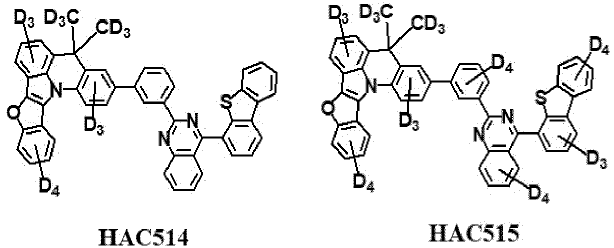

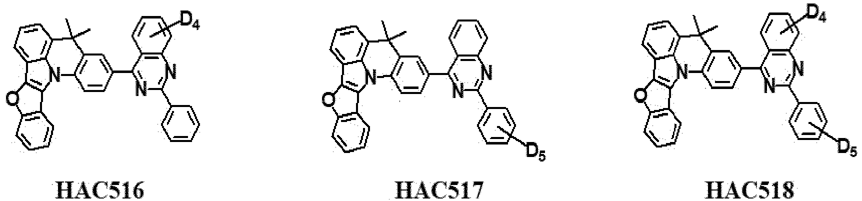

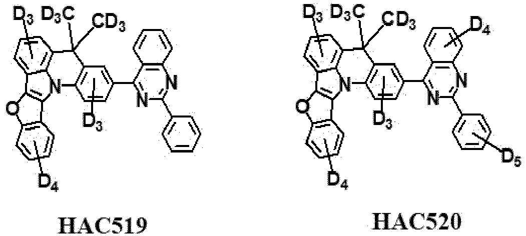

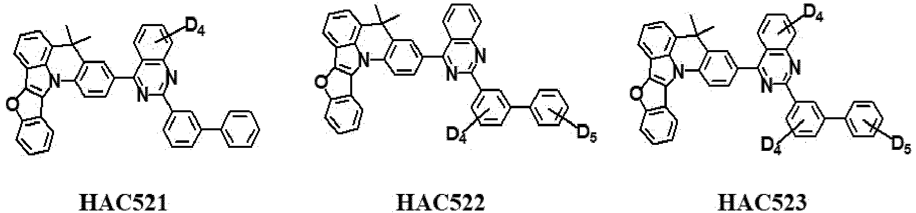

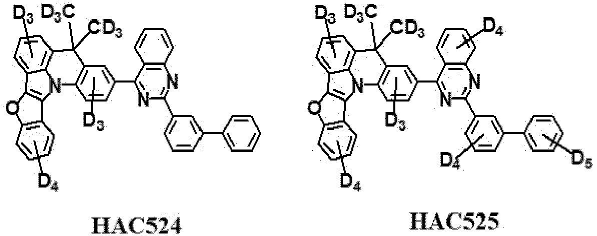

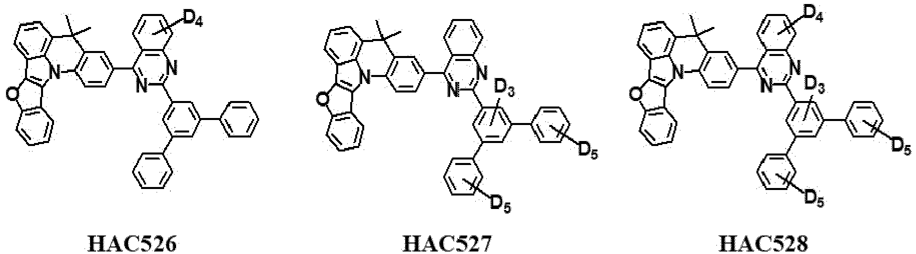

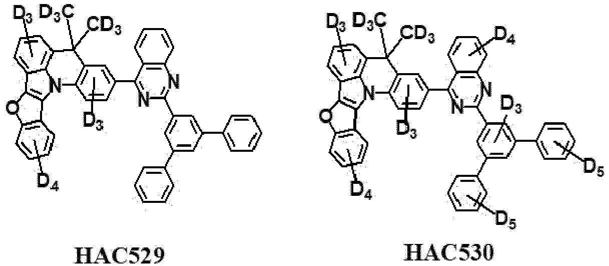

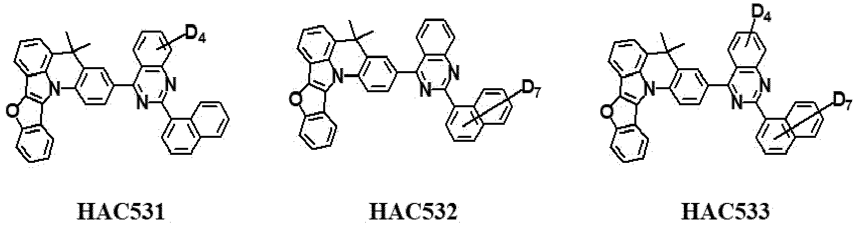

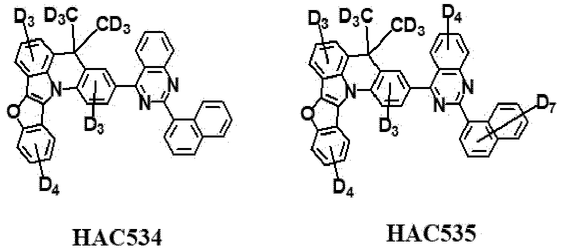

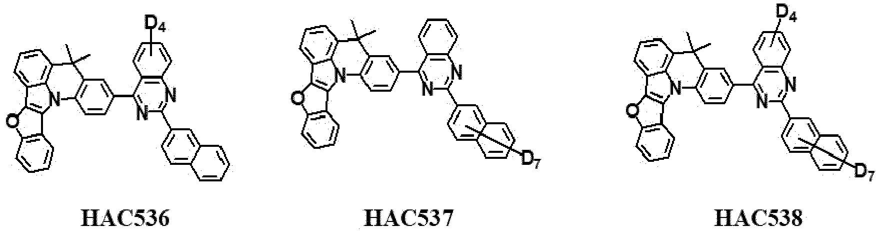

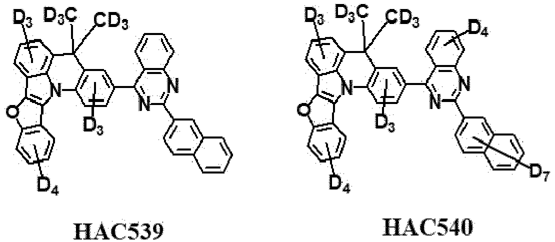

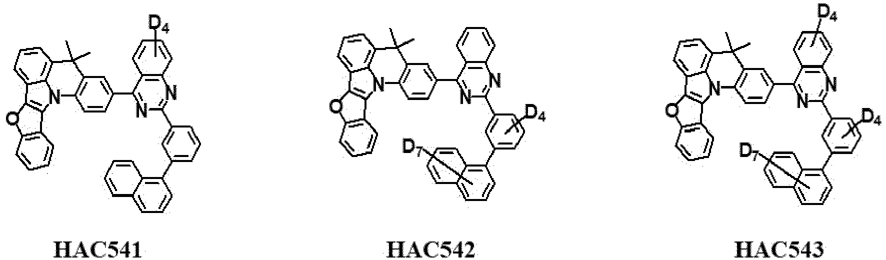

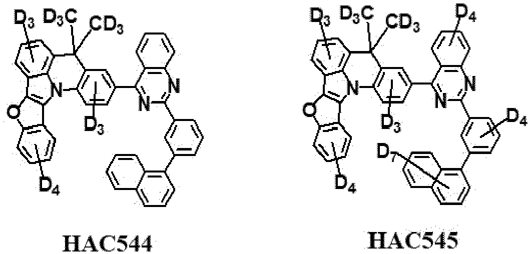

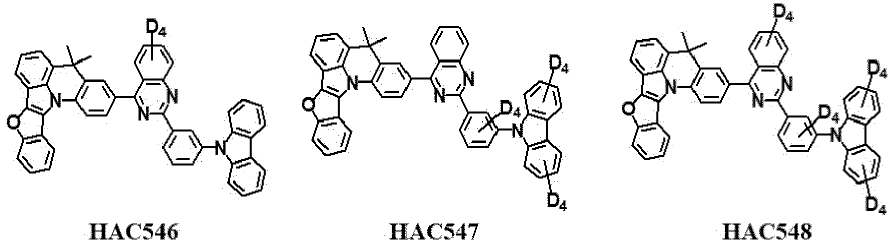

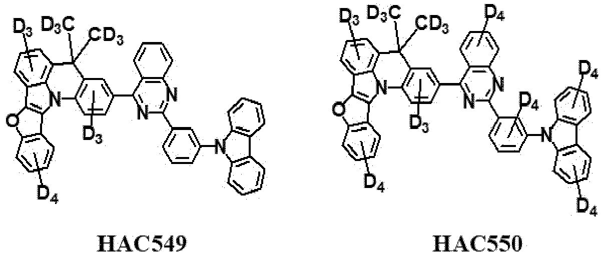

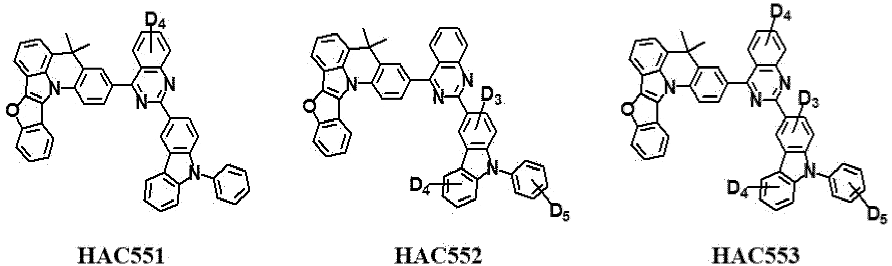

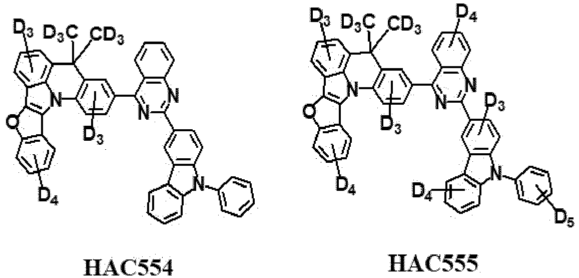

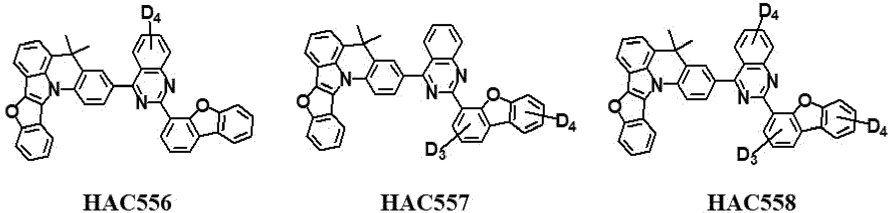

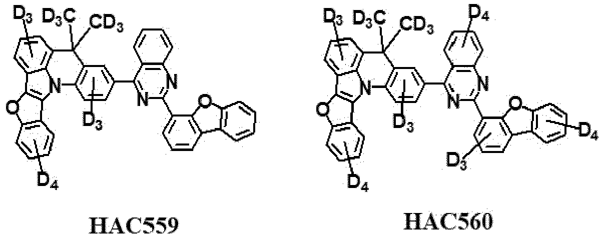

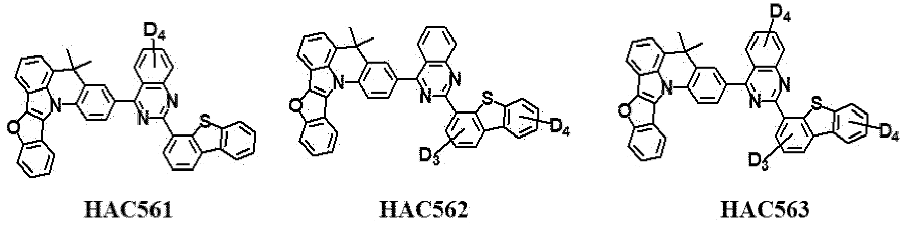

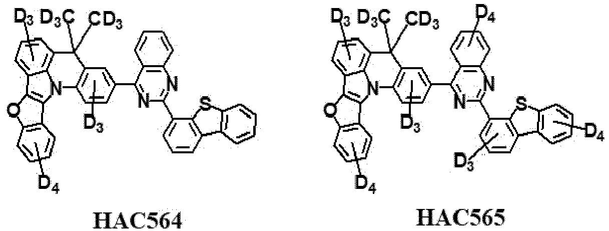

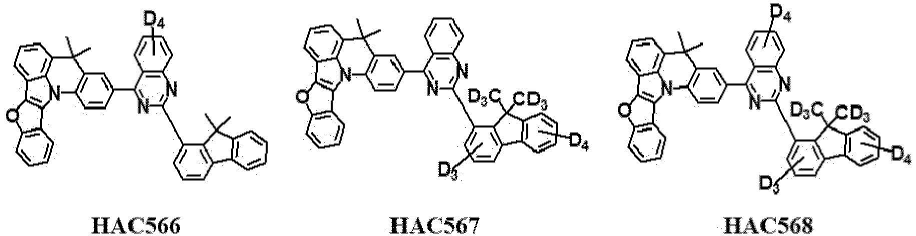

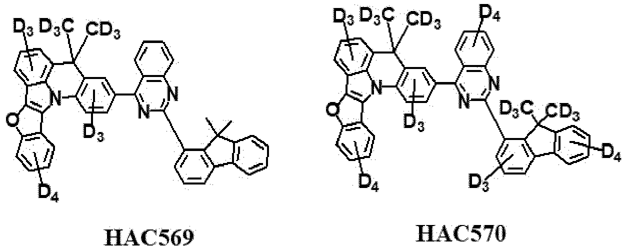

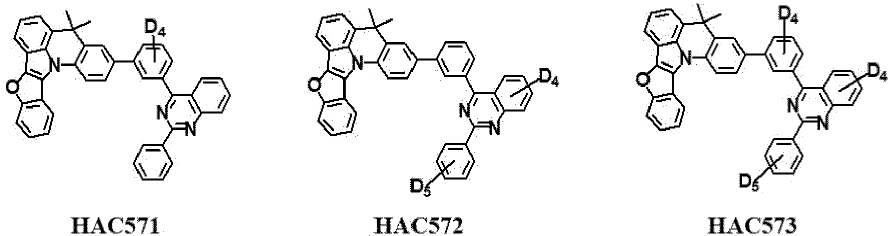

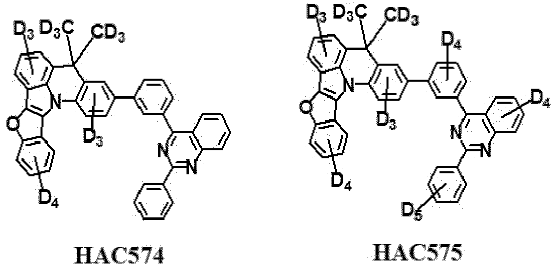

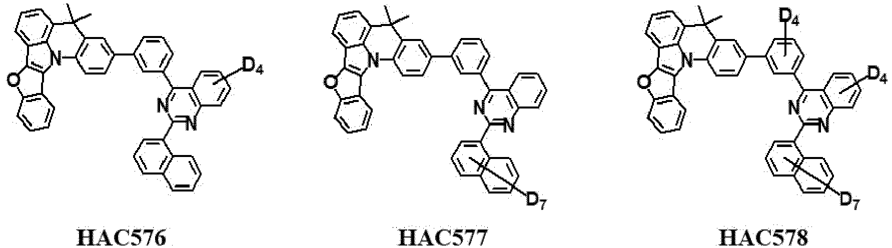

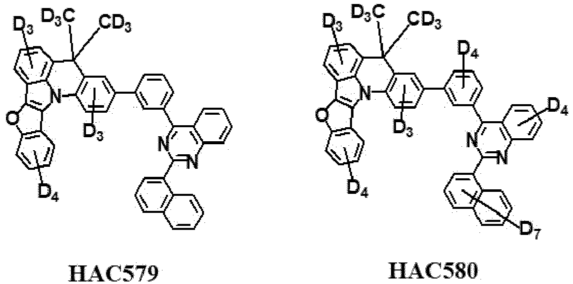

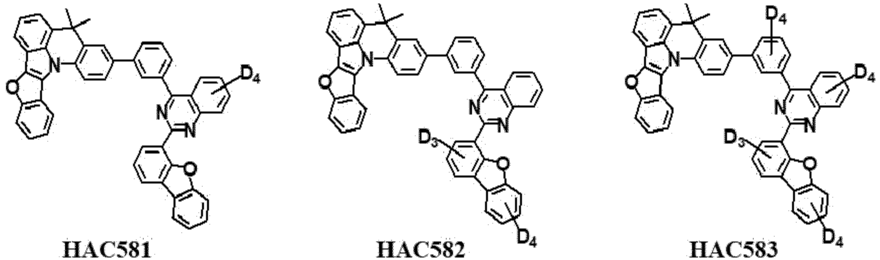

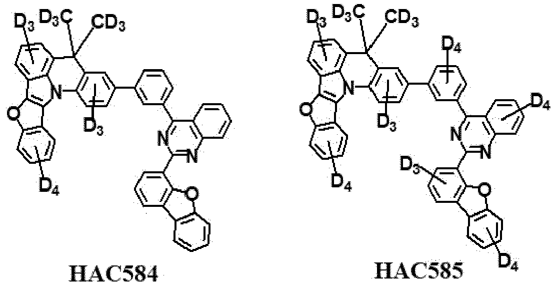

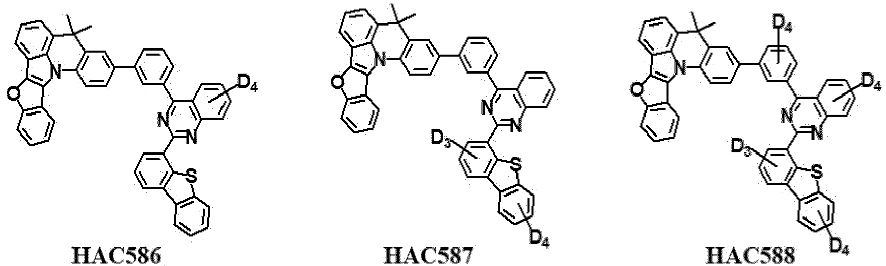

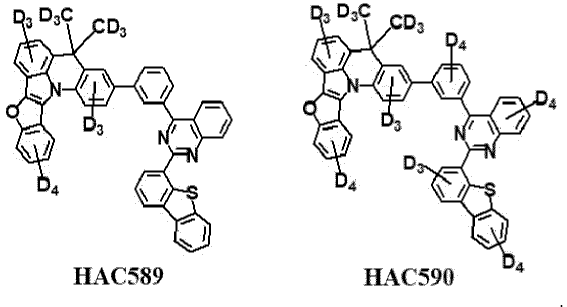

6. An organic light emitting diode (D, D1, D2, D3), including:
a first electrode (210, 510);
a second electrode (220, 520) facing the first electrode (210, 510); and
an emissive layer (230, 530, 530') disposed between the first and second electrodes (210, 220; 510, 520);
wherein the emissive layer (230, 530, 530') includes the organic compound of any of claims 1 to 5.

7. The organic light emitting diode (D, D1, D2, D3) of claim 6, wherein the emissive layer (230, 530, 530') includes at least one emitting material layer (340, 640, 740, 740', 840), and wherein the at least one emitting material layer (340, 640, 740, 740', 840) includes the organic compound.

8. The organic light emitting diode (D, D1, D2, D3) of claim 7, wherein the at least one emitting material layer (340, 640, 740, 740', 840) further includes a spiro-bifluorene-based organic compound having a structure represented by Formula 6: 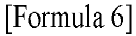 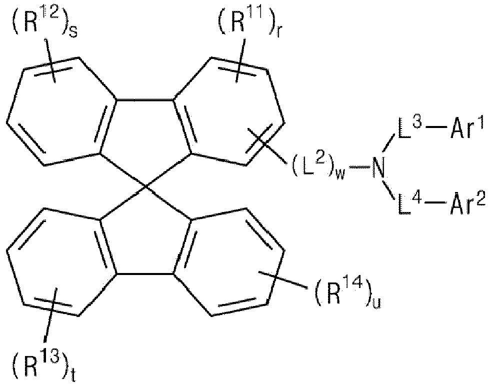
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4,
r is 3 and each of s, t, and u is 4 when each of R¹¹ to R¹⁴ is a protium,
r is 1, 2 or 3, and each of s, t, and u is independently 1, 2, 3 or 4 when each of R¹¹ to R¹⁴ is not a protium,
w is 0, 1, 2 or 3;
each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring.

9. The organic light emitting diode (D, D1, D2, D3) of claim 8, wherein the spiro-bifluorene-based organic compound has a structure represented by Formula 7 or Formula 8: 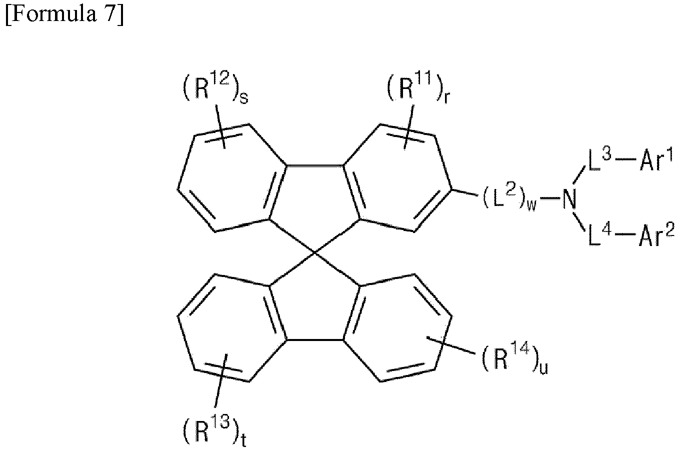 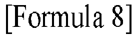 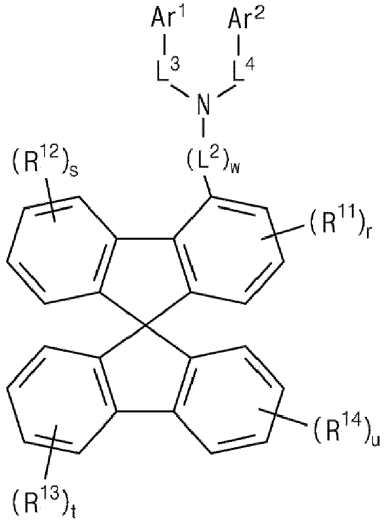
wherein in the Formulae 7 and 8,
each of R¹¹ to R¹⁴, Ar¹, Ar², L² to L⁴, r, s, t, u, and w is as defined in the Formula 6.

10. The organic light emitting diode (D, D1, D2, D3) of claim 8, wherein the spiro-bifluorene-based organic compound includes at least one of the following spiro-bifluorene-based organic compounds:

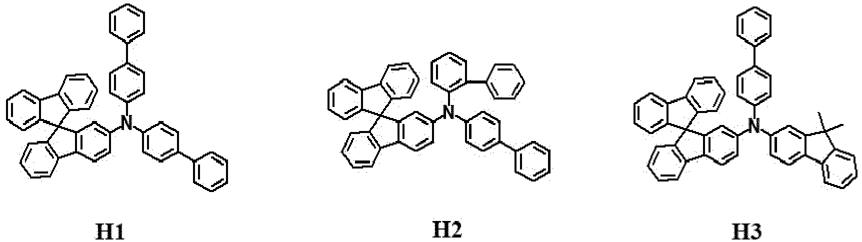

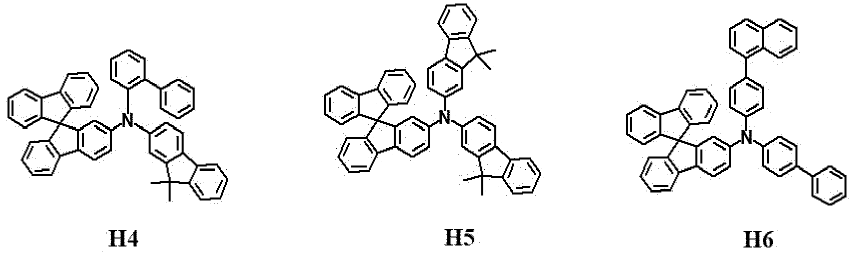

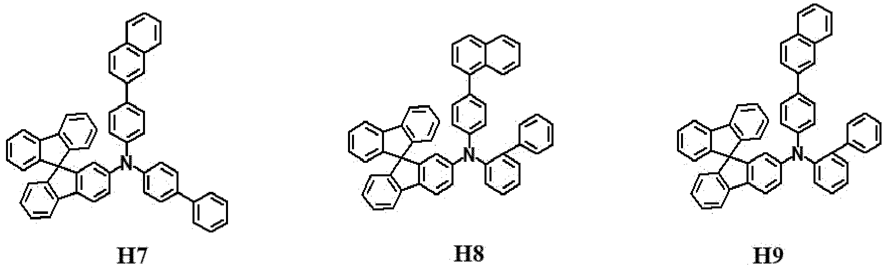

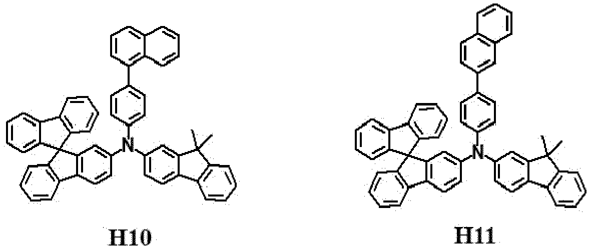

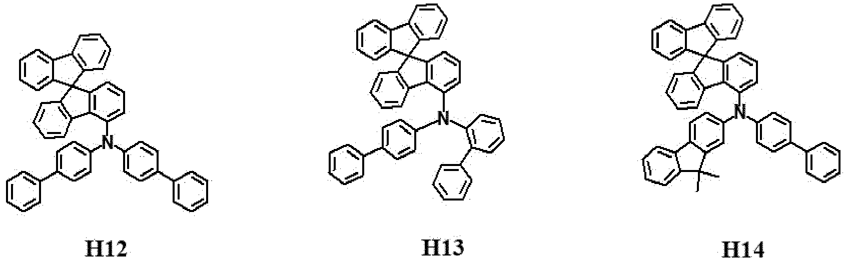

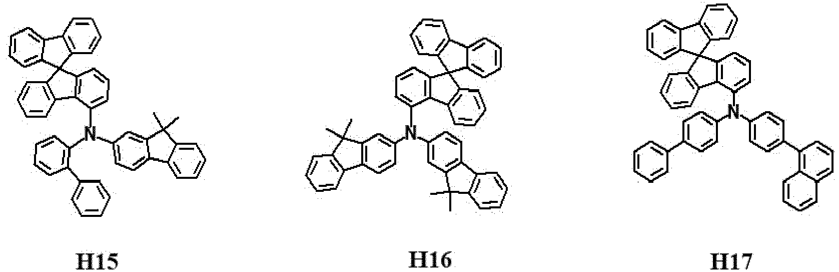

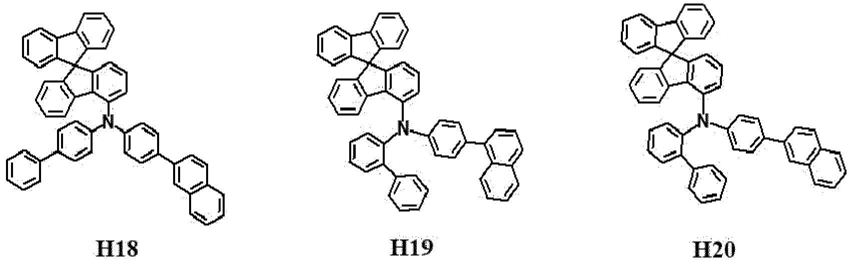

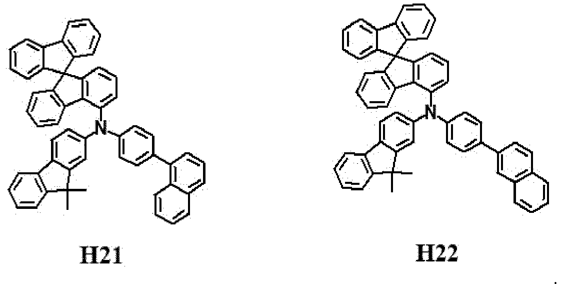

11. The organic light emitting diode (D, D1, D2, D3) of any of claims 7 to 10, wherein the at least one emitting material layer (340, 740, 740') includes a dopant (346, 746),
wherein, preferably, the dopant (346, 746) includes a red dopant.

12. The organic light emitting diode (D, D1, D2, D3) of any of claims 7 to 11, wherein the emissive layer (230, 530, 530') further includes at least one hole transport layer (320, 620, 720, 820) disposed between the first electrode (210, 510) and the at least one emitting material layer (340, 640, 740, 740', 840).

13. The organic light emitting diode (D, D1, D2, D3) of claim 12, wherein the at least one hole transport layer (320, 620, 720, 820) includes a spiro-bifluorene-based organic compound having a structure represented by Formula 6: 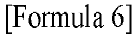 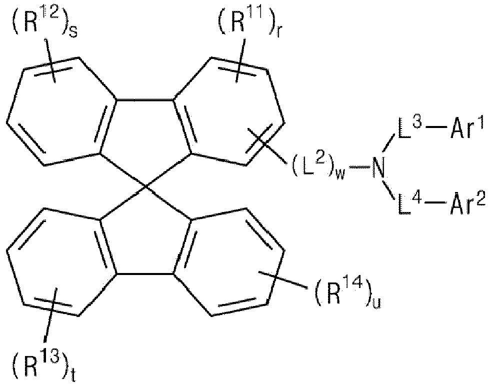
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4,
r is 3 and each of s, t, and u is 4 when each of R¹¹ to R¹⁴ is a protium,
r is 1, 2 or 3, and each of s, t, and u is independently 1, 2, 3 or 4 when each of R¹¹ to R¹⁴ is not a protium, and
w is 0, 1, 2 or 3;
each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring.

14. The organic light emitting diode (D, D1, D2, D3) of claim 13, wherein the spiro-bifluorene-based organic compound has a structure represented by Formula 7 or Formula 8: 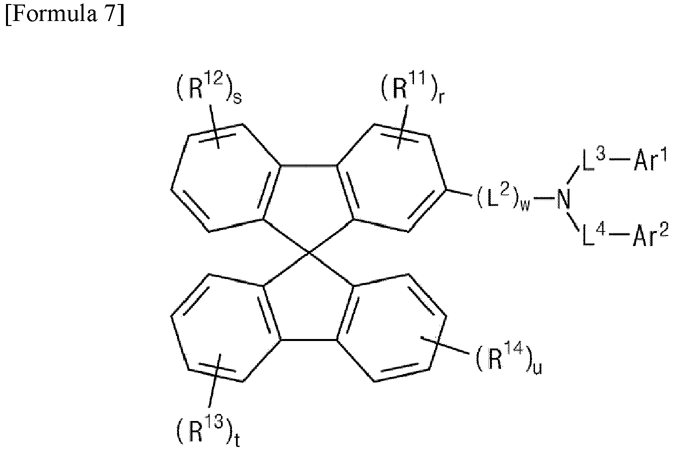 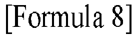 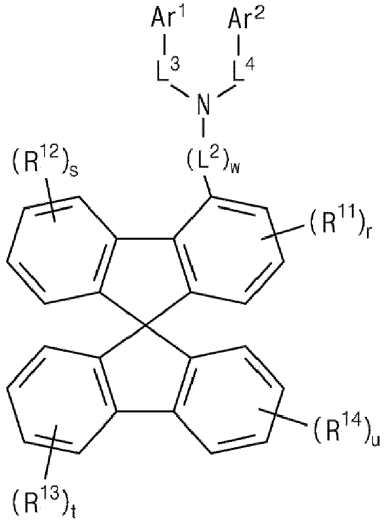
wherein in the Formulae 7 and 8,
each of R¹¹ to R¹⁴, Ar¹, Ar², L² to L⁴, r, s, t, u, and w is as defined in the Formula 6.

15. The organic light emitting diode (D, D1, D2, D3) of claim 13, wherein the spiro-bifluorene-based organic compound includes at least one of the following spiro-bifluorene-based organic compounds:

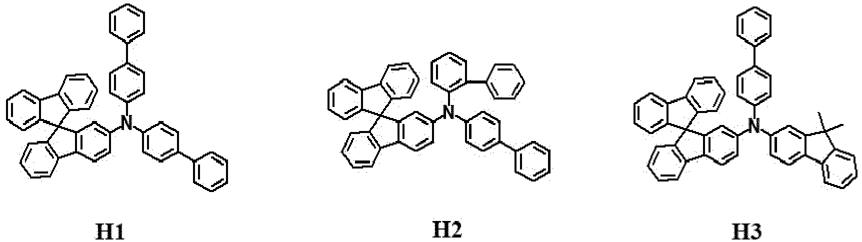

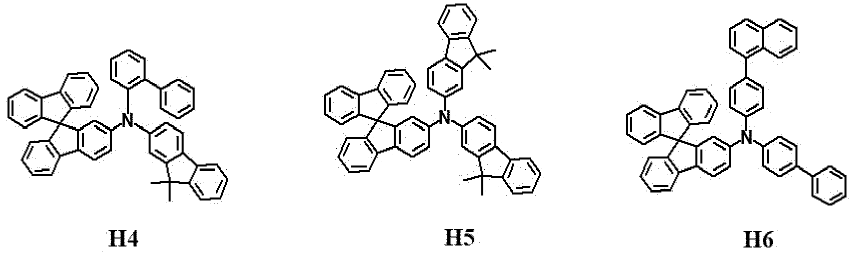

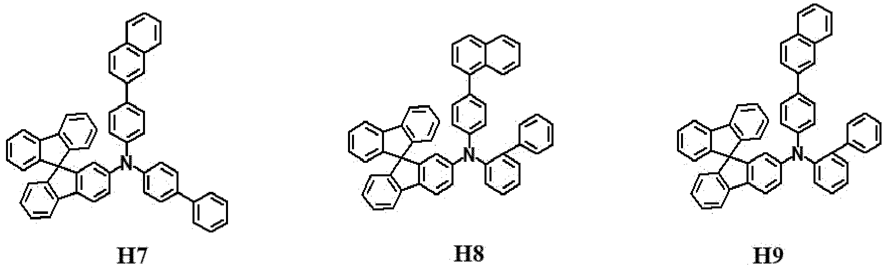

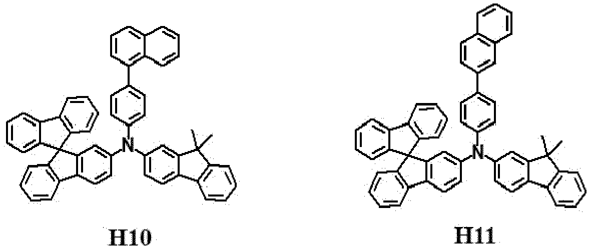

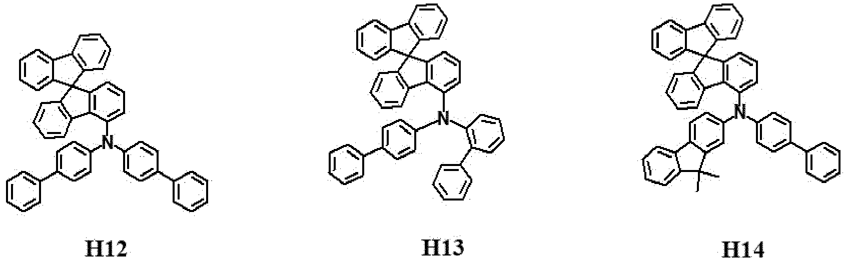

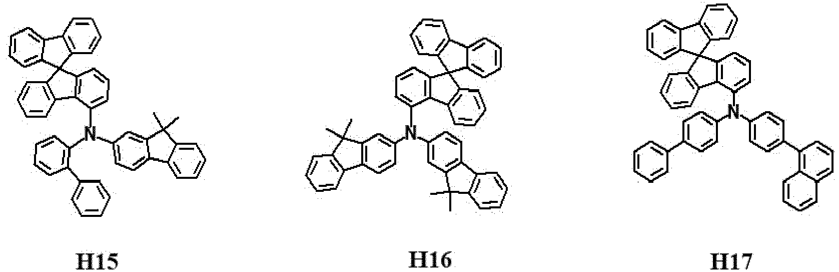

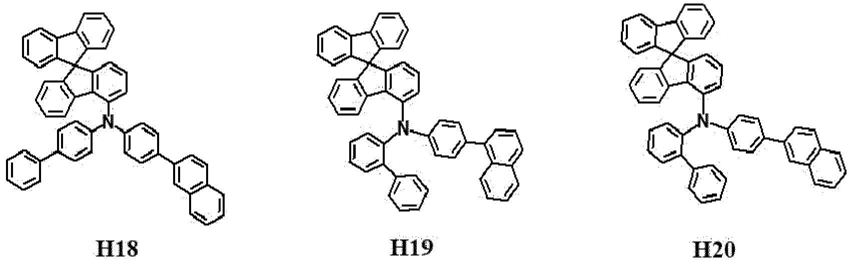

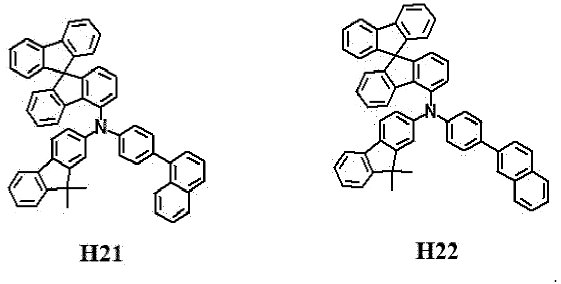

16. The organic light emitting diode (D, D2, D3) of any of claims 6 to 15, wherein the emissive layer (530, 530') includes:
a first emitting part (600) disposed between the first and second electrodes (510, 520) and including a first emitting material layer (640);
a second emitting part (700, 700') disposed between the first emitting part (600) and the second electrode (520) and including a second emitting material layer (740, 740'); and
a first charge generation layer (680) disposed between the first emitting part (600) and the second emitting part (700),
wherein at least one of the first emitting material layer (640) and the second emitting material layer (740, 740') includes the organic compound.

17. The organic light emitting diode (D, D2, D3) of claim 16, wherein the second emitting material layer (740, 740') includes:
a first layer (740A) disposed between the first charge generation layer (680) and the second electrode (520); and
a second layer (740B) disposed between the first layer (740A) and the second electrode (520),
wherein at least one of the first layer (740A) and the second layer (740B) includes the organic compound.

18. The organic light emitting diode of claim 17, wherein the second emitting material layer (740') further includes a third layer (740C) disposed between the first layer (740A) and the second layer (740B),
wherein, preferably, the third layer (740C) is a yellow green emitting material layer.

19. The organic light emitting diode (D, D3) of claim 16 or 17, wherein the emissive layer (530') further includes:
a third emitting part (800) disposed between the second emitting part (700') and the second electrode (520) and including a third emitting material layer (840), and
a second charge generation layer (780) disposed between the second emitting part (700') and the third emitting part (800).

20. The organic light emitting diode of claim 19, wherein the second emitting material layer (740') includes:
a first layer (740A) disposed between the first charge generation layer (680) and the second electrode (520), and
a second layer (740B) disposed between the first layer (740A) and the second electrode (520),
wherein at least one of the first layer (740A) and the second layer (740B) includes the organic compound,
wherein, preferably, the second emitting material layer (740') further includes a third layer (740C) disposed between the first layer (740A) and the second layer (740B),
wherein, further preferably, the third layer (740C) is a yellow green emitting material layer.

21. The organic light emitting diode (D, D2, D3) of any of claims 16 to 20, wherein the first emitting material layer (640) is a blue emitting material layer.

22. The organic light emitting diode (D, D2, D3) of any of claims 16 to 21, wherein the first layer (740A) includes the organic compound and a red dopant (746), and the second layer (740B) is a green emitting material layer,
wherein, preferably, the first layer (740A) further includes a spiro-bifluorene-based organic compound having a structure represented by Formula 6: 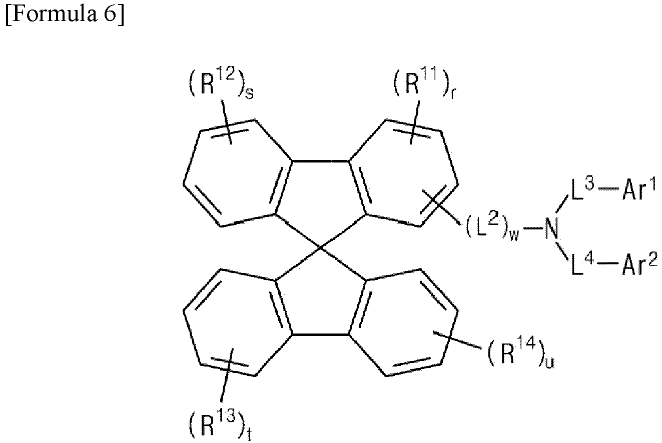
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4,
r is 3 and each of s, t, and u is 4 when each of R¹¹ to R¹⁴ is a protium,
r is 1, 2 or 3, and each of s, t, and u is independently 1, 2, 3 or 4 when each of R¹¹ to R¹⁴ is not a protium,
w is 0, 1, 2 or 3;
each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
wherein, further preferably, the spiro-bifluorene-based organic compound has a structure represented by Formula 7 or Formula 8: 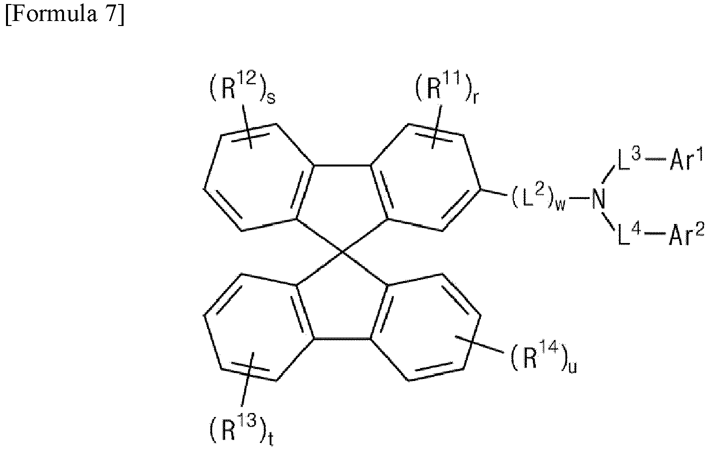 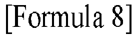 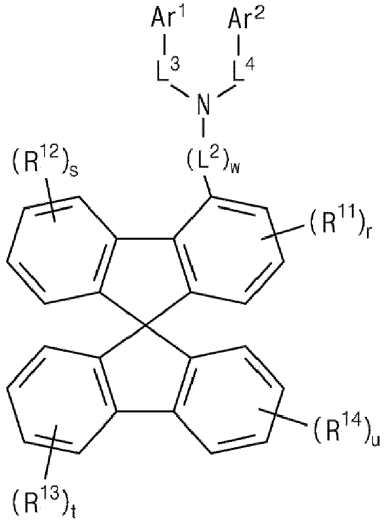
wherein in the Formulae 7 and 8,
each of R¹¹ to R¹⁴, Ar¹, Ar², L² to L⁴, r, s, t, u, and w is as defined in the Formula 6.

23. The organic light emitting diode (D, D2, D3) of any of claims 16 to 22, wherein the second emitting part (700, 700') further includes a hole transport layer (720) disposed between the first charge generation layer (680) and the second emitting material layer (740, 740'), and
wherein the hole transport layer (720) includes a spiro-bifluorene-based organic compound having a structure represented by Formula 6: 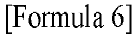 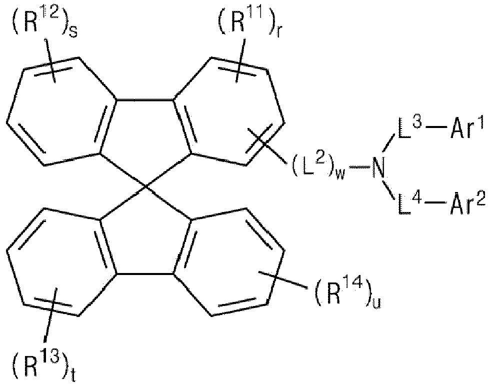
wherein in the Formula 6,
each of R¹¹ to R¹⁴ is independently a protium, a deuterium, a tritium, an unsubstituted or substituted C₁-C₂₀ alkyl, an unsubstituted or substituted C₁-C₂₀ heteroalkyl, an unsubstituted or substituted C₂-C₂₀ alkenyl, an unsubstituted or substituted C₂-C₂₀ heteroalkenyl, an unsubstituted or substituted C₁-C₂₀ alkoxy, an unsubstituted or substituted C₁-C₂₀ alkyl amino, an unsubstituted or substituted C₃-C₂₀ alicyclic group, an unsubstituted or substituted C₃-C₂₀ heteroalicyclic group, an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
each R¹¹ is identical to or different from each other when r is 2 or 3, each R¹² is identical to or different from each other when s is 2, 3 or 4, each R¹³ is identical to or different from each other when t is 2, 3 or 4, and each R¹⁴ is identical to or different from each other when u is 2, 3 or 4,
r is 3 and each of s, t, and u is 4 when each of R¹¹ to R¹⁴ is a protium,
r is 1, 2 or 3, and each of s, t, and u is independently 1, 2, 3 or 4 when each of R¹¹ to R¹⁴ is not a protium,
w is 0, 1, 2 or 3; each of L₂ to L₄ is independently a single bond, an unsubstituted or substituted C₆-C₃₀ arylene, or an unsubstituted or substituted C₃-C₃₀ heteroarylene, optionally, each of the C₆-C₃₀ arylene and the C₃-C₃₀ heteroarylene forms a spiro structure with an unsubstituted or substituted C₆-C₂₀ aromatic ring or an unsubstituted or substituted C₃-C₂₀ heteroaromatic ring;
each of Ar¹ and Ar² is independently an unsubstituted or substituted C₆-C₃₀ aromatic group, or an unsubstituted or substituted C₃-C₃₀ heteroaromatic group, optionally, each of the C₆-C₃₀ aromatic group and the C₃-C₃₀ heteroaromatic group independently forms a spiro structure with an unsubstituted or substituted C₆-C₂₀ aromatic ring or an unsubstituted or substituted C₃-C₂₀ heteroaromatic ring,
wherein, preferably, the spiro-bifluorene-based organic compound has a structure represented by Formula 7 or Formula 8: 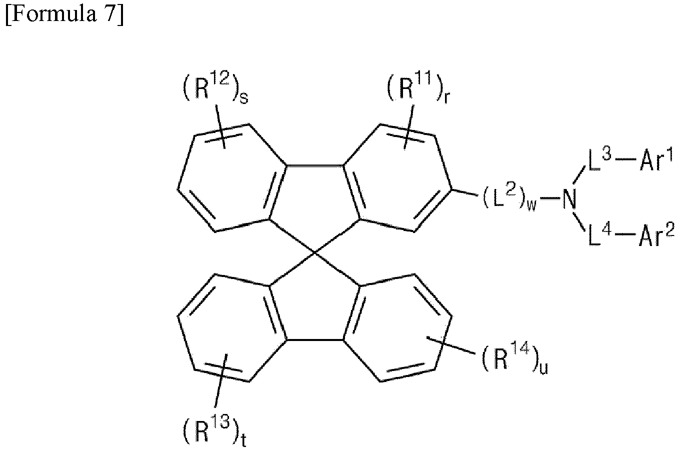 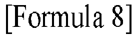 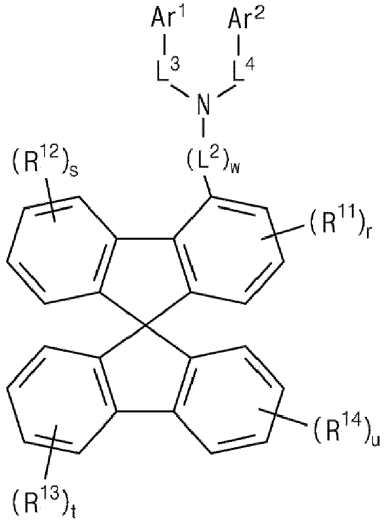
wherein in the Formulae 7 and 8,
each of R¹¹ to R¹⁴, Ar¹, Ar², L² to L⁴, r, s, t, u, and w is as defined in the Formula 6.

24. The organic light emitting diode (D, D3) of any of claims 16 to 18, wherein the emissive layer (530') further includes:
a third emitting part (800) disposed between the second emitting part (700') and the second electrode (520) and including a blue emitting material layer (840); and
a second charge generation layer (780) disposed between the second emitting part (700') and the third emitting part (800).

25. An organic light emitting device (100, 400), including:
a substrate (402); and
the organic light emitting diode (D, D1, D2, D3) of any of claims 6 to 24 disposed on the substrate (402).
